# EUROPEAN PATENT APPLICATION

(11) **EP 2 712 930 A2**
(43) Date of publication of application: **02.04.2014**
(21) Application number: 13179923.1
(22) Date of filing: 06.12.2007
(51) Int. Cl.: C12Q 1/68, A61K 39/395, A61K 39/00, C07K 16/24

(54) **Interferon alpha-induced pharmacodynamic markers**

(30) Priority: 06.12.2006 US 873008 P; 16.04.2007 US 907762 P; 16.04.2007 US 907767 P; 03.05.2007 US 924219 P; 03.05.2007 US 924220 P; 21.05.2007 US 924584 P; 19.09.2007 US 960187 P; 05.11.2007 US 996176 P; 05.11.2007 US 996174 P; 06.11.2007 US 996219 P
(62) Divisional of application: 07867637.6
(71) Applicant: MedImmune, LLC, Gaithersburg, MD 20878 (US)
(72) Inventor: Yao, Yihong, Boyds, MD 20841 (US); Jallal, Bahija, Potomac, MD 20854 (US); Cibotti, Ricardo, Bethesda, MD 20817 (US); Coyle, Anthony, Gaithersburg, MD 20878 (US); Keiner, Peter, Potomac, MD 20854 (US)
(74) Representative: Winter, Christopher Spencer

(57) **Abstract**

A method of monitoring disease progression of a patient receiving treatment with a therapeutic agent that binds to and modulates IFNα activity comprising: obtaining a first IFNα-inducible PD marker expression profile in a first sample from the patient; administering a therapeutic agent that binds to and modulates IFNα activity; obtaining a second IFNα-inducible PD marker expression profile in a second sample from the patient; and comparing the first and the second IFNα-inducible PD marker expression profiles, wherein a variance in the first and the second IFNα-inducible PD marker expression profiles indicates a level of efficacy of the therapeutic agent that binds to and modulates IFNα activity.

## Description

### FIELD OF THE INVENTION

The present invention relates to pharmacodynamic (PD) markers inducible by interferon (IFN) alpha, probes and kits that detect the PD markers, and methods employing the same.

### BACKGROUND OF THE INVENTION

The present invention encompasses PD markers that are induced by IFNα. The PD markers can be used in methods of treating patients with a therapeutic agent that binds to and modulates IFNα activity, methods that identify patients as candidates for a therapeutic agent that binds to and modulates IFNα activity, methods of diagnosing a patient as having a disorder associated with increased IFNα levels, methods of monitoring disease progression of a patient receiving treatment with a therapeutic agent that binds to and modulates IFNα activity, and methods of identifying a candidate therapeutic for treating IFNα-mediated disorders

### SUMMARY OF THE INVENTION

One embodiment of the invention encompasses a method of identifying a patient as a candidate for a therapeutic agent that binds to and modulates IFNα activity. Presence or absence of an IFNα-inducible PD marker expression profile is detected in a sample from the patient.

Another embodiment of the invention encompasses a method of treating a patient having a type I IFN or IFNα-mediated disease or disorder. An agent that binds to and modulates type I IFN or IFNα activity is administered to the patient. The agent neutralizes a type I IFN or IFNα-inducible PD marker expression profile of the patient.

Yet another embodiment of the invention encompasses a method of treating an autoimmune disease patient comprising a moderate or strong type I IFN or an IFNα PD marker profile. An agent that binds to and modulates type I IFN or IFNα activity is administered to the patient. The agent neutralizes the type I IFN or IFNα-inducible PD marker expression profile of the patient.

A further embodiment of the invention encompasses a method of neutralizing a type I IFN or IFNα-inducible PD marker expression profile in a patient in need thereof. An agent that binds to and modulates type I IFN or IFNα activity is administered to the patient. The agent neutralizes the type I IFN or IFNα-inducible PD marker expression profile of the patient.

Another embodiment of the invention encompasses a method of diagnosing a patient as having a disorder associated with increased IFNα levels. Presence or absence of an IFNα-inducible PD marker expression profile is detected in a sample from the patient.

A further embodiment of the invention encompasses a method of monitoring disease progression of a patient receiving treatment with a therapeutic agent that binds to and modulates IFNα activity. A first IFNα-inducible PD marker expression profile is obtained in a first sample from the patient. A therapeutic agent that binds to and modulates IFNα activity is administered to the patient. A second IFNα-inducible PD marker expression profile is obtained from a second sample from the patient. The first and the second IFNα-inducible PD marker expression profiles are compared.

Yet another embodiment of the invention encompasses a method of identifying a candidate therapeutic for treating IFNα-mediated disorders. Cells comprising an IFNα-inducible PD marker expression profile are contacted with an agent. Presence or absence of a change in the IFNα-induced PD marker expression profile of the cells is detected.

A further embodiment of the invention encompasses a set of probes.

Yet a further embodiment of the invention encompasses kits that comprise the probes.

Another embodiment of the invention encompasses a method of detecting IFN activity in a sample. Cells comprising a polynucleotide sequence comprising a reporter gene under the control of an IFN-stimulated response element are incubated with a sample. Expression of the reporter gene is detected.

### BRIEF DESCRIPTION OF THE FIGURES

Figure 1: TaqMan qPCR IF144 gene expression analysis of IFNα-stimulated whole blood of healthy donors.
Figure 2: TaqMan qPCR IRF2 gene expression analysis of IFNα-stimulated whole blood of healthy donors.
Figure 3: TaqMan qPCR RSAD2 gene expression analysis of IFNα-stimulated whole blood of healthy donors.
Figure 4: TaqMan qPCR G1P3 gene expression analysis of IFNα-stimulated whole blood of healthy donors.
Figure 5: TaqMan qPCR HERC5 gene expression analysis of IFNα-stimulated whole blood of healthy donors.
Figure 6: MEDI-545 neutralization of RAB8B gene expression induced by IFN-α in whole blood of healthy donors.
Figure 7: MEDI-545 neutralization of IRF7 gene expression induced by IFN-α in whole blood of healthy donors.
Figure 8: MEDI-545 neutralization of MARCKS gene expression induced by IFN-α in whole blood of healthy donors.
Figure 9: MEDI-545 neutralization of IL6ST gene expression induced by IFN-α in whole blood of healthy donors.
Figure 10: MEDI-545 neutralization of Ly6E gene expression induced by IFN-α in whole blood of healthy donors.
Figure 11: MEDI-545 neutralization of IFIT3 gene expression induced by IFN-α in whole blood of healthy donors.
Figure 12: MEDI-545 neutralization of IFIT1 gene expression induced by IFN-α in whole blood of healthy donors.
Figure 13: MEDI-545 neutralization of HERC5 gene expression induced by IFN-α in whole blood of healthy donors.
Figure 14: MEDI-545 neutralization of OAS 1 gene expression induced by IFN-α in whole blood of healthy donors.
Figure 15: MEDI-545 neutralization of OAS3 gene expression induced by IFN-α in whole blood of healthy donors.
Figure 16: MEDI-545 neutralization of RSAD2 gene expression induced by IFN-α in whole blood of healthy donors.
Figure 17: Ex vivo stimulation in whole blood identifies genes inducible by type I IFN.
Figure 18: MEDI-545 neutralization of top 25 type I IFN inducible genes in individual lupus patients' whole blood.
Figure 19: Heatmap of target modulation and PCA plot using top 25 up-regulated type I IFN, inducible probe sets in whole blood of patient 1541 before and after MEDI-545 treatment.
Figure 20: Heatmap of target modulation and PCA plot based on 25 most up-regulated type I IFN inducible genes in whole blood of patient 1449 before and after MEDI-545 treatment.
Figure 21: Heatmap of target modulation calculated based on 165 type I IFN inducible genes up-regulated in whole blood of one patient treated with 0.3 mg/kg MEDI-545.
Figure 22: PCA using 169 probe sets that are type I IFN inducible - 24/35 SLE patients have statistically significant type I IFN signature in whole blood.
Figure 23: MEDI-545 neutralizes the top 25 most upregulated type I IFN inducible probe sets of lupus patients. Target neutralization of the top 25 most upregulated type I IFN inducible genes was measured at days 1, 4, 7, 14, 28, and 84 for each patient. Dose range was from I (placebo) to 3 mg/kg MedI 545.
Figure 24: MEDI-545 neutralizes the top 25 most upregulated type I IFN inducible probe sets of lupus patients. Target neutralization of the top 25 most upregulated type I IFN inducible genes was measured at days 1, 4, 7, 14, and 28 for each patient. Dose range was from 0 (placebo) to 30 mg/kg MEDI-545.
Figure 25a and b: Heatmap (a) and PCA (b) showing neutralization of the top 25 type I IFN inducible probe sets in whole blood of a SLE patient treated with 30 mg/kg MEDI-545 at 0, 1, 4, 7, and 14 days post-dosing.
Figure 26a and b: PCA plots of lupus patient before (a) and after (b) dosing with placebo control show no trend in the change of type I IFN inducible gene signature. The 25 most upregulated type I IFN inducible probe sets were used to perform the PCA analysis.
Figure 27: Type-I IFNα subtypes are upregulated in the whole blood of individual lupus patients.
Figure 28: Distribution of average fold-change of top 25 type I IFN inducible probe sets in whole blood of individual lupus patients.
Figure 29a-c: Pair-wise fold change ranking test proves MEDI-545 neutralizes type I IFN genes in a clinical trial. Top genes neutralized are shown for (a) SLE patients having a type I IFN gene signature at 14 days following MEDI-545 treatment; (b) SLE patients not having a type I IFN gene signature at 14 days following MEDI-545 treatment; and (c) SLE patients 14 days following treatment with placebo. Genes highlighted in yellow are genes identified as having a type-I IFN signature.

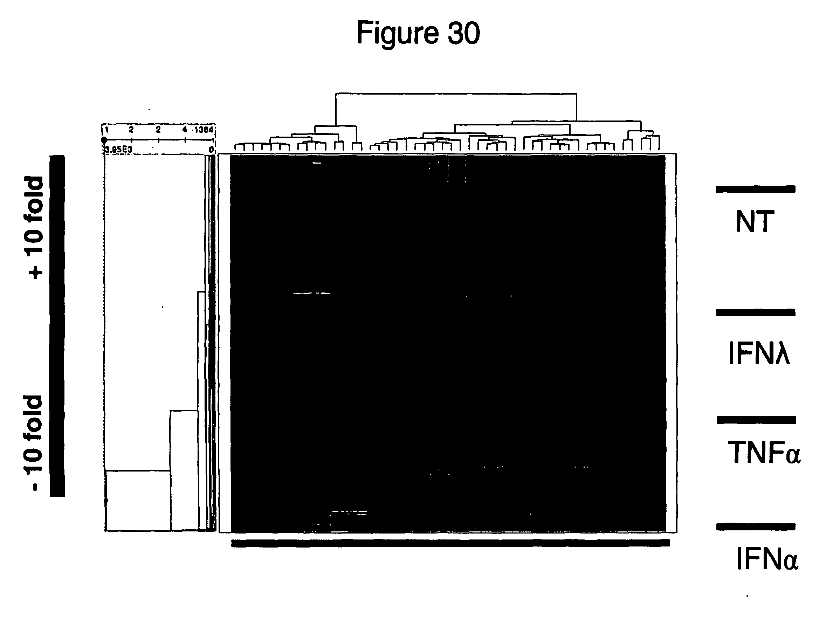

Figure 30: Hierarchical clustering of 1384 probe sets differentially regulated by IFNα subtypes, IFNβ, IFNγ, and TNFα in *ex vivo* stimulated whole blood. Each row corresponds to a single probe set, while each column corresponds to a single sample. The branch lengths indicate the correlation with which probe sets/samples are joined, with a longer branch indicating a weaker correlation. Color represents relative expression level of individual probe sets as compared to the average expression of the no treatment controls. Red indicates up-regulation versus control; green indicates down-regulation versus control; black indicates no change.

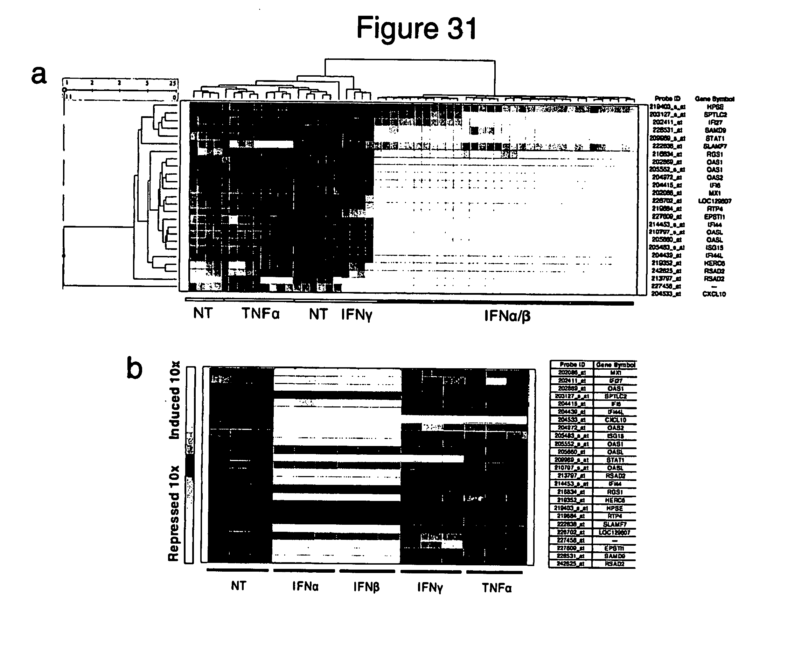

Figure 31a-31b: a. Hierarchical clustering of the relative expression of the top 25 most overexpressed type-I IFN inducible probe sets in whole blood *ex vivo* challenged with a variety of IFNα subtypes, IFNβ, IFNγ, and TNFα. b. Heatmap of the relative expression of the same 25 probe sets compared to no-treatment control in keratinocyte *ex vivo* challenged with IFNα2a, IFNβ, IFNγ, and TNFα. Red indicates upregulated gene expression relative to no treatment control, green indicates downregulated gene expression relative to no treatment control, black indicates no significant change in gene expression of challenged samples relative to control.
Figure 32a-32c: The distribution of the average (a) and median (b) fold change of the top 25 most overexpressed type-I IFN inducible probe sets in 26 pairs of lesional skin compared to non-lesional skin. (c) the average of the average and median fold change of the top 25 most overexpressed type-I IFN inducible probe sets in 26 pairs of lesional and non-lesional skin.
Figure 33a-33d: Relative expression of selected type-I IFN inducible genes ((a)HPSE, (b) OASL, and (c) HERC6) and non type-IFN inducible genes ((d) SERPINB4) in lesional skin (LS) compared to non-lesional skin (NS), and non-lesional skin compared to normal skin (NN) in psoriatic patients based on microarray data. The fold change of these genes in LS is compared to its paired NS, while NS is compared to the average of 21 normal skin controls.
The p value for HPSE, OASL, HERC6, and SERPINB4 is a comparison between NS and
NN, between LS and NS are (listed in pairs): 0.468, <0.00001; 0.376, <0.00001; 0.03, <0.00001; 0.0002, <0.00001.

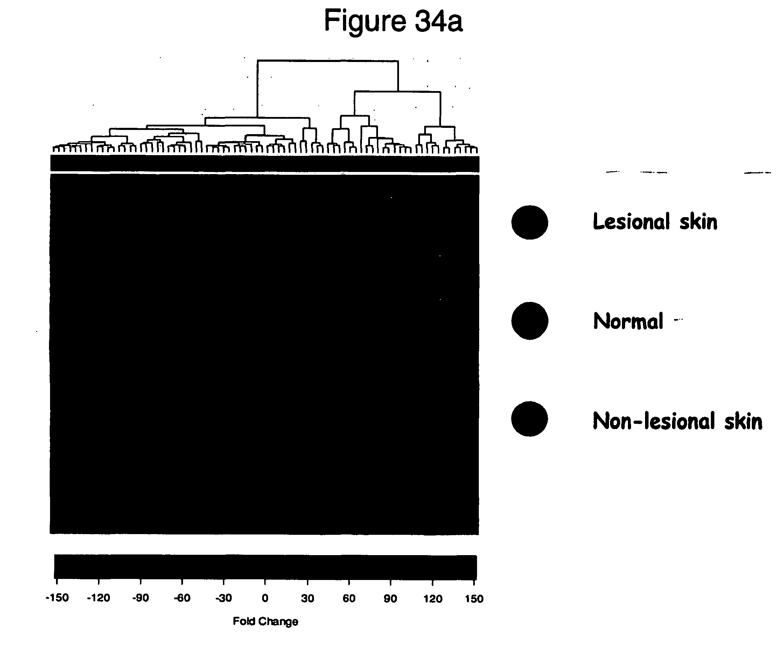

Figure 34a-34b: (a) Hierarchical clustering of all psoriasis samples profiled (21 normal (blue bars)) 26 paired non lesional (black bars) and lesional skin (red bars) from 24 psoriatic patients, and 3 lesional skin (red bars) from 3 psoriatic patients whose paired non lesional skin either did not yield sufficient cRNA for hybridization or scanned arrays had scaling factors that were more than 3 times the average) using the 164 upregulated type-I IFN inducible probe sets in lesional skin compared to those in mostly paired non-lesional skin. Each row corresponds to a single probe set, while each column corresponds to a single sample. The branch lengths indicate the degree of correlation with which samples are joined, with a longer branch indicating a weaker correlation. Color represents relative expression level of individual probe set as compared to the average expression of the 21 normals. Red represents upregulation vs. control and green represents downregulation vs. control. (b) PCA of all psoriasis samples profiled using the 164 upregulated type-I IFN inducible probe sets in lesional skin compared to those in mostly paired non-lesional skin. (PCA is calculated and data is visualized in Spotfire). Each circle represents one sample (blue circles = normal skin; black circles = non-lesional skin; red circles = lesional skin).
Figure 35: Overexpression of selected type-I IFN inducible genes in 18 pairs of lesional and non-lesional skin from 18 psoriatic patients based on taqMan QRT-PCR assays using Fluidigm's BioMark^{™} 48.48 dynamic array.
Figure 36a-36b: Correlation coefficient distribution of overexpressed genes in lesional skin of psoriatic patients between taqMan and array results. The genes are grouped based on correlation coefficient between taqMan QRT-PCR and microarray measurement. (a) correlation coefficient distribution of all 40 upregulated genes in lesional skin that are validated by taqMan QRT-PCR; (b) correlation coefficient distribution of 29 type-IFN inducible genes.
Figure 37a-37d: Comparison of taqMan QRT-PCR based assay using BioMark^{™} 48.48 dynamic array and Affymetrix® genechip results for selected type-I IFN inducible genes ISG15 and MX1.
Figure 38: TaqMan QRT-PCR validation of Affymetrix® genechip results of overexpression of type-I IFN inducible genes IFI27 and CXCL10.

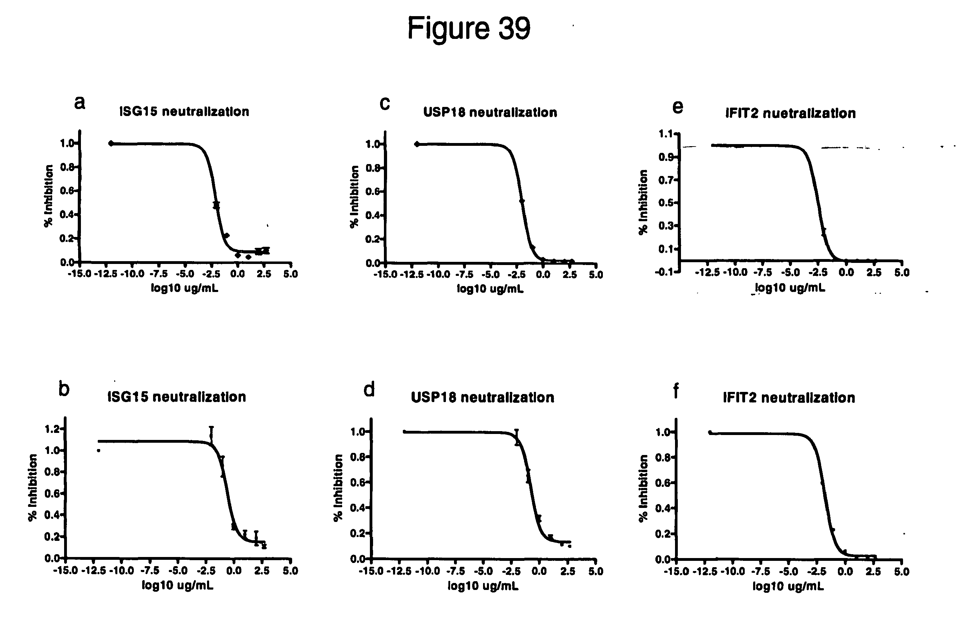

Figure 39a-39f *Ex vivo* stimulation of normal keratinocytes with leukocyte IFN and IFNα2a and dose-dependent neutralization of type-I IFN induced genes by IFNα antibody. (a) neutralization of ISG15 overexpression in response to 350 I.U./mL IFNα2a, (b) neutralization of ISG15 overexpression in response to 150 I.U./mL leukocyte IFN, (c) neutralization of USP18 overexpression in response to 350 I.U./mL IFNα2a, (d) neutralization of USP18 overexpression in response to 150 I.U./mL leukocyte IFN, (e) neutralization of IFIT2 overexpression in response to 350 I.U./mL IFNα2a, and (f) neutralization of IFIT2 overexpression in response to 150 I.U./mL leukocyte IFN. Each dose titration curve is generated on three technical replicates. The overexpression of individual genes with no IFNα antibody is normalized to 1.

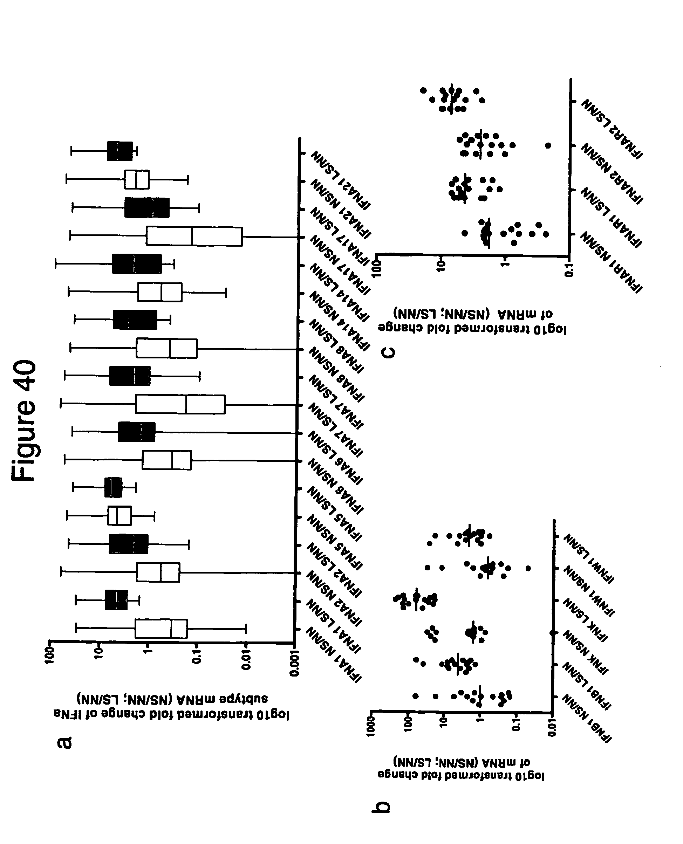

Figure 40a-40c: Relative expression of mRNA and median fold changes of type-I IFNα subtypes (Figure 40a), other members of the type-I IFNs (Figure 40b), and IFNα receptors (Figure 40c) in the lesional skin (LS) or the non-lesional skin (NS) compared to skin from healthy normal controls (NN). The averages of the relative mRNA levels of these cytokines and their receptors in the normal skin of two healthy donors were scaled to be I based on taqMan QRT-PCR assays using TLDA from Applied Biosciences. Black: the relative fold change ofmRNA in the non-lesional skin compared to normal skin (NS/NN); Red: the relative fold change of mRNA in the lesional skin compared to normal skin (LS/NS). The *p* values for the overexpression of these individual genes in the non-lesional skin or lesional skin compared to healthy normal skin (listed in pairs) are as follows: IFNα, 0.303, <0.001; IFNα2, 0.389, 0.072; IFNα5, <0.001, 0.002; IFNα6, 0.664, 0.093; IFNα7, 0.586, 0.077; IFNα8, 0.430, 0.049; IFNα14, 0.224, 0.049; IFNα17, 0.552, 0.0203; IFNα21, 0.113, 0.003; IFNβ, 0.255, 0.022; IFNκ, 0.03, <0.001; IFNω, 0.516, 0.049; IFNAR1, 0.192, <0.001 ; IFNAR2, <0.001, <0.001, respectively.

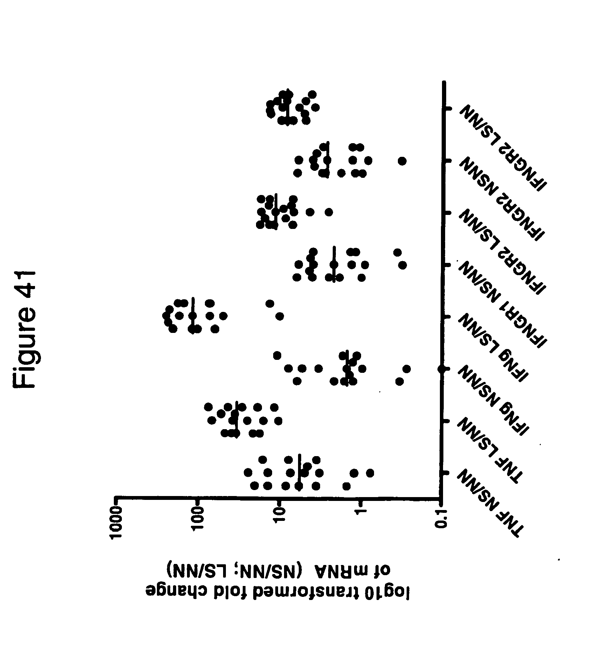

Figure 41 : Relative expression of mRNA and median fold changes of IFNγ, TNFα, and IFNγ receptors in the lesional skin (LS), or the non-lesional skin (NS) compared to skin from healthy normal controls (NN). The averages of the relative mRNA levels of these cytokines and their receptors in the normal skin of two healthy donors were scaled to be I based on taqMan QRT-PCR assays using TLDA from Applied Biosciences. Black: the relative fold change of mRNA in the non-lesional skin compared to normal skin; Red: the relative fold change of mRNA in the lesional skin compared to normal skin. The p values for the overexpression of these individual genes in the non-lesional skin or lesional skin compared to healthy normal skin (listed in pairs) are as follows: IFNγ, 0.02, <0.001; IFNGR1, <0.001, <0.001; IFNGR2, <0.001, <0.001; TNFα, <0.001, <0.001, respectively.
Figure 42: A Venn diagram illustrating both the number of probe sets that are altered by type I IFN, IFNγ, and TNFα during *ex vivo* stimulation, and probe sets that are altered in the lesional skin compared to non-lesional skin. Red numbers: probe sets that show increased expression with cytokine treatment or compared to non-lesional skin baseline; Green numbers: probe sets that show decreased expression with cytokine treatment or compared to non-lesional skin baseline. The intersecting regions represent the probe sets that are common to both comparisons.

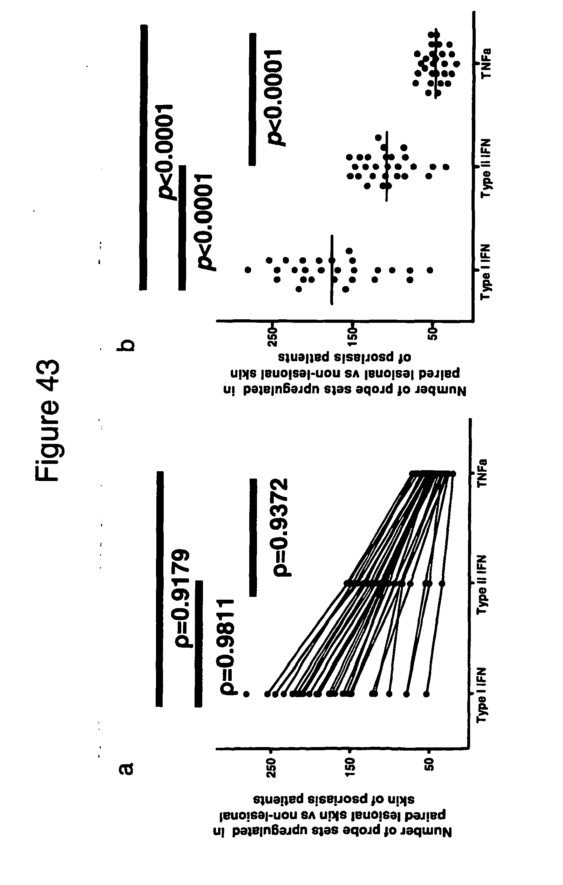

Figure 43a and 43b: Co-overexpression type-I IFN, type-II IFN, and TNF-inducible genes in lesional/non-lesional skin of psoriatic patients based on Affymetrix genechip® results. The type-I IFN, type-II IFN, and TNFα inducible genes were selected based on *ex vivo* stimulation experiments (Examples 10 and 16). A probe set with an at least 2-fold change from non-lesional to lesion skin was considered overexpressed. (a) the number of up-regulated type I IFN, IFNγ, and TNFα inducible genes in the lesional skin shows strong correlation. (b) the number of type I IFN, IFNγ, and TNFα inducible genes in the lesional skin were significantly different amongst pairwise comparisons.

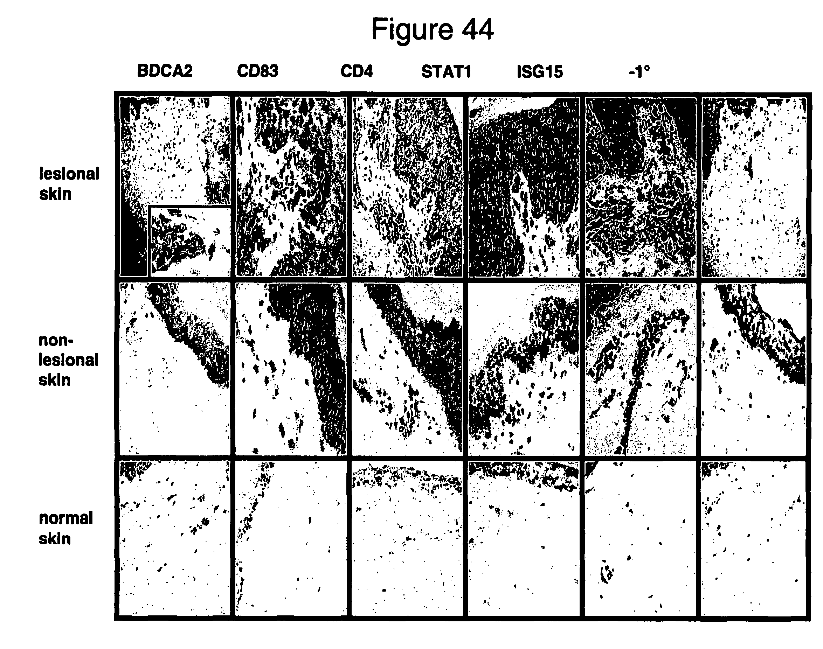

Figure 44: Immunohistochemical analysis of biopsies from psoriatic skin, non-lesional skin and skin from normal donors. BDCA2 is a specific marker for pDCs which are present at greater numbers in lesional skin compared to non-lesional skin, and not at all in normal skin. CD83 is a marker for mDCs, CD4 is present on T cells and dendritic cells. STAT1 protein staining was observed in the epidermis of lesional skin (both nuclear and cytoplasmic) and dermal mononuclear inflammatory cells, but not in non-lesional or normal skin. ISG15 protein increase was observed in psoriatic skin and to a lesser extent in non-lesional skin, but was not detected in normal skin.
Figure 45: A Venn diagram illustrating the number of probe sets that show altered expression at mRNA level in the lesional skin compared to non-lesional skin, or in the non-lesional skin compared to normal skin of psoriatic patients. Values shaded in red indicate the number of probe sets significantly upregulated while those values shaded in green indicate the number of probe sets significantly downregulated. The intersecting region represents probe sets that are common to both comparisons.

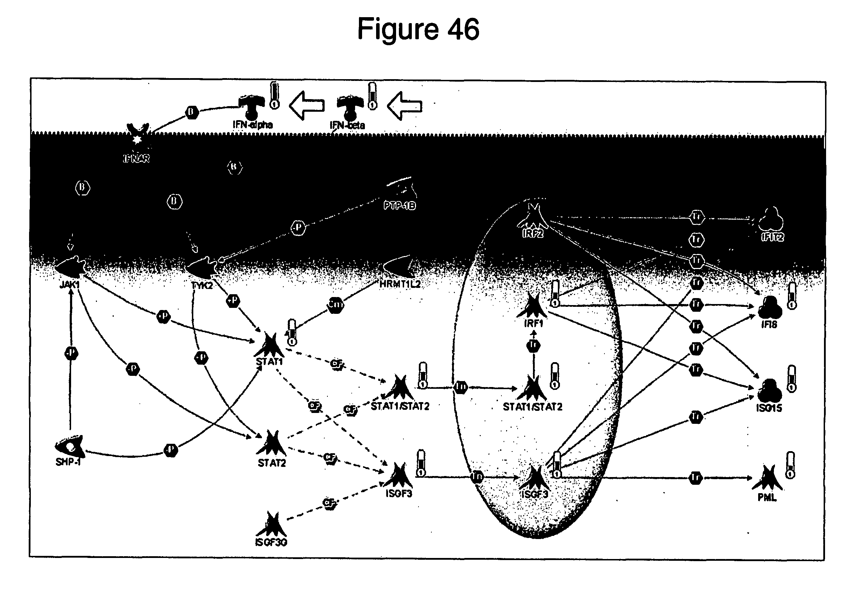

Figure 46: Graphic representation of type-IFN signaling pathway that is activated in the lesional skin of psoriatic patients. Pathway image was generated with GeneGo's MetaCore integrated software suite. Individual symbols within the image represent well characterized proteins or protein complexes. Arrows linking the proteins represent the stimulatory, inhibitory, or interactive effect of the protein on the target protein. Thermometers adjacent to the individual symbols represent relative expression levels (red indicates overexpression, while green indicates underexpression) of transcripts that comprise the protein (or protein complex) within the particular pathway.
Figure 47a and 47b: Table providing fold change (fc; log2 transformed) and q value (calculated by FDR) of the top 100 probe sets upregulated in the lesional skin compared to non-lesional skin in psoriasis. Also listed are the log2 transformed fold change and q values of these genes when comparing non-lesional skin with healthy normal skin controls. Type I IFN inducible genes are listed in bold font.

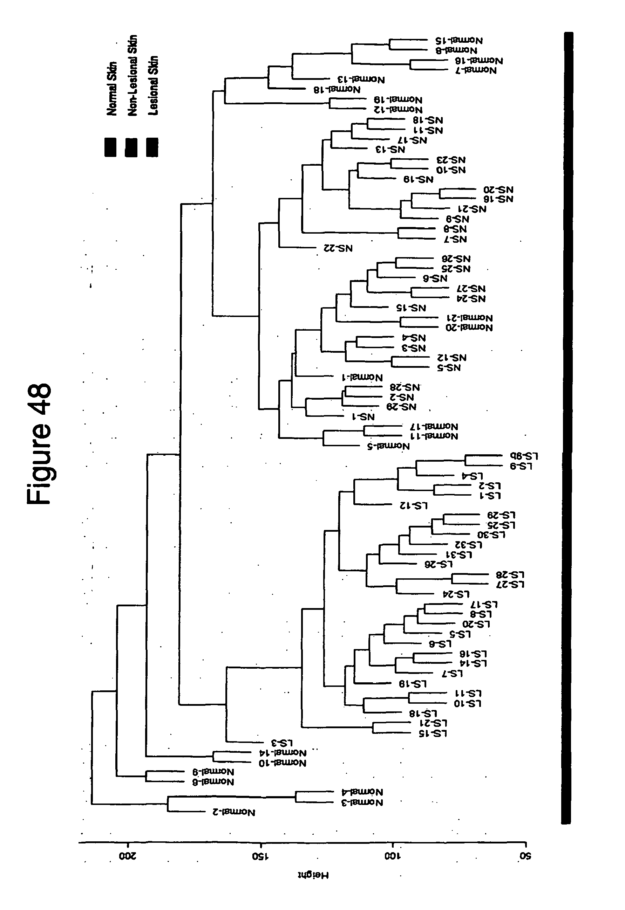

Figure 48: Distinctive separation of the lesional skin from non-lesional skin and normal skin - hierarchical clustering of all samples using transcript profiles of all genes on a whole genome (Affymetrix whole genome U133 plus v2.0 array) array.
Figure 49: Probe sets identified as IFNγ inducible by overlap in Figure 42.
Figure 50: Probe sets identified as TNFα inducible by overlap in Figure 42.
Figure 51: Probe sets identified as type I IFN inducible by overlap in Figure 42.
Figure 52: Immunohistochemical analysis of biopsies from skin lesions of a placebo-treated SLE patient to detect pDC, mDC, and T cell infiltrates.
Figure 53: Immunohistochemical analysis of biopsies from skin lesions of a placebo-treated SLE patient to detect HERC5, ISG15, and IP10 proteins, proteins expressed from type I IFN-induced genes.
Figure 54: Immunohistochemical analysis of biopsies from skin lesions of an SLE patient treated with 10 mg/kg MEDI-545 to detect pDC, mDC, and T cell infiltrates.
Figure 55: Immunohistochemical analysis of biopsies from skin lesions of an SLE patient treated with 10 mg/kg MEDI-545 to detect HERC5, ISG15, and IP10 proteins, proteins expressed from type I IFN-induced genes.
Figure 56: Immunohistochemical analysis of biopsies from skin lesions of an SLE patient treated with 10 mg/kg MEDI-545 to detect pDC, mDC, and T cell infiltrates.
Figure 57: Immunohistochemical analysis of biopsies from skin lesions of an SLE patient treated with 10 mg/kg MEDI-545 to detect HERC5, ISG15, and IP10 proteins, proteins expressed from type I IFN-induced genes.
Figure 58a and 58b: Heatmap (a) and PCA (b) showing neutralization of the top 25 type I IFN inducible genes in a skin biopsy of an SLE patient treated with 10 mg/kg MEDI-545 at 0 and 7 days post-dosing.
Figure 59a-d: Detection of type I and type II IFN activity in an IFN bioassay.
Figure 60a and 60b: Detection of MEDI-545 (a) and MEDI-546 (b)-mediated neutralization of IFNα activity in the IFN bioassay.
Figure 61: Detection of anti-IFNγ-mediated neutralization of IFNγ activity in the IFN bioassay.
Figure 62: Detection of anti-IFNω-mediated neutralization of IFNω activity in the IFN bioassay.
Figure 63: Detection of anti-IFNβ-mediated neutralization of IFNβ activity in the IFN bioassay.
Figure 64: Heat map showing modulation of gene expression in whole blood from healthy donors *ex vivo* stimulated with IFNγ, TNFα, or IFNα/β. Negative control (NT).
Figure 65: Type I IFN-inducible genes were among the most upregulated genes in whole blood of SLE patients.
Figure 66: IFNγ, IFNω, IFNAR1 and IFNAR2 mRNAs are upregulated in whole blood of lupus patients.
Figure 67: Heat map showing modulation of gene expression in healthy donor PBMCs *ex vivo* stimulated with lupus patient serum.
Figure 68a and 68b: (A) PCA plot showing lupus patients having a strong/moderate type I IFN inducible signature (approximately 66% in this sampling) cluster together. (b) Table providing the 25 genes used for PCA analysis.
Figure 69: Confirmation of overexpression of selected type-I IFN inducible genes in lupus patients based on taqMan QRT-PCR assays using Fluidigm's BioMark^{™} 48.48 dynamic array.
Figure 70a and 70b: (a) Ability of four different SLE patient serum samples to induce type I IFN activity in a reporter gene assay. (b) Number of transcripts induced at least 3-fold in healthy human PBMCs by each of the four different SLE patient serum samples following 4 hour co-incubation.
Figure 71a and 71b: The majority of genes neutralized by an anti-IFNα Ab 4 hours post co-incubation of SLE patient serum and healthy human PBMCs are type I IFN genes, while the majority of genes neutralized by the anti-IFNα Ab 18 hours post co-incubation of SLE patient serum and healthy human PBMCs are non-type I IFN genes as shown by (a) heatmap analysis and represented (b) in bar graphs.
Figure 72a and 71b: Provides the (a) type I IFN genes and (b) non-type I IFN genes that were upregulated and neutralized by an anti-IFNα Ab 18 hours post co-incubation of SLE patient serum and healthy human PBMCs, but that were not upregulated 4 hours post co-incubation of SLE patient serum and healthy human PBMCs.
Figure 73: Provides pathways and cell processes neutralized by an anti-IFNα Ab 18 hours following co-incubation of SLE patient serum and healthy human PBMCs.
Figure 74a and 74b: Detection of (a) increased and (b) decreased levels of specific proteins in serum of lupus patients.
Figure 75: QuantiGenePlex 1.0 analysis of IFN-inducible gene signatures from whole blood of 5 healthy donors stimulated with 20 IU/mL IFNα2b.
Figure 76: Dose-dependent changes in gene expression in blood from a single healthy donor treated with multiple concentrations of IFNα2b.
Figure 77: Detection of IFN-inducible transcripts in PAXgene-preserved whole blood samples from SLE subjects with and without detectable serum IFNα activity.
Figure 78: Correlation between QuantiGenePlex and Fluidigm technologies in SLE PAXgene-preserved whole blood samples.
Figure 79: Longitudinal testing of SLE samples following administration of an anti-IFNα monoclonal antibody: comparison of QuantiGenePlex 2.0 and Fluidigm technologies.
Figure 80: Representative heat map visualizing the (in descending order) overexpression of type I IFN gene signature; overexpression of granulocyte signature; underexpression of T-cell signature, underexpression of NK-cell signature, and underexpression of B-cell signature, in whole blood from 46 SLE patients (indicated by red bar under the heat map) compared with whole blood from 24 healthy donors (indicated by blue bar under the heat map) IFN=interferon; SLE=systemic lupus erythematosus.

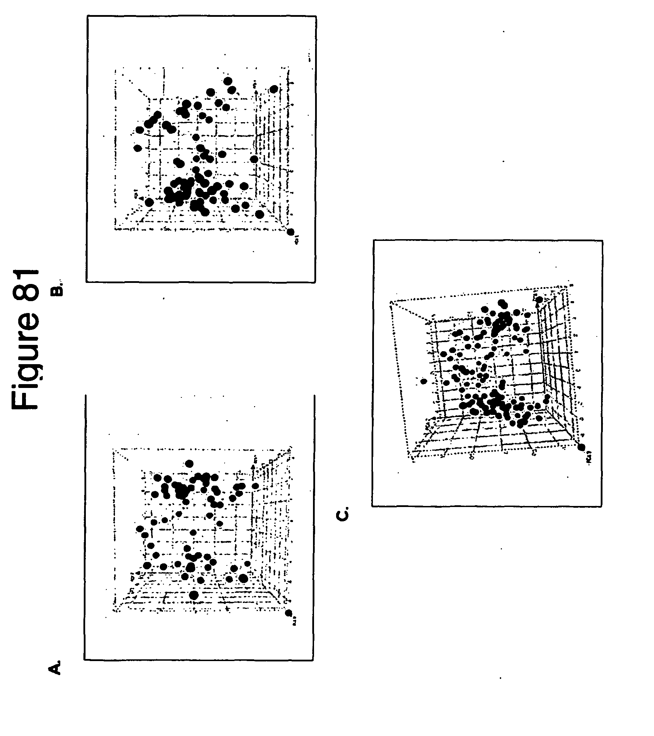

Figure 81a-81c: Type I IFN-inducible genes in whole blood of SLE patients can be used to separate SLE patients with a type I IFN gene signature from healthy normal controls. (a) Three-dimensional PCA plot of whole blood from 46 SLE samples using a 114 type I IFN-inducible probe sets upregulated in whole blood of SLE patients compared with those from 24 healthy donors. (b) PCA plot of whole blood from 54 SLE patients in the prospective study using the 114 upregulated type I IFN-inducible probe set confirmed the overexpression of type I IFN gene signatures in SLE patients. (c) PCA plot of whole blood from 100 SLE samples in both discovery and prospective study using 21 upregulated type I IFN-inducible gene panel in SLE patients compared with 24 healthy donors. Each point represents one sample (blue dots, healthy normals; red dots, SLE patients). IFN=interferon; PCA=principal components analysis; SLE=systemic lupus erythematosus.
Figure 82: Relative expression of mRNAs and median fold changes (horizontal bars) of TNF-α, IFN-γ, and IFN-γ receptors in whole blood of SLE patients compared with healthy controls (P<0.05 for all). Averages of relative mRNA levels of these cytokines and their receptors in whole blood from 24 healthy donors were scaled to 1 based on TaqMan QRT-PCR assays. IFN=interferon; QRT-PCR=quantitative real-time reverse transcriptase polymerase chain reaction; SLE=systemic lupus erythematosus; TNF=tumor necrosis factor.

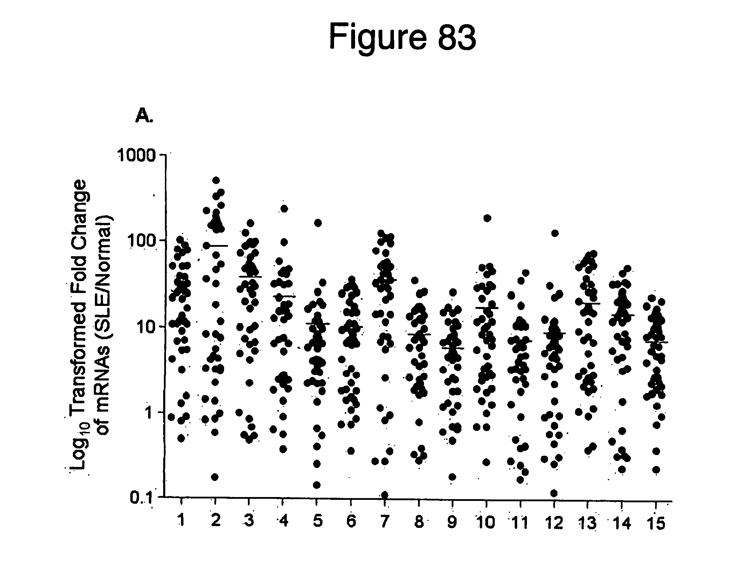

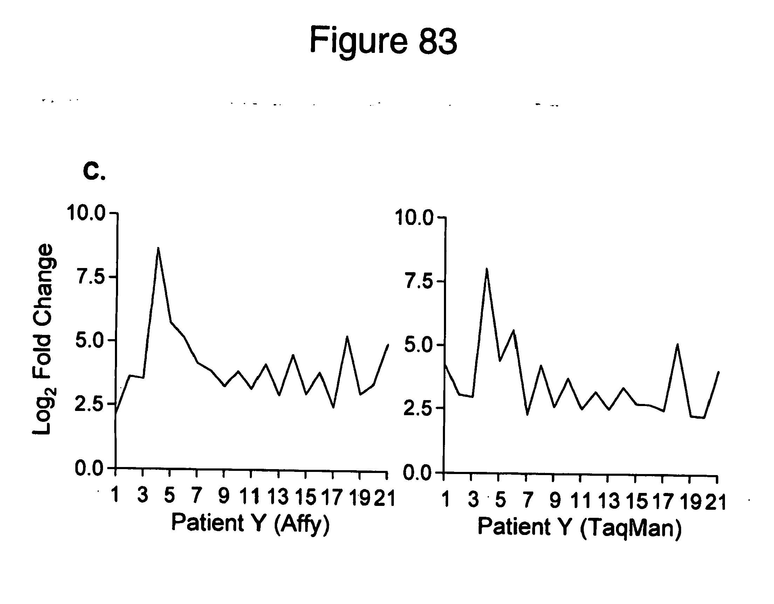

Figure 83a-83c: TaqMan QRT-PCR confirmed the overexpression of type I IFN-inducible genes in whole blood of SLE patients. (a) Relative fold changes of 15 type I IFN-inducible genes (generically labeled 1-15) in SLE patients were compared with healthy donors (p < 0.05 for all). Averages of relative mRNA levels of genes in the pooled RNA from 24 healthy donors were scaled to 1 based on TaqMan QRT-PCR assays. Horizontal bars represent average fold change. (b and c) TaqMan QRT-PCR validation of overexpression of the 21-gene panel of type I IFN-inducible genes in whole blood of SLE patients as determined by whole genome array. The relative overexpression of 21 type I IFN-inducible genes in 2 SLE patients is shown via microarray (left) and TaqMan (right) assays. Correlation coefficients between TaqMan QRT-PCR and microarray were 0.9861 and 0.9888 for patient X and Y, respectively. IFN=interferon; QRT-PCR=quantitative real-time reverse transcriptase polymerase chain reaction; SLE=systemic lupus erythematosus.

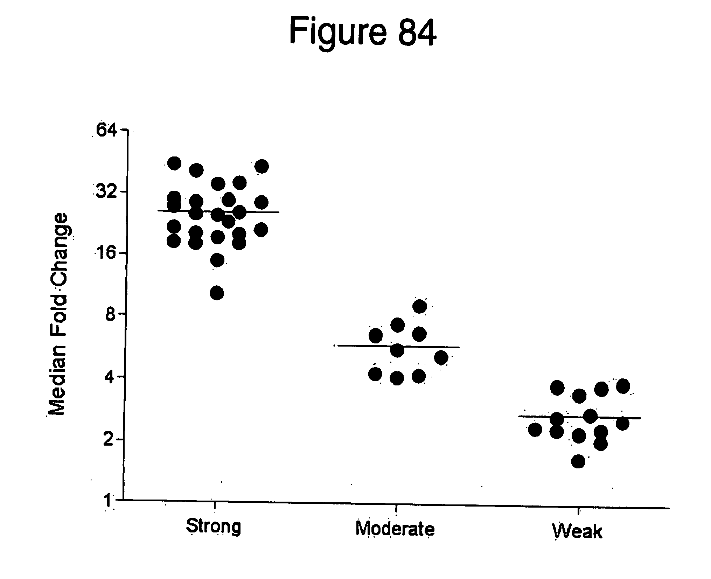

Figure 84: Magnitude of overexpression of type I IFN gene signature in whole blood of SLE patients as measured by the median fold change of the 25 most overexpressed type I IFN-inducible genes or type I IFN gene signature score in individual SLE patients. The horizontal bars represent the median values. Patients whose type I IFN gene signature score was ≥10 were considered to have strong type I IFN gene signatures; those with scores between 4 and 10 were considered to have moderate type I IFN gene signatures, whereas those with scores ≤4 were considered to have weak type I IFN gene signatures. IFN=interferon; SLE=systemic lupus erythematosus.

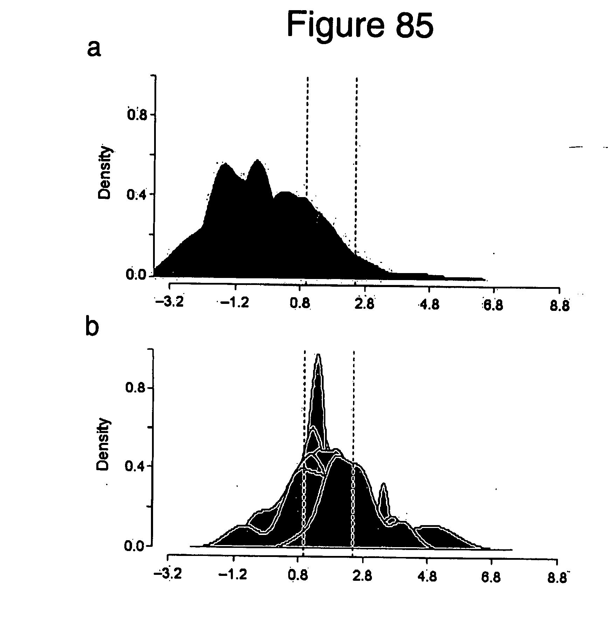

Figure 85a-85c: Stratification of 35 SLE patients into groups of low (a; green), moderate (b; gray), and high (c; red) type I IFN gene signature based on median fold change across the 21-gene panel of type I IFN-inducible genes. Densities for each SLE patient are calculated and graphed using the fold change for each of the 21 genes from each SLE patient on the log2 scale to provide a representation of the distribution of 21 genes fold change values. The vertical dashed lines partition the 3 classes of signature scores: 7 patients with a weak type I IFN gene signature = median fold change <1.91 (0.93 on log2 scale), 8 patients with a moderate type I IFN gene signature = median fold change between 1.91 and 5.53, and 20 patients with a strong type I IFN gene signature = median fold change >5.53 (2.47 on log2 scale). IFN=interferon; SLE=systemic lupus erythematosus.
Figure 86: Dose-dependent neutralization of 21 upregulated IFN-α/β-inducible genes in SLE patients by MEDI-545.
Figure 87a and 87b: Heatmap (a) and PCA (b) showing neutralization of 21 upregulated IFN-α/β-inducible genes in whole blood of an SLE patient treated with 30 mg/kg MEDI-545 (0, 1, 4, 7, and 14 days post-dose).
Figure 88a and 88b: PCA plots prepared using the 21 upregulated IFN-α/β-inducible probe sets do not show IFN signature neutralization in placebo-treated patients.
Figure 89: Neutralization of the 21 upregulated IFN-α/β-inducible probe sets in patients treated with 0.3, 1.0, 3.0, 10.0, and 30.0 mg/kg MEDI-545.

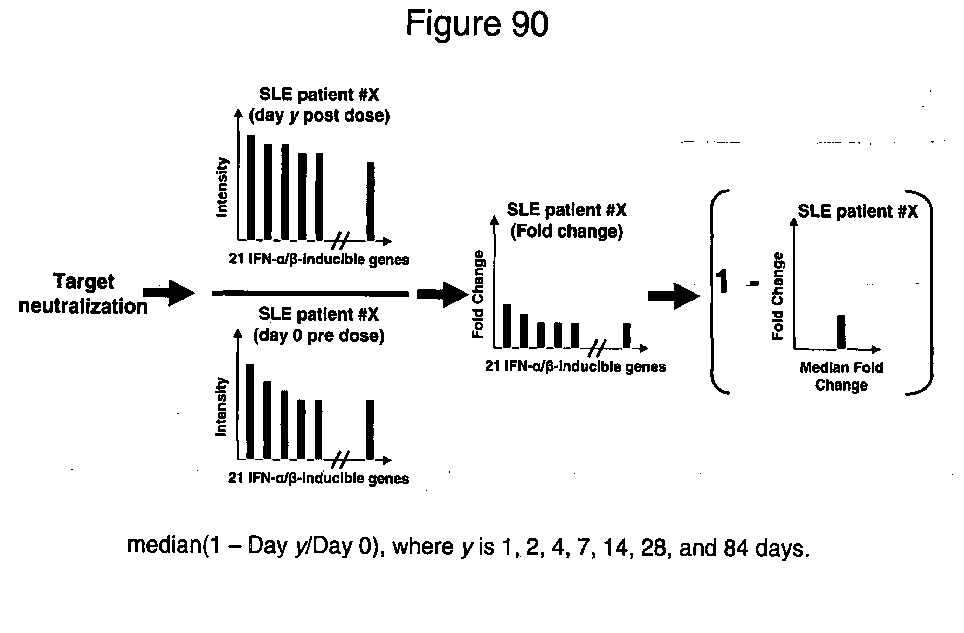

Figure 90: Methodology for calculating target neutralization for Figure 89.

### DETAILED DESCRIPTION

The invention encompasses methods of identifying, diagnosing, treating, and monitoring disease progression in patients. Patients include any animal having a type I IFN or an IFNα-inducible disease, disorder, or condition. The patient may have the disease, disorder, or condition as a result of experimental research, e.g., it may be an experimental model developed for the disease, disorder, or condition. Alternatively, the patient may have the disease, disorder, or condition in the absence of experimental manipulation. Patients include humans, mice, rats, horses, pigs, cats, dogs, and any animal used for research.

The patient may comprise a type I IFN or IFNα-inducible PD marker expression profile. The type I IFN or IFNα-inducible PD marker expression profile may be a strong profile, a moderate profile, or a weak profile. The type I IFN or IFNα-inducible PD marker expression profile can readily be designated as strong, moderate, or weak by determining the fold dysregulation of the type I IFN or IFNα-inducible PD marker expression profile of the patient, (*e.g*., the fold increase in expression of upregulated type I IFN or IFNα-inducible PD markers in the patient), relative to a control sample(s) or control patient(s) and comparing the patient's fold dysregulation to that of other patients having a type I IFN or IFNα-inducible PD marker expression profile. Fold dysregulation can be calculated by well known methods in the art as can the comparing. See, *e.g*., Example 8.

The type I IFN or IFNα-inducible PD marker expression profile may comprise upregulation of any group of genes or group of genes detected by the probes identified in Tables 19, 20, 21, 22, 23, 24, 26, 28, or 30. The group of genes or group of genes detected by the probes identified in Tables 19, 20, 21, 22, 23, 24, 26, 28 or 30 may include any at least 2, any at least 3, any at least 4, any at least 5, any at least 6, any at least 7, any at least 8, any at least 9, any at least 10, any at least 11, any at least 12, any at least 13, any at least 14, any at least 15, any at least 16, any at least 17, any at least 18, any at least 19, any at least 20, any at least 21, any at least 22, any at least 23, any at least 24, any at least 25, any at least 26, any at least 27, any at least 28, any at least 29, any at least 30, any at least 40, or any at least 50 of the genes or genes detected by the probes identified in the Tables.

The group of genes that may be included in the type I IFN or IFNα-inducible PD marker expression profile of the patient may be MX1, LY6E, IFI27, OAS1, IFIT1, IFI6, IFI44L, ISG15, LAMP3, OASL, RASD2, and IFI44. The genes or genes detected by the probes may include IFI44, IFI27, IFI44L, DNAPTP6, LAMP3, LY6E, RSAD2, HERC5, IFI6, ISG15, OAS3, SIGLEC1, OAS2, USP18, RTP4, IFIT1, MX1, OAS1, EPSTI1, PLSCR1, and IFRG28.

The genes may include any at least 2, any at least 3, any at least 4, any at least 5, any at least 6, any at least 7, any at least 8, any at least 9, any at least 10, or any at least 11, or any at least 12, or any at least 13, or any at least 14, or any at least 15, or any at least 16, or any at least 17, or any at least 18, or any at least 19, or at least 20, or any at least 21, or any at least 22, or any at least 23, or any at least 24, or any least 25, or any at least 26, or any at least 27, or any at least 28, or any at least 29, or any at least 30 of LAMP3, DNAPTP6, FLJ31033, HERC6, SERPING1, EPST11, RTP4, OASL, FBXO6, IFIT2, IFI44, OAS3, BATF2, ISG15, IRF7, RSAD2, IFI35, OAS1, LAP3, IFIT1, IFIT5, PLSCR1, IFI44L, MS4A4A, GALM, UBE2L6, TOR1B, SAMD9L, HERC5, TDRD7, TREX1, PARP12, and AXUD1.

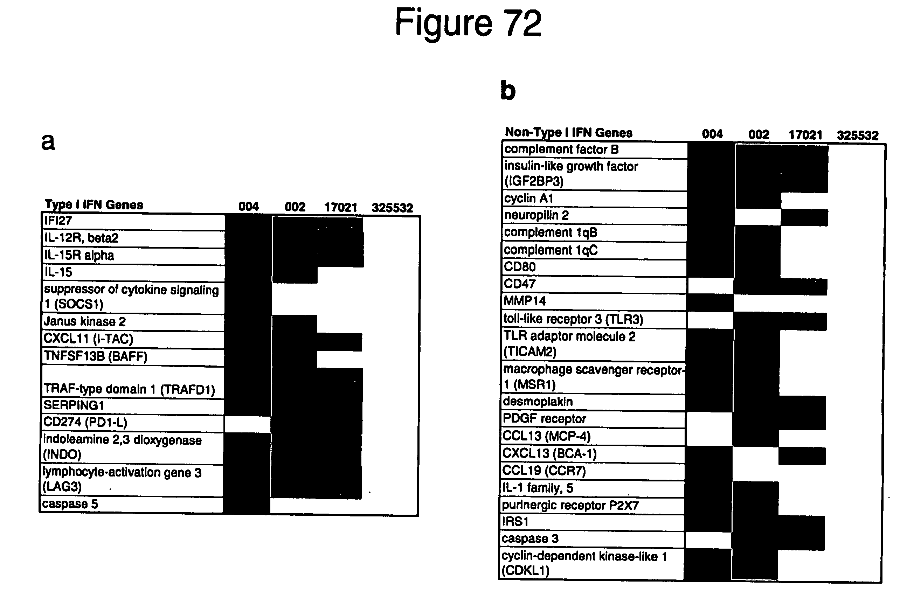

The type I IFN or IFNα-inducible PD marker expression profile may contain upregulation of the entire group of genes or group of genes detected by the probes identified in one of Table 19, or Table 20, or Table 21, or Table 22, or Table 23, or Table 24, or Table 26, or Table 28, or Table 30 or may be any one or more of the genes identified in Figure 72. The type I IFN or IFNα-inducible PD marker expression profile may include upregulation of all the genes identified in Table 24. The type I IFN or IFNα-inducible PD marker expression profile may include upregulation of the genes identified in figure 72A or figure 72b, or figure 72a and figure 72b.

The patient comprising the type I IFN or IFNα-inducible PD marker expression profile may further comprise downregulated type I IFN or IFNα PD marker(s). The downregulated PD markers may include any one, any two, any three, any four, any five, any six, any seven, any eight, any nine, any ten, any 15, any 20, any 25, any 30, any 35, any 40, any 45, or any 50 of the genes in Table 31 or any of CYP1B1, TGST1, RRAGD, IRS2, MGST1, TGFBR3, and RGS2.

The patient comprising the type I IFN or IFNα-inducible PD marker expression profile may further comprise upregulation of expression of any number of IFNα or type-I IFN subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14, IFNα17, IFNα21, IFNβ, or IFNω. The patient may comprise upregulation of expression of IFN subtypes IFNα1, IFNα2, IFNα8, and IFNα14.

Alternatively, a patient treated in the methods encompassed by the invention may simply be one identified as comprising a gene expression profile with upregulation of expression of any number of IFNα or type-I IFN subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14, IFNα17, IFNα21, IFNβ, or IFNω. These subtypes may include IFNα1, IFNα2, IFNα8, and IFNα14.

The patient comprising the type I IFN or IFNα-inducible PD marker expression profile may further comprise upregulation of expression of IFNα receptors, either IFNAR1 or IFNAR2, or both, or TNFα, or IFNγ, or IFNγ receptors (either IFNGR1, IFNGR2, or both IFNGR1 and IFNGR2). The patient may simply be identified as one who comprises upregulation of expression of IFNα receptors, either IFNAR1 or IFNAR2, or both, or TNFα, or IFNγ, or IFNyreceptors (either IFNGR1, IFNGR2, or both IFNGR1 and IFNGR2).

The upregulation or downregulation of the type I IFN or IFNα-inducible PD markers in the patient's expression profile may be by any degree relative to that of a sample from a control (which may be from a sample that is not disease tissue of the patient (e.g., non-lesional skin of a psoriasis patient) or from a healthy person not afflicted with the disease or disorder). The degree upregulation or downregulation may be at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of the control or control sample.

Furthermore, the patient may overexpress or have a tissue that overexpresses a type I IFN subtype at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of the control. The type I IFN subtype may be any one of IFNα1, IFNα2, IFNα4, IFNα5, IFNα6, IFNα7, IFNα8, IFNα10, IFNα14, IFNα17, IFNα21, IFNβ, or IFNω. The type I IFN subtypes may include all of IFNα1, IFNα2, IFNα8, and IFNα14.

The patient may further comprise or alternatively comprise alterations in levels of proteins in serum. The patient may have increased serum levels of proteins such as adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, or vWF. The patient may have increased serum levels of any 1, 2,3, 4, 5, 6, 7, 8, 9, 10, 11, 12, 13 14, 15, 16, 17, 18, 19, 20, 21, o22, 23, 24, 25, or 26 of these proteins in serum. The increased level may be at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of a control, e.g., a healthy subject. The alteration may be a decrease in serum levels of proteins such as BDNK, complement 3, CD40 ligand, EGF, ENA-78, EN-RAGE, IGF-1, MDC, myeloperoxidase, RANTES, or thrombopoietin, The patient may have decreased serum levels of any 1, 2, 3, 4, 5, 6, 7, 8, 9, 10, or 11 or these proteins. The decreased level may be at least 10%, at least 15%, at least 20%, at least 25%, at least 30%, at least 40%, at least 50%, at least 60%, at least 70%, at least 75%, at least 80%, at least 90%, or at least 100% that of a control, e.g., a healthy subject. The PD marker profile may comprise one or more of these increased or decreased serum levels of proteins.

The patient may further comprise auto-antibodies that bind to any one of the following auto-antigens: (a) Myxovirus (influenza virus) resistance 1, interferon-inducible protein p78; (b) surfeit 5, transcript variant c; (c) proteasome (posome, macropain) activator subunit 3 (PA28 gamma; Ki) transc; (d) retinoic acid receptor, alpha; (e) Heat shock 10 kDa protein 1 (chaperonin 10); (f) tropomyosin 3; (g) pleckstrin homology-like domain, family A, member 1; (h) cytoskeleton-associated protein 1; (i) Sjogren syndrome antigen A2 (60 kDa, ribonucleoprotein auto-antigen SS-A/Ro); (j) NADH dehydrogenase (ubiquinone) 1, alpha/beta subcomplex 1, 8 kDa; (k) NudE nuclear distribution gene E homolog 1 (A. nidulans); (1) MutL homolog 1, colon cancer, nonpolyposis type 2 (E. coli); (m) leucine rich repeat (in FLII) interacting protein 2; (n) tropomyosin 1 (alpha); (o) spastic paraplegia 20, spartin (Troyer syndrome); (p) preimplantation protein, transcript variant 1; (r) mitochondrial ribosomal protein L45; (s) Lin-28 homolog (C. elegans); (t) heat shock 90 kDa protein 1, alpha; (u) dom-3 homolog Z (C. elegans); (v) dynein, cytoplasmic, light intermediate polypeptide 2; (w) Ras-related C3 botulinum toxin substrate 1 (rho family, small GTP binding protein); (x) synovial sarcoma, X breakpoint 2, transcript variant 2; (y) moesin; (z) homer homolog (Drosophila), transcript variant 1; (aa) GCN5 general control of amino-acid synthesis 5-like 2 (yeast); (bb) eukaryotic translation elongation factor 1 gamma; (cc) eukaryotic translation elongation factor 1, delta; (dd) DNA-damage-inducible transcript 3; (ee) CCAAT/enhancer binding protein (C/EBP) gamma; and any other auto-antigen described in provisional application entitled "Auto-antibody markers of autoimmune disease" filed May 3, 2007 or in provisional application entitled entitled "Auto-antibody markers of autoimmune disease" to be filed November 6, 2007 (for example, but not limited to, those described on Tables 2, 4, 5, and 9). The patient may comprise auto-antibodies that bind to any number of these auto-antigens, *e.g*., any at least 2, at least 3, at least 4, at least 5, at least 6, at least 7, at least 8, at least 9 at least 10, at least 11, at least 12, at least 13, at least 14, at least 15, at least 20, at least 25.

A type I IFN or an IFNα-inducible disease, disorder, or condition is any that exhibits a type I IFN or an IFNα PD marker expression profile or gene signature. A PD marker expression profile and a gene signature will be understood to be equivalent. These diseases, disorders, or conditions include those with an autoimmune component such as systemic lupus erythematosus, insulin dependent diabetes mellitus, inflammatory bowel disease (including Crohn's disease, ulcerative colitis, and Celiac's disease), multiple sclerosis, psoriasis, autoimmune thyroiditis, rheumatoid arthritis, glomerulonephritis, idiopathic inflammatory myositis, Sjogren's syndrome, vasculitis, dermatomyositis, polymyositis, and sarcoidosis. Other diseases, disorders, or conditions include graft versus host disease and transplant rejection.

The patients may also exhibit any of a number of symptoms as discussed in, *e.g*., provisional patent application Methods of Treating Systemic Lupus Erythematosis filed April 16, 2007, or may have a clinical SLEDAI score or BILAG score as discussed in the same. These symptoms may include fatigue, organ damage, malar rash, and alopecia. The patient may be scored using a known clinical scoring system, e.g., SLEDAI which is an index of SLE disease activity as measured and evaluated within the last 10 days (Bombardier C, Gladman D D, Urowitz M B, Caron D, Chang C H and the Committee on Prognosis Studies in SLE: Derivation of the SLEDAI for Lupus Patients. Arthritis Rheum 35:630-640, 1992.). Disease activity under the SLEDAI scoring system can range from 0 to 105. The following categories of SLEDAI activity have been defined: no activity (SLEDAI = 0); mild activity (SLEDAI = 1-5); moderate activity (SLEDAI = 6-10); high activity (SLEDAI = 11-19); very high activity (SLEDAI = 20 or higher). (Griffiths, et al., Assessment of Patients with Systemic Lupus Erythematosus and the use of Lupus Disease Activity Indices). Another disease scoring index is the BILAG index which is an activity index of SLE that is based on specific clinical manifestations in eight organ systems: general, mucocutaneous, neurological, musculoskeletal, cardiovascular, respiratory, renal, and hematology results. Scoring is based on a letter system, but weighted numerical scores can also be assigned to each letter, making it possible to calculate a BILAG score in the range of 0-72. (Griffiths, et al., Assessment of Patients with Systemic Lupus Erythematosus and the use of Lupus Disease Activity Indices). Other scoring indices include the PGA score, the composite responder index (CRI), and the ANAM4™ test. The methods described herein, *e.g*., of treating an autoimmune disorder, may be used for any subject identified as having any activity level of disease activity as measured by any classification methodology known in the art, *e.g*., mild, moderate, high, or very high. The methods described herein, e.g., of treating an autoimmune disorder, may result in a decrease in a patient's symptoms or may result in an improvement in a score of disease for the patient's type I IFN or an IFNα-inducible disease, disorder, or condition.

A therapeutic agent may be administered to a patient or a patient may be identified as a candidate for administration of an agent or a therapeutic agent. A therapeutic agent is any molecule that binds to and modulates type I IFN or IFNα activity. The therapeutic agent may be a small molecule or a biological agent. If the therapeutic agent is a small molecule it may be synthesized or identified and isolated from a natural source.

If the therapeutic agent is a biological agent, it may be an antibody specific for any subtype(s) of type I IFN or IFNα. For instance, the antibody may be specific for any one of IFNα1, IFNα2, IFNα4, IFNα5, IFNα6, IFNα7, IFNα8, IFNα10, IFNα14, IFNα17, IFNα21, IFNβ, or IFNω. Alternatively, the antibody may be specific for any two, any three, any four, any five, any six, any seven, any eight, any nine, any ten, any eleven, any twelve type I IFN of IFNα subtypes. If the antibody is specific for more than one type I IFN subtype, the antibody may be specific for IFNα1, IFNα2, IFNα4, IFNα5, IFNα8, IFNα10, and IFNα21; or it may be specific for IFNα1, IFNα2, IFNα4, IFNα5, IFNα8, and IFNα10; or it may be specific for IFNα1, IFNα2, IFNα4, IFNα5, IFNα8, and IFNα21; or it may be specific for IFNα1, IFNα2, IFNα4, IFNα5, IFNα10, and IFNα21. Antibodies specific for type I IFN or IFNα include MEDI-545, any biologic or antibody other than MEDI-545, antibodies described in U.S. patent applications 11/009,410 filed December 10, 2004 and 11/157,494 filed June 20, 2005, 9F3 and other antibodies described in U.S. Patent No. 7,087,726 (Example I and Example 2, those disclosed in Table 3 and Table 4, and/or those disclosed in the table entitled "Deposit of Material" on lines 25-54, column 56), NK-2 and YOK5/19 (WO 84/03105), LO-22 (U.S. Patent 4,902,618), 144 BS (U.S. Patent 4,885,166), and EBI-1, EBI-2, and EBI-3 (EP 119476). A therapeutic agent that modulates IFNα activity may neutralize IFNα activity. One of skill in the art is well aware of preparation and formulation of such biological agents and methods of their administration.

The antibody may be a synthetic antibody, a monoclonal antibody, polyclonal antibodies, a recombinantly produced antibody, an intrabody, a multispecific antibody (including bi-specific antibodies), a human antibody, a humanized antibody, a chimeric antibody, a single-chain Fv (scFv) (including bi-specific scFv), a BiTE molecule, a single chain antibody, a Fab fragments, a F(ab') fragment, a disulfide-linked Fv (sdFv), or an epitope-binding fragment of any of the above. The antibody may be any of an immunoglobulin molecule or immunologically active portion of an immunoglobulin molecule. Furthermore, the antibody may be of any isotype. For example, it may be any of isotypes IgG1, IgG2, IgG3 or IgG4. The antibody may be a full-length antibody comprising variable and constant regions, or an antigen-binding fragment thereof, such as a single chain antibody, or a Fab or Fab'2 fragment. The antibody may also be conjugated or linked to a therapeutic agent, such as a cytotoxin or a radioactive isotope.

In the methods of treatment a second agent other than the agent that binds to modulates IFNα activity may be administered to the patient. Second agents include, but are not limited to non-steroidal anti-inflammatory drugs such as ibuprofen, naproxen, sulindac, diclofenac, piroxicam, ketoprofen, diflunisal, nabumetone, etodolac, and oxaprozin, indomethacin; anti-malarial drugs such as hydroxychloroquine; corticosteroid hormones, such as prednisone, hydrocortisone, methylprednisolone, and dexamethasone; methotrexate; immunosuppressive agents, such as azathioprine and cyclophosphamide; and biologic agents that, *e.g*., target T cells such as Alefacept and Efalizumab, or target TNFα, such as, Enbrel, Remicade, and Humira.

Treatment with the agent may result in neutralization of the type I IFN or IFNα-inducible profile. Treatment with the agent may result in a decrease in one or more symptoms of the type I IFN or an IFNα-mediated disease or disorder. Treatment with the agent may result in fewer flare-ups related to the type I IFN or an IFNα-mediated disease or disorder. Treatment with the agent may result in improved prognosis for the patient having the type I IFN or an IFNα-mediated disease or disorder. Treatment with the agent may result in a higher quality of life for the patient. Treatment with the agent may alleviate the need to co-administer second agents or may lessen the dosage of administration of the second agent to the patient. Treatment with the agent may reduce the number of hospitalizations of the patient that are related to the type I IFN or an IFNα-mediated disease or disorder.

The agent that binds to and modulates type I IFN or IFNα activity may neutralize a type I IFN or IFNα-inducible profile. Neutralization of the type I IFN or IFNα-inducible profile may be a reduction in at least one, at least two, at least three, at least five, at least seven, at least eight, at least ten, at least twelve, at least fifteen, at least twenty, at least twenty five, at least thirty, at least thirty five, at least forty, at least forty five, or at least fifty genes up-regulated by type I IFN or IFNα. The genes upregulated by type I IFN or IFNα may be any group of genes in Tables 19, 20, 21, 22, 23, 24, 26, 28, or 30 as discussed above. Neutralization of the type I IFN or IFNα-inducible profile is a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, at least seven, at least eight, at least ten, at least twelve, at least fifteen, at least twenty, at least twenty five, at least thirty, at least thirty five, at least forty, at least forty five, or at least fifty genes up-regulated in any type 1 IFN or IFNα-inducible profile. Alternatively, neutralization of the type I IFN or IFNα-inducible profile refers to a reduction of expression of up-regulated type I IFN or IFNα-inducible genes that is within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those type I IFN or IFNα-inducible genes in a control sample. If the agent that binds to and modulates type I IFN or IFNα activity is a biologic agent, such as an antibody, the agent may neutralize the type I IFN or IFNα profile at doses of 0.3 to 30 mg/kg, 0.3 to 10 mg/kg, 0.3 to 3 mg/kg, 0.3 to I mg/kg, 1 to 30 mg/kg, 3 to 30 mg/kg, 5 to 30 mg/kg, 10 to 30 mg/kg, 1 to 10 mg/kg, 3 to 10 mg/kg, or I to 5 mg/kg.

Neutralization of the type 1 IFN or IFNα-inducible profile may be increased expression of at least one, at least two, at least three, at least five, at least seven, at least eight, at least ten, at least twelve, at least fifteen, at least twenty, at least twenty five, at least thirty, at least thirty five, at least forty, at least forty five, or at least fifty genes whose expression is reduced by type I IFN or IFNα. The genes whose expression is reduced by type I IFN or IFNα may be any group of genes in Table 30. Neutralization of down-regulated genes in a type I IFN or IFNα-inducible profile is an increase of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90%, or at least 100%, or at least 125%, or at least 130%, or at least 140%, or at least 150%, or at least 175%, or at least 200%, or at least 250%, or at least 300%, or at least 500% of any of the at least one, at least two, at least three, at least five, at least seven, at least eight, at least ten, at least twelve, at least fifteen, at least twenty, or at least twenty five genes whose expression is downregulated in any type I IFN or IFNα-inducible profile. Alternatively, neutralization of the type I IFN or IFNα-inducible profile refers to an increase in expression of type I IFN or IFNα-inducible genes to within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those type I IFN or IFNα-inducible (downregulated) genes in a control sample. If the agent that binds to and modulates type I IFN or IFNα activity is a biologic agent, such as an antibody, the agent may neutralize the type I IFN or IFNα profile at doses of 0.3 to 30 mg/kg, 0.3 to 10 mg/kg, 0.3 to 3 mg/kg, 0.3 to I mg/kg, 1 to 30 mg/kg, 3 to 30 mg/kg, 5 to 30 mg/kg, 10 to 30 mg/kg, 1 to 10 mg/kg, 3 to 10 mg/kg, or I to 5 mg/kg.

The agent that binds to and modulates type I IFN or IFNα activity may further or alternatively neutralize expression of one or more type I IFN or IFNα subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14,IFNα17, IFNα21-, IFNβ, or IFNω. These subtypes may include all of IFNα1, IFNα2, IFNα8, and IFNα14.Alternatively, these subtypes may include IFNα1, IFNα2, IFNα4, IFNα5, IFNα8, IFNα10, IFNα21. Neutralization of the IFNα or type-I IFN subtypes may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, at least seven, at least eight, or at least ten of the subtypes. Neutralization of the IFNα or type-I IFN subtypes may be a reduction in expression of IFNα or type-I IFN subtype genes that is within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those IFNα or type I IFN subtypes in a control sample. If the agent that binds to and modulates IFNα activity or type I IFN activity is a biologic agent, such as an antibody, the agent may neutralize the IFNα or type I IFN subtypes at doses of 0.3 to 30 mg/kg, 0.3 to 10 mg/kg, 0.3 to 3 mg/kg, 0.3 to I mg/kg, I to 30 mg/kg, 3 to 30 mg/kg, 5 to 30 mg/kg, 10 to 30 mg/kg, I to 10 mg/kg, 3 to 10 mg/kg, or I to 5 mg/kg.

The agent that binds to and modulates type I IFN or IFNα activity may further or alternatively neutralize expression of IFNα receptors, either IFNAR1 or IFNAR2, or both, or TNFα, or IFNγ, or IFNγ receptors (either IFNGR1, IFNGR2, or both IFNGR1 and IFNGR2). Neutralization of expression of IFNα receptors, either IFNAR1 or IFNAR2, or both, or TNFα, or IFNγ, or IFNγ receptors (either IFNGR1, IFNGR2, or both IFNGR1 and IFNGR2) may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, or at least six of these genes. Neutralization of expression of IFNα receptors, either IFNAR1 or IFNAR2, or TNFα, or IFNγ, or IFNγ receptors (either IFNGR1, IFNGR2, or both IFNGR1 and IFNGR2) is a reduction of expression of at most 50%, at most 45%, at most 40%, at most 35%, at most 30%, at most 25%, at most 20%, at most 15%, at most 10%, at most 5%, at most 4%, at most 3%, at most 2%, or at most I % of expression levels of these genes in a control sample. If the agent that binds to and modulates type I IFN or IFNα activity is a biologic agent, such as an antibody, the agent may neutralize expression of IFNα receptors IFNAR1 or IFNAR2, or TNFα, or IFNγ, or IFNγ receptors IFNGR1 or IFNGR2 at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates type I IFN or IFNα activity may further or alternatively neutralize alterations of levels of proteins in serum, *e.g*., increase levels of those proteins whose serum levels are downregulated or decrease levels of those proteins whose serum levels are upregulated to levels closer to those of control subjects. Neutralization of expression of proteins in serum, such as adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, vWF, BDNK, complement 3, CD40 ligand, EGF, ENA-78, EN-RAGE, IGF-1, MDC, myeloperoxidase, RANTES, or thrombopoietin may be by bringing the level of at least one, at least two, at least three, at least five, at least six, at least seven, at least eight, at least nine, at least ten, at least twelve, at least fifteen, at least twenty, or at least 25 of these proteins to within at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% levels of the protein in serum of a healthy subject. If the agent that binds to and modulates type I IFN or IFNα activity is a biologic agent, such as an antibody, the agent may neutralize levels of the serum proteins, *e.g*., adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, vWF, BDNK, complement 3, CD40 ligand, EGF, ENA-78, EN-RAGE, IGF-1, MDC, myeloperoxidase, RANTES, or thrombopoietin, at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates type I IFN or IFNα activity may further or alternatively reduce number or level of auto-antibodies that bind to any one, any at least 2, any at least 3, any at least 4, any at least 5, any at least 6, any at least 7, any at least 8, any at least 9, any at least 10, any at least 15, or any at least 20 of the following auto-antigens: (a) Myxovirus (influenza virus) resistance 1, interferon-inducible protein p78; (b) surfeit 5, transcript variant c; (c) proteasome (posome, macropain) activator subunit 3 (PA28 gamma; Ki) transc; (d) retinoic acid receptor, alpha; (e) Heat shock 10 kDa protein 1 (chaperonin 10); (f) tropomyosin 3; (g) pleckstrin homology-like domain, family A, member 1; (h) cytoskeleton-associated protein 1; (i) Sjogren syndrome antigen A2 (60 kDa, ribonucleoprotein auto-antigen SS-A/Ro); (j) NADH dehydrogenase (ubiquinone) 1, alpha/beta subcomplex 1, 8 kDa; (k) NudE nuclear distribution gene E homology 1 (A. nidulans); (1) MutL homolog 1, colon cancer, nonpolyposis type 2 (E. coli); (m) leucine rich repeat (in FLII) interacting protein 2; (n) tropomyosin I (alpha); (o) spastic paraplegia 20, spartin (Troyer syndrome); (p) preimplantation protein, transcript variant 1; (r) mitochondrial ribosomal protein L45; (s) Lin-28 homolog (C. elegans); (t) heat shock 90 kDa protein 1, alpha; (u) dom-3 homolog Z (C. elegans); (v) dynein, cytoplasmic, light intermediate polypeptide 2; (w) Ras-related C3 botulinum toxin substrate I (rho family, small GTP binding protein); (x) synovial sarcoma, X breakpoint 2, transcript variant 2; (y) moesin; (z) homer homolog (Drosophila), transcript variant 1; (aa) GCN5 general control of amino-acid synthesis 5-like 2 (yeast); (bb) eukaryotic translation elongation factor I gamma; (cc) eukaryotic translation elongation factor 1, delta; (dd) DNA-damage-inducible transcript 3; (ee) CCAAT/enhancer binding protein (C/EBP) gamma; and any other auto-antigen described in provisional application entitled "Auto-antibody markers of autoimmune disease" filed May 3, 2007; and any other auto-antigen described in provisional application entitled "Auto-antibody markers of autoimmune disease" filed November 6, 2007 (for example, but not limited to, those described on Tables 2, 4, 5, and 9). Reduction in level of auto-antibody may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% in presence of any of the auto-antibodies. If the agent that binds to and modulates type I IFN or IFNα activity is a biologic agent, such as an antibody, the agent may reduce number or level or auto-antibodies at doses of 0.3 to 30 mg/kg, 0.3 to 10 mg/kg, 0.3 to 3 mg/kg, 0.3 to I mg/kg, 1 to 30 mg/kg, 3 to 30 mg/kg, 5 to 30 mg/kg, 10 to 30 mg/kg, I to 10 mg/kg, 3 to 10 mg/kg, or 1 to 5 mg/kg.

The agent that binds to and modulates type I IFN or IFNα activity may not neutralize expression of genes that are not included in an interferon-inducible signature or PD marker profile.

Samples may also be obtained from patients in the methods of the invention. Samples include any biological fluid or tissue, such as whole blood, saliva, urine, synovial fluid, bone marrow, cerebrospinal fluid, nasal secretions, sputum, amniotic fluid, bronchoalveolar lavage fluid, peripheral blood mononuclear cells, total white blood cells, lymph node cells, spleen cells, tonsil cells, or skin. The samples may be obtained by any means known in the art.

IFNα-inducible PD marker expression profiles may include up-regulated expression or activity of genes in cells exposed to elevated IFNα levels relative to baseline. Up-regulated expression or activity of genes includes an increase in expression of mRNA from a gene, an increase in expression of a protein encoded by a gene, or an increase in activity of a protein encoded by a gene. The expression or activity of the genes may be up-regulated as a direct or indirect response to IFNα.

The up-regulated expression or activity of any gene detected in a sample, by probes, or by probes in kits in an IFNα-inducible PD marker expression profile may be at least 1.2-fold, at least 1.25-fold, at least 1.3-fold, at least 1.4-fold, at least 1.5-fold, at least 2.0-fold, at least 2.25-fold, at least 2.5-fold, at least 2.75-fold, at least 3.0-fold, at least 3.5-fold, at least 4.0-fold, at least 4.5-fold, at least 5.0-fold, at least 6.0-fold, at least 7.0-fold, at least 8.0-fold, at least 9.0-fold, at least 10.0-fold, at least 15.0-fold, at least 20.0-fold, at least 25.0-fold, or at least 50.0-fold relative to baseline levels of control cells, *e.g*., cells of healthy volunteers or cells of control animals or cells not exposed to IFNα in culture. All of the genes in the IFNα-inducible PD marker expression profile may have up-regulated expression or activity at the same fold increase. Alternatively, the genes in the PD marker expression profile may have varying levels of up-regulated expression or activity.

The down-regulated expression or activity of any gene detected in a sample, by probes, or by probes in kits in an IFNα-inducible PD marker expression profile may be at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least97%, at least 98%, or at least 99% relative to baseline levels of control cells, *e.g*., cells of healthy volunteers or cells of control animals or cells not exposed to IFNα in culture. All of the genes in the IFNα-inducible PD marker expression profile may have down-regulated expression or activity at the same fold decrease. Alternatively, the genes in the PD marker expression profile may have varying levels of down-regulated expression or activity.

The number of genes included in IFNα-inducible PD marker expression profile may be at least 2, at least 3, at least 4, at least 5, at least 10, at least 20, at least 25 at least 30, at least 50, at least 75, at least 100, at least 150, at least 200, at least 250, at least 300, at least 400, at least 500, at least 750, at least 1000, at least 1500, at least 2000, at least 2500, at least 5000, at least 10000, or at least 15000 genes. These genes may include those listed in Tables 19 and/or 20 and/or 21 and/or 22 and/or 23 and/or 24 and/or 26 and/or 28 and/or 30 and/or 31 and/or any of the genes identified in Figures 72, 74, 75, or 77. The genes included in IFNα-inducible PD marker expression profile may be up-regulated genes, down-regulated genes, or a combination of up- and down-regulated genes.

The genes included in the IFNα-inducible PD marker expression profile may be the genes provided in Tables 19 and/or 20 and/or 21 and/or 22 and/or 23 and/or 24 and/or 26 and/or 28 and/or 30 and/or 31 and/or any of the genes identified in Figures 72, 74, 75, or 77. The genes included in the IFNα-inducible PD marker expression profile may consist of or comprise at least 10%, at least 20%, at least 25%, at least 30%, at least 40%, at least 50%, at least 60%, at least 75%, at least 80%, at least 85% at least 90%, at least 95%, or at least 100% of the genes provided in Tables 19 and/or 20 and/or 21 and/or 22 and/or 23 and/or 24 and/or 26 and/or 28 and/or 30 and/or 31 and/or any of the genes identified in Figures 72, 74, 75, or 77.

The IFNα-inducible PD markers in an expression profile may include any at least 5 genes such as, for example: MX1, LLY6E, IFI27, OAS1, IFIT1; or MX1, LLY6E, IFI27, OAS1, IFI6; or MX1, LLY6E, IFI27, OAS1, IFI44L; or MX1, LLY6E, IFI27, OAS1, ISG15; or MX1, LLY6E, IFI27, OAS1, LAMP3; or MX1, LLY6E, IFI27, OAS1, OASL; or MX1, LLY6E, IFI27, OAS1, RSAD2; or MX1, LLY6E, IFI27, OAS1, IFI44; or MX1, LLY6E, IFI27, OAS1, IFIT2; or MX1, LLY6E, IFI27, OAS1, OAS3; or MX1, LLY6E, IFI27, OAS1, USP18; or MX1, LLY6E, IFI27, OAS1, SIGLEC1; or MX1, LLY6E, IFI27, OAS1, HERC5; or MX1, LLY6E, IFI27, OAS1, DNAPTP6; or MX1, LLY6E, IFI27, OAS1, LOC129607; or MX I , LLY6E, IFI27, OAS1, EPSTI1; or MX1, LLY6E, IFI27, OAS1, BIRC4BP; or MX1, LLY6E, IFI27, OAS1, SIGLEC1; or MX1, LLY6E, IFI27, OAS1, gene detected by probe 229450_at; or MX1, LLY6E, IFI27, OAS1, gene detected by probe 235276_at; or LLY6E, IFI27, OAS1, IFIT1, IFI6; or LLY6E, IFI27, OAS1, IFIT1, IFI44L; or LLY6E, IFI27, OAS1, IFIT1, ISG15; or LLY6E, IFI27, OAS1, IFIT1, LAMP3; or LLY6E, IFI27, OAS1, IFIT1, OASL; or LLY6E, IFI27, OAS1, IFIT1, RSAD2; or LLY6E, IFI27, OAS1, IFIT1, IFI44; or LLY6E, IFI27, OAS1, IFIT1, IFIT2; or LLY6E, IFI27, OAS1, IFIT1, OAS3; or LLY6E, IFI27, OAS1, IFIT1, USP18; or LLY6E, IFI27, OAS1, IFIT1, SIGLEC1; or LLY6E, IFI27, OAS1, IFIT1, HERC5; or LLY6E, IFI27, OAS1, IFIT1, DNAPTP6; or LLY6E, IFI27, OAS1, IFIT1, LOC129607; or LLY6E, IFI27, OAS1, IFIT1, EPSTI1; or LLY6E, IFI27, OASI, IFIT1, BIRC4BP; or LLY6E, IFI27, OAS1, IFIT1, SIGLEC1; or LLY6E, IFI27, OAS1, IFIT1, gene detected by probe 229450_at; or LLY6E, IFI27, OAS1, IFIT1, gene detected by probe 235276_at; or IFI27, OAS1, IFIT1, IFI6, IFI44L, ISG15; or IFI27, OAS1, IFIT1, IFI6, LAMP3; or IFI27, OAS1, IFIT1, IFI6, OASL; or IFI27, OAS1, IFIT1, IFI6, RSAD2; or IFI27, OAS1, IFIT1, IFI6, IFI44; or IFI27, OAS1, IFIT1, IFI6, IFIT2; or IFI27, OAS1, IFIT1, IFI6, OAS3; or IFI27, OAS1, IFIT1, IFI6, USP18; or IFI27, OAS1, IFIT1, IFI6, SIGLEC1; or IFI27, OAS1, IFIT1, IFI6, HERC5; or IFI27, OAS1, IFITI, IFI6, DNAPTP6; or IFI27, OAS I, IFIT1, IFI6, LOC129607; or IFI27, OAS1, IFIT1, IFI6, EPSTI1; or IFI27, OAS1, IFIT1, IFI6, BIRC4BP; or IFI27, OAS1, IFIT1, IFI6, SIGLEC1; or IFI27, OAS1, IFIT1, IFI6, gene detected by probe 229450_at; or IFI27, OAS1, IFIT1, IFI6, gene detected by probe 235276_at; or OAS1, IFIT1, IFI6, IFI44L, ISG15; or OAS1, IF1T1, IFI6, IFI44L, LAMP3; or OAS1, IFIT1, IFI6, IFI44L, OASL; or OAS1, IFIT1, IFI6, IFI44L, RSAD2; or OAS1, IFIT1, IFI6, IFI44L, IFI44; or OAS1, IFIT1, IFI6, IFI44L, IFIT2; or OAS1, IFIT1, IFI6, IFI44L, OAS3; or OAS1, IFIT1, IFI6, IFI44L, USP18; or OAS1, IFIT1, IFI6, IFI44L, SIGLEC1; or OAS1, IFIT1, IFI6, IFI44L, HERC5; or OAS1, IFIT1, IFI6, IFI44L, DNAPTP6; or OAS1, IFIT1, IFI6, IFI44L, LOC129607; or OAS1, IFIT1, IFI6, IFI44L, EPSTI1; or OAS1, IFIT1, IFI6, IFI44L, BIRC4BP; or OAS1, IFIT1, IFI6, IFI44L, SIGLEC1; or OAS1, IFIT1, IFI6, IFI44L, gene detected by probe 229450_at; or OAS1, IFIT1, IFI6, IFI44L, gene detected by probe 235276_at; or IFIT1, IFI6, IFI44L, ISG15, LAMP3; or IFIT1, IFI6, IFI44L, ISG15, OASL; or IFIT1, IFI6, IFI44L, ISG15, RSAD2; or IFIT1, IFI6, IFI44L, ISG15, IFI44; or IFIT1, IFI6, IFI44L, ISG15, IFIT2 or IFIT1, IFI6, IFI44L, ISG15, OAS3; or IFIT1, IFI6, IFI44L, ISG15, USP18; or IFIT1, IFI6, IFI44L, ISG15, SIGLEC1; or IFIT1, IFI6, IFI44L, ISG15, HERC5; or IFIT1, IFI6, IFI44L, ISG15, DNAPTP6; or IFIT1, IFI6, IFI44L, ISG15, LOC129607; or IFIT1, IFI6, IFI44L, ISG15, EPSTI1; or IFIT1, IFI6, IFI44L, ISG15, BIRC4BP; or IFIT1, IFI6, IFI44L, ISG15, gene detected by probe 229450_at; or IFIT1, IFI6, IFI44L, ISG15, gene detected by probe 235276_at; or IFI6, IFI44L, ISG15, LAMP3, HERC5; or IFI6, IFI44L, ISG15, LAMP3, DNAPTP6; or IFI6, IFI44L, ISG15, LAMP3, LOC129607; or IFI6, IFI44L, ISG15, LAMP3, EPSTI1; or IFI6, IFI44L, ISG15, LAMP3, BIRC4BP; or IFI6, IFI44L, ISG15, LAMP3, gene detected by probe 229450_at; or IFI6, IFI44L, ISG15, LAMP3, gene detected by probe 235276_at; or IFI6, IFI44L, ISG15, LAMP3, SIGLEC1; or IFI6, IFI44L, ISG15, LAMP3, USP18; or IFI6, IFI44L, ISG15, LAMP3, OAS3; or IFI6, IFI44L, ISG15, LAMP3, IFIT2; or IFI6, IFI44L, ISG15, LAMP3, IFI44; or IFI6, IFI44L, ISG15, LAMP3, RSAD2; or IFI6, IFI44L, ISG15, LAMP3, OASL; or IFI44L, ISG15, LAMP3, OASL, RSAD2; or IFI44L, ISG15, LAMP3, OASL, IFI44; or IFI44L, ISG15, LAMP3, OASL, IFIT2; or IFI44L, ISG15, LAMP3, OASL, OAS3; or IFI44L, ISG15, LAMP3, OASL, USP18; or IFI44L, ISG15, LAMP3, OASL, SIGLEC1; or IFI44L, ISG15, LAMP3, OASL, HERC5; or IFI44L, ISG15, LAMP3, OASL, DNAPTP6; or IFI44L, ISG15, LAMP3, OASL, LOC129607; or IFI44L, ISG15, LAMP3, OASL, EPSTI1;or IFI44L, ISG15, LAMP3, OASL, BIRC4BP; or IFI44L, ISG15, LAMP3, OASL, gene detected by probe 229450_at; or IFI44L, ISG15, LAMP3, OASL, gene detected by probe 235276_at; or ISG15, LAMP3, OASL, RSAD2, IFI44; or ISG15, LAMP3, OASL, RSAD2, IFIT2; or ISG15, LAMP3, OASL, RSAD2, OAS3; or ISG15, LAMP3, OASL, RSAD2, USP18; or ISG15, LAMP3, OASL, RSAD2, SIGLEC1; or ISG15, LAMP3, OASL, RSAD2, HERC5; or ISG15, LAMP3, OASL, RSAD2, DNAPTP6; or ISG15, LAMP3, OASL, RSAD2, LOC129607; or ISG15, LAMP3, OASL, RSAD2, EPSTI1; or ISG 15, LAMP3, OASL, RSAD2, BIRC4BP; or ISG15, LAMP3, OASL, RSAD2, gene detected by probe 229450_at; or ISG15, LAMP3, OASL, RSAD2, gene detected by probe 235276_at; or LAMP3, OASL, RSAD2, IFI44, IFIT2; or LAMP3, OASL, RSAD2, IFI44, OAS3; or LAMP3, OASL, RSAD2, IFI44, USP18; or LAMP3, OASL, RSAD2, IFI44, SIGLEC1; or LAMP3, OASL, RSAD2, IFI44, HERC5; or LAMP3, OASL, RSAD2, IFI44, DNAPTP6; or LAMP3, OASL, RSAD2, IFI44, LOC129607; or LAMP3, OASL, RSAD2, IFI44, EPSTI1; or LAMP3, OASL, RSAD2, IFI44, BIRC4BP; or LAMP3, OASL, RSAD2, IFI44, gene detected by probe 229450_at; or LAMP3, OASL, RSAD2, IFI44, gene detected by probe 235276_at; or OASL, RSAD2, IFI44, IFIT2, OAS3; or OASL, RSAD2, IFI44, IFIT2, USP18; or OASL, RSAD2, IFI44, IFIT2, SIGLEC1; or OASL, RSAD2, IFI44, IFIT2, HERC5; or OASL, RSAD2, IFI44, IFIT2, DNAPTP6; or OASL, RSAD2, IFI44, IFIT2, LOC129607; or OASL, RSAD2, IFI44, IFIT2, EPSTI1; or OASL, RSAD2, IFI44, IFIT2, BIRC4BP; or OASL, RSAD2, IFI44, IFIT2, gene detected by probe 229450_at; or OASL, RSAD2, IFI44, IFIT2, gene detected by probe 235276_at; or RSAD2, IFI44, IFIT2, OAS3, USP18; or RSAD2, IFI44, IFIT2, OAS3, SIGLECI; or RSAD2, IFI44, IFIT2, OAS3, HERC5; or RSAD2, IFI44, IFIT2, OAS3, DNAPTP6; or RSAD2, IFI44, IFIT2, OAS3, LOC129607; or RSAD2, IFI44, IFIT2, OAS3, EPSTI1; or RSAD2, IFI44, IFIT2, OAS3, BIRC4BP; or RSAD2, IFI44, IFIT2, OAS3, gene detected by probe 229450_at; or RSAD2, IFI44, IFIT2, OAS3, gene detected by probe 235276_at; or IFI44, IFIT2, OAS3, USP18, SIGLEC1; or IFI44, IFIT2, OAS3, USP18, HERC5; or IFI44, IFIT2, OAS3, USP18, DNAPTP6; or IFI44, IFIT2, OAS3, USP18, LOC129607; or IFI44, IFIT2, OAS3, USP18, EPSTI1; or IFI44, IFIT2, OAS3, USP18, BIRC4BP; or IFI44, IFIT2, OAS3, USP18, gene detected by probe 229450_at; or IFI44, IFIT2, OAS3, USP18, gene detected by probe 235276_at; or IFIT2, OAS3, USP18, SIGLEC1, HERC5; or IFIT2, OAS3, USP18, SIGLEC1, DNAPTP6; or IFIT2, OAS3, USP18, SIGLEC1, LOC129607; or IFIT2, OAS3, USP18, SIGLEC1, EPSTI1; or IFIT2, OAS3, USP18, SIGLEC1, BIRC4BP; or IFIT2, OAS3, USP18, SIGLEC1, gene detected by probe 229450_at; or IFIT2, OAS3, USP18, SIGLEC1, gene detected by probe 235276_at; or OAS3, USP18, SIGLEC1, HERC5, DNAPTP6; or OAS3, USP18, SIGLEC1, HERC5, LOC129607; or OAS3, USP18, SIGLEC1, HERC5, EPSTI1; or OAS3, USP18, SIGLEC1, HERC5, BIRC4BP; or OAS3, USP18, SIGLEC1, HERC5, gene detected by probe 229450_at; or OAS3, USP18, SIGLEC1, HERC5, gene detected by probe 235276_at; or USP18, SIGLEC1, HERC5, DNAPTP6, LOC129607; or USP18, SIGLEC1, HERC5, DNAPTP6, EPSTI1; or USP18, SIGLEC1, HERC5, DNAPTP6, BIRC4BP; or USP18, SIGLEC1, HERC5, DNAPTP6, gene detected by probe 229450_at; or USP18, SIGLEC1, HERC5, DNAPTP6, gene detected by probe 235276_at; or SIGLEC1, HERC5, DNAPTP6, LOC129607, EPSTI1; or SIGLEC1, HERC5, DNAPTP6, LOC129607, BIRC4BP; or SIGLEC1, HERC5, DNAPTP6, LOC129607, gene detected by probe 229450_at; or SIGLEC1, HERC5, DNAPTP6, LOC129607, gene detected by probe 235276_at; or HERC5, DNAPTP6, LOC129607, EPSTI1, BIRC4BP; or HERC5, DNAPTP6, LOC129607, EPSTI1, gene detected by probe 229450_at; or HERC5, DNAPTP6, LOC129607, EPSTI1, gene detected by probe 235276_at; or DNAPTP6, LOC129607, EPSTI1, BIRC4BP, gene detected by probe 229450_at; or DNAPTP6, LOC129607, EPSTI1, BIRC4BP, gene detected by probe 235276_at; or LOC129607, EPSTI1, BIRC4BP, gene detected by probe 229450_at, gene detected by probe 235276_at. The IFNα-inducible PD markers in such an expression profile may further include at least one or more gene listed in Tables 19 and/or 20 and/or 21 and/or 22 and/or 23 and/or 24 and/or 26 and/or 28 and/or 30.

The IFNα-inducible PD markers in an expression profile may include any at least 6 genes such as, for example: MX1, LLY6E, IFI27, OAS1, IFIT1, IFI6; or MX1, LLY6E, IFI27, OAS1, IFIT1, IFI44L; or MX1, LLY6E, IFI27, OAS1, IFIT1, ISG15; or MX1, LLY6E, IFI27, OAS1, IFIT1, LAMP3; or MX1, LLY6E, IFI27, OAS1, IFIT1, OASL; or MX1, LLY6E, IFI27, OAS1, IFIT1, RSAD2; or MX1, LLY6E, IFI27, OAS1, IFIT1, IFI44; or MX1, LLY6E, IF127, OAS1, IFIT1, IFIT2; or MX1, LLY6E, IFI27, OAS1, IFIT1, OAS3; or MX1, LLY6E, IFI27, OAS1, IFIT1, USP18; or MX1, LLY6E, IFI27, OAS1, IFIT1, SIGLEC1; or MX1, LLY6E, IFI27, OAS1, IFIT1, HERC5; or MX1, LLY6E, IFI27, OAS1, IFIT1, DNAPTP6; or MX1 LLY6E, IFI27, OAS1, IFIT1, LOC129607; or MX1, LLY6E, IFI27, OAS1, IFIT1, EPSTI1; or MX1, LLY6E, IFI27, OAS1, IFITI, BIRC4BP;or MX1, LLY6E, IFI27, OAS1, IFIT1, gene detected by probe 229450_at; or MX1, LLY6E, IFI27, OAS1, IFIT1, gene detected by probe 235276_at; or LLY6E, IFI27, OAS1, IFIT1, IFI6, IFI44L; or LLY6E, IFI27, OAS1, IFIT1, IFI6, ISG15; or LLY6E, IFI27, OAS1, IFITI, IPI6, LAMP3; or LLY6E, IFI27, OAS1, IFIT1, IFI6, OASL ; or LLY6E, IFI27, OAS1, IFIT1, IFI6, RSAD2; or LLY6E, IFI27, OAS1, IFIT1, IFI6, IFI44; or LLY6E, IFI27, OAS1, IFIT1, IFI6, IFIT2; or LLY6E, IFI27, OAS1, IFIT1, IFI6, OAS3; or LLY6E, IFI27, OAS1, IFIT1, IFI6, USP18; or LLY6E, IFI27, OAS1, IFIT1, IFI6, SIGLEC1; or LLY6E, IFI27, OAS1, IFIT1, IFI6, HERC5; or LLY6E, IFI27, OAS1, IFIT1, IFI6, DNAPTP6; or LLY6E, IFI27, OAS1, IFIT1, IFI6, LOC129607; or LLY6E, IFI27, OAS1, IFIT1, IFI6, EPSTI1; or LLY6E, IFI27, OAS1, IFIT1, IFI6, BIRC4BP; or LLY6E, IFI27, OAS1, IFIT1, IFI6, gene detected by probe 229450_at; or LLY6E, IFI27, OAS1, IFIT1, IFI6, gene detected by probe 235276_at; or IFI27, OAS1, IFIT1, IFI6, IFI44L, ISG15; or IFI27, OAS1, IFIT1, IFI6, IFI44L, LAMP3; or IFI27, OAS1, IFIT1, IFI6, IFI44L, OASL; or IFI27, OAS1, IFIT1, IFI6, IFI44L, RSAD2; or IFI27, OAS1, IFIT1, IFI6, IFI44L, IF144; or IFI27, OAS1, IFIT1, IFI6, IFI44L, IFIT2; or IFI27, OAS1, IFIT1, IFI6, IFI44L, OAS3; or IFI27, OAS1, IFIT1, IFI6, IFI44L, USP18; or IFI27, OAS1, IFIT I , IFI6, IFI44L, SIGLEC1; or IFI27, OAS1, IFIT1, IFI6, IFI44L, HERC5; or IFI27, OAS1, IFIT1, IFI6, IFI44L, DNAPTP6; or IFI27, OAS1, IFIT1, IFI6, IFI44L, LOC129607; or IFI27, OAS1, IFIT1, IFI6, IFI44L, EPSTI1; or IFI27, OAS1, IFIT1, IFI6, IFI44L, BIRC4BP; or IFI27, OAS1, IFIT1, IFI6, IFI44L, gene detected by probe 229450_at; or IFI27, OAS1, IFIT1, IFI6, IFI44L, gene detected by probe 235276_at; or OAS1, IFIT1, IFI6, IFI44L, ISG15, LAMP3; or OAS1, IFIT1, IFI6, IFI44L, ISG15, OASL; or OAS1, IFIT1, IFI6, IFI44L, ISG15, RSAD2; or OAS1, IFIT1, IFI6, IFI44L, ISG15, IFI44; or OAS1, IFIT1, IFI6, IFI44L, ISG15, IFIT2; or OAS1, IFIT1, IFI6, IFI44L, ISG15, OAS3; or OAS1, IFIT1, IFI6, IFI44L, ISG15, USP18; or OAS1, IFIT1, IFI6, IFI44L, ISG15, SIGLEC1; or OAS1, IFIT1, IFI6, IFI44L, ISG15, HERC5; or OAS1, IFIT1, IFI6, IFI44L, ISG15, DNAPTP6; or OAS1, IFIT1, IFI6, IFI44L, ISG15, LOC129607; or OAS1, IFIT1, IFI6, IFI44L, ISG15, EPSTI1; or OAS1, IFIT1, IFI6, IFI44L, ISG15, BIRC4BP; or OAS1, IFIT1, IFI6, IFI44L, ISG15, gene detected by probe 229450_at; or OAS1, IFIT1, IFI6, IFI44L, ISG15, gene detected by probe 235276_at; or IFIT1, IFI6, IFI44L, ISG15, LAMP3, OASL; or IFIT1, IFI6, IFI44L, ISG15, LAMP3, RSAD2; or IFIT1, IFI6, IFI44L, ISG15, LAMP3, IFI44; or IFIT1, IFI6, IFI44L, ISG15, LAMP3, IFIT2; or IFIT1, IFI6, IFI44L, ISG15, LAMP3, OAS3; or IFIT1, IFI6, IFI44L, ISG15, LAMP3, USP18; or IFIT1, IFI6, IFI44L, ISG15, LAMP3, SIGLEC1; or IFIT1, IFI6, IFI44L, ISG15, LAMP3, HERC5; or IFIT1, IFI6, IFI44L, ISG15, LAMP3, DNAPTP6; or IFIT1, IFI6, IFI44L, ISG15, LAMP3, LOC129607; or IFIT1, IFI6, IFI44L, ISG15, LAMP3, EPSTI1; or IFIT1, IFI6, IFI44L, ISG15, LAMP3, BIRC4BP; or IFIT1, IFI6, IFI44L, ISG15, LAMP3, gene detected by probe 229450_at; or IFIT1, IFI6, IFI44L, ISG15, LAMP3, gene detected by probe 235276_at; or IFI6, IFI44L, ISG15, LAMP3, OASL, RSAD2; or IFI6, IFI44L, ISG15, LAMP3, OASL, IFI44; or IFI6, IFI44L, ISG15, LAMP3, OASL, IFIT2; or IFI6, IFI44L, ISG15, LAMP3, OASL, OAS3; or IF16, IFI44L, ISG15, LAMP3, OASL, USP18; or IFI6, IFI44L, ISG15, LAMP3, OASL, SIGLEC1; or IFI6, IFI44L, ISG15, LAMP3, OASL, HERC5; or IFI6, IFI44L, ISG15, LAMP3, OASL, DNAPTP6; or IFI6, IFI44L, ISG15, LAMP3, OASL, LOC129607; or IFI6, IFI44L, ISG15, LAMP3, OASL, EPSTI1; or IFI6, IFI44L, ISG15, LAMP3, OASL, BIRC4BP; or IFI6, IFI44L, ISG15, LAMP3, OASL, gene detected by probe 229450_at; or IFI6, IFI44L, ISG15, LAMP3, OASL, gene detected by probe 235276_at; or IFI44L, ISG15, LAMP3, OASL, RSAD2, IFI44; or IFI44L, ISG15, LAMP3, OASL, RSAD2, IFIT2; or IFI44L, ISG15, LAMP3, OASL, RSAD2, OAS3; or IFI44L, ISG15, LAMP3, OASL, RSAD2, USP18; or IFI44L, ISG15, LAMP3, OASL, RSAD2, SIGLEC1; or IFI44L, ISG15, LAMP3, OASL, RSAD2, HERC5; or IFI44L, ISG15, LAMP3, OASL, RSAD2, DNAPTP6; or IFI44L, ISG15, LAMP3, OASL, RSAD2, LOC129607; or IFI44L, ISG15, LAMP3, OASL, RSAD2, EPSTI1; or IFI44L, ISG15, LAMP3, OASL, RSAD2, BIRC4BP; or IFI44L, ISG15, LAMP3, OASL, RSAD2, gene detected by probe 229450_at; or IFI44L, ISG15, LAMP3, OASL, RSAD2, gene detected by probe 235276_at; or ISG15, LAMP3, OASL, RSAD2, IFI44, IFIT2; or ISG15, LAMP3, OASL, RSAD2, IFI44, OAS3; or ISG15, LAMP3, OASL, RSAD2, IFI44, USP18; or ISG15, LAMP3, OASL, RSAD2, IFI44, SIGLEC1; or ISG15, LAMP3, OASL, RSAD2, IFI44, HERC5; or ISG15, LAMP3, OASL, RSAD2, IFI44, DNAPTP6; or ISG15, LAMP3, OASL, RSAD2, IFI44, LOC129607; or ISG15, LAMP3, OASL, RSAD2, IFI44, EPSTI1; or ISG15, LAMP3, OASL, RSAD2, IFI44, BIRC4BP; or ISG15, LAMP3, OASL, RSAD2, IFI44, gene detected by probe 229450_at; or ISG15, LAMP3, OASL, RSAD2, IFI44, gene detected by probe 235276_at; or LAMP3, OASL, RSAD2, IFI44, IFIT2, OAS3; or LAMP3, OASL, RSAD2, IFI44, IFIT2, USP18; or LAMP3, OASL, RSAD2, IFI44, IFIT2, SIGLEC1; or LAMP3, OASL, RSAD2, IFI44, IFIT2, HERC5; or LAMP3, OASL, RSAD2, IFI44, IFIT2, DNAPTP6; or LAMP3, OASL, RSAD2, IFI44, IFIT2, LOC129607; or LAMP3, OASL, RSAD2, IFI44, IFIT2, EPST11; or LAMP3, OASL, RSAD2, IFI44, IFIT2, BIRC4BP; or LAMP3, OASL, RSAD2, IFI44, IFIT2, gene detected by probe 229450_at; or LAMP3, OASL, RSAD2, IFI44, IFIT2, gene detected by probe 235276_at; or OASL, RSAD2, IFI44, IFIT2, OAS3, USP18; or OASL, RSAD2, IFI44, IFIT2, OAS3, SIGLEC1; or OASL, RSAD2, IFI44, IFIT2, OAS3, HERC5; or OASL, RSAD2, IFI44, IFIT2, OAS3, DNAPTP6; or OASL, RSAD2, IFI44, IFIT2, OAS3, LOC129607; or OASL, RSAD2, IFI44, IFIT2, OAS3, EPSTI1; or OASL, RSAD2, IFI44, IFIT2, OAS3, BIRC4BP; or OASL, RSAD2, IFI44, IFIT2, OAS3, gene detected by probe 229450_at; or OASL, RSAD2, IFI44, IFIT2, OAS3, gene detected by probe 235276_at; or RSAD2, IFI44, IFIT2, OAS3, USP 18, SIGLEC1; or RSAD2, IFI44, IFIT2, OAS3, USP 18, HERC5; or RSAD2, IFI44, IFIT2, OAS3, USP18, DNAPTP6; or RSAD2, IFI44, IFIT2, OAS3, USP18, LOC 129607; or RSAD2, IFI44, IFIT2, OAS3, USP 18, EPSTI1; or RSAD2, IFI44, IFIT2, OAS3, USP18, BIRC4BP; or RSAD2, IFI44, IFIT2, OAS3, USP18, gene detected by probe 229450_at; or RSAD2, IFI44, IFIT2, OAS3, USP18, gene detected by probe 235276_at; or IFI44, IFIT2, OAS3, USP18, SIGLEC1, HERC5; or IFI44, IFIT2, OAS3, USP18, SIGLEC1, DNAPTP6; or IFI44, IFIT2, OAS3, USP18, SIGLEC1, LOC129607; or IFI44, IFIT2, OAS3, USP18, SIGLEC1, EPSTI1; or IFI44, IFIT2, OAS3, USP18, SIGLEC1, BIRC4BP; or IFI44, IFIT2, OAS3, USP18, SIGLEC1, gene detected by probe 229450_at; or IFI44, IFIT2, OAS3, USP18, SIGLEC1, gene detected by probe 235276_at; or IFIT2, OAS3, USP18, SIGLEC1, HERC5, DNAPTP6; or IFIT2, OAS3, USP18, SIGLEC1, HERC5, LOC129607; or IFIT2, OAS3, USP18, SIGLEC1, HERC5, EPSTI1; or IFIT2, OAS3, USP18, SIGLEC1, HERC5, BIRC4BP; or IFIT2, OAS3, USP18, SIGLEC1, HERC5, gene detected by probe 229450_at; or IFIT2, OAS3, USP18, SIGLEC1, HERC5, gene detected by probe 235276_at; or OAS3, USP18, SIGLEC1, HERC5, DNAPTP6, LOC129607; or OAS3, USP18, SIGLEC1, HERC5, DNAPTP6, EPSTI1; or OAS3, USP18, SIGLEC1, HERC5, DNAPTP6, BIRC4BP; or OAS3, USP18, SIGLEC1, HERC5, DNAPTP6, gene detected by probe 229450_at; or OAS3, USP18, SIGLEC1, HERC5, DNAPTP6, gene detected by probe 235276_at; or USP18, SIGLEC1, HERC5, DNAPTP6, LOC129607, EPSTI1; or USP18, SIGLEC1, HERC5, DNAPTP6, LOC129607, BIRC4BP; or USP18, SIGLEC1, HERC5, DNAPTP6, LOC129607, gene detected by probe 229450_at; or USP18, SIGLEC1, HERC5, DNAPTP6, LOC129607, gene detected by probe 235276_at; or SIGLEC1, HERC5, DNAPTP6, LOC129607, EPSTI1, BIRC4BP; or SIGLEC1, HERC5, DNAPTP6, LOC129607, EPSTI 1, gene detected by probe 229450_at; or SIGLEC1, HERC5, DNAPTP6, LOC 129607, EPSTI1, gene detected by probe 235276_at; or HERC5, DNAPTP6, LOC129607, EPSTI1, BIRC4BP, gene detected by probe 229450_at; or HERC5, DNAPTP6, LOC129607, EPSTI1, BIRC4BP, gene detected by probe 235276_at; or DNAPTP6, LOC129607, EPSTI1, BIRC4BP, gene detected by probe 229450_at, gene detected by probe 235276_at. The IFNα-inducible PD markers in such an expression profile may further include at least one or more gene listed in Tables 19 and/or 20 and/or 21 and/or 22 and/or 23 and/or 24 and/or 26 and/or 28 and/or 30.

The IFNα-inducible PD markers in an expression profile may include any at least 7 genes such as, for example: MX1, LLY6E, IFI27, OAS1, IFIT1, IFI6, IFI44L; or MX1, LLY6E, IFI27, OAS1, IFIT1, IFI6, ISG15; or MX1, LLY6E, IFI27, OAS1, IFIT1, IFI6, LAMP3; or MX1, LLY6E, IFI27, OAS1, IFIT1, IFI6, OASL; or MX1, LLY6E, IFI27, OAS1, IFIT1, IFI6, RSAD2; or MX1, LLY6E, IFI27, OAS1, IFIT1, IFI6, IFI44; or MX1, LLY6E, IFI27, OAS1, IFIT1, IFI6, IFIT2; or MX1, LLY6E, IFI27, OAS1, IFIT1, IFI6, OAS3; or MX1, LLY6E, IFI27, OAS1, IFIT1, IFI6, USP18; or MX1, LLY6E, IFI27, OAS1, IFIT1, IFI6, SIGLEC1; or MX1, LLY6E, IFI27, OAS1, IFIT1, IFI6, HERC5; or MX1, LLY6E, IFI27, OAS1, IFIT1, IFI6, DNAPTP6; or MX1, LLY6E, IFI27, OAS1, IFIT1, IFI6, LOC129607; or MX1, LLY6E, IFI27, OAS1, IFIT1, IFI6, EPSTI1; or MX1, LLY6E, IFI27, OAS1, IFIT1, IFI6, BIRC4BP; or MX1, LLY6E, IFI27, OAS1, IFIT1, IFI6, gene detected by probe 229450_at; or MX1, LLY6E, IFI27, OAS1, IFIT1, IFI6, gene detected by probe 235276_at; or LLY6E, IFI27, OAS1, IFIT1, IFI6, IFI44L, ISG15; or LLY6E, IFI27, OAS1, IFIT1, IFI6, IFI44L, LAMP3; or LLY6E, IFI27, OAS1, IFIT1, IFI6, IFI44L, OASL; or LLY6E, IFI27, OAS1, IFIT1, IFI6, IFI44L, RSAD2; or LLY6E, IFI27, OAS1, IFIT1, IFI6, IFI44L, IFI44; or LLY6E, IFI27, OAS1, IFIT1, IFI6, IFI44L, IFIT2; or LLY6E, IFI27, OAS1, IFIT1, IFI6, IFI44L, OAS3; or LLY6E, IFI27, OAS1, IFIT1, IFI6, IFI44L, USP18; or LLY6E, IFI27, OAS1, IFIT1, IFI6, IFI44L, SIGLEC1; or LLY6E, IFI27, OAS1, IFIT1, IFI6, IFI44L, HERC5; or LLY6E, IFI27, OAS1, IFIT1, IFI6, IFI44L, DNAPTP6; or LLY6E, IFI27, OAS1, IFIT1, IFI6, IFI44L, LOC129607; or LLY6E, IFI27, OAS1, IFIT1, IFI6, IFI44L, EPSTI1; or LLY6E, IFI27, OAS1, IFIT1, IFI6, IFI44L, BIRC4BP; or LLY6E, IFI27, OAS1, IFIT1, IFI6, IFI44L, gene detected by probe 229450_at; or LLY6E, IFI27, OAS1, IFIT1, IFI6, IFI44L, gene detected by probe 235276_at; or IFI27, OAS1, IFIT1, IFI6, IFI44L, ISG15, LAMP3; or IFI27, OAS1, IFIT1, IFI6, IFI44L, ISG15, OASL; or IF127, OAS1, IFIT1, IFI6, IFI44L, ISG15, RSAD2; or IFI27, OAS1, IFIT1, IFI6, IFI44L, ISG15, IFI44; or IFI27, OAS1, IFITI, IFI6, IFI44L, ISG15, IFIT2; or IFI27, OASI, IFIT1, IFI6, IFI44L, ISG15, OAS3; or IFI27, OAS1, IFIT1, IFI6, IFI44L, ISG15, USP18; or IFI27, OAS1, IFITI, IFI6, IFI44L, ISG15, SIGLEC1; or IFI27, OAS1, IFITI, IFI6, IFI44L, ISG15, HERC5; or IFI27, OAS1, IFITI, IFI6, IFI44L, ISG15, DNAPTP6; or IFI27, OASI, IFIT1, IF16, IFI44L, ISG15, LOC 129607; or IFI27, OAS1, IFIT1, IFI6, IFI44L, ISG15, EPSTI1; or IFI27, OAS1, IFIT1, IFI6, IFI44L, ISG15, BIRC4BP; or IFI27, OAS1, IFIT1, IFI6, IFI44L, ISG15, gene detected by probe 229450_at; or IFI27, OAS1, IFIT1, IFI6, IFI44L, ISG15, gene detected by probe 235276_at; or OAS1, IFIT1, IFI6, IFI44L, ISG15, LAMP3, OASL; or OAS1, IFIT1, IFI6, IFI44L, ISG15, LAMP3, RSAD2; or OAS1, IFIT1, IFI6, IFI44L, ISG15, LAMP3, IFI44; or OAS1, IFIT1, IFI6, IFI44L, ISG15, LAMP3, IFIT2; or OAS1, IFIT1, IFI6, IFI44L, ISG15, LAMP3, OAS3; or OAS1, IFIT1, IFI6, IFI44L, ISG15, LAMP3, USP18; or OAS1, IFIT1, IFI6, IFI44L, ISG15, LAMP3, SIGLEC1; or OAS1, IFITI, IFI6, IFI44L, ISGI5, LAMP3, HERC5; or OAS1, IFIT1, IFI6, IFI44L, ISG15, LAMP3, DNAPTP6; or OAS1, IFIT1, IFI6, IFI44L, ISG15, LAMP3, LOC129607; or OAS1, IFIT1, IFI6, IFI44L, ISG15, LAMP3, EPSTI1; or OAS1, IFIT1, IFI6, IFI44L, ISG15, LAMP3, BIRC4BP; or OAS1, IFIT1, IFI6, IFI44L, ISG15, LAMP3, gene detected by probe 229450_at; or OAS1, IFIT1, IFI6, IFI44L, ISG15, LAMP3, gene detected by probe 235276_at; or IFIT1, IFI6, IFI44L, ISG15, LAMP3, OASL, RSAD2; or IFIT1, IFI6, IFI44L, ISG15, LAMP3, OASL, IFI44; or IFIT1, IFI6, IFI44L, ISG15, LAMP3, OASL, IFIT2; or IFIT1, IFI6, IFI44L, ISG15, LAMP3, OASL, OAS3; or IFIT1, IFI6, IFI44L, ISG15, LAMP3, OASL, USP18; or IFIT1, IFI6, IFI44L, ISG15, LAMP3, OASL, SIGLEC1; or IFIT1, IFI6, IFI44L, ISG15, LAMP3, OASL, HERC5; or IFIT1, IFI6, IFI44L, ISG15, LAMP3, OASL, DNAPTP6; or IFIT1, IFI6, IFI44L, ISG15, LAMP3, OASL, LOC129607; or IFIT1, IFI6, IFI44L, ISG15, LAMP3, OASL, EPSTI1; or IFIT1, IFI6, IFI44L, ISG15, LAMP3, OASL, BIRC4BP; or IFIT1, IFI6, IFI44L, ISG15, LAMP3, OASL, gene detected by probe 229450_at; or IFIT1, IFI6, IFI44L, ISG15, LAMP3, OASL, gene detected by probe 235276_at; or IFI6, IFI44L, ISG15, LAMP3, OASL, RSAD2, IFI44; or IFI6, IFI44L, ISG15, LAMP3, OASL, RSAD2, IFIT2; or IFI6, IFI44L, ISG15, LAMP3, OASL, RSAD2, OAS3; or IFI6, IFI44L, ISG15, LAMP3, OASL, RSAD2, USP18; or IFI6, IFI44L, ISG15, LAMP3, OASL, RSAD2, SIGLEC1; or IFI6, IFI44L, ISG15, LAMP3, OASL, RSAD2, HERC5; or IFI6, IFI44L, ISG15, LAMP3, OASL, RSAD2, DNAPTP6; or IFI6, IFI44L, ISG15, LAMP3, OASL, RSAD2, LOC129607; or IFI6, IFI44L, ISG15, LAMP3, OASL, RSAD2, EPSTI1; or IFI6, IFI44L, ISG15, LAMP3, OASL, RSAD2, BIRC4BP; or IFI6, IFI44L, ISG15, LAMP3, OASL, RSAD2, gene detected by probe 229450_at; or IFI6, IFI44L, ISG15, LAMP3, OASL, RSAD2, gene detected by probe 235276_at; or IFI44L, ISG15, LAMP3, OASL, RSAD2, IFI44, IFIT2; or IFI44L, ISG15, LAMP3, OASL, RSAD2, IFI44, OAS3; or IFI44L, ISG15, LAMP3, OASL, RSAD2, IFI44, USP18; or IFI44L, ISG15, LAMP3, OASL, RSAD2, IFI44, SIGLEC1; or IFI44L, ISG15, LAMP3, OASL, RSAD2, IFI44, HERC5; or IFI44L, ISG15, LAMP3, OASL, RSAD2, IFI44, DNAPTP6; or IFI44L, ISG15, LAMP3, OASL, RSAD2, IFI44, LOC129607; or IFI44L, ISG15, LAMP3, OASL, RSAD2, IFI44, EPSTI1; or IFI44L, ISG15, LAMP3, OASL, RSAD2, IFI44, BIRC4BP; or IFI44L, ISG15, LAMP3, OASL, RSAD2, IFI44, gene detected by probe 229450_at; or IFI44L, ISG15, LAMP3, OASL, RSAD2, IFI44, gene detected by probe 235276_at; or ISG15, LAMP3, OASL, RSAD2, IFI44, IFIT2, OAS3; or ISG15, LAMP3, OASL, RSAD2, IFI44, IFIT2, USP18; or ISG15, LAMP3, OASL, RSAD2, IFI44, IFIT2, SIGLEC1; or ISG15, LAMP3, OASL, RSAD2, IFI44, IFIT2, HERC5; or ISG15, LAMP3, OASL, RSAD2, IFI44, IFIT2, DNAPTP6; or ISG15, LAMP3, OASL, RSAD2, IFI44, IFIT2, LOC129607; or ISG15, LAMP3, OASL, RSAD2, IFI44, IFIT2, EPST11; or ISG15, LAMP3, OASL, RSAD2, IFI44, IFIT2, BIRC4BP; or ISG15, LAMP3, OASL, RSAD2, IFI44, IFIT2, gene detected by probe 229450_at; or ISG15, LAMP3, OASL, RSAD2, IFI44, IFIT2, gene detected by probe 235276_at; or LAMP3, OASL, RSAD2, IFI44, IFIT2, OAS3, USP18; or LAMP3, OASL, RSAD2, IFI44, IFIT2, OAS3, SIGLEC1; or LAMP3, OASL, RSAD2, IFI44, IFIT2, OAS3, HERC5; or LAMP3, OASL, RSAD2, IFI44, IFIT2, OAS3, DNAPTP6; or LAMP3, OASL, RSAD2, IFI44, IFIT2, OAS3, LOC129607; or LAMP3, OASL, RSAD2, IFI44, IFIT2, OAS3, EPSTI1; or LAMP3, OASL, RSAD2, IFI44, IFIT2, OAS3, BIRC4BP; or LAMP3, OASL, RSAD2, IFI44, IFIT2, OAS3, gene detected by probe 229450_at; or LAMP3, OASL, RSAD2, IFI44, IFIT2, OAS3, gene detected by probe 235276_at; or OASL, RSAD2, IFI44, IFIT2, OAS3, USP18, SIGLECI ; or OASL, RSAD2, IFI44, IFIT2, OAS3, USP18, HERC5; or OASL, RSAD2, IFI44, IFIT2, OAS3, USP18, DNAPTP6; or OASL, RSAD2, IFI44, IFIT2, OAS3, USP18, LOC129607; or OASL, RSAD2, IFI44, IFIT2, OAS3, USP18, EPST11; or OASL, RSAD2, IFI44, IFIT2, OAS3, USP18, BIRC4BP; or OASL, RSAD2, IFI44, IFIT2, OAS3, USP18, gene detected by probe 229450_at; or OASL, RSAD2, IFI44, IFIT2, OAS3, USP18, gene detected by probe 235276_at; or RSAD2, IFI44, IFIT2, OAS3, USP18, SIGLEC1, HERC5; or RSAD2, IFI44, IFIT2, OAS3, USP18, SIGLEC1, DNAPTP6; or RSAD2, IFI44, IFIT2, OAS3, USP18, SIGLEC1, LOC129607; or RSAD2, IFI44, IFIT2, OAS3, USP18, SIGLEC1, EPST11; or RSAD2, IFI44, IFIT2, OAS3, USP18, SIGLEC1, BIRC4BP; or RSAD2, IFI44, IFIT2, OAS3, USP18, SIGLEC1, gene detected by probe 229450_at; or RSAD2, IFI44, IFIT2, OAS3, USP18, SIGLECI, gene detected by probe 235276_at; or IFI44, IFIT2, OAS3, USP18, SIGLEC1, HERC5, DNAPTP6; or IFI44, IFIT2, OAS3, USP18, SIGLEC1, HERC5, LOC129607; or IFI44, IFIT2, OAS3, USP18, SIGLEC1, HERC5, EPSTI1; or IFI44, IFIT2, OAS3, USP18, SIGLEC1, HERC5, BIRC4BP; or IFI44, IFIT2, OAS3, USP18, SIGLEC1, HERC5, gene detected by probe 229450_at; or IFI44, IFIT2, OAS3, USP18, SIGLEC1, HERC5, gene detected by probe 235276_at; or IFIT2, OAS3, USP18, SIGLEC1, HERC5, DNAPTP6, LOCI29607; or IFIT2, OAS3, USP18, SIGLEC1, HERC5, DNAPTP6, EPSTI11; or IFIT2, OAS3, USP18, SIGLEC1, HERC5, DNAPTP6, BIRC4BP; or IFIT2, OAS3, USP18, SIGLEC1, HERC5, DNAPTP6, gene detected by probe 229450_at ; or IFIT2, OAS3, USP18, SIGLEC1, HERC5, DNAPTP6, gene detected by probe 235276_at; or OAS3, USP18, SIGLEC1, HERC5, DNAPTP6, LOCI29607, EPSTI1; or OAS3, USP18, SIGLEC1, HERC5, DNAPTP6, LOC129607, BIRC4BP; or OAS3, USP18, SIGLEC1, HERC5, DNAPTP6, LOC129607, gene detected by probe 229450_at; or OAS3, USP18, SIGLEC1, HERC5, DNAPTP6, LOC129607, gene detected by probe 235276_at; or USP18, SIGLEC1, HERC5, DNAPTP6, LOC129607, EPSTI1, BIRC4BP; or USPI8, SIGLEC1, HERC5, DNAPTP6, LOC129607, EPSTI1, gene detected by probe 229450_at; or USP18, SIGLEC1, HERC5, DNAPTP6, LOC129607, EPSTI1, gene detected by probe 235276_at; or SIGLEC1, HERC5, DNAPTP6, LOC129607, EPSTI1, BIRC4BP, gene detected by probe 229450_at; or SIGLEC1, HERC5, DNAPTP6, LOC129607, EPSTI1, BIRC4BP, gene detected by probe 235276_at; or HERC5, DNAPTP6, LOC129607, EPSTI1, BIRC4BP, gene detected by probe 229450_at, gene detected by probe 235276_at. The IFNα-inducible PD markers in such an expression profile may further include at least one or more gene listed in Tables 19 and/or 20 and/or 21 and/or 22 and/or 23 and/or 24 and/or 26 and/or 28 and/or 30.

The IFNα-inducible PD markers in an expression profile may include any at least 8 genes such as, for example: MX1, LLY6E, IFI27, OASI, IFIT1, IFI6, IFI44L, ISG15; or MX1, LLY6E, IFI27, OAS1, IFIT1, IFI6, IFI44L, LAMP3; or MX1, LLY6E, IFI27, OAS1, IFIT1, IFI6, IFI44L, OASL; or MX1, LLY6E, IFI27, OAS1, IFIT1, IFI6, IFI44L, RSAD2; or MX1, LLY6E, IFI27, OAS1, IFIT1, IFI6, IFI44L, IFI44; or MX1, LLY6E, IFI27, OAS1, IFIT1, IFI6, IFI44L, IFIT2; or MX1, LLY6E, IFI27, OAS1, IFIT1, IFI6, IFI44L, OAS3; or MX1, LLY6E, IFI27, OAS1, IFIT1, IFI6, IFI44L, USP18; or MX1, LLY6E, IFI27, OAS1, IFIT1, IFI6, IFI44L, SIGLEC1; or MX1, LLY6E, IFI27, OAS1, IFIT1, IFI6, IF144L, HERC5; or MX1, LLY6E, IFI27, OAS1, IFITI, IFI6, IFI44L, DNAPTP6; or MX1, LLY6E, IFI27, OAS1, IFIT1, IFI6, IFI44L, LOC129607; or MX1, LLY6E, IFI27, OAS1, IFIT1, IFI6, IFI44L, EPSTI1; or MX1, LLY6E, IFI27, OAS1, IFIT1, IFI6, IFI44L, BIRC4BP; or MX1, LLY6E, IFI27, OAS1, IFIT1, IFI6, IFI44L, gene detected by probe 229450_at; or MX1, LLY6E, IFI27, OAS1, IFIT1, IFI6, IFI44L, gene detected by probe 235276_at; or LLY6E, IFI27, OAS1, IFIT1, IFI6, IFI44L, ISG15, LAMP3; or LLY6E, IFI27, OAS1, IFIT1, IFI6, IFI44L, ISG15, OASL; or LLY6E, IFI27, OAS1, IFIT1, IFI6, IFI44L, ISG15, RSAD2; or LLY6E, IFI27, OAS1, IFIT1, IFI6, IFI44L, ISG15, IFI44; or LLY6E, IFI27, OAS1, IFIT1, IFI6, IFI44L, ISG15, IFIT2; or LLY6E, IF127, OAS1, IFIT1, IFI6, IFI44L, ISG15, OAS3; or LLY6E, IFI27, OAS1, IFIT1, IFI6, IFI44L, ISG15, USP18; or LLY6E, IFI27, OAS1, IFIT1, IFI6, IFI44L, ISG15, SIGLEC1; or LLY6E, IFI27, OAS1, IFIT1, IFI6, IFI44L, ISG15, HERC5; or LLY6E, IFI27, OAS1, IFIT1, IFI6, IFI44L, ISG15, DNAPTP6; or LLY6E, IFI27, OAS1, IFIT1, IFI6, IFI44L, ISG15, LOC129607; or LLY6E, IFI27, OAS1, IFIT1, IFI6, IFI44L, ISG 15, EPSTI 1; or LLY6E, IFI27, OAS 1, IFIT1, IFI6, IFI44L, ISG15, BIRC4BP; or LLY6E, IFI27, OAS1, IFITI, IFI6, IFI44L, ISG15, gene detected by probe 229450_at; or LLY6E, IFI27, OAS1, IFIT1, IFI6, IFI44L, ISG15, gene detected by probe 235276_at; orIF127, OAS1, IFIT1, IFI6, IFI44L, ISG15, LAMP3, OASL; or IFI27, OAS1, IFIT1, IFI6, IFI44L, ISG15, LAMP3, RSAD2; or IFI27, OAS1, IFIT1, IFI6, IFI44L, ISG15, LAMP3, IFI44; or IFI27, OAS1, IFIT1, IFI6, IFI44L, ISG15, LAMP3, IFIT2; or IFI27, OAS1, IFIT1, IFI6, IFI44L, ISG15, LAMP3, OAS3; or IFI27, OAS1, IFIT1, IFI6, IFI44L, ISG15, LAMP3, USP18; or IFI27, OAS1, IFIT1, IFI6, IFI44L, ISG15, LAMP3, SIGLEC1; or IFI27, OAS1, IFIT1, IFI6, IFI44L, ISG15, LAMP3, HERC5; or IFI27, OAS1, IFIT1, IFI6, IFI44L, ISG15, LAMP3, DNAPTP6; or IFI27, OAS1, IFIT1, IFI6, IFI44L, ISG15, LAMP3, LOC129607; or IFI27, OAS1, IFIT1, IFI6, IFI44L, ISG15, LAMP3, EPSTI1; or IFI27, OAS1, IFIT1, IFI6, IFI44L, ISG15, LAMP3, gene detected by probe 229450_at; or IFI27, OAS1, IFIT1, IFI6, IFI44L, ISG15, LAMP3, BIRC4BP; or IFI27, OAS1, IFIT1, IFI6, IFI44L, ISG15, LAMP3, gene detected by probe 235276_at; or OAS1, IFIT1, IFI6, IFI44L, ISG15, LAMP3, OASL, RSAD2; or OAS1, IFIT1, IFI6, IFI44L, ISG15, LAMP3, OASL, IFI44; or OAS1, IFIT1, IFI6, IFI44L, ISG15, LAMP3, OASL, IFIT2; or OAS I, IFIT1, IFI6, IFI44L, ISG15, LAMP3, OASL, OAS3; or OAS1, IFIT1, IFI6, IFI44L, ISG15, LAMP3, OASL, USP18; or OAS1, IFIT1, IFI6, IFI44L, ISG15, LAMP3, OASL, SIGLEC1; or OAS1, IFIT1, IFI6, IFI44L, ISG15, LAMP3, OASL, HERC5; or OAS1, IFIT1, IFI6, IFI44L, ISG15, LAMP3, OASL, DNAPTP6; or OAS1, IFIT1, IFI6, IFI44L, ISG15, LAMP3, OASL, LOC129607; or OAS1, IFIT1, IFI6, IFI44L, ISG15, LAMP3, OASL, EPSTI1; or OAS1, IFIT1, IFI6, IFI44L, ISG15, LAMP3, OASL, BIRC4BP; or OAS1, IFIT1, IFI6, IFI44L, ISG15, LAMP3, OASL, gene detected by probe 229450_at; or OAS1, IFIT1, IFI6, IFI44L, ISG15, LAMP3, OASL, gene detected by probe 235276_at; or IFIT1, IFI6, IFI44L, ISG15, LAMP3, OASL, RSAD2, IFI44; or IFIT1, IFI6, IFI44L, ISG15, LAMP3, OASL, RSAD2, IFIT2; or IFIT1, IFI6, IF144L, ISG15, LAMP3, OASL, RSAD2, OAS3; or IFIT1, IFI6, IFI44L, ISG15, LAMP3, OASL, RSAD2, USP18; or IFIT1, IFI6, IFI44L, ISG15, LAMP3, OASL, RSAD2, SIGLEC1; or IFIT1 IFI6, IFI44L, ISG15, LAMP3, OASL, RSAD2, HERC5; or IFIT1, IF16, IFI44L, ISG15, LAMP3, OASL, RSAD2, DNAPTP6; or IFIT1, IFI6, IFI44L, ISG15, LAMP3, OASL, RSAD2, LOC129607; or IFIT1, IFI6, IFI44L, ISG15, LAMP3, OASL, RSAD2, EPSTI1; or IFIT1, IFI6, IFI44L, ISG15, LAMP3, OASL, RSAD2, BIRC4BP; or IFIT1, IFI6, IFI44L, ISG15, LAMP3, OASL, RSAD2, gene detected by probe 229450_at; or IF1T1, IFI6, IFI44L, ISG15, LAMP3, OASL, RSAD2, gene detected by probe 235276_at; or IFI6, IFI44L, ISG15, LAMP3, OASL, RSAD2, IFI44, IFIT2; or IFI6, IPI44L, ISG15, LAMP3, OASL, RSAD2, IFI44, OAS3; or IFI6, IFI44L, ISG15, LAMP3, OASL, RSAD2, IFI44, USP18; or IFI6, IFI44L, ISG15, LAMP3, OASL, RSAD2, IFI44, SIGLEC1; or IFI6, IFI44L, ISG15, LAMP3, OASL, RSAD2, IFI44, HERC5; or IFI6, IFI44L, ISG15, LAMP3, OASL, RSAD2, IFI44, DNAPTP6; or IFI6, IFI44L, ISG15, LAMP3, OASL, RSAD2, IFI44, LOC129607; or IFI6, IFI44L, ISG15, LAMP3, OASL, RSAD2, IFI44, EPSTI1; or IFI6, IFI44L, ISG15, LAMP3, OASL, RSAD2, IFI44, BIRC4BP; or IFI6, IFI44L, ISG15, LAMP3, OASL, RSAD2, IFI44, gene detected by probe 229450_at; or IFI6, IFI44L, ISG15, LAMP3, OASL, RSAD2, IFI44, gene detected by probe 235276_at; or IFI44L, ISG15, LAMP3, OASL, RSAD2, IFI44, IFIT2, OAS3; or IFI44L, ISG15, LAMP3, OASL, RSAD2, IFI44, IFIT2, USP18; or IFI44L, ISG15, LAMP3, OASL, RSAD2, IFI44, IFIT2, SIGLEC1; or IFI44L, ISG15, LAMP3, OASL, RSAD2, IFI44, IFIT2, HERC5; or IFI44L, ISG15, LAMP3, OASL, RSAD2, IFI44, IFIT2, DNAPTP6; or IFI44L, ISG15, LAMP3, OASL, RSAD2, IFI44, IFIT2, LOC129607; or IFI44L, ISG15, LAMP3, OASL, RSAD2, IFI44, IFIT2, EPSTI1; or IFI44L, ISG15, LAMP3, OASL, RSAD2, IFI44, IFIT2, BIRC4BP; or IFI44L, ISG15, LAMP3, OASL, RSAD2, IFI44, IFIT2, gene detected by probe 229450_at; or IFI44L, ISG15, LAMP3, OASL, RSAD2, IFI44, IFIT2, gene detected by probe 235276_at; or ISG15, LAMP3, OASL, RSAD2, IFI44, IFIT2, OAS3, USP18; or ISG15, LAMP3, OASL, RSAD2, IFI44, IFIT2, OAS3, SIGLEC1; or ISG15, LAMP3, OASL, RSAD2, IFI44, IFIT2, OAS3, HERC5; or ISG15, LAMP3, OASL, RSAD2, IFI44, IFIT2, OAS3, DNAPTP6; or ISG15, LAMP3, OASL, RSAD2, IFI44, IFIT2, OAS3, LOC129607; or ISG15, LAMP3, OASL, RSAD2, IFI44, IFIT2, OAS3, EPSTI1; or ISG15, LAMP3, OASL, RSAD2, IFI44, IFIT2, OAS3, BIRC4BP; or ISG15, LAMP3, OASL, RSAD2, IPI44, IFIT2, OAS3, gene detected by probe 229450_at; or ISG15, LAMP3, OASL, RSAD2, IFI44, IFIT2, OAS3, gene detected by probe 235276_at; or LAMP3, OASL, RSAD2, IFI44, IFIT2, OAS3, USP18, SIGLEC1; or LAMP3, OASL, RSAD2, IFI44, IFIT2, OAS3, USP18, HERC5; or LAMP3, OASL, RSAD2, IFI44, IFIT2, OAS3, USP18, DNAPTP6; or LAMP3, OASL, RSAD2, IFI44, IFIT2, OAS3, USP18, LOC129607; or LAMP3, OASL, RSAD2, IFI44, IFIT2, OAS3, USP18, EPSTI1; or LAMP3, OASL, RSAD2, IPI44, IFIT2, OAS3, USP18, BIRC4BP; or LAMP3, OASL, RSAD2, IFI44, IFIT2, OAS3, USP18, gene detected by probe 229450_at; or LAMP3, OASL, RSAD2, IFI44, IFIT2, OAS3, USP18, gene detected by probe 235276_at; or OASL, RSAD2, IFI44, IFIT2, OAS3, USP18, SIGLEC1, HERC5; or OASL, RSAD2, IFI44, IFIT2, OAS3, USP18, SIGLEC1, DNAPTP6; or OASL, RSAD2, IFI44, IFIT2, OAS3, USP18, SIGLEC1, LOC129607; or OASL, RSAD2, IFI44, IFIT2, OAS3, USP18, SIGLEC1, EPSTI1; or OASL, RSAD2, IFI44, IFIT2, OAS3, USP18, SIGLEC1, BIRC4BP; or OASL, RSAD2, IFI44, IFIT2, OAS3, USP18, SIGLEC1, gene detected by probe 229450_at; or OASL, RSAD2, IFI44, IFIT2, OAS3, USP18, SIGLEC1, gene detected by probe 235276_at; or RSAD2, IFI44, IFIT2, OAS3, USP18, SIGLEC1, HERC5, DNAPTP6; or RSAD2, IFI44, IFIT2, OAS3, USP18, SIGLEC1, HERC5, LOC129607; or RSAD2, IFI44, IFIT2, OAS3, USP18, SIGLEC1, HERC5, EPSTII; or RSAD2, IFI44, IFIT2, OAS3, USP18, SIGLEC1, HERC5, gene detected by probe 229450_at; or RSAD2, IFI44, IFIT2, OAS3, USP 18, SIGLEC1, HERC5, BIRC4BP; or RSAD2, IFI44, IFIT2, OAS3, USP18, SIGLEC1, HERC5, gene detected by probe 235276_at; or IFI44, IFIT2, OAS3, USP18, SIGLEC1, HERC5, DNAPTP6, LOC129607; or IFI44, IFIT2, OAS3, USP18, SIGLEC1, HERC5, DNAPTP6, EPSTI1; or IFI44, IFIT2, OAS3, USP18, SIGLEC1, HERC5, DNAPTP6, BIRC4BP; or IFI44, IFIT2, OAS3, USP18, SIGLEC1, HERC5, DNAPTP6, gene detected by probe 229450_at; or IFI44, IFIT2, OAS3, USP18, SIGLEC1, HERC5, DNAPTP6, gene detected by probe 235276_at; or IFIT2, OAS3, USP18, SIGLEC1, HERC5, DNAPTP6, LOC129607, EPSTI1; or IFIT2, OAS3, USP18, SIGLECI, HERC5, DNAPTP6, LOC129607, BIRC4BP; or IFIT2, OAS3, USP18, SIGLEC1, HERC5, DNAPTP6, LOC129607, gene detected by probe 229450_at; or IFIT2, OAS3, USP18, SIGLEC1, HERC5, DNAPTP6, LOC129607, gene detected by probe 235276_at; or OAS3, USP18, SIGLEC1, HERC5, DNAPTP6, LOC129607, EPSTI1, BIRC4BP; or OAS3, USP18, SIGLEC1, HERC5, DNAPTP6, LOC129607, EPSTII, gene detected by probe 229450_at; or OAS3, USP18, SIGLEC1, HERC5, DNAPTP6, LOC129607, EPSTI1, gene detected by probe 235276_at; or USP18, SIGLEC1, HERC5, DNAPTP6, LOC129607, EPSTI1, BIRC4BP, gene detected by probe 229450_at; or USP18, SIGLEC1, HERC5, DNAPTP6, LOC129607, EPSTI1, BIRC4BP, gene detected by probe 235276_at; or SIGLEC1, HERC5, DNAPTP6, LOC129607, EPSTI1, BIRC4BP, gene detected by probe 229450_at, gene detected by probe 235276_at. The IFNα-inducible PD markers in such an expression profile may further include at least one or more gene listed in Tables 19 and/or 20 and/or 21 and/or 22 and/or 23 and/or 24 and/or 26 and/or 28 and/or 30.

The IFNα-inducible PD markers in an expression profile may include any at least 12 genes such as, for example: MX1, LLY6E, IFI27, OAS1, IFIT1, IFl6, IFI44L, ISG15, LAMP3, OASL, RSAD2, IFI44; or MX1, LLY6E, IFI27, OAS1, IFIT1, IFI6, IFI44L, ISG15, LAMP3, OASL, RSAD2, IFIT2; or MX1, LLY6E, IFI27, OAS1, IFIT1, IFI6, IFI44L, ISG15, LAMP3, OASL, RSAD2, OAS3; or MX1, LLY6E, IFI27, OAS1, IFIT1, IFI6, IFI44L, ISG15, LAMP3, OASL, RSAD2, USP18; or MX1, LLY6E, IFI27, OAS1, IFIT1, IFI6, IFI44L, ISG15, LAMP3, OASL, RSAD2, SIGLEC1; or MX1, LLY6E, IFI27, OAS1, IFIT1, IFI6, IFI44L, ISG15, LAMP3, OASL, RSAD2, HERC5; or MX1, LLY6E, IFI27, OAS1, IFIT1, IFI6, IFI44L, ISG15, LAMP3, OASL, RSAD2, DNAPTP6; or MX1, LLY6E, IFI27, OAS1, IFITI, IFI6, IFI44L, ISG15, LAMP3, OASL, RSAD2, LOC129607; or MX1, LLY6E, IFI27, OAS1, IFIT1, IFI6, IFI44L, ISG15, LAMP3, OASL, RSAD2, EPSTI1; or MX1, LLY6E, IFI27, OAS1, IFIT1, IFI6, IFI44L, ISG15, LAMP3, OASL, RSAD2, BIRC4BP; or MX1, LLY6E, IFI27, OAS1, IFIT1, IFI6, IFI44L, ISG15, LAMP3, OASL, RSAD2, gene detected by probe 229450_at; or MX1, LLY6E, IFI27, OAS1, IFIT1, IFI6, IFI44L, ISG15, LAMP3, OASL, RSAD2, gene detected by probe 235276_at; or LLY6E, IFI27, OAS1, IFIT1, IFI6, IFI44L, ISG15, LAMP3, OASL, RSAD2, IFI44, IFIT2; or LLY6E, IFI27, OAS1, IFIT1, IFI6, IFI44L, ISG15, LAMP3, OASL, RSAD2, IFI44, OAS3; or LLY6E, IFI27, OAS1, IFIT1, IFI6, IFI44L, ISG15, LAMP3, OASL, RSAD2, IFI44, USP18; or LLY6E, IFI27, OAS1, IFIT1, IFI6, IFI44L, ISG15, LAMP3, OASL, RSAD2, IFI44, SIGLEC1; or LLY6E, IFI27, OAS1, IFIT1, IFI6, IFI44L, ISG15, LAMP3, OASL, RSAD2, IFI44, HERC5; or LLY6E, IFI27, OAS1, IFIT1, IFI6, IFI44L, ISG15, LAMP3, OASL, RSAD2, IFI44, DNAPTP6; or LLY6E, IFI27, OAS1, IFIT1, IFI6, IFI44L, ISG15, LAMP3, OASL, RSAD2, IFI44, LOC129607; or LLY6E, IFI27, OAS1, IFIT1, IFI6, IFI44L, ISG15, LAMP3, OASL, RSAD2, IFI44, EPSTI1; or LLY6E, IFI27, OAS1, IFIT1, IFI6, IFI44L, ISG15, LAMP3, OASL, RSAD2, IFI44, BIRC4BP; or LLY6E, IFI27, OAS1, IFIT1, IFI6, IFI44L, ISG15, LAMP3, OASL, RSAD2, IFI44, gene detected by probe 229450_at; or LLY6E, IFI27, OAS1, IFIT1, IFI6, IFI44L, ISG15, LAMP3, OASL, RSAD2, IFI44, gene detected by probe 235276_at; or IFI27, OAS1, IFIT1, IFI6, IFI44L, ISG15, LAMP3, OASL, RSAD2, IFI44, IFIT2, OAS; or IFI27, OAS1, IFIT1, IFI6, IFI44L, ISG15, LAMP3, OASL, RSAD2, IFI44, IFIT2, USP18; or IFI27, OAS1, IFIT1, IFI6, IFI44L, ISG15, LAMP3, OASL, RSAD2, IFI44, IFIT2, SIGLEC1; or IFI27, OAS1, IFIT1, IFI6, IFI44L, ISG15, LAMP3, OASL, RSAD2, IFI44, IFIT2, HERC5; or IFI27 OAS1, IFIT1, IFI6, IFI44L, ISG15, LAMP3, OASL, RSAD2, IFI44, IFIT2, DNAPTP6; or IFI27, OAS1, IFIT1, IFI6, IFI44L, ISG15, LAMP3, OASL, RSAD2, IFI44, IFIT2, LOC129607; or IFI27, OAS1, IFIT1, IFI6, IFI44L, ISG15, LAMP3, OASL, RSAD2, IFI44, IFIT2, EPSTI1; or IFI27, OAS1, IFIT1, IFI6, IFI44L, ISG15, LAMP3, OASL, RSAD2, IFI44, IFIT2, BIRC4BP; or IFI27, OAS1, IFIT1, IFI6, IFI44L, ISG15, LAMP3, OASL, RSAD2, IFI44, IFIT2, gene detected by probe 229450_at; or IFI27, OAS1, IFIT1, IFI6, IFI44L, ISG15, LAMP3, OASL, RSAD2, IFI44, IFIT2, gene detected by probe 235276_at; or OAS1, IFIT1, IFI6, IFI44L, ISG15, LAMP3, OASL, RSAD2, IFI44, IFIT2, OAS3, USP18; or OAS1, IFIT1, IFI6, IFI44L, ISG15, LAMP3, OASL, RSAD2, IFI44, IFIT2, OAS3, SIGLEC1; or OAS1, IFIT1, IFI6, IFI44L, ISG15, LAMP3, OASL, RSAD2, IFI44, IFIT2, OAS3, HERC5; or OAS1, IFIT1, IFI6, IFI44L, ISG15, LAMP3, OASL, RSAD2, IFI44, IFIT2, OAS3, DNAPTP6; or OAS1, IFIT1, IFI6, IFI44L, ISG15, LAMP3, OASL, RSAD2, IFI44, IFIT2, OAS3, LOC129607; or OAS1, IFIT1, IFI6, IFI44L, ISG15, LAMP3, OASL, RSAD2, IFI44, IFIT2, OAS3, EPSTI1; or OAS1, IFIT1, IFI6, IFI44L, ISG15, LAMP3, OASL, RSAD2, IFI44, IFIT2, OAS3, BIRC4BP; or OAS1, IFIT1, IFI6, IFI44L, ISG15, LAMP3, OASL, RSAD2, IFI44, IFIT2, OAS3, gene detected by probe 229450_at; or OAS1, IFIT1, IFI6, IFI44L, ISG15, LAMP3, OASL, RSAD2, IFI44, IFIT2, OAS3, gene detected by probe 235276_at; or IFIT1, IFI6, IFI44L, ISG15, LAMP3, OASL, RSAD2, IFI44, IFIT2, OAS3, USP18, SIGLEC1; or IFIT1, IFI6, IFI44L, ISG15, LAMP3, OASL, RSAD2, IFI44, IFIT2, OAS3, USP18, HERC5; or IFIT1, IFI6, IFI44L, ISG15, LAMP3, OASL, RSAD2, IFI44, IFIT2, OAS3, USP18, DNAPTP6; or IFIT1, IFI6, IFI44L, ISG15, LAMP3, OASL, RSAD2, IFI44, IFIT2, OAS3, USP18, LOC129607; or IFIT1, IFI6, IFI44L, ISG15, LAMP3, OASL, RSAD2, IFI44, IFIT2, OAS3, USP18, EPSTI1; or IFIT1, IFI6, IFI44L, ISG15, LAMP3, OASL, RSAD2, IFI44, IFIT2, OAS3, USP18, BIRC4BP; or IFIT1, IFI6, IFI44L, ISG15, LAMP3, OASL, RSAD2, IFI44, IFIT2, OAS3, USP18, gene detected by probe 229450_at; or IFIT1, IFI6, IFI44L, ISG15, LAMP3, OASL, RSAD2, IFI44, IFIT2, OAS3, USP18, gene detected by probe 235276_at; or IFI6, IFI44L, ISG15, LAMP3, OASL, RSAD2, IFI44, IFIT2, OAS3, USP18, SIGLEC1, HERC5; or IFI6, IFI44L, ISG15, LAMP3, OASL, RSAD2, IFI44, IFIT2, OAS3, USP18, SIGLECI, DNAPTP6; or IFI6, IFI44L, ISG15, LAMP3, OASL, RSAD2, IFI44, IFIT2, OAS3, CUSP 18, SIGLEC1, LOC129607; or IFI6, IFI44L, ISG15, LAMP3, OASL, RSAD2, IFI44, IFIT2, OAS3, USP18, SIGLEC1, EPSTI1; or IFI6, IFI44L, ISG15, LAMP3, OASL, RSAD2, IFI44, IFIT2, OAS3, USP18, SIGLEC1, BIRC4BP; or IFI6, IFI44L, ISG15, LAMP3, OASL, RSAD2, IFI44, IFIT2, OAS3, USP18, SIGLEC1, gene detected by probe 229450_at; or IFI6, IFI44L, ISG15, LAMPS, OASL, RSAD2, IFI44, IFIT2, OAS3, USP18, SIGLEC1, gene detected by probe 235276_at; or IFI44L, ISG15, LAMP3, OASL, RSAD2, IFI44, IFIT2, OAS3, USP18, SIGLEC1, HERC5, DNAPTP6; or IFI44L, ISG15, LAMP3, OASL, RSAD2, IFI44, IFIT2, OAS3, USP18, SIGLEC1, HERC5, LOC129607; or IFI44L, ISG15, LAMP3, OASL, RSAD2, IFI44, IFIT2, OAS3, USP18, SIGLEC1, HERC5, EPSTI1; or IFI44L, ISG15, LAMP3, OASL, RSAD2, IFI44, IFIT2, OAS3, USP18, SIGLEC 1, HERC5, BIRC4BP; or IFI44L, ISG15, LAMP3, OASL, RSAD2, IFI44, IFIT2, OAS3, USP18, SIGLEC1, HERC5, gene detected by probe 229450_at; or IFI44L, ISG15, LAMP3, OASL, RSAD2, IFI44, IFIT2, OAS3, USP18, SIGLEC1, HERC5, gene detected by probe 235276_at; or ISG15, LAMP3, OASL, RSAD2, IFI44, IFIT2, OAS3, USP18, SIGLEC1, HERC5, DNAPTP6, LOC 129607; or ISG15, LAMP3, OASL, RSAD2, IFI44, IFIT2, OAS3, USP18, SIGLEC1, HERC5, DNAPTP6, EPSTII; or ISG15, LAMP3, OASL, RSAD2, IFI44, IFIT2, OAS3, USP18, SIGLEC1, HERC5, DNAPTP6, BIRC4BP; or ISG15, LAMP3, OASL, RSAD2, IFI44, IFIT2, OAS3, USP18, SIGLEC1, HERC5, DNAPTP6, gene detected by probe 229450_at; or ISG15, LAMP3, OASL, RSAD2, IFI44, IFIT2, OAS3, USP18, SIGLEC1, HERC5, DNAPTP6, gene detected by probe 235276_at; or LAMP3, OASL, RSAD2, IF144, IFIT2, OAS3, USP18, SIGLEC1, HERC5, DNAPTP6, LOC129607, EPSTI1; or LAMP3, OASL, RSAD2, IFI44, IFIT2, OAS3, USP18, SIGLEC1, HERC5, DNAPTP6, LOC129607, BIRC4BP; or LAMP3, OASL, RSAD2, IFI44, IFIT2, OAS3, USP18, SIGLEC1, HERC5, DNAPTP6, LOC129607, gene detected by probe 229450_at; or LAMP3, OASL, RSAD2, IFI44, IFIT2, OAS3, USP18, SIGLEC1, HERC5, DNAPTP6, LOC129607, gene detected by probe 235276_at; or OASL, RSAD2, IFI44, IFIT2, OAS3, USP18, SIGLEC1, HERC5, DNAPTP6, LOC129607, EPSTI1, BIRC4BP; or OASL, RSAD2, IFI44, IFIT2, OAS3, USP18, SIGLEC1, HERC5, DNAPTP6, LOC129607, EPSTI I , gene detected by probe 229450_at; or OASL, RSAD2, IFI44, IFIT2, OAS3, USP18, SIGLEC1, HERC5, DNAPTP6, LOC129607, EPSTI1, gene detected by probe 235276_at; or RSAD2, IFI44, IFIT2, OAS3, USP18, SIGLEC1, HERC5, DNAPTP6, LOC129607, EPSTI1, BIRC4BP, gene detected by probe 229450_at; or RSAD2, IFI44, IFIT2, OAS3, USP18, SIGLEC1, HERC5, DNAPTP6, LOC129607, EPSTI1, BIRC4BP, gene detected by probe 229450_at; or IFI44, IFIT2, OAS3, USP18, SIGLEC1, HERC5, DNAPTP6, LOC129607, EPSTII, BIRC4BP, gene detected by probe 229450_at gene detected by probe 235276_at. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5, OAS1. The IFNα-inducible PD markers in such an expression profile may further include at least one or more gene listed in Table 19 and/or 20 and/or 21, and/or 22, and/or 23, and/or 24, and/or 26, and/or 28, and/or 30.

The IFNα-inducible PD markers in an expression profile may include at least genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes SERVING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, EPSTI1, and RSAD2. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes BCL2, BAK1, BAD, BAX, and BCL2L1. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes RTP4, RSAD2, HERC5, SIGLEC1, USP18, LY6E, ETV7, SERPING1, IFIT3, OAS1, HSXIAPAF1, G1P3, MX1, OAS3, IFI27, DNAPTP6, LAMP3, EPSTI1, IFI44, OAS2, IFIT2, and ISG 15. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes LAMP3, SIGLEC1, DNAPTP6, IFIT2, ETV7, RTP4, SERPRNG1, HERC5, XAF1, MX1, EPSTI1, OAS2, OAS1, OAS3, IFIT3, IFI6, USP18, RSAD2, IFI44, LY6E, ISG15, and IFI27. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes DNAPTP6, EPSTI1, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes SAMD9L, IFI6, IFI44, IFIT2, ZC3HAV1, ETV6, DAPP1, ILIRN, CEACAM1, OAS1, IFI27, OAS3, IFI44L, HERC5, IFIT1, EPSTI1, ISG15, SERPING1 , OASL, GBP1, and MX1. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes IFI6, RSAD2, IFI44, IFI44L, IFI27, MX1, IFIT1, ISG15, LAMP3, OAS3, OAS1, EPSTI1, IFIT3, OAS2, SIGLEC1, and USP18. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes IFI6, RSAD2, IFI44, IFI44L, IFI27, MX1, IFIT1, HERC5, ISG15, LAMP3, OAS3, OAS1, EPSTI1, IFIT3, OAS2, LY6E, SIGLEC1, and USP18. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes IF16, RSAD2, IFI44, IFI44L, IFI27, MX1, and IFIT1. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes IFI6, RSAD2, IFI44, IFI44L, and IFI27. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes SAMD9L, IFI6, IFI44, IFIT2, OAS1, IFI27, OAS3, IFI44L, HERC5, IFIT1, EPSTI1, ISG15, SERPING1, OASL, GBP1, and MX1. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes IFI27, IL-121R beta2, IL-15R alpha, IL-15, suppressor of cytokine signaling 1 (SOCS1), janus kinase 2, CXCL11 (T-TAC), TNFSF13B (BAFF), TRAF-type domain 1 (TRAFD1), SERVING1, CD274 (PD1-L), indoleamine 2,3 dioxygenase (INDO), lymphocyte-activation gene 3 (LAG3), and caspase 5. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include at least genes complement factor B, insulin-like growth factor (IGF2BP3), cyclin A1, neuropilin 2, complement 1qB, complement 1qC, CD80, CD47, MMP14, toll-like receptor 3 (TLR3), TLR adaptor molecule 2 (TICAM2), macrophage scavenger receptor-1 (MSR1), desmoplakin, PDGR receptor, CCL13 (MCP-4), CXCL13 (BCA-1), CCL19 (CCR7), IL-1 family 5, purinergic receptor P2X7, IRS1, caspase 3, and cyclin-dependent kinase-like 1 (CDKL1). The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include alterations in any one or more of serum protein levels of adiponectin, alpha-fetoprotein, apolipoprotein CIII, beta-2 microglobulin, cancer antigen 125, cancer antigen 19-9, eotaxin, FABP, factor Vll, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, vWF, BDNK, complement 3, CD40 ligand, EGF, ENA-78, EN-RAGE, IGF-1, MDC, myeloperoxidase, RANTES, or thrombopoietin.

The IFNα-inducible PD markers in an expression profile may include alterations in any one or more of serum protein levels of adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1 ra, IL-3, MCP-1, MMP-3, myoglobin, SGOT, tissue factor, TIMP-1, TNF RII, TNF-alpha, VCAM-1, or vWF. The IFNα-inducible PD markers in such an expression profile may further include at least one or more gene listed in Table 19 and/or 20 and/or 21, and/or 22, and/or 23, and/or 24, and/or 26, and/or 28 and/or 30.

The IFNα-inducible PD markers in an expression profile may include alterations in any one or more of serum protein levels of BDNK, complement 3, CD40 ligand, EGF, ENA-78, EN-RAGE, IGF-1, MDC, myeloperoxidase, RANTES, or thrombopoietin. The IFNα-inducible PD markers in such an expression profile may further include at least one or more gene listed in Table 19 and/or 20 and/or 21, and/or 22, and/or 23, and/or 24, and/or 26, and/or 28 and/or 30.

An IFNα-inducible PD marker expression profile may further include genes whose expression or activity is down-regulated in cells exposed to non-baseline IFNα levels. The genes whose expression or activity is down-regulated may be any of the genes that are identified in Table 31. The genes may include any one or more of SLC4A1, PRSS33, FCER1A, BACH2, KERB1, D4S234E, T cell receptor alpha locus/T cell receptor delta locus, FEZ1, AFF3, CD160, ABCB1, PTCH1, OR2W3, IGHD, NOG, NR3C2, TNS1, PDZK1IP1, SH2D1B, STRBP, ZMYND11, TMOD1 , FCRLA, DKFZp761P0423, EPB42, NR6A1, LOC341333, MS4A1, IGHM, SIGLECP3, KIR2DS2, PKIA, BLR1, C5orf4, MYLK, LOC283663, MAD1L1, CXCL5, D4S234E, FCRLA, KRT1, c16orf74, ABCB4, or GRASP1. Any number of these genes may serve as PD markers in an IFNα-inducible PD marker expression profile. For example, at least 2, at least 3, at least 4, at least 5, at least 6, at least 7, at least 8, at least 9, at least 10, at least 11, at least 12 at least 15, at least 20, at least 25, at least 30, at least 35, at least 40, at least 45, or at least 50 down-regulated genes may be included in the IFNα-inducible PD marker expression profile. The IFNα-inducible PD marker expression profile may further include genes listed in Tables 19 and/or 20 and/or 21 and/or 22 and/or 23 and/or 24 and/or 26 and/or 28.

The IFNα-inducible PD marker expression profile may include gene FEZ1, or may include genes FEZ1 and NOG, or may include gene NOG, or may include genes FEZ1, NOG, and SLC4A1, or may include gene SLC4A1, or may include genes NOG and SLC4A1, or may include genes FEZ1, NOG, SLC4A1, and D4S234E, or may include genes FEZ1, NOG, SLC4A1, D4S234E, and PRSS33. The IFNα-inducible PD marker expression profile may further include genes listed in Tables 19 and/or 20 and/or 21 and/or 22 and/or 23 and/or 24 and/or 26 and/or 28 and/or 30, and/or 31.

Down-regulated genes may have down-regulated expression or activity of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, or at least 99% that of control cells, e.g., cells of healthy volunteers or cells of control animals or cells not exposed to IFNα in culture.

Up- or down-regulation of gene expression or activity of IFNα-inducible PD markers may be determined by any means known in the art. For example, up- or down-regulation of gene expression may be detected by determining mRNA levels. mRNA expression may be determined by northern blotting, slot blotting, quantitative reverse transcriptase polymerase chain reaction, or gene chip hybridization techniques. See U.S. Pat. Nos. 5,744,305 and 5,143,854 for examples of making nucleic acid arrays for gene chip hybridization techniques.

Up- or down-regulation of gene expression or activity of IFNα-inducible PD markers may be determined by detecting protein levels. The up- or down-regulated gene whose protein levels are detected may be any one, any two, any three, any four, any five, any six, any seven, any eight, any nine, any ten, any twelve, any fifteen, any twenty, any twenty five, any thirty, any thirty five, or more of adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, vWF, BDNK, complement 3, CD40 ligand, EGF, ENA-78, EN-RAGE, IGF-1, MDC, myeloperoxidase, RANTES, or thrombopoietin. Methods for detecting protein expression levels include immuno-based assays such as enzyme-linked immunosorbant assays, western blotting, protein arrays, and silver staining.

An IFNα-inducible PD marker expression profile may comprise a profile of protein activity. Up- or down-regulation of gene expression or activity of IFNα-inducible PD markers may be determined by detecting activity of proteins including, but not limited to, detectable phosphorylation activity, de-phosphorylation activity, or cleavage activity. Furthermore, up- or down-regulation of gene expression or activity of IFNα-inducible PD markers may be determined by detecting any combination of these gene expression levels or activities.

A candidate therapeutic for treating IFNα-mediated disorders may be identified by the methods encompassed by the invention. Candidate therapeutics may be any type of molecule including a small molecule or a biological agent. A candidate therapeutic identified by the methods encompassed by the invention may immediately be identified as useful as a therapeutic for a disease, disorder, or condition. Alternatively, a candidate therapeutic identified by the methods encompassed by the invention may need to be further tested and/or modified before selection 'for treating patients. Alternatively, a candidate therapeutic identified by the methods encompassed by the invention may, after further testing, be deselected as a molecule for treating patients.

In methods that identify candidate therapeutics, cells comprising an IFNα-inducible PD marker expression profile are contacted with an agent. The cells may be any type of cells, such as commercially available immortalized cell lines that comprise an IFNα-inducible PD marker expression profile, commercially available immortalized cell lines that have been treated with IFNα to induce an IFNα-inducible PD marker expression profile, cells isolated from a patient having an IFNα-inducible PD marker expression profile, or cells isolated from a healthy patient and treated with IFNα to induce an IFNα-inducible PD marker expression profile.

Presence or absence of a change in the IFNα-inducible PD marker expression profile of the cells is detected following contacting the cells with the agent. Presence of change may be any change in IFNα-inducible PD marker expression profile including at least a 10% decrease in up-regulated expression or activity of at least 1 gene in the IFNα-inducible PD marker expression profile, at least a 20% decrease of the at least 1 up-regulated gene, at least a 30% decrease of the at least up-regulated 1 gene, at least a 40% decrease of the at least 1 up-regulated gene, at least a 50% decrease of the at least 1 up-regulated gene, at least a 60% decrease of the at least 1 up-regulated gene, at least a 70% decrease of the at least I up-regulated gene, at least a 75% decrease of the at least 1 up-regulated gene, at least an 80% decrease of the at least 1 up-regulated gene, at least an 85% decrease of the at least 1 up-regulated gene, at least a 90% decrease of the at least I up-regulated gene, at least a 95% decrease of the at least 1 up-regulated gene, at least a 96% decrease of the at least I up-regulated gene, at least a 97% decrease of the at least 1 up-regulated gene, at least a 98% decrease of the at least I up-regulated gene, at least a 99% decrease of the at least I up-regulated gene, or a 100% decrease of the at least I up-regulated gene. Alternatively, or in addition, presence of change may be any change in IFNα-inducible PD marker expression profile including at least a 10% increase in expression or activity of at least I down-regulated gene in the IFNα-inducible PD marker expression profile, at least a 20% increase of the at least 1 down-regulated gene, at least a 30% increase of the at least 1 down-regulated gene, at least a 40% increase of the at least 1 down-regulated gene, at least a 50% increase of the at least I down-regulated gene, at least a 60% increase of the at least 1 down-regulated gene, at least a 70% increase of the at least I down-regulated gene, at least a 75% increase of the at least 1 down-regulated gene, at least an 80% increase of the at least 1 down-regulated gene, at least an 85% increase of the at least 1 down-regulated gene, at least a 90% increase of the at least 1 down-regulated gene, at least a 95% increase of the at least I down-regulated gene, at least a 96% increase of the at least 1 down-regulated gene, at least a 97% increase of the at least 1 down-regulated gene, at least a 98% increase of the at least 1 down-regulated gene, at least a 99% increase of the at least 1 down-regulated gene, or a 100% increase of the at least I down-regulated gene.

In methods of monitoring disease progression of a patient samples from the patient may be obtained before and after administration of an agent, e.g., an agent that binds to and modulates type I IFN or IFNα activity, or an agent that binds to and does not modulate type I IFN or IFNα activity, or a combination of agents that may or may not include an agent that binds to and modulates type I IFN or IFNα activity. Type I IFN or IFNα inducible PD marker expression profiles are obtained in the (before and after agent administration) samples. The type I IFN or IFNα inducible PD marker expression profiles in the samples are compared. Comparison may be of the number of type I IFN or IFNα inducible PD markers present in the samples or may be of the quantity of type I IFN or IFNα inducible PD markers present in the samples, or any combination thereof. Variance indicating efficacy of the therapeutic agent may be indicated if the number or level (or any combination thereof) of up-regulated type I IFN or IFNα inducible PD markers decreases in the sample obtained after administration of the therapeutic agent relative to the sample obtained before administration of the therapeutic agent. The number of up-regulated type I IFN or IFNα inducible PD markers may decrease by at least 1, at least 2, at least 3, at least 4, at least 5, at least 6, at least 7, at least 8, at least 9, or at least 10. The level of any given up-regulated type I IFN or IFNα inducible PD marker may decrease by at least 10%, at least 20%, at least 25%, at least 30%, at least 35%, at least 40%, at least 50%, at least 60%, at least 70%, at least 80%, at least 90%, or at least 95%. The number of up-regulated type I IFN or IFNα inducible PD markers with decreased levels may be at least 1, at least 2, at least 3, at least 4, at least 5, at least 6, at least 7, at least 8, at least 9, at least 10, at least 15, at least 20, at least 25, at least 30, or at least 35. Any combination of decreased number and decreased level of up-regulated type I IFN or IFNα inducible PD markers may indicate efficacy. Variance indicating efficacy of the therapeutic agent may be indicated if the number or level (or any combination thereof) of down-regulated type I IFN or IFNα inducible PD markers decreases in the sample obtained after administration of the therapeutic agent relative to the sample obtained before administration of the therapeutic agent. The number of down-regulated type I IFN or IFNα inducible PD markers may decrease by at least 1, at least 2, at least 3, at least 4, at least 5, at least 6, at least 7, at least 8, at least 9, or at least 10. The level of any given down-regulated type I IFN or IFNα inducible PD marker may increase by at least 10%, at least 20%, at least 25%, at least 30%, at least 35%, at least 40%, at least 50%, at least 60%, at least 70%, at least 80%, at least 90%, or at least 95%. The number of down-regulated type I IFN or IFNα inducible PD markers with increased levels may be at least 1, at least 2, at least 3, at least 4, at least 5, at least 6, at least 7, at least 8, at least 9, at least 10, at least 15, at least 20, at least 25, at least 30, or at least 35. Any combination of decreased number and increased level of down-regulated type I IFN or IFNα inducible PD markers may indicate efficacy.

The sample obtained from the patient may be obtained prior to a first administration of the agent, i.e., the patient is naive to the agent. Alternatively, the sample obtained from the patient may occur after administration of the agent in the course of treatment. For example, the agent may have been administered prior to the initiation of the monitoring protocol. Following administration of the agent an additional samples may be obtained from the patient and type I IFN or IFNα inducible PD markers in the samples are compared. The samples may be of the same or different type, e.g., each sample obtained may be a blood sample, or each sample obtained may be a serum sample. The type I IFN or IFNα inducible PD markers detected in each sample may be the same, may overlap substantially, or may be similar.

The samples may be obtained at any time before and after the administration of the therapeutic agent. The sample obtained after administration of the therapeutic agent may be obtained at least 2, at least 3, at least 4, at least 5, at least 6, at least 7, at least 8, at least 9, at least 10, at least 12, or at least 14 days after administration of the therapeutic agent. The sample obtained after administration of the therapeutic agent may be obtained at least 2, at least 3, at least 4, at least 5, at least 6, at least 7, or at least 8 weeks after administration of the therapeutic agent. The sample obtained after administration of the therapeutic agent may be obtained at least 2, at least 3, at least 4, at least 5, or at least 6 months following administration of the therapeutic agent.

Additional samples may be obtained from the patient following administration of the therapeutic agent. At least 2, at least 3, at least 4, at least 5, at least 6, at least 7, at least 8, at least 9, at least 10, at least 12, at least 15, at least 20, at least 25 samples may be obtained from the patient to monitor progression or regression of the disease or disorder over time. Disease progression may be monitored over a time period of at least 1 week, at least 2 weeks, at least 3 weeks, at least 4 weeks, at least 5 weeks, at least 6 weeks, at least 7 weeks, at least 2 months, at least 3 months, at least 4 months, at least 5 months, at least 6 months, at least I year, at least 2 years, at least 3 years, at least 4 years, at least 5 years, at least 10 years, or over the lifetime of the patient. Additional samples may be obtained from the patient at regular intervals such as at monthly, bi-monthly, once a quarter year, twice a year, or yearly intervals. The samples may be obtained from the patient following administration of the agent at regular intervals. For instance, the samples may be obtained from the patient at one week following each administration of the agent, or at two weeks following each administration of the agent, or at three weeks following each administration of the agent, or at one month following each administration of the agent, or at two months following each administration of the agent. Alternatively, multiple samples may be obtained from the patient following an or each administration of the agent.

Disease progression in a patient may similarly be monitored in the absence of administration of an agent. Samples may periodically be obtained from the patient having the disease or disorder. Disease progression may be identified if the number of type I IFN or IFNα inducible PD markers increases in a later-obtained sample relative to an earlier obtained sample. The number of type I IFN or IFNα inducible PD markers may increase by at least 1, at least 2, at least 3, at least 4, at least 5, at least 6, at least 7, at least 8, at least 9, or at least 10. Disease progression may be identified if level of any given up-regulated type I IFN or IFNα inducible PD marker increases by at least 10%, at least 20%, at least 25%, at least 30%, at least 35%, at least 40%, at least 50%, at least 60%, at least 70%, at least 80%, at least 90%, or at least 95%. Disease progression may be identified if level of any given down-regulated type I IFN or IFNα inducible PD marker decreases by at least 10%, at least 20%, at least 25%, at least 30%, at least 35%, at least 40%, at least 50%, at least 60%, at least 70%, at least 80%, at least 90%, or at least 95%. The number of up-regulated type I IFN or IFNα inducible PD markers with increased levels may be at least 1, at least 2, at least 3, at least 4, at least 5, at least 6, at least 7, at least 8, at least 9, at least 10, at least 15, at least 20, at least 25, at least 30, or at least 35. The number of down-regulated type I IFN or IFNα inducible PD markers with decreased levels may be at least 1, at least 2, at least 3, at least 4, at least 5, at least 6, at least 7, at least 8, at least 9, at least 10, at least 15, at least 20, at least 25, at least 30, or at least 35. Any combination of increased number and increased level of up-regulated type I IFN or IFNα inducible PD marker may indicate disease progression. Alternatively, or in combination, any combination of decreased number and decreased level of down-regulated type I IFN or IFNα inducible PD marker may indicate disease progression. Disease regression may also be identified in a patient having a disease or disorder, not treated by an agent. In this instance, regression may be identified if the number of type I IFN or IFNα inducible PD markers decreases in a later-obtained sample relative to an earlier obtained sample. The number of type I IFN or IFNα inducible PD markers may decrease by at least I, at least 2, at least 3, at least 4, at least 5, at least 6, at least 7, at least 8, at least 9, or at least 10. Disease regression may be identified if level of any given up-regulated type I IFN or IFNα inducible PD marker decreases by at least 10%, at least 20%, at least 25%, at least 30%, at least 35%, at least 40%, at least 50%, at least 60%, at least 70%, at least 80%, at least 90%, or at least 95%. Disease regression may be identified if level of any given down-regulated type I IFN or IFNα inducible PD marker increases by at least 10%, at least 20%, at least 25%, at least 30%, at least 35%, at least 40%, at least 50%, at least 60%, at least 70%, at least 80%, at least 90%, or at least 95%. The number of up-regulated type I IFN or IFNα inducible PD markers with decreased levels may be at least 1, at least 2, at least 3, at least 4, at least 5, at least 6, at least 7, at least 8, at least 9, at least 10, at least 15, at least 20, at least 25, at least 30, or at least 35. The number of down-regulated type I IFN or IFNα inducible PD markers with increased levels may be at least 1, at least 2, at least 3, at least 4, at least 5, at least 6, at least 7, at least 8, at least 9, at least 10, at least 15, at least 20, at least 25, at least 30, or at least 35. Disease progression or disease regression may be monitored by obtaining samples over any period of time and at any interval. Disease progression or disease regression may be monitored by obtaining samples over the course of at least 1 week, at least 2 weeks, at least 3 weeks, at least 4 weeks, at least 5 weeks, at least 6 weeks, at least 7 weeks, at least 2 months, at least 3 months, at least 4 months, at least 5 months, at least 6 months, at least I year, at least 2 years, at least 3 years, at least 4 years, at least 5 years, at least 10 years, or over the lifetime of the patient. Disease progression or disease regression may be monitored by obtaining samples at least monthly, bi-monthly, once a quarter year, twice a year, or yearly. The samples need not be obtained at strict intervals.

The invention also encompasses kits and probes. The probes may be any molecule that detects any expression or activity of any gene that may be included in an IFNα-inducible PD marker expression profile.

The invention also encompasses methods of detecting IFN activity. These methods may employ cells comprising a polynucleotide sequence comprising a reporter gene under the control of an interferon-stimulated response element. The cells comprising the polynucleotide sequence may be any cells amenable to transfection or transformation with a polynucleotide sequence and that can be maintained in culture. These cells include animal cells, bacterial cells, yeast cells, insect cells, or plant cells. These cells may be adherent or may grow in suspension. If the cells are animal cells, they may be from a known cell line such as HeLa, COS, NIH3T3, AGS, 293, CHO, Huh-7, HUVEC, MCF-7, C6, BHK-21, BNL CL 2, C2C12, HepG2, and ATDC5. Countless other cell lines are known and can be obtained by those of skill in the art. The cells may alternatively be primary cells that have or have not been immortalized.

The cells may comprise a polynucleotide sequence comprising a reporter gene under the control of an interferon-stimulated response element. The polynucleotide sequence may be stably integrated in the DNA of the cell or may be an extrachomosomal element that is stably or transiently in the cell. The polynucleotide may have been introduced to the cell as a naked polynucleotide molecule, a polynucleotide molecule complexed with lipids or other molecules, or a polynucleotide in a virus particle.

If the polynucleotide was introduced as a naked polynucleotide molecule, the polynucleotide may have been a linear or a circular molecule. Non-limiting examples of circular polynucleotide molecules include plasmids, and artificial chromosomes. These vectors may be cleaved with enzymes, for example, to generate linear polynucleotide molecules.

Furthermore, if the polynucleotide was introduced as a naked polynucleotide it may have been introduced into the cells by any of many well known techniques in the art. These techniques include, but are not limited to, electroporation, microinjection, and biolistic particle delivery. See, also, *e.g*., Loeffler and Behr, 1993, Meth. Enzymo/. 217:599-618; Cohen et al., 1993, Meth. Enzymol. 217:618-644; Clin. Pharma. Ther. 29:69-92 (1985), Sambrook, et al. Molecular Cloning: A Laboratory Manual. 2nd, ed., Cold Spring Harbor Laboratory, Cold Spring Harbor Laboratory Press, Cold Spring Harbor, N.Y., 1989 and Ausubel et al., ed. Current Protocols in Molecular Biology, John Wiley & Sons, Inc., N.Y., N.Y. (1987-2001).

If the polynucleotide was introduced as a complex with lipids or liposomes, it too may have been introduced by one of many known techniques to the skilled artisan. Lipids or liposomes comprise a mixture of fat particles or lipids which bind to DNA or RNA to provide a hydrophobic coated delivery vehicle. Suitable liposomes may comprise any of the' conventional synthetic or natural phospholipid liposome materials including phospholipids from natural sources such as egg, plant or animal sources such as phosphatidylcholine, phosphatidylethanolamine, phosphatidylglycerol, sphingomyelin, phosphatidylserine or phosphatidylinositol. Synthetic phospholipids also may be used, e.g., dimyristoylphosphatidylcholine, dioleoylphosphatidylcholine, dioleoylphosphatidycholine and corresponding synthetic phosphatidylethanolamines and phosphatidylglycerols. Lipids or liposomes that may be conjugated with the vector are also commercially available to the skilled artisan. Examples of commercially available lipid or liposome transfection reagents known to those of skill in the art include LIPOFECTAMINE™ (Invitrogen), GENEJUICE® (Novagen), GENEJAMMER® (Stratagene), FUGENE® HD (Roche), MEGAFECTIN™ (Qbiogene), SUPERFECT® (Qiagen), and EFFECTENE® (Qiagen).

If the polynucleotide was introduced as a complex with other molecule it may have been compacted or in a nanosphere. Compacted polynucleotide complexes are described in U.S. Patents 5,972,901, 6,008,336, and 6,077,835. Nanospheres are described in U.S. Patent Nos. 5,718,905 and 6,207,195. These compacted polynucleotide complexes and nanospheres that complex nucleic acids utilize polymeric cations. Typical polymeric cations include gelatin, poly-L-lysine, and chitosan. Alternatively, the polynucleotide may have been complexed with DEAE-dextran, or transfected using techniques such as calcium phosphate coprecipitation, or calcium chloride coprecipitation.

If the polynucleotide was introduced associated with a virus, the virus may have been any well known suitable virus for polynucleotide delivery. Example viruses that may be used as vectors include adenovirus, adeno-associated virus, lentivirus, retrovirus, herpes virus (e.g. herpes simplex virus), vaccina virus, papovirus, Sendai virus, SV40 virus, respiratory syncytial virus, etc.

The polynucleotide sequence may include a reporter gene and an interferon-stimulated response element. The reporter gene may be any one of luciferase, chloramphenicol acetyl transferase, β-galactosidase, green fluorescent protein, β-glucuronidase, or secreted placental alkaline phosphatase. Variations of many of these reporter genes, *e.g*., green fluorescent protein and luceriferase, are known and can be readily identified and/or produced by those of skill in the art. Other reporter genes in addition to those listed will also be known to those of skill in the art and are readily available. Interferon-stimulated response elements are also known to those of skill in the art. They may be obtained from commercial vendors such as Stratagene, Clonetech, and Biomyx. They have also been reported in, for instance, Alcantara et al. (Nuc. Acid. Res. 30 (2002):2068-2075 and Kirchhoff et al. (Oncogene 18 (1999):3725-3736).

The cells employed in the assay may be incubated with a sample. The sample may be obtained from a patient, from a vendor with patient samples, or a control sample used for calibration or as a control. If the sample is obtained from a patient it may be any biological fluid or tissue, such as whole blood, saliva, urine, synovial fluid, bone marrow, cerebrospinal fluid, nasal secretions, sputum, amniotic fluid, bronchoalveolar lavage fluid, peripheral blood mononuclear cells, total white blood cells, lymph node cells, spleen cells, tonsil cells, or skin.

Expression of the reporter gene is detected by any well known means in the art. The expression, even if "0" indicates IFN activity in the sample. One of skill in the art may further quantitate any level of expression of the reporter gene which may then correlate to level of IFN activity in the sample.

Applicants provide a set of non-limiting embodiments to describe some of the aspects of the invention.

### Embodiments

Embodiment 1. A method of treating a patient having a type I IFN or an IFNα-mediated disease or disorder comprising:
   administering an agent that binds to and modulates type I IFN or IFNα activity;
      wherein the patient comprises a type I IFN or IFNα-inducible PD marker expression profile;
      and wherein the agent neutralizes the type I IFN or IFNα-inducible PD marker expression profile of the patient.
Embodiment 2. The method of 1 further comprising detecting neutralization of the type I IFN or IFNα-inducible PD marker expression profile of the patient.
Embodiment 3. The method of embodiment I wherein the type I IFN or IFNα-inducible PD marker expression profile comprises up-regulated expression or activity of genes MX1, LY6E, IFI27, OAS1 IFIT1, IFI6, IFI44L, ISG15, LAMP3, OASL, RSAD2, and IFI44.
Embodiment 4. The method of embodiment I wherein the agent is a biologic agent.
Embodiment 5. The method of embodiment 4 wherein the agent is an antibody.
Embodiment 6. The method of embodiment 5 wherein the antibody is MEDI-545.
Embodiment 7. The method of embodiment 5 wherein the antibody is specific for one or more type I IFN or IFNα subtype but is not MEDI-545.
Embodiment 8. The method of embodiment 1 wherein the administering the agent alleviates one or more symptoms of the disease or disorder.
Embodiment 9. The method of embodiment 5 wherein the antibody is administered at a dose between approximately .03 and 30 mg/kg.
Embodiment 10. The method of embodiment 9 wherein the antibody is administered at a dose between 0.3 and 3 mg/kg.
Embodiment 11. The method of embodiment 10 wherein the antibody is administered at a dose between .03 and 1 mg/kg.
Embodiment 12. The method of any one of embodiments 9-11 wherein the agent neutralizes the type I IFN or IFNα-inducible PD marker expression profile of the patient by at least 10%.
Embodiment 13. The method of embodiment 12 wherein the agent neutralizes the type I IFN or IFNα-inducible PD marker expression profile of the patient by at least 20%.
Embodiment 14. The method of embodiment 13 wherein the agent neutralizes the type I IFN or IFNα-inducible PD marker expression profile of the patient by at least 30%.
Embodiment 15. The method of embodiment 14 wherein the agent neutralizes the type I IFN or IFNα-inducible PD marker expression profile of the patient by at least 40%.
Embodiment 16. The method of embodiment 15 wherein the agent neutralizes the type I IFN or IFNα-inducible PD marker expression profile of the patient by at least 50%.
Embodiment 17. The method of embodiment 1 wherein the type I IFN or an IFNα-mediated disease or disorder is one of lupus, psoriasis, vasculitis, sarcoidosis, Sjogren's syndrome, or idiopathic inflammatory myositis.
Embodiment 18. The method of embodiment 17 wherein the type I IFN or an IFNα-mediated disease or disorder is lupus.
Embodiment 19. The method of embodiment 17 wherein the type I IFN or an IFNα-mediated disease or disorder is psoriasis.
Embodiment 20. The method of embodiment 1 wherein the type I IFN or I FNα- inducible PD marker expression profile comprises up-regulated expression or activity of at least IFNα subtypes 1, 2, 8, and 14.
Embodiment 21. The method of embodiment 1 wherein the type I IFN or IFNα-inducible PD marker expression profile comprises transcripts of PD marker genes.
Embodiment 22. The method of embodiment 1 wherein the type I IFN or IFNα-inducible PD marker expression profile comprises polypeptides expressed from PD marker genes.
Embodiment 23. The method of embodiment I wherein the type I IFN or IFNα-inducible PD marker expression profile comprises up-regulated expression or activity of genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5,OAS1
Embodiment 24. The method of embodiment 1 wherein the type I IFN or IFNα-inducible PD marker expression profile comprises up-regulated expression or activity of genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2
Embodiment 25. The method of embodiment 1 wherein the type I IFN or IFNα-inducible PD marker expression profile comprises up-regulated expression or activity of genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1.
Embodiment 26. The method of embodiment 1 wherein the type 1 IFN or IFNα-inducible PD marker expression profile comprises up-regulated expression or activity of genes RTP4, RSAD2, HERC5, SIGLECI, USP18, LY6E, ETV7, SERPING1, IFIT3, OAS1, HSXIAPAF1, GIP3, MX1, OAS3, IFI27, DNAPTP6, LAMP3, EPSTI1, IFI44, OAS2, IFIT2, and ISG 15.
Embodiment 27. The method of embodiment I wherein the type I IFN or IFNα-inducible PD marker expression profile comprises up-regulated expression or activity of genes LAMP3, SIGLEC1, DNAPTP6, IFIT2, ETV7, RTP4, SERVING1, HERC5, XAF1, MX1, EPSTI1, OAS2, OAS1, OAS3, IFIT3, IFI6, USP18, RSAD2, IFI44, LY6E, ISG15, and IFI27.
Embodiment 28. The method of embodiment I wherein the type I IFN or IFNα-inducible PD marker expression profile comprises up-regulated expression or activity of genes DNAPTP6, EPSTI1, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18.
Embodiment 29. The method of embodiment I wherein the type I IFN or IFNα-inducible PD marker expression profile comprises up-regulated expression or activity of genes SAMD9L, IFI6, IFI44, IFIT2, ZC3HAV1, ETV6, DAPP1, ILIRN, CEACAM1, OAS1, IFI27, OAS3, IFI44L, HERC5, IFIT1, EPSTI1, ISG15, SERPING1, OASL, GBP1, and MX1.
Embodiment 30. The method of embodiment I wherein the type I IFN or IFNα-inducible PD marker expression profile comprises up-regulated expression or activity of genes SAMD9L, IFI6, IFI44, IFIT2, OAS1, IFI27, OAS3, IFI44L, HERC5, IFIT1, EPSTI1, ISG15, SERPING1, OASL, GBP1, and MX1.
Embodiment 31. The method of embodiment I wherein the type I IFN or IFNα-inducible PD marker expression profile comprises up-regulated expression or activity of genes IF16, RSAD2, IFI44, IFI44L, IFI27, MX1, IFIT1, ISG15, LAMP3, OAS3, OASI, EPSTI1, IFIT3, OAS2, SIGLEC1, and USP18.
Embodiment 32. The method of embodiment I wherein the type I IFN or IFNα-inducible PD marker expression profile comprises up-regulated expression or activity of genes IFI6, RSAD2, IFI44, IFI44L, and IFI27.
Embodiment 33. The method of embodiment 32 wherein the type I IFN or IFNα-inducible PD marker expression profile further comprises up-regulated expression or activity of genes MX1 and IFIT1.
Embodiment 34. The method of embodiment 33 wherein the type I IFN or IFNα-inducible PD marker expression profile further comprises up-regulated expression or activity of genes OAS2 and OAS1.
Embodiment 35. The method of any one of embodiments 3 or 23-33 wherein the type I IFN or IFNα-inducible PD marker expression profile further comprises down-regulated expression or activity of genes NOG, SLC4A1, PRSS33, and FEZ1.
Embodiment 36. The method of embodiment 1 wherein the type I IFN or IFNα-inducible PD marker expression profile comprises down-regulated expression or activity of genes NOG, SLC4A1, PRSS33, and FEZ1.
Embodiment 37. The method of embodiment 22 wherein the polypeptides are detected at increased levels in serum.
Embodiment 38. The method of embodiment 37 wherein polypeptides include cancer antigen 125, ferritin, tissue factor, and MMP-3.
Embodiment 39. The method of embodiment 22 wherein the polypeptides are detected at decreased levels in serum.
Embodiment 40. The method of embodiment 39 wherein the polypeptides include EGF, thrombopoietin, and CD40 ligand.
Embodiment 41. A method of treating an autoimmune disease patient comprising a moderate or strong type I IFN or an IFNα PD marker profile comprising:
   administering an agent that binds to and modulates type I IFN or IFNα activity;
      wherein the agent neutralizes the type I IFN or IFNα-inducible PD marker expression profile of the patient.
Embodiment 42. The method of 41 further comprising detecting neutralization of the type I IFN or IFNα-inducible PD marker expression profile of the patient.
Embodiment 43. The method of embodiment 41 wherein the type I IFN or IFNα-inducible PD marker expression profile comprises up-regulated expression or activity of genes MX1, LY6E, IFI27, OAS1 IFIT1, IFI6, IFI44L, ISG15, LAMP3, OASL, RSAD2, and IFI44.
Embodiment 44. The method of embodiment 41 wherein the type I IFN or IFNα-inducible PD marker expression profile comprises up-regulated expression or activity of genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5, OAS1
Embodiment 45. The method of embodiment 41 wherein the type I IFN or IFNα-inducible PD marker expression profile comprises up-regulated expression or activity of genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2
Embodiment 46. The method of embodiment 41 wherein the type I IFN or IFNα-inducible PD marker expression profile comprises up-regulated expression or activity of genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1.
Embodiment 47. The method of embodiment 41 wherein the type I IFN or IFNα-inducible PD marker expression profile comprises up-regulated expression or activity of genes RTP4, RSAD2, HERC5, SIGLEC1, USP18, LY6E, ETV7, SERPING1, IFIT3, OAS1, HSXIAPAF1, G1P3, MX1, OAS3, IFI27, DNAPTP6, LAMP3, EPSTI1, IFI44, OAS2, IFIT2, and ISG15.
Embodiment 48. The method of embodiment 41 wherein the type I IFN or IFNα-inducible PD marker expression profile comprises up-regulated expression or activity of genes LAMP3, SIGLEC1, DNAPTP6, IFIT2, ETV7, RTP4, SERPING1, HERC5, XAF1, MX1, EPSTI1, OAS2, OAS1, OAS3, IFIT3, IFI6, USP18, RSAD2, IFI44, LY6E, ISG15, and IFI27.
Embodiment 49. The method of embodiment 41 wherein the type I IFN or IFNα-inducible PD marker expression profile comprises up-regulated expression or activity of genes DNAPTP6, EPSTI1, HERC5, IFI27, IFI44, IFI44L, IF16, IFIT1, IFIT3, ISG15, LAMP3, LY6E, MX I, OAS1, OAS2, OAS3, PLSCR1, RSAD2, RTP4, SIGLEC1, and USP18.
Embodiment 50. The method of embodiment 41 wherein the type I IFN or IFNα-inducible PD marker expression profile comprises up-regulated expression or activity of genes SAMD9L, IFI6, IFI44, IFIT2, ZC3HAV1, ETV6, DAPP1, IL1RN, CEACAM1, OAS1, IFI27, OAS3, IFI44L, HERC5, IFIT1, EPSTI1, ISG15, SERPING1, OASL, GBP1, and MX1.
Embodiment 51. The method of embodiment 41 wherein the type I IFN or IFNα-inducible PD marker expression profile comprises up-regulated expression or activity of genes SAMD9L, IFI6, IFI44, IFIT2, OAS1, IFI27, OAS3, IFI44L, HERC5, IFIT1, EPSTI1, ISG15, SERPING1, OASL, GBP1, and MX1.
Embodiment 52. The method of embodiment 41 wherein the type I IFN or IFNα-inducible PD marker expression profile comprises up-regulated expression or activity of genes IFI6, RSAD2, IFI44, IFI44L, IFI27, MX1, IFIT1, ISG15, LAMP3, OAS3, OAS1, EPSTI1, IFIT3, OAS2, SIGLEC1, and USP18.
Embodiment 53. The method of embodiment 41 wherein the type I IFN or IFNα-inducible PD marker expression profile comprises up-regulated expression or activity of genes IFI6, RSAD2, IFI44, and IFI27.
Embodiment 54. The method of embodiment 53 wherein the type I IFN or IFNα-inducible PD marker expression profile further comprises up-regulated expression or activity of genes MX1 and IFIT1.
Embodiment 55. The method of embodiment 41 wherein the type I IFN or IFNα-inducible PD marker expression profile comprises up-regulated expression or activity of at least IFNα subtypes 1, 2, 8, and 14.
Embodiment 56. The method of embodiment 41 wherein the agent is a biologic agent.
Embodiment 57. The method of embodiment 41 wherein the agent is an antibody.
Embodiment 58. The method of embodiment 57 wherein the antibody is MEDI-545.
Embodiment 59. The method of embodiment 57 wherein the antibody is specific for one or more type I IFN or IFNα subtype but is not MEDI-545.
Embodiment 60. The method of embodiment 41 wherein the administering the agent alleviates one or more symptoms of the disease or disorder.
Embodiment 61. The method of embodiment 57 wherein the antibody is administered at a dose between approximately .03 and 30 mg/kg.
Embodiment 62. The method of embodiment 57 wherein the antibody is administered at a dose between 0.3 and 3 mg/kg.
Embodiment 63. The method of embodiment 57 wherein the antibody is administered at a dose between .03 and 1 mg/kg.
Embodiment 64. The method of embodiment 41 wherein the wherein the agent neutralizes the type I IFN or IFNα-inducible PD marker expression profile by at least 10%.
Embodiment 65. The method of embodiment 64 wherein the wherein the agent neutralizes the type I IFN or IFNα-inducible PD marker expression profile by at least 20%.
Embodiment 66. The method of embodiment 65 wherein the agent neutralizes the type I IFN or IFNα-inducible PD marker expression profile by at least 30%.
Embodiment 67. The method of embodiment 66 wherein the wherein the agent neutralizes the type I IFN or IFNα-inducible PD marker expression profile by at least 40%.
Embodiment 68. The method of embodiment 67 wherein the wherein the agent neutralizes the type I IFN or IFNα-inducible PD marker expression profile by at least 50%.
Embodiment 69. The method of embodiment 41 wherein the autoimmune disease patient is a lupus, psoriasis, vasculitis, sarcoidosis, Sjogren's syndrome, or idiopathic inflammatory myositis patient.
Embodiment 70. The method of embodiment 69 wherein the patient is a lupus patient.
Embodiment 71. The method of embodiment 69 wherein the patient is a psoriasis patient.
Embodiment 72. A method of neutralizing a type I IFN or IFNα-inducible PD marker expression profile in a patient in need thereof, comprising:
   administering an agent that binds to and modulates type I IFN or IFNα activity to the patient;
      wherein the agent neutralizes the type I IFN or IFNα-inducible PD marker expression profile of the patient.
Embodiment 73. The method of 72 further comprising detecting neutralization of the type I IFN or IFNα-inducible PD marker expression profile of the patient.
Embodiment 74. The method of embodiment 72 wherein the type I IFN or IFNα-inducible PD marker expression profile comprises up-regulated expression or activity of genes MX1, LY6E, IFI27, OAS1 IFIT1, IFI6, IFI44L, ISG15, LAMP3, OASL, RSAD2, and IFI44.
Embodiment 75. The method of embodiment 72 wherein the agent is a biologic agent.
Embodiment 76. The method of embodiment 75 wherein the agent is an antibody.
Embodiment 77. The method of embodiment 76 wherein the antibody is MEDI-545.
Embodiment 78. The method of embodiment 76 wherein the antibody is specific for one or more type I IFN or IFNα subtype but is not MEDI-545.
Embodiment 79. The method of embodiment 72 wherein the administering the agent alleviates one or more symptoms of the disease or disorder.
Embodiment 80. The method of embodiment 76 wherein the antibody is administered at a dose between approximately .03 and 30 mg/kg.
Embodiment 81. The method of embodiment 80 wherein the antibody is administered at a dose between 0.3 and 3 mg/kg.
Embodiment 82. The method of embodiment 81 wherein the antibody is administered at a dose between .03 and 1 mg/kg.
Embodiment 83. The method of any one of embodiments 80-82 wherein the agent neutralizes the type I IFN or IFNα-inducible PD marker expression profile of the patient by at least 10%.
Embodiment 84. The method of embodiment 83 wherein the agent neutralizes the type 1 IFN or IFNα-inducible PD marker expression profile of the patient by at least 20%.
Embodiment 85. The method of embodiment 84 wherein the agent neutralizes the type I IFN, or IFNα-inducible PD marker expression profile of the patient at least 30%.
Embodiment 86. The method of embodiment 85 wherein the agent neutralizes the type I IFN or IFNα-inducible PD marker expression profile of the patient at least 40%.
Embodiment 87. The method of embodiment 86 wherein the agent neutralizes the type I IFN or IFNα-inducible PD marker expression profile of the patient at least 50%.
Embodiment 88. The method of embodiment 72 wherein the patient is a lupus, psoriasis, vasculitis, sarcoidosis, Sjogren's syndrome, or idiopathic inflammatory myositis patient.
Embodiment 89. The method of embodiment 88 wherein the patient is a lupus patient.
Embodiment 90. The method of embodiment 88 wherein the patient is a psoriasis patient.
Embodiment 91. The method of embodiment 72 wherein the type I IFN or IFNα-inducible PD marker expression profile comprises up-regulated expression or activity of at least IFNα subtypes 1, 2, 8, and 14.
Embodiment 92. The method of embodiment 72 wherein the type I IFN or IFNα-inducible PD marker expression profile comprises transcripts of PD marker genes.
Embodiment 93. The method of embodiment 72 wherein the type I IFN or IFNα-inducible PD marker expression profile comprises polypeptides expressed from PD marker genes.
Embodiment 94. The method of embodiment 72 wherein the type I IFN or IFNα-inducible PD marker expression profile comprises up-regulated expression or activity of genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5, OAS1.
Embodiment 95. The method of embodiment 72 wherein the type I IFN or IFNα-inducible PD marker expression profile comprises up-regulated expression or activity of genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2.
Embodiment 96. The method of embodiment 72 wherein the type I IFN or IFNα-inducible PD marker expression profile comprises up-regulated expression or activity of genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1.
Embodiment 97. The method of embodiment 72 wherein the type I IFN or IFNα-inducible PD marker expression profile comprises up-regulated expression or activity of genes RTP4, RSAD2, HERC5, SIGLEC1, USP18, LY6E, ETV7, SERPING1, IFIT3, OAS1, HSXIAPAF1, GIP3, MX1, OAS3, IFI27, DNAPTP6, LAMP3, EPSTI1, IFI44, OAS2, IFIT2, and ISG15.
Embodiment 98. The method of embodiment 72 wherein the type I IFN or IFNα-inducible PD marker expression profile comprises up-regulated expression or activity of genes LAMP3, SIGLEC1, DNAPTP6, IFIT2, ETV7, RTP4, SERPTNG1, HERC5, XAF1, MX1, EPSTI1, OAS2, OAS1, OAS3, IFIT3, IFI6, USP18, RSAD2, IFI44, LY6E, ISG15, and IFI27.
Embodiment 99. The method of embodiment 72 wherein the type I IFN or IFNα-inducible PD marker expression profile comprises up-regulated expression or activity of genes DNAPTP6, EPSTI1, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18.
Embodiment 100. The method of embodiment 72 wherein the type I IFN or IFNα-inducible PD marker expression profile comprises up-regulated expression or activity of genes SAMD9L, IFI6, IFI44, IFIT2, ZC3HAV1, ETV6, DAPP1, IL1RN, CEACAM1, OAS1, IFI27, OAS3, IFI44L, HERC5, IFIT1, EPSTI1, ISG15, SERPING1, OASL, GBP1, and MX1.
Embodiment 101. The method of embodiment 72 wherein the type I IFN or IFNα-inducible PD marker expression profile comprises up-regulated expression or activity of genes SAMD9L, IFI6, IFI44, IFIT2, OAS1, IFI27, OAS3, IFI44L, HERC5, IFIT1, EPSTI1, ISG15, SERPING1, OASL, GBP1, and MX1.
Embodiment 102. The method of embodiment 72 wherein the type I IFN or IFNα-inducible PD marker expression profile comprises up-regulated expression or activity of genes IF16, RSAD2, IFI44, IFI44L, IFI27, MX1, IFIT1, ISG15,-LAMP3, OAS3, OAS1, EPSTI1, IFIT3, OAS2, SIGLEC1, and USP18.
Embodiment 103. The method of embodiment 72 wherein the type I IFN or IFNα-inducible PD marker expression profile comprises up-regulated expression or activity of genes IFI6, RSAD2, IFI44, IFI44L, and IFI27.
Embodiment 104. The method of embodiment 103 wherein the type I IFN or IFNα-inducible PD marker expression profile further comprises up-regulated expression or activity of genes MX1 and IFIT1.
Embodiment 105. The method of any one of embodiments 74 or 94-104 wherein the type I IFN or IFNα-inducible PD marker expression profile further comprises down-regulated expression or activity of genes NOG, SLC4A1, PRSS33, and FEZ1.
Embodiment 106. The method of embodiment 72 wherein the type I IFN or IFNα-inducible PD marker expression profile comprises down-regulated expression or activity of genes NOG, SLC4A1, PRSS33, and FEZ1.
Embodiment 107. The method of embodiment 93 wherein the polypeptides are detected at increased levels in serum.
Embodiment 108. The method of embodiment 107 wherein polypeptides include cancer antigen 125, ferritin, tissue factor, and MMP-3.
Embodiment 109. The method of embodiment 93 wherein the polypeptides are detected at decreased levels in serum.
Embodiment 110. The method of embodiment 109 wherein the polypeptides include EGF, thrombopoietin, and CD40 ligand.
Embodiment 111. A method of monitoring or prognosing autoimmune disease progression of a patient comprising:
   obtaining, a first IFNα-inducible PD marker expression profile in a first sample from a patient.
Embodiment 112. The method of embodiment 111 wherein the first IFNα-inducible PD marker expression profile is a strong profile and the patient prognosis is disease progression. Embodiment 113. The method of embodiment 112 wherein the autoimmune disease is SLE and the progression is an SLE flare.
Embodiment 114. The method of embodiment 111 wherein the first IFNα-inducible PD marker expression profile is a weak profile and the patient prognosis is disease regression.
Embodiment 115. The method of embodiment 111 further comprising:
   obtaining a second IFNα-inducible PD marker expression profile in a second sample from a patient;
   wherein an increase in number or level of type I IFN or IFNα inducible PD markers in the second relative to the first expression profile prognoses disease progression; or
   wherein a decrease in number or level of type I IFN or IFNα inducible PD markers in the second relative to the first expression profile prognoses disease regression.
Embodiment 116. A method of monitoring disease progression of a patient receiving treatment with a therapeutic agent that binds to and modulates IFNα activity comprising:
   obtaining a first IFNα-inducible PD marker expression profile in a first sample from the patient;
   administering a therapeutic agent that binds to and modulates IFNα activity;
   obtaining a second IFNα-inducible PD marker expression profile in a second sample from the patient; and
   comparing the first and the second IFNα-inducible PD marker expression profiles,
      wherein a variance in the first and the second IFNα-inducible PD marker expression profiles indicates a level of efficacy of the therapeutic agent that binds to and modulates IFNα activity.
Embodiment 117. The method of embodiment 116 wherein the first IFNα-inducible PD marker expression profile comprises up-regulated expression or activity of genes MX1, LY6E, IFI27, OAS1, IFIT1, IFI6, IFI44L, ISG15, LAMP3, OASL, RSAD2, and IFI44
Embodiment 118. The method of embodiment 116 wherein the first type I IFN or IFNα-inducible PD marker expression profile comprises up-regulated expression or activity of genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5, OAS 1.
Embodiment 119. The method of embodiment 116 wherein the first type I IFN or IFNα-inducible PD marker expression profile comprises up-regulated expression or activity of genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2.
Embodiment 120. The method of embodiment 116 wherein the first type I IFN or IFNα-inducible PD marker expression profile comprises up-regulated expression or activity of genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1.
Embodiment 121. The method of embodiment 116 wherein the first type I IFN or IFNα-inducible PD marker expression profile comprises up-regulated expression or activity of genes RTP4, RSAD2, HERC5, SIGLEC1, USP18, LY6E, ETV7, SERVING1, IFIT3, OAS1, HSXIAPAF1, GIP3, MX1, OAS3, IFI27, DNAPTP6, LAMP3, EPSTI1, IFI44, OAS2, IFIT2, and ISG15.
Embodiment 122. The method of embodiment 116 wherein the first type I IFN or IFNα-inducible PD marker expression profile comprises up-regulated expression or activity of genes LAMP3, SIGLEC1, DNAPTP6, IFIT2, ETV7, RTP4, SERPING1, HERC5, XAF1, MX1, EPSTI1, OAS2, OAS1, OAS3, IFIT3, IFI6, USP18, RSAD2, IFI44, LY6E, ISG15, and IFI27.
Embodiment 123. The method of embodiment 116 wherein the first type I IFN or IFNα-inducible PD marker expression profile comprises up-regulated expression or activity of genes DNAPTP6, EPSTI1, HERC5, IFI27, IFI44, IFI44L, IFI6, IFITI, IFIT3, ISG15, LAMP3, LY6E, MX1, OAS1, OAS2, OAS3, PLSCR1, RSAD2, RTP4, SIGLEC1, and USP18.
Embodiment 124. The method of embodiment 116 wherein the first type I IFN or IFN-α inducible PD marker expression profile comprises up-regulated expression or activity of genes SAMD9L, IFI6, IFI44, IFIT2, ZC3HAV1, ETV6, DAPP1, IL1RN, CEACAM1, OAS1, IFI27, OAS3, IFI44L, HERC5, IFIT1, EPSTI1, ISG15, SERPING1, OASL, GBP1, and MX1.
Embodiment 125. The method of embodiment 116 wherein the first type I IFN or IFNα-inducible PD marker expression profile comprises up-regulated expression or activity of genes SAMD9L, IFI6, IFI44, IFIT2, OAS1, IFI27, OAS3, IFI44L, HERC5, IFIT1, EPSTI1, ISG15, SERPING1, OASL, GBP1, and MX1.
Embodiment 126. The method of embodiment 116 wherein the first type I IFN or IFNα-inducible PD marker expression profile comprises up-regulated expression or activity of genes IFI6, RSAD2, IFI44, IFI44L, IFI27, MX1, IFIT1, ISG15, LAMP3, OAS3, OAS1, EPSTI1, IFIT3, OAS2, SIGLEC1, and USP18.
Embodiment 127. The method of embodiment 116 wherein the first type I IFN or IFNα-inducible PD marker expression profile comprises up-regulated expression or activity of genes IFI6, RSAD2, IFI44, IFI44L, and IFI27.
Embodiment 128. The method of embodiment 116 wherein the variance is a decrease in up-regulated expression of activity levels of the genes.
Embodiment 129. The method of embodiment 116 wherein the disease is lupus, idiopathic inflammatory myositis, Sjogren's syndrome, vasculitis, sarcoidosis, and psoriasis.
Embodiment 130. The method of embodiment 131 wherein the disease is lupus.
Embodiment 131. The method of embodiment 116 wherein the therapeutic agent is a small molecule or a biologic agent.
Embodiment 132. The method of embodiment 131 wherein the biologic agent is an antibody.
Embodiment 133. The method of embodiment 132 wherein the antibody is MEDI-545
Embodiment 134. The method of embodiment 116 wherein the first IFNα-inducible PD marker expression profile is obtained prior to administration of the therapeutic agent.
Embodiment 135. The method of embodiment 116 wherein the first IFNα-inducible PD marker expression profile is obtained at the time of administration of the therapeutic agent.
Embodiment 136. The method of embodiment 116 wherein the first and the second sample are whole blood or serum.
Embodiment 137. The method of embodiment 116 further comprising obtaining a third IFNα-inducible PD marker expression profile in a third sample from the patient.
Embodiment 138. The method of 137 further comprising obtaining a fourth IFNα-inducible PD marker expression profile in a fourth sample from the patient.
Embodiment 139. The method of 138 further comprising obtaining a fifth IFNα-inducible PD marker expression profile in a fifth sample from the patient.
Embodiment 140. The method of 139 further comprising obtaining a sixth IFNα-inducible PD marker expression profile in a sixth sample from the patient.
Embodiment 141. The method of 116 wherein the second sample is obtained at least one week, at least 2 weeks, at least three weeks, at least one month or at least two months following administration of the therapeutic agent.
Embodiment 142. The method of 137 wherein the third sample is obtained at least 2 days, at least 5 days, at least one week, at least 2 weeks, at least three weeks, at least one month or at least two months following obtaining the second sample.
Embodiment 143. The method of 138 wherein the fourth sample is obtained at least 2 days, at least 5 days, at least one week, at least 2 weeks, at least three weeks, at least one month or at least two months following obtaining the third sample.
Embodiment 144. The method of 139 wherein the fifth sample is obtained at least 2 days, at least 5 days, at least one week, at least 2 weeks, at least three weeks, at least one month or at least two months following obtaining the fourth sample.
Embodiment 145. The method of embodiment 116 wherein variance is a decrease in up-regulated expression or activity of the gene.
Embodiment 146. The method of embodiment 145 wherein the decrease is at least 10%, at least 20%, at least 25%, at least 30%, at least 40%, at least 45%, at least 50%, at least 60%, at least 70%, at least 75%, at least 80%, at least 85%, at least 90%, at least 95%, at least 96%, at least 97%, at least 98%, at least 99%, or 100%.
Embodiment 147. A method of identifying a patient as a candidate for a therapeutic agent that binds to and modulates IFNα activity comprising:
   detecting presence or absence of an IFNα-inducible PD marker expression profile in a sample from the patient,
      wherein detecting presence of the IFNα-induced PD marker expression profile identifies the patient as a candidate for the therapeutic agent that binds to and modulates IFNα activity.
Embodiment 148. The method of embodiment 147 wherein the IFNα-inducible PD marker expression profile comprises up-regulated expression or activity of genes MX1, LY6E, IFI27, OAS1, IFITI, IFI6, IFI44L, ISG15, LAMP3, OASL, RSAD2, and IFI44.
Embodiment 149. The method of embodiment 147 wherein type I IFN or IFNα-inducible PD marker expression profile comprises up-regulated expression or activity of genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5, OAS1.
Embodiment 150. The method of embodiment 147 wherein the type I IFN or IFNα-inducible PD marker expression profile comprises up-regulated expression or activity of genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2.
Embodiment 151. The method of embodiment 147 wherein the type I IFN or IFNα-inducible PD marker expression profile comprises up-regulated expression or activity of genes SERVING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1.
Embodiment 152. The method of embodiment 147 wherein the type I IFN or IFNα-inducible PD marker expression profile comprises up-regulated expression or activity of genes RTP4, RSAD2, HERC5, SIGLEC1, USP18, LY6E, ETV7, SERPING1, IFIT3, OAS1, HSXIAPAF1, GIP3, MX1, OAS3, IFI27, DNAPTP6, LAMP3, EPSTI1, IFI44, OAS2, IFIT2, and ISG15.
Embodiment 153. The method of embodiment 147 wherein the type I IFN or IFNα-inducible PD marker expression profile comprises up-regulated expression or activity of genes LAMP3, SIGLEC1, DNAPTP6, IFIT2, ETV7, RTP4, SERPING1, HERC5, XAF1, MX1, EPSTI1, OAS2, OAS1, OAS3, IFIT3, IFI6, USP18, RSAD2, IFI44, LY6E, ISG15, and IFI27.
Embodiment 154. The method of embodiment 147 wherein the type I IFN or IFNα-inducible PD marker expression profile comprises up-regulated expression' or activity of genes DNAPTP6, EPSTI1, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18.
Embodiment 155. The method of embodiment 147 wherein the type I IFN or IFNα-inducible PD marker expression profile comprises up-regulated expression or activity of genes SAMD9L, IFI6, IFI44, IFIT2, ZC3HAV1, ETV6, DAPP1, IL1RN, CEACAM1, OAS1, IFI27, OAS3, IFI44L, HERC5, IFIT1, EPSTI1, ISG15, SERVING1, OASL, GBP1, and MX1.
Embodiment 156. The method of embodiment 147 wherein the type I IFN or IFNα-inducible PD marker expression profile comprises up-regulated expression or activity of genes SAMD9L, IFI6, IFI44, IFIT2, OAS1, IFI27, OAS3, IFI44L, HERC5, IFIT1, EPSTI1, ISG15, SERPING1, OASL, GBP1, and MX1.
Embodiment 157. The method of embodiment 147 wherein the type I IFN or IFNα-inducible PD marker expression profile comprises up-regulated expression or activity of genes IFI6, RSAD2, IFI44, IFI44L, IFI27, MX1, IFIT1, ISG15, LAMP3, OAS3, OAS1, EPSTI1, IFIT3, OAS2, SIGLEC1, and USP18.
Embodiment 158. The method of embodiment 147 wherein the type I IFN or IFNα-inducible PD marker expression profile comprises up-regulated expression or activity of genes IFI6, RSAD2, IFI44, IFI44L, and-IFI27.
Embodiment 159. The method of embodiment 147 wherein the patient has been diagnosed as having a disorder selected from the group consisting of lupus, idiopathic inflammatory myositis, Sjogren's syndrome, vasculitis, sarcoidosis, and psoriasis.
Embodiment 160. The method of embodiment 159 wherein the disorder is lupus.
Embodiment 161. The method of embodiment 147 wherein the therapeutic agent is a small molecule or a biologic agent.
Embodiment 162. The method of embodiment 161 wherein the biologic agent is an antibody.
Embodiment 163. The method of embodiment 162 wherein the antibody is MEDI-545.
Embodiment 164. The method of any one of embodiments 148-158 wherein the up-regulated expression or activity comprises at least a 2-fold increase in expression of one or more of the genes.
Embodiment 165. The method of any one of embodiments 148-158 wherein the up-regulated expression or activity comprises at least a 3-fold increase in expression of one or more of the genes.
Embodiment 166. The method of any one of embodiments 148-158 wherein the up-regulated expression or activity comprises an increase in mRNA levels of one or more of the genes.
Embodiment 167. The method of any one of embodiments 148-158 wherein the up-regulated expression or activity comprises an increase in protein levels of one or more of the genes.
Embodiment 168. The method of any one of embodiments 148-158 wherein the up-regulated expression or activity comprises an increase in enzymatic activity of a protein expressed from one or more of the genes.
Embodiment 169. The method of embodiment 147 wherein the sample is whole blood.
Embodiment 170. The method of embodiment 147 wherein the type I IFN or IFNα-inducible PD marker expression profile comprises down-regulated expression or activity of genes NOG, SLC4A1, PRSS33, and FEZ1.
Embodiment 171. The method of embodiment 147 wherein the type I IFN or IFNα- inducible PD marker expression profile comprises increased serum levels of polypeptides cancer antigen 125, ferritin, tissue factor, and MMP-3.
Embodiment 172. The method of embodiment 147 wherein the type I IFN or IFNα-inducible PD marker expression profile comprises decreased serum levels of polypeptides EGF, thrombopoietin, and CD40 ligand.
Embodiment 173. A method of diagnosing a patient as a having a disorder associated with increased IFNα levels comprising:
   detecting presence or absence of an IFNα-inducible PD marker expression profile in a sample from the patient,
      wherein detecting presence of the IFNα-induced PD marker expression profile identifies the patient as having a disorder associated with increased IFNα levels.
Embodiment 174. The method of embodiment 173 wherein the IFNα-inducible PD marker expression profile comprises up-regulated expression or activity of genes MX1, LY6E, IFI27, OAS1, IFIT1, IFI6, IFI44L, ISG15, LAMP3, OASL, RSAD2, and IFI44.
Embodiment 175. The method of embodiment 173 wherein the IFNα-inducible PD marker expression profile comprises up-regulated expression or activity of genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5, OAS1.
Embodiment 176. The method of embodiment 173 wherein the IFNα-inducible PD marker expression profile comprises up-regulated expression or activity of genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2.
Embodiment 177 The method of embodiment 173 wherein the IFNα-inducible PD marker expression profile comprises up-regulated expression or activity of genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1.
Embodiment 178. The method of embodiment 173 wherein the IFNα-inducible PD marker expression profile comprises up-regulated expression or activity of genes RTP4, RSAD2, HERC5, SIGLEC1, USP18, LY6E, ETV7, SERPING1, IFIT3, OAS1, HSXIAPAF1, G1P3, MX1, OAS3, IFI27, DNAPTP6, LAMP3, EPSTI1, IFI44, OAS2, IFIT2, and ISG15.
Embodiment 179. The method of embodiment 173 wherein the IFNα-inducible PD marker expression profile comprises up-regulated expression or activity of genes LAMP3, SIGLEC1, DNAPTP6, IFIT2, ETV7, RTP4, SERPING1, HERC5, XAF1, MX1 , EPSTI1, OAS2, OAS1, OAS3, IFIT3, IFI6, USP18, RSAD2, IFI44, LY6E, ISG15, and IFI27.
Embodiment 180. The method of embodiment 173 wherein the IFNα-inducible PD marker expression profile comprises up-regulated expression or activity of genes DNAPTP6, EPSTI1, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18.
Embodiment 181. The method of embodiment 173 wherein the IFNα-inducible PD marker expression profile comprises up-regulated expression or activity of genes SAMD9L, IF16, IFI44, IFIT2, ZC3HAV1, ETV6, DAPP1, ILIRN, CEACAM1, OAS1, IFI27, OAS3, IFI44L, HERC5, IFIT1, EPSTI1, ISG15, SERVING 1, OASL, GBP1, and MX1.
Embodiment 182. The method of embodiment 173 wherein the IFNα-inducible PD marker expression profile comprises up-regulated expression or activity of genes SAMD9L, IFI6, IFI44, IFIT2, OAS1, IFI27, OAS3, IFI44L, HERC5, IFIT1, EPSTI1, ISG15, SERPING1, OASL, GBP1, and MX1.
Embodiment 183. The method of embodiment 173 wherein the IFNα-inducible PD marker expression profile comprises up-regulated expression or activity of genes IF16, RSAD2, IFI44, IFI44L, IFI27, MX1, IFIT1, ISG15, LAMP3, OAS3, OAS1, EPSTI1, IFIT3, OAS2, SIGLEC1, and USP18.
Embodiment 184. The method of embodiment 173 wherein the IFNα-inducible PD marker expression profile comprises up-regulated expression or activity of genes IFI6, RSAD2, IFI44, IFI44L, and IFI27.
Embodiment 185. The method of embodiment 173 wherein the disorder is lupus, idiopathic inflammatory myositis, Sjogren's syndrome, vasculitis, sarcoidosis, or psoriasis.
Embodiment 186. The method of embodiment 185 wherein the disorder is lupus.
Embodiment 187. The method of any one of embodiments 174-184 wherein the up-regulated expression or activity comprises at least a 2-fold increase in expression or activity of one or more of the genes.
Embodiment 188. The method of embodiment 187 wherein the up-regulated expression or activity comprises at least a 3-fold increase in expression or activity of one or more of the genes.
Embodiment 189. The method of any one of embodiments 174-184 wherein the up-regulated expression or activity comprises an increase in mRNA levels of one or more of the genes.
Embodiment 190. The method of any one of embodiments 174-184 wherein the up-regulated expression or activity comprises an increase in protein levels of one or more of the genes.
Embodiment 191. The method of any one of embodiments 174-184 wherein the up-regulated expression or activity comprises an increase in enzymatic activity of a protein expressed from one or more of the genes.
Embodiment 192. The method of any one of embodiments 174-184 wherein the type 1 IFN or IFNα-inducible PD marker expression profile further comprises down-regulated expression or activity of genes NOGSLC4A1, PRSS33, and FEZ1.
Embodiment 193. The method any one of embodiments 174-184 wherein the type I IFN or IFNα-inducible PD marker expression profile further comprises increased serum levels of polypeptides cancer antigen 125, ferritin, tissue factor, and MMP-3.
Embodiment 194. The method of any one of embodiments 174-184 wherein the type I IFN or IFNα-inducible PD marker expression profile further comprises decreased serum levels of polypeptides EGF, thrombopoietin, and CD40 ligand.
Embodiment 195. A method of identifying a candidate therapeutic for treating IFNα-mediated disorders comprising:
   contacting cells comprising an IFNα-inducible PD marker expression profile with an agent; and
   detecting presence or absence of a change in the IFNα-induced PD marker expression profile of the cells,
      wherein the presence of a change comprising a reduction in the up-regulation of the genes of the IFNα-inducible PD marker expression profile indicates the agent is a candidate therapeutic agent.
Embodiment 196. The method of embodiment 195 wherein the IFNα-inducible PD marker expression profile comprises up-regulated expression or activity of genes MX1, LY6E, IFI27, OAS1, IFIT1, IFI6, IFI44L, ISG15, LAMP3, OASL, RSAD2, and IFI44.
Embodiment 197. The method of embodiment 195 wherein the IFNα-inducible PD marker expression profile comprises up-regulated expression or activity of genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5, and OAS1.
Embodiment 198. The method of embodiment 195 wherein the IFNα-inducible PD marker expression profile comprises up-regulated expression or activity of genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2.
Embodiment 199 The method of embodiment 195 wherein the IFNα-inducible PD marker expression profile comprises up-regulated expression or activity of genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTII.
Embodiment 200. The method of embodiment 195 wherein the IFNα-inducible PD marker expression profile comprises up-regulated expression or activity of genes RTP4, RSAD2, HERC5, SIGLEC1, USP18, LY6E, ETV7, SERPINGI, IFIT3, OAS1, HSXIAPAF1, GIP3, MX1, OAS3, IFI27, DNAPTP6, LAMP3, EPSTI1, IFI44, OAS2, IFIT2, and ISG15.
Embodiment 201. The method of embodiment 195 wherein the IFNα-inducible PD marker expression profile comprises up-regulated expression or activity of genes LAMP3, SIGLEC1, DNAPTP6, IFIT2, ETV7, RTP4, SERPING1, HERC5, XAF1, MX1, EPSTI1, OAS2, OAS1, OAS3, IFIT3, IFI6, USP18, RSAD2, IFI44, LY6E, ISG15, and IFI27.
Embodiment 202. The method of embodiment 195 wherein the IFNα-inducible PD marker expression profile comprises up-regulated expression or activity of genes DNAPTP6, EPSTII, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18.
Embodiment 203. The method of embodiment 195 wherein the IFNα-inducible PD marker expression profile comprises up-regulated expression or activity of genes SAMD9L, IFI6, IFI44, IFIT2, ZC3HAV1, ETV6, DAPPI, IL1RN, CEACAM 1, OAS1, IFI27, OAS3, IFI44L, HERC5, IFIT1, EPSTI1, ISG15, SERPING1, OASL, GBP1, and MX1.
Embodiment 204. The method of embodiment 195 wherein the IFNα-inducible PD marker expression profile comprises up-regulated expression or activity of genes SAMD9L, IFI6, IFI44, IFIT2, OAS1, IFI27, OAS3, IFI44L, HERC5, IFIT1, EPSTI1, ISG15, SERPING1, OASL, GBP1, and MX1.
Embodiment 205. The method of embodiment 195 wherein the IFNα-inducible PD marker expression profile comprises up-regulated expression or activity of genes IFI6, RSAD2, IFI44, IFI44L, IFI27, MX1, IFIT1, ISG15, LAMP3, OAS3, OAS1, EPSTI1, IFIT3, OAS2, SIGLEC1, and USP18.
Embodiment 206. The method of embodiment 195 wherein the IFNα-inducible PD marker expression profile comprises up-regulated expression or activity of genes IFI6, RSAD2, IFI44, IFI44L, and IFI27.
Embodiment 207. The method of embodiment 195 wherein the cells obtained from a patient comprising a disorder associated with increased IFNα levels.
Embodiment 208. The method of embodiment 195 wherein the cells are cells treated with IFNα to induce the IFNα-inducible PD marker expression profile.
Embodiment 209. The method of embodiment 195 wherein the up-regulation of the genes of the IFNα-inducible PD marker expression profile is at least a 2-fold increase in expression of one or more of the genes of the profile.
Embodiment 210. The method of embodiment 195 wherein the up-regulation of the genes of the IFNα-inducible PD marker expression profile is at least a 3-fold increase in expression of one or more of the genes of the IFNα-inducible PD marker expression profile.
Embodiment 211. The method of embodiment 195 wherein the up-regulation of the genes of the IFNα-inducible PD marker expression profile comprises an increase in mRNA levels of one or more of the genes of the IFNα-inducible PD marker expression profile.
Embodiment 212. The method of embodiment 195 wherein the up-regulation of the genes of the IFNα-inducible PD marker expression profile comprises an increase in protein levels of one or more of the genes of the IFNα-inducible PD marker expression profile.
Embodiment 213. The method of embodiment 195 wherein the up-regulation of the genes of the IFNα-inducible PD marker expression profile comprises an increase in enzymatic activity of a protein expressed from one or more of the genes of the IFNα-inducible PD marker expression profile.
Embodiment 214. The method of any one of embodiments 196-206 wherein the type I IFN or IFNα-inducible PD marker expression profile further comprises down-regulated expression or activity of genes NOGSLC4A1, PRSS33, and FEZ1; and
   wherein the presence of a change comprising an increase in expression or activity of the down-regulated genes indicates the agent is a candidate therapeutic agent.
Embodiment 215. The method of any one of embodiments 196-206 wherein the type I IFN or IFNα-inducible PD marker expression profile further comprises increased serum levels of polypeptides cancer antigen 125, ferritin, tissue factor, and MMP-3; and
   wherein the presence of a change comprising a decrease in serum levels of the polypeptide indicates the agent is a candidate therapeutic agent.
Embodiment 216. The method of any one of embodiments 196-206 wherein the type I IFN or IFNα-inducible PD marker expression profile further comprises decreased serum levels of polypeptides EGF, thrombopoietin, and CD40 ligand
   wherein the presence of a change comprising an increase in serum levels of the polypeptide indicates the agent is a candidate therapeutic agent.
Embodiment 217. A set of probes comprising:
   polynucleotides that specifically detect expression of any one of the sets of genes:
      (a) MX1, LY6E, IFI27, OAS1, IFIT1, IFI6, IFI44L, ISG15, LAMP3, OASL, RSAD2, and IFI44; or
      (b) IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX 1, OAS2, OASL, CCL2, HERC5, OAS1; or
      (c) IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2; or
      (d) SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1; or
      (e) RTP4, RSAD2, HERC5, SIGLEC1, USP18, LY6E, ETV7, SERPING1, IFIT3, OAS1, HSXIAPAF1, G1P3, MX1, OAS3, IFI27, DNAPTP6, LAMP3, EPSTI1, IFI44, OAS2, IFIT2, and ISG15; or
      (f) LAMP3, SIGLEC1, DNAPTP6, IFIT2, ETV7, RTP4, SERPING1, HERC5, XAF1, MX1, EPSTI1, OAS2, OAS1, OAS3, IFIT3, IFI6, USP18, RSAD2, IFI44, LY6E, ISG 15, and IFI27; or
      (g) DNAPTP6, EPSTI1, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18; or
      (h) SAMD9L, IFI6, IFI44, IFIT2, ZC3HAV1, ETV6, DAPP1, ILIRN, CEACAM1, OAS1, IFI27, OAS3, IFI44L, HERC5, IFIT1, EPSTI1, ISG15, SERPING1, OASL, GBP1, and MX1; or
      (i) SAMD9L, IFI6, IFI44, IFIT2, OAS1, IFI27, OAS3, IFI44L, HERC5, IFIT1, EPSTI1, ISG15, SERPING1, OASL, GBP1, and MX1; or
      (j) IFI6, RSAD2, IFI44, IFI44L, IFI27, MX1, IFIT1, ISG15, LAMP3, OAS3, OAS1, EPSTI1, IFIT3, OAS2, SIGLEC1, and USP18; or
      (k) IFI6, RSAD2, IFI44, IFI44L, and IFI27; or
      (l) NOGSLC4A1, PRSS33, and FEZ1.
Embodiment 218. A kit comprising any of the set of probes recited in embodiment 217.
Embodiment 219. A method of detecting IFN activity in a sample comprising:
   incubating cells comprising a polynucleotide sequence comprising a reporter gene under the control of an interferon-stimulated response element with a sample; and
   detecting expression of the reporter gene,
      wherein expression of the reporter gene indicates IFN activity in the sample. Embodiment 220. The method of embodiment 219 wherein cells are HEK293H cells.
Embodiment 221. The method of embodiment 219 wherein the reporter gene is luciferase, chloramphenicol acetyl transferase, β-galactosidase, green fluorescent protein, β-glucuronidase, or secreted placental alkaline phosphatase.
Embodiment 222. The method of embodiment 221 wherein the reporter gene is luciferase.
Embodiment 223. The method of embodiment 222 wherein the luciferase is *Gaussia princeps* luciferase.
Embodiment 224. The method of embodiment 219 further comprising quantitating level of expression of the reporter gene.
Embodiment 225. The method of embodiment 224 further comprising correlating the level of expression of the reporter gene to level of IFN activity in the sample.

All publications, patents and patent applications mentioned in this specification are herein incorporated by reference into the specification to the same extent as if each individual publication, patent or patent application was specifically and individually indicated to be incorporated herein by reference.

This application claims the benefit of priority of U.S. Provisional Application Serial No. 60/873,008 filed December 6, 2006, U.S. Provisional Application Serial No. 60/907,762 filed April 16, 2007, U.S. Provisional Application Serial No. 60/924,219 filed May 3, 2007, U.S. Provisional Application Serial No. 60/924,584 filed May 21, 2007, U.S. Provisional Application Serial No. 60/960,187 filed September 19, 2007, and U.S. Provisional Application Serial No. 60/996,176 filed November 5, 2007, herein incorporated by reference for all purposes. This application also claims the benefit of priority of U.S. Provisional Application Serial No. 60/924,220 filed May 3, 2007, U.S. Provisional Application entitled "Auto-Antibody Markers of Autoimmune Disease" filed November 6, 2007 (Attorney docket no. IA201P2), and U.S. Provisional Application entitled "Auto-Antibody Markers of Autoimmune Disease" filed December 6, 2007 (Attorney docket no. IA201P3), herein incorporated by reference for all purposes. This application further claims the benefit of priority of U.S. Provisional Application Serial No. 60/907,767 filed April 16, 2007, U.S. Provisional Application Serial No. 60/996,174 filed November 5, 2007, and PCT application entitled Methods of Treating Systemic Lupus Erythematosus", filed December 6, 2007 (Attorney docket no. IA210PCT), herein incorporated by reference for all purposes.

The set of examples that follow are provided for the purpose of illustration only and the invention should in no way be construed as being limited to these examples.

### EXAMPLES

### Example 1a: Initial Identification of Up-Regulated Genes in Lupus Patients

Gene expression in whole blood of 5 (2 cutaneous and 3 severe) lupus patients and 5 healthy volunteers was profiled using Affymetrix whole genome array technology and qPCR validation. Gene expression fold-change values were determined by calculating the log₂ signal intensity difference between individual lupus patient samples and the mean log₂ signal intensity for the 5 healthy donor samples. 118 genes were identified as up-regulated by at least 2-fold in whole blood of all 5 lupus patients relative to the healthy volunteers.

Table I provides a summary for 71 of the 118 annotated genes identified as up-regulated by at least 2-fold in all 5 lupus patients. Table 2 provides the fold-up-regulation in gene expression for a subset of the 118 genes for each of the five lupus patients relative to the healthy volunteers. Table 2 also provides a comparison between fold-change values determined on two unique platforms (Affy GeneChip and TaqMan (i.e. qPCR)).

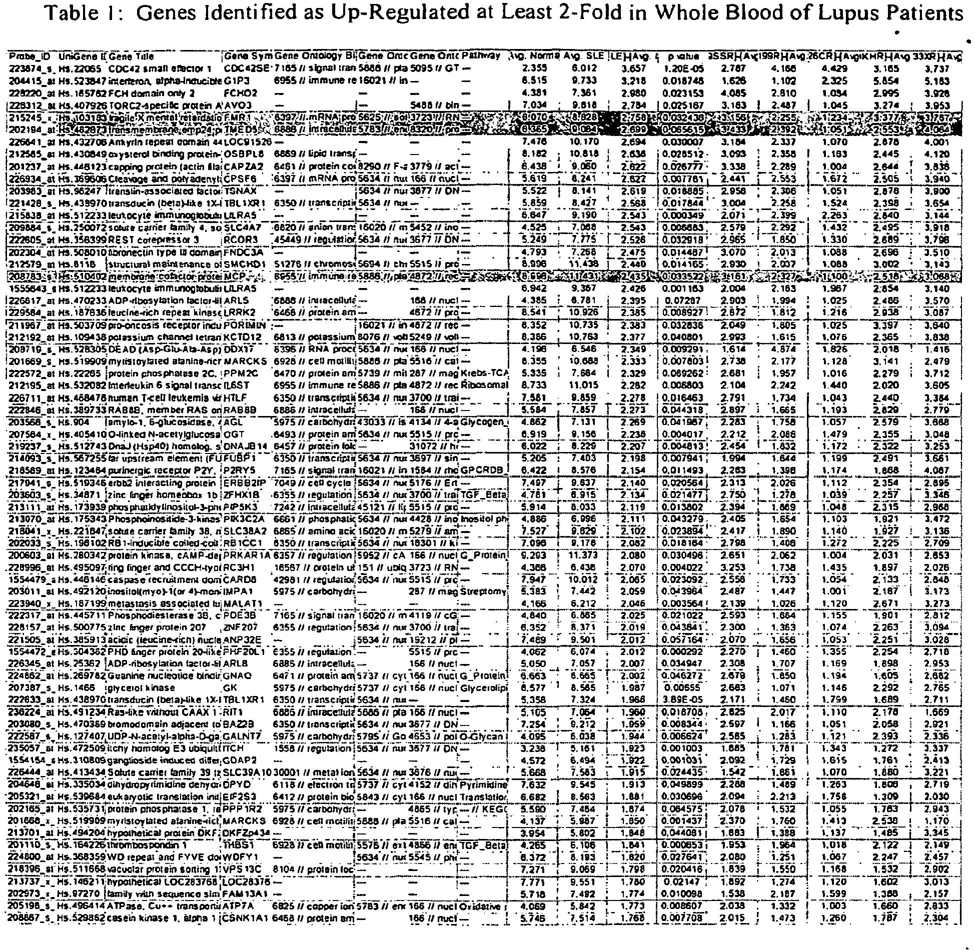

**Table 2: Up-Regulation in Gene Expression for a Set of Genes for each of Five Lupus Patients**

| **Probe_ID** | **Gene Symbol** | **I29KHR.SLE (TaqMan)** | **I29KHR.S LE (Affy)** | **RH33XR.SLE (TaqMan)** | **RH33XR.S LE (Affy)** | **J9SSR (Ta** | **OJ9SSR (A** | **499R (Ta** | **4499R (A** | **4126CR(Ta** | **aMI)26CR(AA** |
|---|---|---|---|---|---|---|---|---|---|---|---|
| 228220_at | FCHO2 | **3.24** | **39.86** | **3.30** | **76.15** | **33.05** | **84.86** | **22.36** | **35.07** | **17.77** | **10.61** |
| 205483_s_at | G1P3 | **86.13** | **146.74** | **80.55** | **92.15** | **4.45** | **7.83** | **2.90** | **5.45** | **5.06** | **12.71** |
| 212195_at | IL6ST | **3.87** | **9.18** | **3.63** | **27.52** | **6.60** | **9.73** | **4.95** | **10.70** | **2.35** | **6.14** |
| 203275_at | IRF2 | **8.10** | **6.46** | **5.00** | **4.80** | **5.07** | **6.54** | **4.12** | **4.32** | **2.44** | **4.66** |
| 1555643_s_at | LILRA5 | **16.43** | **12.00** | **27.25** | **14.64** | **11.22** | **6.66** | **6.82** | **7.44** | **4.86** | **6.49** |
| 205170_at | STAT2 | **11.55** | **8.67** | **9.74** | **2.25** | **8.08** | **2.16** | **6.37** | **2.92** | **4.12** | **2.62** |

### Example 1a: Validation of Genes Identified as Up-Regulated Genes in Lupus Patients

To further identify candidate PD markers for anti-IFN-α mAb clinical trials in SLE, the Affymetrix Human Genome U133 Plus 2.0 GeneChip^{®} array platform was used to profile WB from 46 SLE patients and WB from 24 age- and sex-matched healthy donors. It was observed that 245 and 77 probe sets were upregulated and downregulated, respectively, in WB of SLE patients compared with that from healthy control donors.

Of the 245 probe sets upregulated in WB of SLE patients, 114 were type I IFN inducible. Table 30 lists the 50 most upregulated probe sets in WB of these SLE patients; 76% of them are type I IFN inducible. Table 30 also lists the prevalence of the overexpression of these genes in WB of SLE patients. The majority of these genes are overexpressed by at least 2-fold in 65% to 80% of the patients profiled. The robust and prevalent overexpression of a large number of type I IFN-inducible genes in SLE patients suggests that they might be suitable PD markers for clinical trials that investigate an anti-IFN-α mAb therapy for SLE.

**Table 30: 50 most upregulated probe sets in whole blood of SLE patients**

| **Probe ID** | **Gene Title** | **Gene Symbol** | **log₂ fc** | **q Value (FDR)** | **Prevalence** |
|---|---|---|---|---|---|
| **202411_at** | **interferon, alpha-inducible protein 27** | **IFI27** | **4.60** | **8.41E-07** | **73.91** |
| **219519_s_at** | **sialic acid binding Ig-like lectin 1, sialoadhesin** | **SIGLEC1** | **3.52** | **7.28E-07** | **65.22** |
| **214059_at** | **Interferon-induced protein 44** | **IFI44** | **3.51** | **8.04E-07** | **73.91** |
| **213797_at** | **radical S-adenosyl methionine domain containing 2** | **RSAD2** | **3.29** | **9.86E-06** | **71.74** |
| **204415_at** | **interferon, alpha-inducible protein 6** | **IFI6** | **3.21** | **2.25E-09** | **82.61** |
| **242625_at** | **radical S-adenosyl methionine domain containing 2** | **RSAD2** | **3.19** | **1.55E-06** | **69.57** |
| **204439_at** | **interferon-induced protein 44-like** | **IFI44L** | **3.14** | **4.99E-06** | **71.74** |
| **219211_at** | **ubiquitin specific peptidase 18** | **USP18** | **2.84** | **2.23E-06** | **67.39** |
| **214453_s_at** | **interferon-induced protein 44** | **IFI44** | **2.72** | **1.07E-05** | **71.74** |
| **202145_at** | **lymphocyte antigen 6 complex, locus E** | **LY6E** | **2.53** | **7.28E-07** | **63.04** |
| 207329_at | matrix metallopeptidase 8 (neutrophil collagenase) | MMP8 | 2.51 | 0.00111 | 60.87 |
| **202869_at** | **2',5'-oligoadenylate synthetase 1, 40/46kDa** | **OAS1** | **2.33** | **1.66E-06** | **69.57** |
| **222154_s_at** | **DNA polymerase-transactivated protein 6** | **DNAPTP6** | **2.32** | **1.14E-05** | **65.22** |
| **44673_at** | **sialic acid binding lg-like lectin 1, sialoadhesin** | **SICLEC1** | **2.31** | **2.23E-06** | **58.70** |
| **242234_at** | **XIAP associated factor-1** | **BIRC4BP** | **2.31** | **8.41E-07** | **65.22** |
| **203153_at** | **interferon-induced protein with tetratricopeptide repeats 1** | **IFIT1** | **2.25** | **9.53E-05** | **67.39** |
| **218400_at** | **2'-5'-oligoadenylate synthetase 3, 100kDa** | **OAS3** | **2.24** | **1.23E-05** | **67.39** |
| 212768_s_at | olfactomedin 4 | OLFM4 | 2.23 | 0.00608 | 60.87 |
| 241869_at | apolipoprotein L, 6 | APOL6 | 2.22 | 0.00045 | 80.43 |
| **235643_at** | **sterile alpha motif domain containing 9-like** | **SAMD9L** | **2.22** | **1.37E-06** | **84.78** |
| 231688_at | Transcribed locus | --- | 2.22 | 0.00248 | 63.04 |
| 208470_s_at | haptoglobin /// haptoglobin-related protein | HP /// HPR | 2.20 | 2.48E-05 | 80.43 |
| **239979_at** | **Epithelial stromal interaction 1 (breast)** | **EPSTI1** | **2.20** | **5.44E-06** | **65.22** |
| 206697_s_at | haptoglobin | HP | 2.19 | 2.96E-05 | 73.91 |
| **205552_s_at** | **2',5'-oligoadenylate synthetase 1, 40/46kDa** | **OAS1** | **2.18** | **4.98E-07** | **65.22** |
| **205483_s_at** | **ISC15 ubiquitin-like modifier** | **ISG15** | **2.16** | **2.73E-06** | **65.22** |
| **227609_at** | **epithelial stromal interaction 1 (breast)** | **EPSTI1** | **2.15** | **4.99E-06** | **67.39** |
| 1555643_s_at | leukocyte immunoglobulin-like receptor, subfamily A | LILRAS | 2.14 | 8.41 E-07 | 76.09 |
| **222816_s_at** | **zinc finger, CCHC domain containing 2** | **ZCCHC2** | **2.09** | **5.43E-05** | **80.43** |
| **205569_at** | **lysosomal-associated membrane protein 3** | **LAMP3** | **2.08** | **2.74E-06** | **65.22** |
| **226702**_at | **hypothetical protein LOC129607** | **LOC12960 7** | **2.07** | **5.96E-05** | **67.39** |
| 215838_at | leukocyte immunoglobulin-like receptor, subfamily A | LILRA5 | 2.07 | 1.87E-05 | 71.74 |
| **219863_at** | **hect domain and RLD 5** | **HERC5** | **2.03** | **1.53E-05** | **67.39** |
| **204747_at** | **interferon-induced protein with tetratricopeptide repeats 3** | **IFIT3** | **2.01** | **1.55E-06** | **67.39** |
| **200986_at** | **serpin peptidase inhibitor, clade G (C1 inhibitor), member 1** | **SERPING 1** | **1.98** | **0.00013** | **67.39** |
| **224225_s_at** | **ets variant gene 7 (TEL2 oncogene)** | **ETV7** | **1.98** | **2.48E-05** | **58.70** |
| **219684_at** | **receptor (chemosensory) transporter protein 4** | **RTP4** | **1.96** | **2.74E-06** | **63.04** |
| **206133_at** | **XIAP associated factor-1** | **BIRC4BP** | **1.96** | **7.28E-07** | **65.22** |
| 206871_at | elastase 2, neutrophil | ELA2 | 1.95 | 0.00316 | 54.35 |
| **2.17502_at** | **interferon-induced protein with tetratricopeptide repeats 2** | **IFIT2** | **1.95** | **4.86E-06** | **71.74** |
| 237340_at | solute carrier family 26, member 8 | SLC26A8 | 1.93 | 6.68E-06 | 60.87 |
| **235276_at** | **---** | **---** | **1.93** | **6.44E-06** | **65.22** |
| 203757_s_at | carcinoembryonic antigen-related cell adhesion molecule 6 | CEACAM 6 | 1.91 | 0.00124 | 47.83 |
| **202086_at** | **myxovirus (influenza virus) resistance 1, interferon-inducible protein p78 (mouse) /// myxovirus (influenza virus) resistance 1, interferon-inducible protein p78 (mouse)** | **MX1** | **1.90** | **2.66E-05** | **67.39** |
| **241916_at** | **Phospholipid scramblase 1** | **PLSCR1** | **1.89** | **4.86E-06** | **73.91** |
| **203595_s_at** | **interferon-induced protein with tetratricopeptide repeats 5** | **IFIT5** | **1.89** | **2.81E-08** | **69.57** |
| **205660_at** | **2'-5'-olipoadenylate synthetase-like** | **OASL** | **1.89** | **1.94E-05** | **65.22** |
| **219352_at** | **hect domain and RLD 6** | **HERC6** | **1.87** | **9.79E-06** | **63.04** |
| 211657_at | carcinoembryonic antigen-related cell adhesion molecule 6 | CEACAM 6 | 1.86 | 0.00667 | 60.87 |
| **228439_at** | **basic leucine zipper transcription factor, ATF-like 2** | **BATF2** | **1.86** | **2.63E-05** | **63.04** |

| | | | | | |
|---|---|---|---|---|---|
| Data were generated from 46 SLE patients and 24 healthy controls using SAM and FDR in R (see Methods). Type I IFN-inducible genes are highlighted in bold. FDR=false discovery rate; SAM=significance analysis of microarrays; SLE=systemic lupus erythematosus; WB=whole blood. | | | | | |

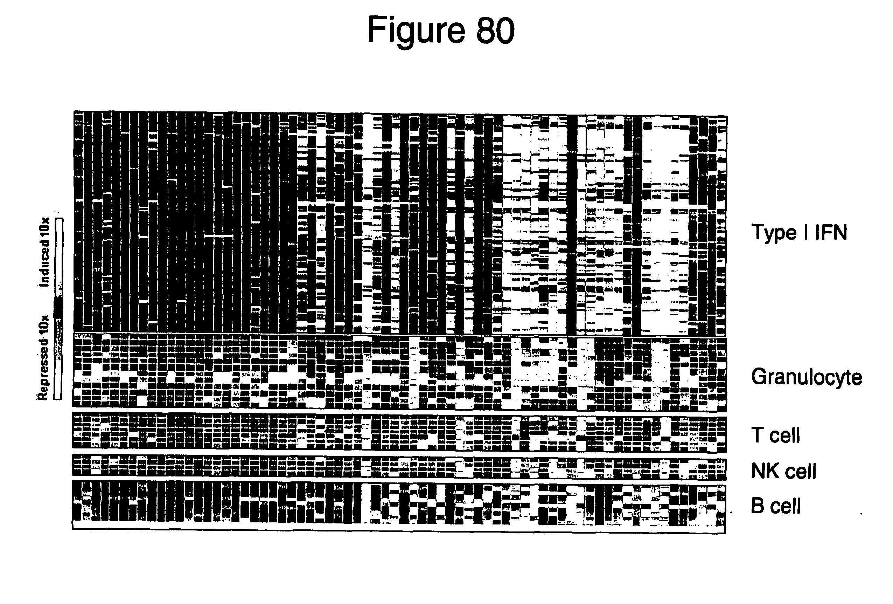

Figure 80 (top panel) shows a heat map of the expression of the 114 upregulated type I IFN-inducible probe sets in SLE patients and healthy controls. A total of 32/46 of the SLE patients profiled showed significant overexpression of the type I IFN gene signature. To confirm the observation that type I IFN-inducible genes are overexpressed in WB of SLE patients, WB was procured from 54 SLE patients in a prospective study. Figure 81A shows the PCA plot of the 46 SLE patients in the first study using the 114 overexpressed type I IFN-inducible probes. A clear difference was observed between SLE patients that had distinct overexpression of type I IFN gene signature from healthy donors and SLE patients that had weak or nondetectable type I IFN gene signature in WB. Figure 81B shows the PCA plot from the 54 SLE patients in the prospective study using the same 114 type I IFN-inducible probe sets identified. A similar separation of SLE patients was observed based on type 1 IFN gene signature as in Figure 81A. The distribution of the type I IFN gene signature scores in the prospective study was also similar to that of the first study (data not shown). The ability to use the overexpressed type I IFN-inducible genes identified to segregate SLE patients into 2 distinct groups-patients with or without type I IFN gene signature-validated the accurate identification of overexpression in the type I IFN gene signature in WB of SLE patients.

In addition to the overexpression of a type I IFN gene signature, the overexpression of a gene signature that is indicative of granulocyte activation in WB of SLE patients was observed. The granulocyte gene signature included (but was not limited to) the following genes: AZU, DEFA1, DEFA4, ELA2, MMP8, MMP9, RNAS2, MPO, CAMP, FCAR, and CYBB (Figure 80, second panel). The granulocyte gene signature was present in about 50% of the SLE patients profiled.

The 50 most downregulated probe sets observed in WB of SLE patients are shown in Table 31. The downregulation of T, NK, and B cell gene signatures was observed in WB of SLE patients (Figure 80, panels three, four, and five, respectively); this is in agreement with the observation of lymphopenia in SLE patients previously reported in the literature (Bennett L, Palucka AK, Arce E et al.: Interferon and granulopoiesis signatures in systemic lupus erythematosus blood. J Exp Med. 197(6), 711-723 (2003), Rivero SJ, Diaz-Jouanen E and Alarcon-Segovia D: Lymphopenia in systemic lupus erythematosus. Clinical, diagnostic, and prognostic significance. Arthritis Rheum. 21 (3), 295-305 (1978).

**Table 31: Top 50 most downregulated transcripts in whole blood of SLE patients**

| **Probe ID** | **Gene Title** | **Gene Symbol** | **log₂ fc** | **q Value (FDR)** | **Prevalence** |
|---|---|---|---|---|---|
| 1552713_a_at | solute carrier family 4, anion exchanger, member 1 (erythrocyte membrane protein band 3, Diego blood group) | SLC4A1 | -1.82 | 0.00021 | 69.57 |
| 1552348_at | protease, serine, 33 | PRSS33 | -1.71 | 0.00046 | 63.04 |
| 211734_s_at | Fc fragment of IgE, high affinity I, receptor for; alpha polypeptide /// Fc fragment of IgE, high affinity 1, receptor for; alpha polypeptide | FCER1A | -1.59 | 0.00083 | 54.35 |
| 236307_at | BTB and CNC homology 1, basic leucine zipper transcription factor 2 | BACH2 | -1.51 | 0.00012 | 54.35 |
| 214470_at | killer cell lectin-like receptor subfamily B, member 1 /// killer cell lectin-like receptor subfamily B, member 1 | KLRB1 | -1.50 | 0.00000 | 58.70 |
| 209570_s_at | DNA segment on chromosome 4 (unique) 234 expressed sequence | D4S234E | -1.46 | 0.00000 | 65.22 |
| 217143_s_at | T cell receptor alpha locus /// T cell receptor delta locus | TRA@ /// TRD@ | -1.38 | 0.00001 | 58.70 |
| 203562_at | fasciculation and elongation protein zeta 1 (zygin 1) | FEZ1 | -1.36 | 0.00028 | 89.13 |
| 227198_at | AF4/FMR2 family, member 3 | AFF3 | -1.35 | 0.00046 | 45.65 |
| 207840_at | CD 160 molecule | CD160 | -1.34 | 0.00079 | 47.83 |
| 232286_at | AF4/FMR2 family, member 3 | AFF3 | -1.34 | 0.00003 | 56.52 |
| 209993_at | ATP-binding cassette, sub-family B (MDR/TAP), member 1 | ABCB1 | -1.32 | 0.00002 | 63.04 |
| 20951_5_at | patched homolog 1 (Drosophila) | PTCH1 | -1.29 | 0.00003 | 54.35 |
| 241881_at | olfactory receptor, family 2, subfamily W, member 3 | OR2W3 | -1.29 | 0.01736 | 50.00 |
| 213674_x_at | immunoglobulin heavy constant delta | IGHD | -1.29 | 0.01801 | 50.00 |
| 231798_at | Noggin | NOG | -1.28 | 0.00234 | 73.91 |
| 239673_at | Nuclear receptor subfamily 3, group C, member 2 | NR3C2 | -1.27 | 0.00004 | 56.52 |
| 221748_s_at | tensin 1 /// tensin 1 | TNS1 | -1.23 | 0.00953 | 50.00 |
| 218864_at | tensin 1 | TNS1 | -1.22 | 0.00718 | 50.00 |
| 219630_at | PDZK1 interacting protein 1 | PDZK11P | -1.20 | 0.00528 | 56.52 |
| 1553177_at | SH2 domain containing 1B | SH2D1B | -1.20 | 0.00187 | 47.83 |
| 229513_at | Spermatid perinuclear RNA binding protein | STRBP | -1.20 | 0.00017 | 58.70 |
| 243054_at | Zinc finger, MYND domain containing 11 | ZMYND11 | -1.20 | 0.00101 | 60.87 |
| 236796_at | BTB and CNC homology 1, basic leucine zipper transcription factor 2 | BACH2 | -1.20 | 0.00004 | 56.52 |
| 203661_s_at | tropomodulin 1 | TMOD1 | -1.19 | 0.00675 | 50.00 |
| 239278_at | CDNA clone IMAGE:5301129 | --- | -1.17 | 0.00002 | 65.22 |
| 235400_at | Fc receptor-like A | FCRLA | -1.17 | 0.00099 | 52.17 |
| 240690_at | Homolog of rat pragma of Rnd2 | DKFZp761P0 423 | -1.17 | 0.00012 | 52.17 |
| 210746_s_at | erythrocyte membrane protein band 4.2 /// erythrocyte membrane protein band 4.2 | EPB42 | -1.16 | 0.00552 | 45.65 |
| 232478_at | Nuclear receptor subfamily 6, group A, member 1 | NR6A1 | -1.15 | 0.00004 | 47.83 |
| 243810_at | Similar to Heterogeneous nuclear ribonucleoprotein A1 (Helix-destabilizing protein) (Single-strand RNA-binding protein) (hnRNP core protein A1) | LOC341333 | -1.15 | 0.00014 | 47.83 |
| 228599_at | membrane-spanning 4-domains, subfamily A, member 1 | MS4A1 | -1.14 | 0.00454 | 45.65 |
| 212827_at | immunoglobulin heavy constant mu /// immunoglobulin heavy constant mu | IGHM | -1.14 | 0.00324 | 45.65 |
| 1552349_a_at | protease, serine, 33 | PRSS33 | -1.13 | 0.02357 | 47.83 |
| 216191_s_at | T cell receptor alpha locus /// T cell receptor delta locus /// B-cell CLL/lymphoma 11B (zinc finger protein) | TRA@ /// TRD@ /// BCL11B | -1.12 | 0.01073 | 50.00 |
| 232686_at | static acid binding Ig-like lectin, pseudogene 3 | SIGLECP3 | -1.12 | 0.00003 | 58.70 |
| 211532_x_at | killer cell immunoglobulin-like receptor, two domains, short cytoplasmic tail, 2 | KIR2DS2 | -1.10 | 0.04011 | 54.35 |
| 1563217_at | Protein kinase (cAMP-dependent, catalytic) inhibitor alpha | PKIA | -1.10 | 0.00024 | 58.70 |
| 243798_at | Burkitt lymphoma receptor 1, GTP binding protein (chemokine (C-X-C motif) receptor 5) | BLR1 | -1.10 | 0.00044 | 54.35 |
| 220751_s_at | chromosome 5 open reading frame 4 | C5orf4 | -1.09 | 0.00531 | 50.00 |
| 202555_s_at | myosin, light chain kinase /// myosin, light chain kinase | MYLK | -1.09 | 0.00149 | 52.17 |
| 230245_s_at | hypothetical protein LOC283663 | LOC283663 | -1.09 | 0.00977 | 47.83 |
| 233921_s_at | MAD1 mitotic arrest deficient-like 1 (yeast) | MADIL1 | -1.08 | 0.00001 | 41.30 |
| 214974_x_at | chemokine (C-X-C motif) ligand 5 | CXCL5 | -1.08 | 0.00717 | 54.35 |
| 209569_x_at | DNA segment on chromosome 4 (unique) 234 expressed sequence | D4S234E | -1.08 | 0.00005 | 58.70 |
| 235401_s_at | Fc receptor-like A | FCRLA | -1.08 | 0.00173 | 50.00 |
| 205900_at | keratin 1 (epidermolytic hyperkeratosis) | KRT1 | -1.08 | 0.04518 | 43.48 |
| 242509_at | Chromosome 16 open reading frame 74 | C16orf74 | -1.08 | 0.00016 | 47.83 |
| 209994_s_at | ATP-binding cassette, sub-family B (MDR/TAP), member 1 /// ATP-binding cassette, sub-family B (MDR/TAP), member 4 | ABCB1 /// ABCB4 | -1.08 | 0.00000 | 56.52 |
| 204793_at | G protein-coupled receptor associated sorting protein 1 | GPRASP1 | -1.08 | 0.00026 | 45.65 |

| | | | | | |
|---|---|---|---|---|---|
| Data were generated from 46 SLE patients and 24 healthy controls using SAM and FDR in R (see Methods). FDR=false discovery rate; SLE=systemic lupus erythematosus; SAM=significance analysis of microarrays. WB=whole blood. | | | | | |

To further confirm the observation of overexpression of the type I IFN and granulocyte signatures and to identify other signaling pathways that may be altered in SLE, a pathway and network analysis was carried out with GeneGo software (see Methods). Overall, for SLE, this pathway analysis confirmed the activation of the type I IFN pathway, along with the activation of a granulocyte signature, and the underexpression of the T-cell signaling pathway. Additionally, in the patients profiled, the activation of the IL-10 signaling pathway was among the other notable pathways found to be altered. This may suggest B cell activation and be indicative of the abnormal apoptosis of T-cell subsets observed in SLE patients. (Diaz-Alderete A, Crispin JC, Vargas-Rojas MI and Alcocer-Varela J: IL-10 production in B cells is confined to CD154+ cells in patients with systemic lupus erythematosus. J Autoimmun. 23(4), 379-383 (2004), Wang H, Xu J, Ji X et al.: The abnormal apoptosis of T cell subsets and possible involvement of IL-10 in systemic lupus erythematosus. Cell Immunol. 235(2), 117-121 (2005)).

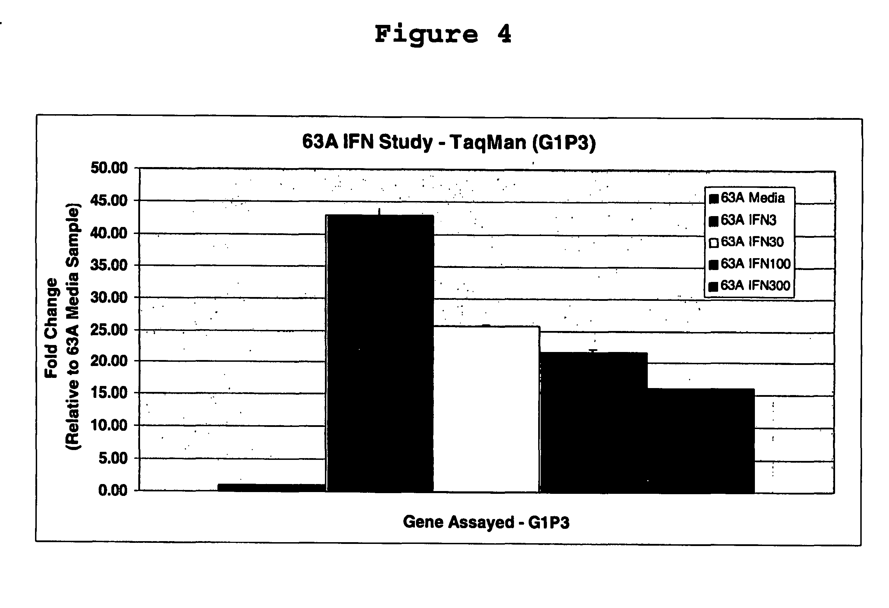

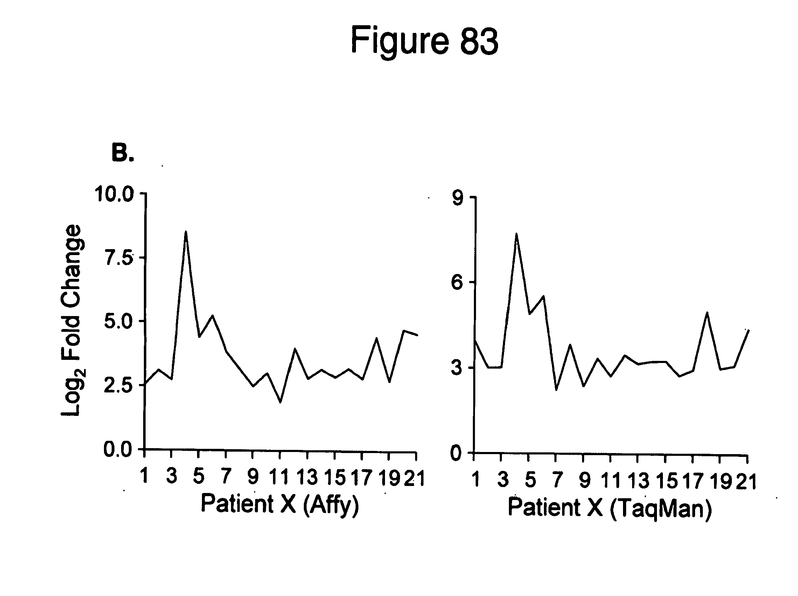

*Confirmation of overexpression of type I IFN-inducible genes*: To confirm the overexpression of type I IFN-inducible genes in SLE that were observed in the microarray analyses, a BioMark^{™} 48.48 dynamic array was used to perform high throughput (HTP) TaqMan QRT-PCR on 40 of the type I IFN-inducible genes (selected based on their magnitude and prevalence of overexpression in whole blood of SLE patients). TaqMan QRT-PCR assays confirmed the overexpression of all 40 genes in whole blood of 35 of the originally profiled 46 SLE patients. The overexpression of 15 of the 40 type I IFN-inducible genes using TaqMan assays is shown in Figure 83A. These genes were upregulated by an average of 8- to 92-fold, and all were significantly overexpressed (*P*<0.05). These observations provide evidence that type I IFN-inducible genes are significantly overexpressed in SLE patients. The consistency of the results among microarray and TaqMan assays and the strong correlation (correlation coefficient >0.98) between microarray and TaqMan assays for 21 IFN-inducible genes in 2 example SLE patients (Figures 83B and 4C) argues for their potential as PD and diagnostic markers in clinical trials that investigate anti-IFN-α approaches in the treatment of SLE.

### Example 2: Potential PD Markers Selected from Genes Up-Restated in Lupus Patients

Using the whole genome profiling data described in Example 1a, a group of candidate PD markers were selected. These candidate markers are provided in Table 3.

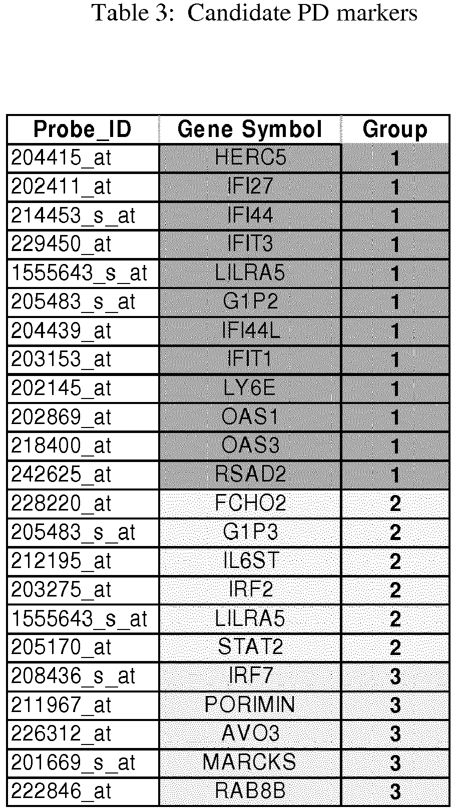

### Example 3: Candidate PD Markers Exhibit Minimal Variation in Healthy Donors

qPCR was conducted for a selected group of candidate PD markers to determine whether they exhibited variation at baseline in the whole blood of healthy volunteers. qPCR indicated that baseline variation was minimal. See Table 4, which provides the baseline qPCR data (healthy volunteers shown in shaded columns).

**Table 4: Baseline Variation of Candidate PD Markers**

| **Gene** | **102-PAX** | **129-PAX** | **I29KHR-SLE** | **RH33XR-SLE** |
|---|---|---|---|---|
| CDC42SE1 | 0.589 | 1.000 | 2.622 | 1.996 |
| FCHO2 | 0.872 | 1.000 | 3.235 | 3.298 |
| GIP3 | 2.059 | 1.000 | 86.130 | 80.545 |
| HERC5 | 3.638 | 1.000 | 638.073 | 159.621 |
| IFI27 | 0.246 | 1.000 | 508.346 | 14.012 |
| IFI44 | 5.194 | 1.000 | 636.965 | 338.921 |
| IFIT3 | 1.413 | 1.000 | 104.166 | 59.344 |
| IL6ST | 0.337 | 1.000 | 3.873 | 3.628 |
| IRF2 | 1.486 | 1.000 | 8.096 | 4.998 |
| LILRA5 | 1.48177 | 1.000 | 16.433182 | 27.248745 |
| BAFF | 0.433 | 1.000 | 2.478 | 4.679 |
| GIP2 | 0.571 | 1.000 | 22.168 | 13.634 |
| IFI44L | 2.581 | 1.000 | 407.035 | 259.517 |
| IFIT1 | 4.018 | 1.000 | 128.164 | 151.301 |
| LY6E | 0.442 | 1.000 | 10.095 | 5.181 |
| OAS1 | 0.817 | 1.000 | 16.650 | 10.379 |
| OAS3 | 2.517 | 1.000 | 75.542 | 32.355 |
| RSAD2 | 2.425 | 1.000 | 310.575 | 217.885 |
| STAT2 | 1.526 | 1.000 | 11.551 | 9.735 |

### Example 4. IFNα Stimulates Up-Regulation in Expression of Candidate PD Markers in Whole Blood of Healthy Volunteers

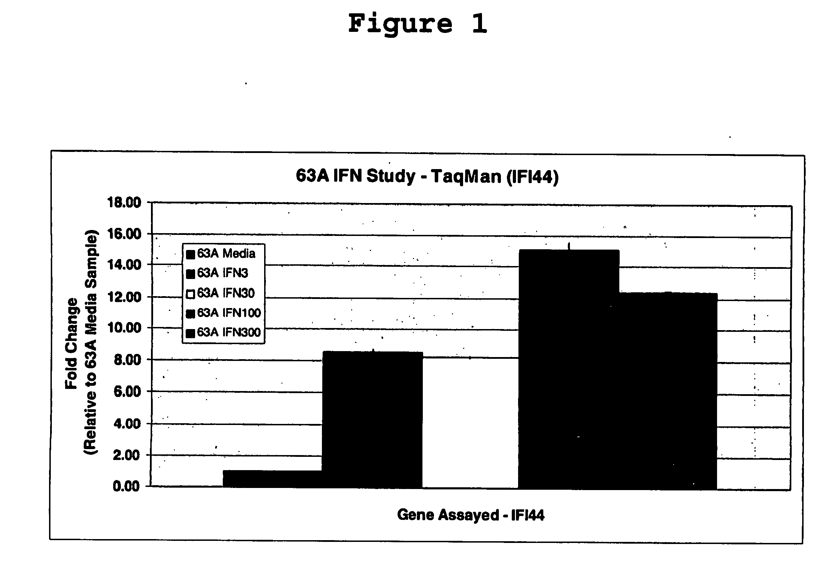

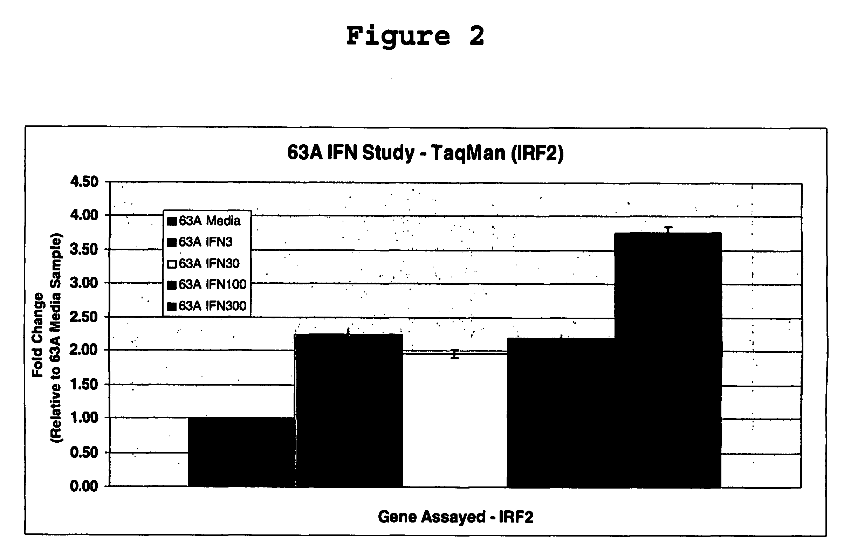

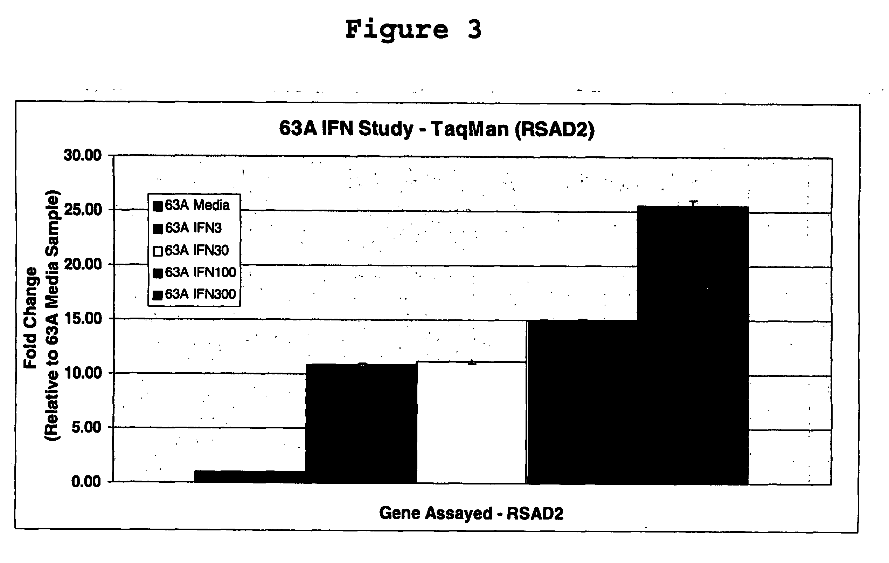

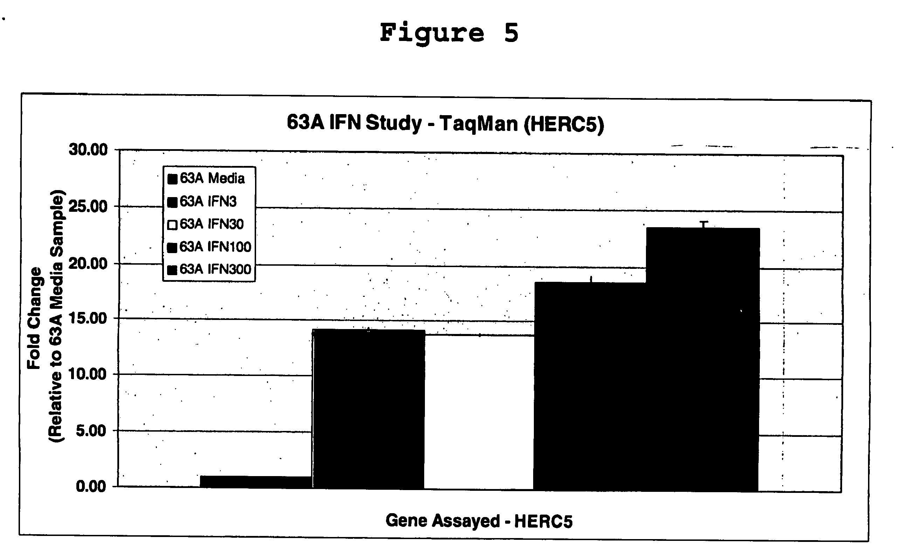

A study was performed to determine whether IFNα could stimulate expression of candidate PD markers in whole blood of healthy volunteers. Whole blood of healthy volunteers was collected in heparinized tubes, transferred to the appropriate wells of 6-well culture plates, and incubated with leukocyte IFN doses of 3, 30, 100, and 300 I.U. and then incubated for 4 hours at 37"C, 5% CO₂. Fold-induction of expression of candidate PD markers for genes IFI44, IRF2, RSAD2, GIP3, and HERC5 was determined using RNA isolated from PBMCs (Peripheral Blood Mononuclear Cells) with Qiagen's RNAeasy kit. As shown in Table 5 (IFI44 and IRF2), Table 6 (RSAD2), and Table 7 (G1P3 and HERC5) leukocyte IFN causes up-regulation in expression of each of these candidate PD markers. See also Figure 1 (IFI44), Figure 2 (IRF2), Figure 3 (RSAD2), Figure 4 (G1P3), and Figure 5 (HERC5) for a graphical analysis of these candidate PD marker expression results.

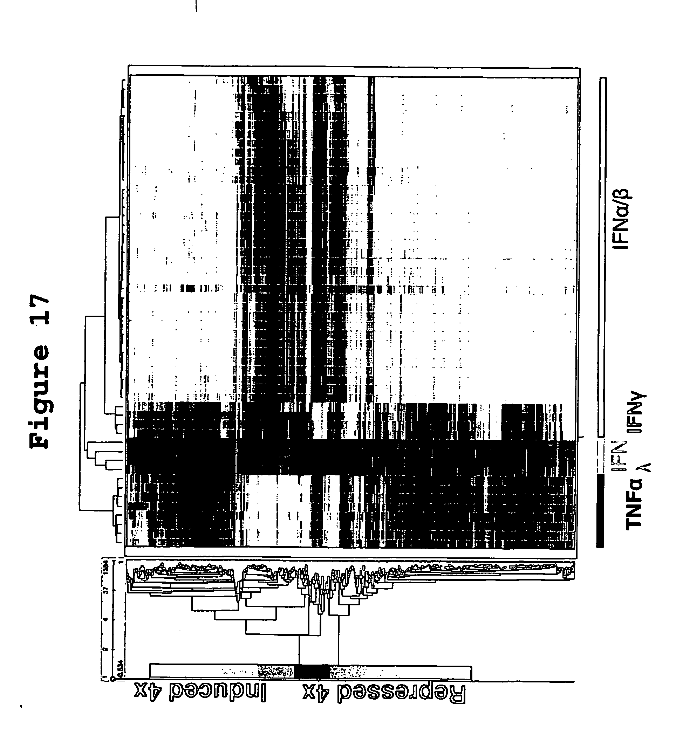

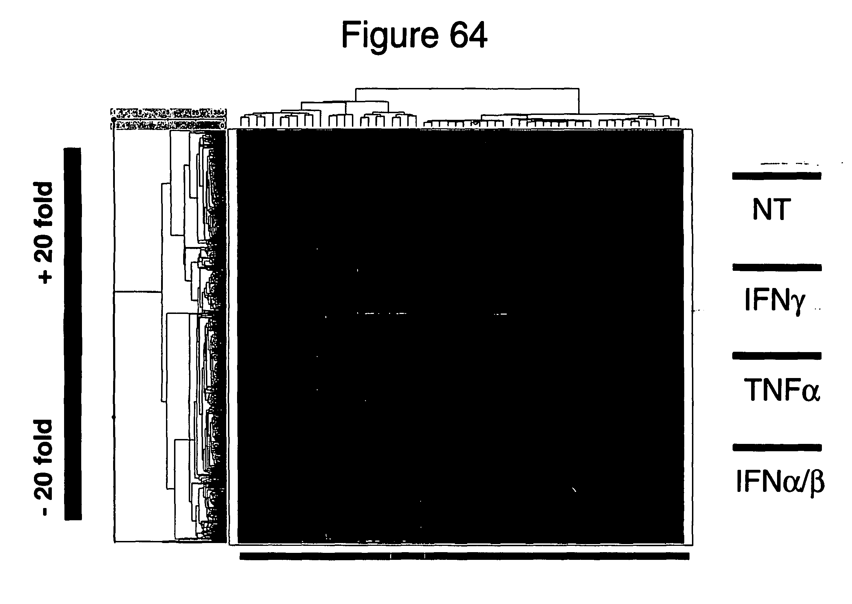

A summary hierarchical clustering of all samples using 1384 genes differentially regulated by IFN type 1, IFN type 2, or TNFα obtained from a separate experiment is shown in Figure 17. A heat map with a summary hierarchical clustering is also provided for 689 type I IFN inducible probe sets used on whole blood samples from healthy donors *ex vivo* stimulated with IFN type 1, IFN type 2, or TNFα. See figure 64.

**Table 5: Induced IFI44 and IRF2 Expression Following Leukocyte IFN Stimulation of Healthy Volunteer's Whole Blood**

| **Sample** | **Gene** | **Average FC** | **StDev** |
|---|---|---|---|
| **63A Media** | IFI44 | **1.00** | |
| **63A IFN3** | IFI44 | **8.58** | 0.16 |
| **63A IFN30** | IFI44 | **8.27** | 0.07 |
| **63A IFN100** | IFI44 | **15.12** | 0.50 |
| **63A IFN300** | IFI44 | **12.42** | 0.04 |
| | | | |

| **Sample** | **Gene** | **Average FC** | **StDev** |
|---|---|---|---|
| **63A Media** | IRF2 | **1.00** | |
| **63A IFN3** | IRF2 | **2.25** | 0.08 |
| **63A IFN30** | IRF2 | **1.96** | 0.06 |
| **63A IFN100** | IRF2 | **2.19** | 0.06 |
| **63A IFN300** | IRF2 | **3.75** | 0.10 |

**Table 6: Induced RSAD2 Expression Following Leukocyte IFN Stimulation of Healthy Volunteer's Whole Blood**

| **Sample** | **Gene** | **Average FC** | **StDev** |
|---|---|---|---|
| **63A Media** | RSAD2 | **1.00** | |
| **63A IFN3** | RSAD2 | **10.88** | 0.11 |
| **63A IFN30** | RSAD2 | **11.14** | 0.21 |
| **63A IFN100** | RSAD2 | **14.96** | 0.12 |
| **63A IFN300** | RSAD2 | **25.50** | 0.50 |

**Table 7: Induced G1P3 and HERC5 Expression Following Leukocyte IFN Stimulation of Healthy Volunteer's Whole Blood**

| **Sample** | **Gene** | **Average FC** | **StDev** |
|---|---|---|---|
| **63A Media** | G1P3 | **1.00** | |
| **63A IFN3** | G1P3 | **42.88** | 1.03 |
| **63A IFN30** | G1P3 | **25.76** | 0.10 |
| **63A IFN100** | G1P3 | **21.72** | 0.48 |
| **63A IFN300** | G1P3 | **16.02** | 0.06 |
| | | | |

| **Sample** | **Gene** | **Average FC** | **StDev** |
|---|---|---|---|
| **63A Media** | HERC5 | **1.00** | |
| **63A IFN3** | HERC5 | **14.17** | 0.12 |
| **63A IFN30** | HERC5 | **13.74** | 0.12 |
| **63A IFN100** | HERC5 | **18.51** | 0.58 |
| **63A IFN300** | HERC5 | **23.55** | 0.54 |

### Example 5. IFNα Ab Neutralizes IFNα-Induced Candidate PD Marker Expression in Healthy Volunteers' Whole Blood

### Source of Interferon= IFNα2a

Because IFNα treatment of healthy volunteers' whole blood induced expression of candidate PD markers, it was determined whether IFNα Ab, MEDI-545, could neutralize the induction of expression of these markers.

Blood was drawn from each of three donors into heparin tubes. Aliquots of 2.5 ml of drawn blood were added to each of 4 wells of 6- or 24-well treatment plates. The 4 wells were designated for treatment as follows: (a) blood + vehicle, (b) blood + 100 IU IFNα2a, (c) blood + 100 IU IFNα2a + MEDI-545 (IFNα Ab), and (d) blood + 100 IU IFNα2a + R347 (control Ab).

Wells containing blood to be treated with Ab were first incubated with either MEDI-545 (IFNα Ab; well (c)) or R347 (control Ab; well (d)) for 30 minutes. Following Ab treatment, vehicle (well (a)) or IFN α2a (wells (b), (c), and (d)) was added to the appropriate wells and was then incubated for an additional 4 hours at 37°C, 5% CO₂. The samples were then transferred to PAXgene tubes and incubated at room temperature for 2 hr. Following the 2 hr incubation the tubes were transferred to -80 °C for storage.

Following, at least, an overnight incubation at -80°C the total RNA of the cells was prepared according to the PAXgene protocol. First and second strand cDNA was prepared via Affy GRP methods and TaqMan was conducted on the cDNA samples.

Expression of at least 11 candidate PD markers, previously identified as up-regulated in lupus patients, could be neutralized by MEDI-545 in the IFNα2a-stimulated whole blood. See Table 8 (RAB8B), Table 9 (IRF7), Table 10 (MARCKS), Table 11 (IL6ST), Table 12 (LY6E), Table 13 (IFIT3), Table 14 (IFIT1), Table 15 (HERC5), Table 16 (OAS1), Table 17 (OAS3), and Table 18 (RSAD2), which provide quantitative gene expression analysis for each of these 11 genes in the whole blood of each of the 3 healthy volunteers.

**Table 8: IFN α2a-Induced RAB8B Gene Expression is Neutralized by MEDI-545**

| **Sample** | **Gene** | **Average** | **StDev** |
|---|---|---|---|
| **107 VEH** | RAB8B | **1.00** | |
| **107 IFN** | RAB8B | **3.45** | 0.31 |
| **107 IFN+545** | RAB8B | **1.30** | 0.04 |
| **107 IFN+R347** | RAB8B | **3.15** | 0.03 |
| | | | |
| **163 VEH** | RAB8B | **0.70** | 0.01 |
| **163 IFN** | RAB8B | **2.20** | 0.04 |
| **163 IFN+545** | RAB8B | **1.18** | 0.01 |
| **163 IFN+R3437** | RAB8B | **3.71** | 0.02 |
| | | | |
| **175 VEH** | RAB8B | **0.64** | 0.01 |
| **175 IFN** | RAB8B | **2.63** | 0.04 |
| **175 IFN+545** | RAB8B | **1.15** | 0.02 |
| **175 IFN+R347** | RAB8B | **2.51** | 0.05 |

**Table 9: IFN α2a-Induced IRF7 Gene Expression is Neutralized by MEDI-545**

| **Sample** | **Gene** | **Average** | **StDev** |
|---|---|---|---|
| **107 VEH** | IRF7 | **1.00** | |
| **107 IFN** | IRF7 | **18.53** | 3.32 |
| **107 IFN+545** | IRF7 | **3.42** | 0.33 |
| **107 IFN+R347** | IRF7 | **19.48** | 1.67 |
| | | | |
| **163 VEH** | IRF7 | **0.91** | 0.02 |
| **163 IFN** | IRF7 | **17.16** | 1.39 |
| **163 IFN +545** | IRF7 | **2.92** | 0.22 |
| **163 IFN+R3437** | IRF7 | **23.28** | 1.46 |
| | | | |
| **175 VEH** | IRF7 | **1.25** | 0.10 |
| **175 IFN** | IRF7 | **24.65** | 0.80 |
| **175 IFN+545** | IRF7 | **2.43** | 0.08 |
| **175 IFN+R347** | IRF7 | **26.34** | 8.61 |

**Table 10: IFN α2a-Induced MARCKS Gene Expression is Neutralized by MEDI-545**

| **Sample** | **Gene** | **Average** | **StDev** |
|---|---|---|---|
| **107 VEH** | MARCKS | **1.00** | |
| **107 FN** | MARCKS | **3.97** | 0.09 |
| **107 IFN+545** | MARCKS | **1.30** | 0.08 |
| **107 IFN+R347** | MARCKS | **2.99** | 0.10 |
| | | | |
| **163 VEH** | MARCKS | **0.56** | 0.01 |
| **163 IFN** | MARCKS | **2.59** | 0.12 |
| **163 IFN +545** | MARCKS | **1.55** | 0.05 |
| **163 IFN+R3437** | MARCKS | **4.42** | 0.07 |
| | | | |
| **175 VEH** | MARCKS | **0.41** | 0.01 |
| **175 IFN** | MARCKS | **2.59** | 0.06 |
| **175 IFN+545** | MARCKS | **0.55** | 0.02 |
| **175 IFN+R347** | MARCKS | **3.38** | 0.05 |

**Table 11: IFN α2a-Induced IL6ST Gene Expression is Neutralized by MEDI-545**

| **Sample** | **Gene** | **Average** | **StDev** |
|---|---|---|---|
| **107 VEH** | IL6ST | **1.00** | |
| **107 IFN** | IL6ST | **3.54** | 0.60 |
| **107 IFN+545** | IL6ST | **2.62** | 0.16 |
| **107 IFN+R347** | IL6ST | **8.19** | 0.54 |
| | | | |
| **163 VEH** | IL6ST | **2.50** | 0.58 |
| **163 IFN** | IL6ST | **7.69** | 0.47 |
| **163 IFN +545** | IL6ST | **4.18** | 0.44 |
| **163 IFN+R3437** | IL6ST | **13.24** | 0.12 |
| | | | |
| **175 VEH** | IL6ST | **1.37** | 0.09 |
| **175 IFN** | IL6ST | **7.62** | 0.56 |
| **175 IFN+545** | IL6ST | **2.95** | 0.38 |
| **175 IFN+R347** | IL6ST | **23.91** | 2.77 |

**Table 12: IFN α2a-Induced LY6E Gene Expression is Neutralized by MEDI-545**

| **Sample** | **Gene** | **Average** | **StDev** |
|---|---|---|---|
| **107 VEH** | LY6E | **1.00** | |
| **107 IFN** | LY6E | **19.09** | 0.03 |
| **107 IFN+545** | LY6E | **3.50** | 0.15 |
| **107 IFN+R347** | LY6E | **12.54** | 0.20 |
| | | | |
| **163 VEH** | LY6E | **1.02** | 0.04 |
| **163 IFN** | LY6E | **13.52** | 0.35 |
| **163 IFN +545** | LY6E | **4.80** | 0.18 |
| **163 IFN+R3437** | LY6E | **22.56** | 0.35 |
| | | | |
| **175 VEH** | LY6E | **1.61** | 0.15 |
| **175 IFN** | LY6E | **19.32** | 0.68 |
| **175 IFN+545** | LY6E | **3.74** | 0.00 |
| **175 IFN+R347** | LY6E | **15.57** | 0.44 |

**Table 13: IFN α2a-Induced IFIT3 Gene Expression is Neutralized by MEDI-545**

| **Sample** | **Gene** | **Average** | **StDev** |
|---|---|---|---|
| **107 VEH** | IFIT3 | **1.00** | |
| **107 IFN** | IFIT3 | **38.43** | 0.78 |
| **107 IFN+545** | IFIT3 | **6.78** | 0.14 |
| **107 IFN+R347** | IFIT3 | **42.59** | 0.75 |
| | | | |
| **163 VEH** | IFIT3 | **0.62** | 0.01 |
| **163 IFN** | IFIT3 | **25.94** | 0.57 |
| **163 IFN+545** | IFIT3 | **4.58** | 0.08 |
| **163 IFN+R3437** | IFIT3 | **44.83** | 0.44 |
| | | | |
| **175 VEH** | IFIT3 | **1.32** | 0.02 |
| **175 IFN** | IFIT3 | **35.02** | 0.48 |
| **175 IFN+545** | IFIT3 | **5.28** | 0.05 |
| **175 IFN+R347** | IFIT3 | **29.71** | 0.79 |

**Table 14: IFN α2a-Induced IFIT1 Gene Expression is Neutralized by MEDI-545**

| **Sample** | **Gene** | **Average** | **StDev** |
|---|---|---|---|
| **107 VEH** | IFIT1 | **1.00** | |
| **107 IFN** | IFIT1 | **80.21** | 3.44 |
| **107 IFN+545** | IFIT1 | **13.14** | 0.02 |
| **107 IFN+R347** | IFIT1 | **86.44** | 0.57 |
| | | | |
| **163 VEH** | IFIT1 | **0.92** | 0.03 |
| **163 IFN** | IFIT1 | **51.65** | 1.21 |
| **163 IFN+545** | IFIT1 | **7.60** | 0.05 |
| **163 IFN+R3437** | IFIT1 | **86.63** | 2.67 |
| | | | |
| **175 VEH** | IFIT1 | **1.47** | 0.17 |
| **175 IFN** | IFIT1 | **82.98** | 2.94 |
| **175 IFN+545** | IFIT1 | **8.40** | 0.24 |
| **175 IFN+R347** | IFIT1 | **58.50** | 1.47 |

**Table 15: IFN α2a-Induced HERC5 Gene Expression is Neutralized by MEDI-545**

| **Sample** | **Gene** | **Average** | **StDev** |
|---|---|---|---|
| **107 VEH** | HERC5 | **1.00** | |
| **107 IFN** | HERC5 | **41.12** | 2.87 |
| **107 IFN+545** | HERC5 | **6.29** | 0.49 |
| **107 IFN+R347** | HERC5 | **55.04** | 0.69 |
| | | | |
| **163 VEH** | HERC5 | **1.05** | 0.07 |
| **163 IFN** | HERC5 | **75.81** | 0.50 |
| **163 IFN+545** | HERC5 | **7.83** | 0.00 |
| **163 IFN+R3437** | HERC5 | **95.44** | 7.79 |
| | | | |
| **175 VEH** | HERC5 | **1.19** | 0.06 |
| **175 IFN** | HERC5 | **74.58** | 5.79 |
| **175 IFN+545** | HERC5 | **6.89** | 0.13 |
| **175 IFN+R347** | HERC5 | **98.15** | 19.40 |

**Table 16: IFN α2a-Induced OAS 1 gene Expression is Neutralized by MEDI-545**

| **Sample** | **Gene** | **Average** | **StDev** |
|---|---|---|---|
| **107 VEH** | OAS1 | **1.00** | |
| **107 IFN** | OAS1 | **15.11** | 4.27 |
| **107 IFN+545** | OAS1 | **3.45** | 1.03 |
| **107 IFN+R347** | OAS1 | **17.82** | 3.93 |
| | | | |
| **163 VEH** | OAS1 | **0.77** | 0.22 |
| **163 IFN** | OAS1 | **14.19** | 3.14 |
| **163 IFN +545** | OAS1 | **3.05** | 0.75 |
| **163 IFN+R3437** | OAS1 | **22.44** | 3.49 |
| | | | |
| **175 VEH** | OAS1 | **1.62** | 0.38 |
| **175 IFN** | OAS1 | **22.09** | 0.97 |
| **175 IFN+545** | OAS1 | **4.04** | 0.45 |
| **175 IFN+R347** | OAS1 | **15.22** | 4.48 |

**Table 17: IFN α2a-Induced OAS3 Gene Expression is Neutralized by MEDI-545**

| **Sample** | **Gene** | **Average** | **StDev** |
|---|---|---|---|
| **107 VEH** | OAS3 | **1.00** | |
| **107 IFN** | OAS3 | **49.04** | 13.74 |
| **107 IFN+545** | OAS3 | **7.03** | 0.84 |
| **107 IFN+R347** | OAS3 | **76.88** | 13.69 |
| | | | |
| **163 VEH** | OAS3 | **0.49** | 0.06 |
| **163 IFN** | OAS3 | **42.01** | 10.01 |
| **163 IFN +545** | OAS3 | **14.60** | 4.53 |
| **163 IFN+R3437** | OAS3 | **52.60** | 7.04 |
| | | | |
| **175 VEH** | OAS3 | **1.27** | 0.14 |
| **175 IFN** | OAS3 | **37.87** | 3.57 |
| **175 IFN+545** | OAS3 | **3.92** | 0.06 |
| **175 IFN+R347** | OAS3 | **34.91** | 2.07 |

**Table 18: IFN α2a-Induced RSAD2 Gene Expression is Neutralized by MEDI-545**

| **Sample** | **Gene** | **Average** | **StDev** |
|---|---|---|---|
| **107 VEH** | RSAD2 | **1.00** | |
| **107 IFN** | RSAD2 | **109.64** | 36.65 |
| **107 IFN+545** | RSAD2 | **9.88** | 0.32 |
| **107 IFN+R347** | RSAD2 | **107.32** | 35.38 |
| | | | |
| **163 VEH** | RSAD2 | **0.56** | 0.11 |
| **163 IFN** | RSAD2 | **71.47** | 21.17 |
| **163 IFN +545** | RSAD2 | **4.39** | 0.60 |
| **163 IFN+R3437** | RSAD2 | **114.51** | 28.63 |
| | | | |
| **175 VEH** | RSAD2 | **1.88** | 0.43 |
| **175 IFN** | RSAD2 | **126.27** | 22.95 |
| **175 IFN+545** | RSAD2 | **8.43** | 0.36 |
| **175 IFN+R347** | RSAD2 | **90.97** | 7.42 |

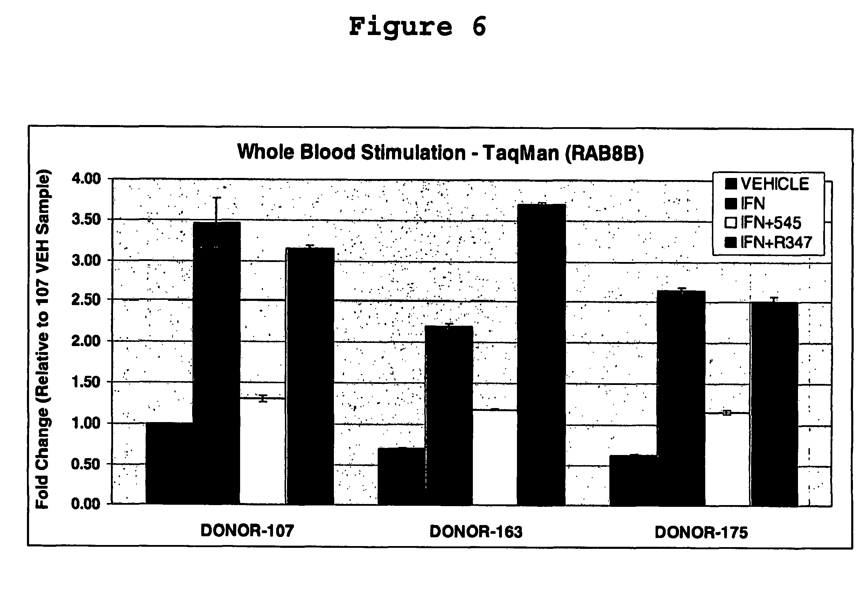

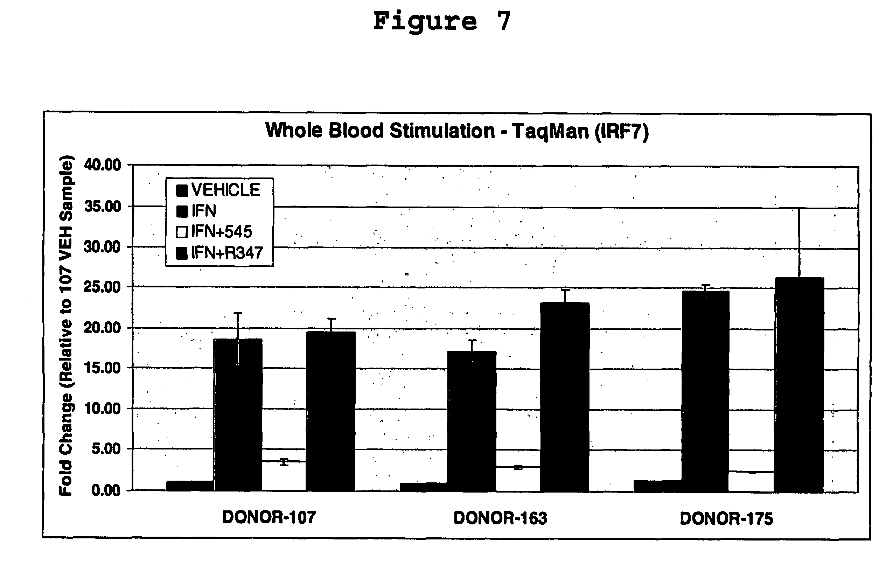

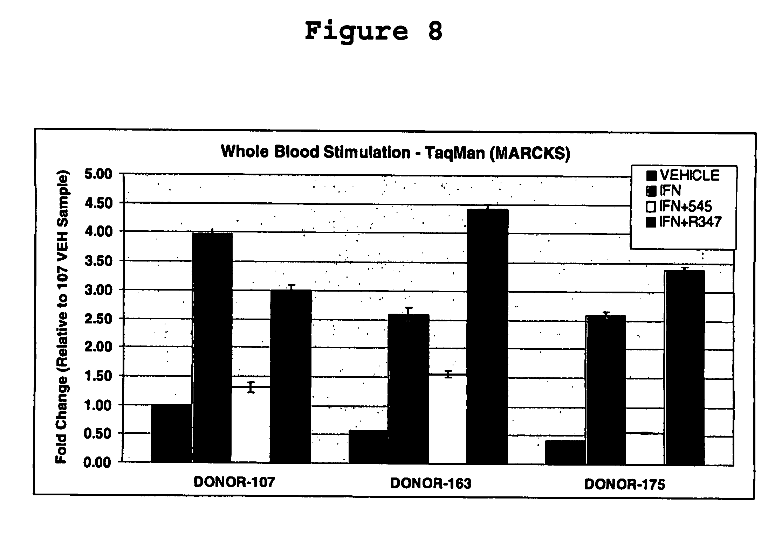

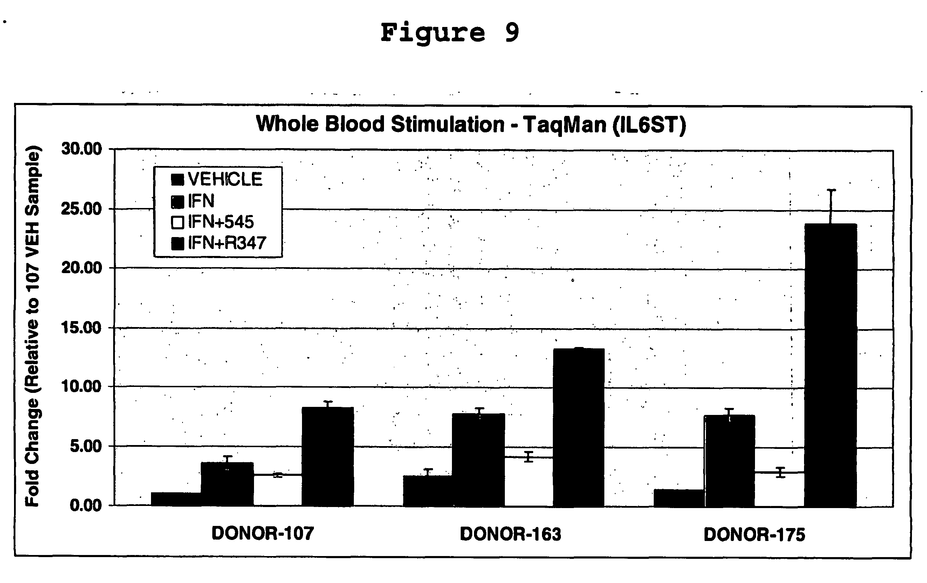

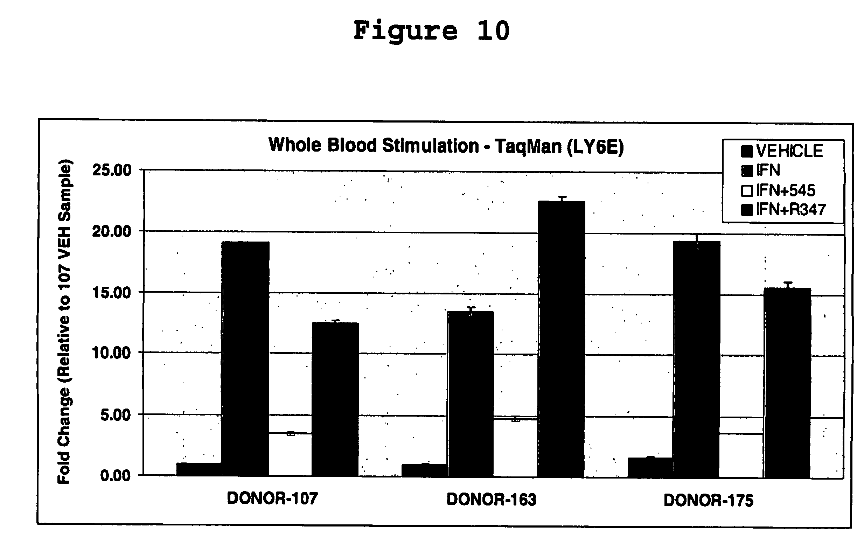

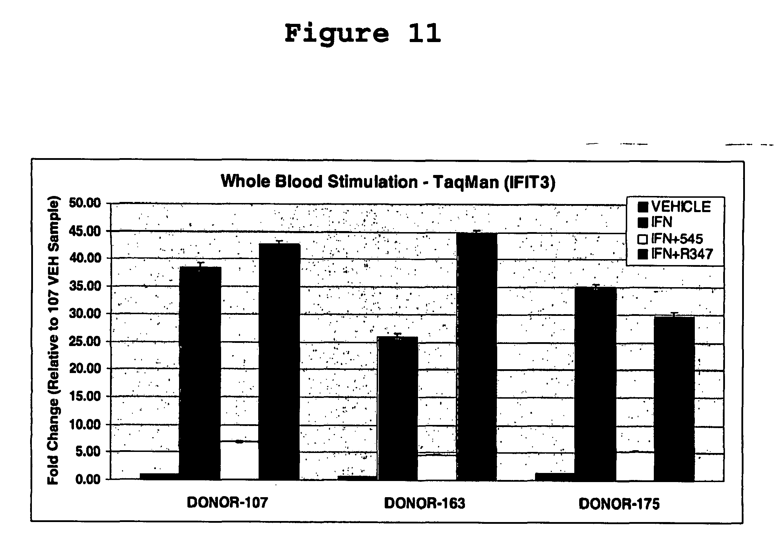

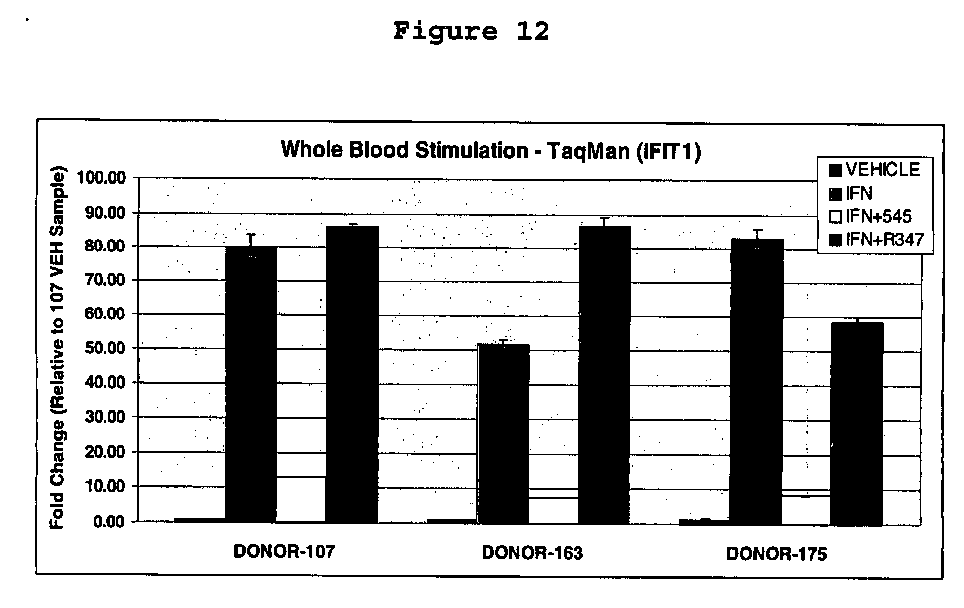

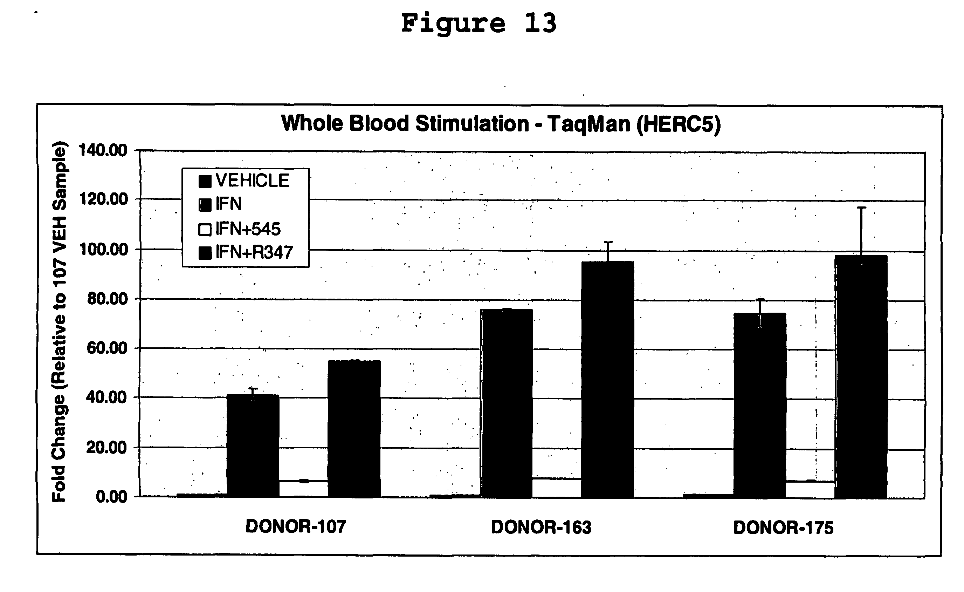

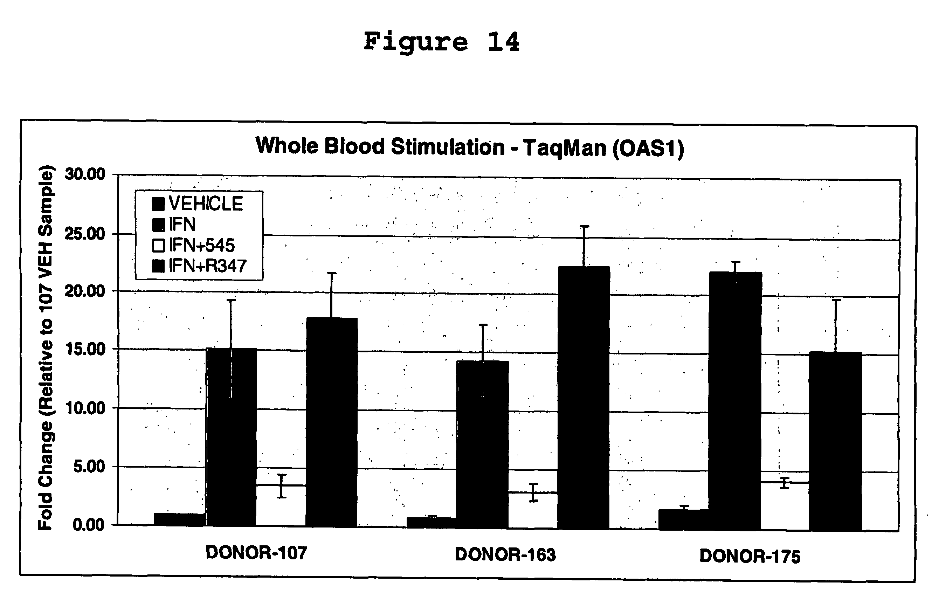

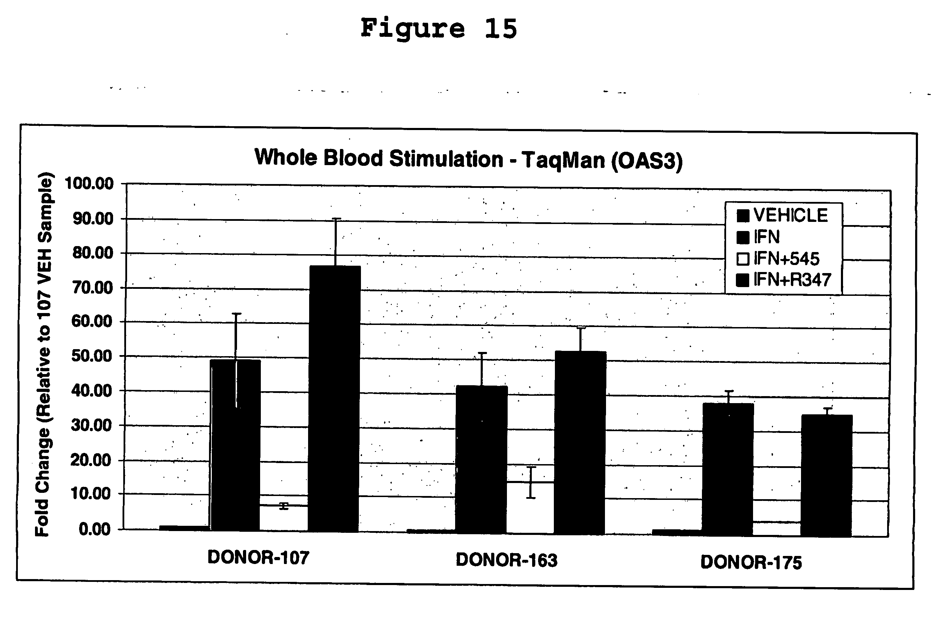

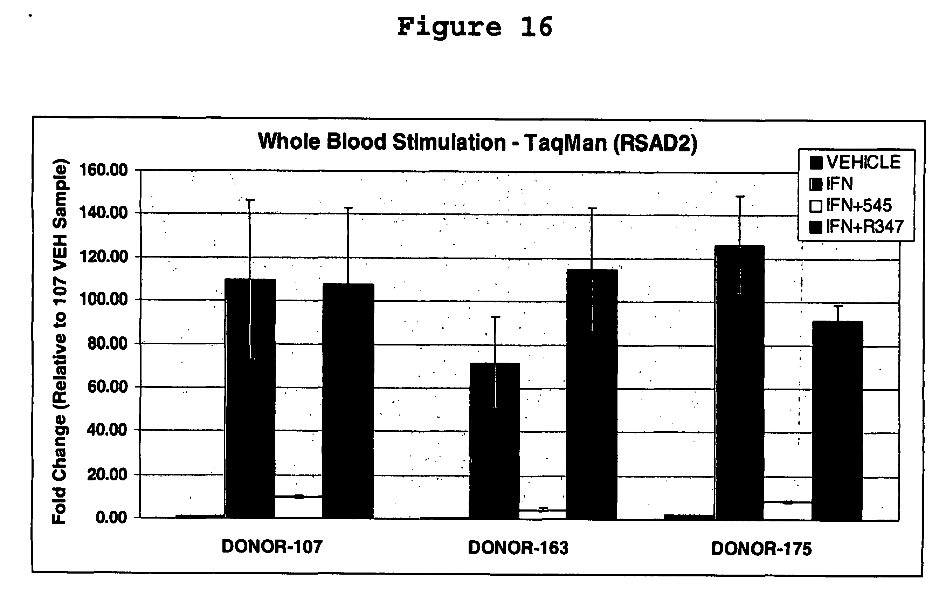

See also Figure 6 (RAB8B), Figure 7 (IRF7), Figure 8 (MARCKS), Figure 9 (IL6ST), Figure 10 (LY6E), Figure 11 (IFIT3), Figure 12 (IFIT1), Figure 13, (HERC5), Figure 14 (OAS1), Figure 15 (OAS3), and Figure 16 (RSAD2) for graphical representations of the gene expression data for each of the 11 genes.

### Source of Interferon=SLE patient serum

*(a)* Neutralization of type I IFN-induced genes by MEDI-545 could also be observed in whole blood of healthy volunteers that had been stimulated with serum obtained from lupus patients. Serum samples were obtained from SLE patients that had been tested in an IFN bioassay. Whole blood was collected from healthy donors in heparinized vacutainer tubes and PBMC were isolated using Ficoll gradient centrifugation method. PBMC were resuspended at 1x10⁷ cells/mL in RPMI media with 10% fetal bovine serum (FBS) and 125 µL of cells were aliquoted into each well of a 24 well flat bottom plate (1.25x106cells/well). Serum from SLE patients was preincubated for one hour with MEDI-545 (0.1, 1, 10 µg/mL), anti-IFN-γ antibody (1 µg/mL) or control antibody (10 µg/mL). SLE serum was added to the PBMC at a final concentration 25% (62.5 µL per well). Additional volume of RPMI + 10% FBS was added to the wells to obtain a final volume of 250 µL per well. Plates were incubated at 37°C for either 4 or 18 hours. Following the incubation, RNA was harvested by adding 750 µL of Trizol LS to each well. Samples were frozen at -70°C until the time of RNA isolation. Table 21 provides the MEDI-545 blockade of 74 type I IFN genes in healthy volunteers' whole blood stimulated *ex vivo* with SLE patient serum.

**Table 21: MEDI-545 blocks overexpression of type I IFN genes in whole blood of healthy volunteers stimulated ex vivo with lupus patient serum**

| Probe ID | D1_002_545.10 | D1_004_345.10 | D1_17021_545.10 | UniGene.ID | Gene.symbol |
|---|---|---|---|---|---|
| 219211_at | -3.1949 | -4.9995 | -4.0543 | Hs.38260 | USP18 |
| 217502_at | -3.1886 | -4.2648 | -3.0247 | Hs.437609 | IFIT2 |
| 218400_at | -3.1235 | -4.3204 | -3.9594 | Hs.528634 | OAS3 |
| 213797_at | -3.0752 | -3.3250 | -2.5795 | Hs.17518 | RSAD2 |
| 203153_at | -2.8862 | -4.6545 | -4.7890 | Hs.20315 | IFIT1 |
| 242625_at | -2.8104 | -2.9506 | -2.2214 | Hs.17518 | RSAD2 |
| 204747_at | -2.7900 | -3.6590 | -2.9676 | Hs.47338 | IFIT3 |
| 205483_s_at | -2.5237 | -2.9955 | -3.1568 | Hs.458485 | ISG15 |
| 204439_at | -2.5133 | -3.5887 | -3.5926 | Hs.389724 | IFI44L |
| 202145_at | -2.4809 | -3.0198 | -3.5950 | Hs.521903 | LY6E |
| 202869_at | -2.4582 | -3.5402 | -3.2304 | Hs.524760 | OAS1 |
| 235643_at | -2.4535 | -3.3586 | -2.9115 | Hs.489118 | SAMD9L |
| 219352_at | -2.4496 | -3.5983 | -3.8692 | Hs.529317 | HERC6 |
| 204415_at | -2.4417 | -2.5228 | -2.3149 | Hs.523847 | IFI6 |
| 219684_at | -2.4167 | -2.8965 | -2.1421 | Hs.43388 | RTP4 |
| 236156_at | -2.4160 | -2.5440 | -2.8885 | Hs.127445 | UPA |
| 205552_s_at | -2.3880 | -3.3679 | -2-7561 | Hs.524760 | OAS1 |
| 206133_at | -2.3139 | -3.0772 | -2.4787 | Hs.441975 | BIRC4BP |
| 214453_s_at | -2.2965 | -3.1707 | -3.3204 | Hs.82316 | IFI44 |
| 1556643_at | -2.2666 | -2.0429 | -1.7120 | Hs.515243 | LOC93343 |
| 228607_at | -2.2597 | -2.1659 | -2.3234 | Hs.414332 | OAS2 |
| 218943_s_at | -2.2563 | -2.4118 | -2.6600 | Hs.190622 | DDX58 |
| 242020_s_at | -2.2542 | -2.6436 | -1.7975 | Hs.302123 | ZBP1 |
| 204959_at | -2.2501 | -1.3731 | -1.5552 | Hs.153837 | MNDA |
| 226757_at | -2.2481 | -2.9288 | -2.3984 | Hs.437609 | IFIT2 |
| 219863_at | -2.2465 | -3.0980 | -3.8114 | Hs.26663 | HERC5 |
| 229450_at | -2.2281 | -3.2200 | -2.2151 | - | - |
| 214059_at | | -3.2929 | -3.5281 | Hs.82316 | IFI44 |
| 232517_s_at | -2.1925 | -2.2750 | -2.4569 | Hs.517180 | PRIC285 |
| 232666_at | -2.1925 | -1.9206 | -1.4938 | Hs.528634 | OAS3 |
| 230036_at | -2.1654 | -3.0256 | -2.4879 | Hs.489118 | SAMD9L |
| 227009_at | -2.1548 | -2.5608 | -1.1577 | Hs.546467 | EPSTI1 |
| 226702_at | -2.1420 | -3.0150 | -3.0155 | Hs.7155 | LOC129607 |
| 226603_at | -2.1183 | -2.8672 | -2.4103 | Hs.489118 | SAMD9L |
| 210397_at | -2.1095 | -0.5687 | -2.0322 | Hs.32949 | DEFB1 |
| 204994_at | -2.0685 | -3.2727 | -3.6132 | Hs.926 | MX2 |
| 202086_at | -2.0661 | -3.2741 | -3.6406 | Hs.517307 | MX1 |
| 228617_at | -2.0596 | -2.5832 | -2.3139 | Hs.441975 | BIRC4BP |
| 219364_at | -2.0583 | -2.3774 | -2.4651 | Hs.55918 | LGP2 |
| 209417_s_at | -2.0364 | -2.5262 | -2.4132 | Hs.632258 | IFI35 |
| 222154_s_at | -2.0330 | -2.4542 | -2.6425 | Hs.120323 | DNAPTP6 |
| 228230_at | -2.0323 | -2.9621 | -3.0255 | Hs.517180 | PRIC285 |
| 242234_at | -2.0161 | -3.0047 | -3.1633 | Hs.441975 | BIRC4BP |
| 219519_s_at | -2.0077 | -0.2596 | -3.1621 | Hs.31869 | SIGLEC1 |
| 207713_s_at | -1.9940 | -1.0134 | -1.6345 | Hs.247280 | C20orf18 |
| 218974_at | -1.8904 | -2.5122 | -2.5244 | Hs.445244 | FLJ10159 |
| 1552309_a_at | -1.8820 | -2.4284 | -2.7221 | Hs.632387 | NEXN |
| 210873_x_at | -1.8424 | -1.2891 | -1.2710 | Hs.348983 | APOBEC3A |
| 243277_at | -1.8388 | -2.2657 | -2.0595 | Hs.489118 | SAMD9L |
| 202411_at | -1.8385 | -0.1345 | -2.4757 | Hs.532634 | IFI27 |
| 222793_at | -1.8137 | -2.4540 | -2.6576 | Hs.190622 | DDX58 |
| 235276_at | -1.8007 | -2.6121 | -1.4780 | - | - |
| 203236_s_at | -1.7926 | -1.9069 | -2.6425 | Hs.81337 | LGALS9 |
| 225291_at | -1.7801 | -2.0167 | -2.4613 | Hs.388733 | PNPT1 |
| 44673_at | -1.7547 | -0.1337 | -2.3913 | Hs.31869 | SIGLEC1 |
| 213294_at | -1.7361 | -2.4393 | -2.5907 | Hs.546523 | - |
| 211122_s_at | -1.7296 | -3.0816 | -1.5743 | Hs.632592 | CXCL11 |
| 224701_at | -1.6827 | -1.7880 | -1.2356 | Hs.583792 | PARP14 |
| 230314_at | -1.6795 | -2.2159 | -2.3476 | Hs.112420 | - |
| 218986_s_at | -1.6648 | -2.1615 | -2.0204 | Hs.591710 | FLJ20035 |
| 205569_at | -1.6647 | -2.5741 | -2.6878 | Hs.516448 | LAMP3 |
| 219691_at | -1.6420 | -1.8434 | -1.8310 | Hs.65641 | SAMD9 |
| 204211_x_at | -1.6244 | -2.0612 | -2.3379 | Hs.131431 | EIF2AK2 |
| 220146_at | -1.6033 | -2.7419 | -1.7471 | Hs.443036 | TLR7 |
| 241916_at | -1.6026 | -1.5906 | -1.3802 | Hs.130759 | PLSCR1 |
| 229350_x_at | -1.5906 | -1.7395 | -1.3577 | Hs.348609 | PARP10 |
| 1555464_at | -1.5866 | -1.7397 | -1.3101 | Hs.163173 | IFIH1 |
| 204972_at | -1.5822 | -2.8402 | -2.8355 | Hs.414332 | OAS2 |
| 204698_at | -1.5277 | -1.5978 | -1.6553 | Hs.459265 | ISG20 |
| 203595_s_at | -1.4853 | -1.8724 | -1.5442 | Hs.252639 | IFIT5 |
| 220576_at | -1.4834 | -1.7834 | -1.0040 | Hs.229988 | PGAP1 |
| 1555491_a_at | -1.4739 | -1.0165 | -1.4991 | - | FLJ11286 |
| 1565752_at | -1.4418 | -0.0040 | -1.0835 | Hs.509664 | FGD2 |
| 203596_s_at | -1.4389 | -2.0356 | -1.9284 | Hs.252839 | IFIT5 |

Analysis of the genes uniquely activated at the 18 hour time point revealed upregulation of genes involved in the innate immune response (TLR, NFκB), adaptive immune response (NFAT, IL-1/IL-6), complement activation as well as leukocyte chemotaxis and adhesion. It is possible that neutralization of the type IFN pathway has the potential to modify downstream pathways that may significantly impact the pathogenesis of SLE.

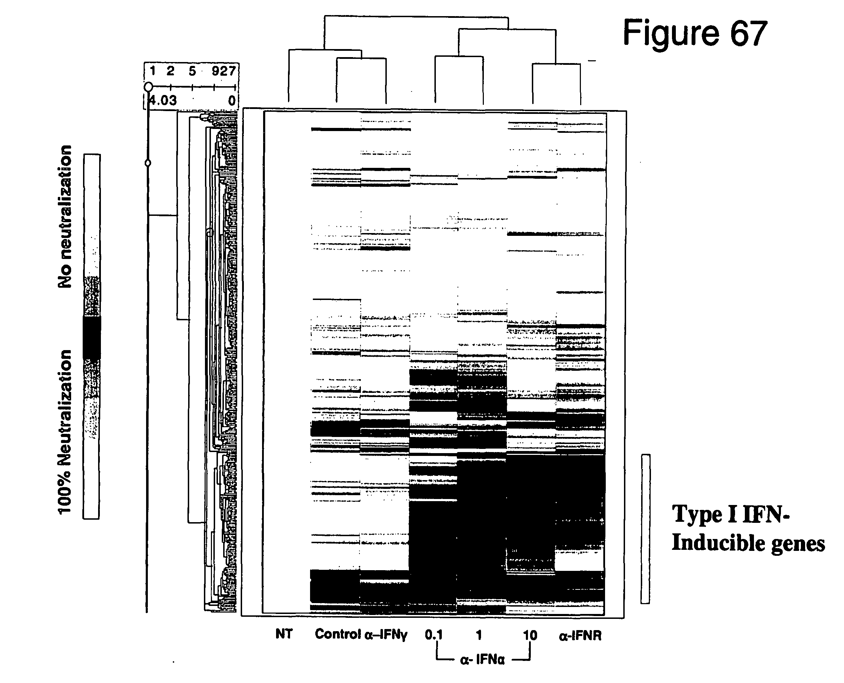

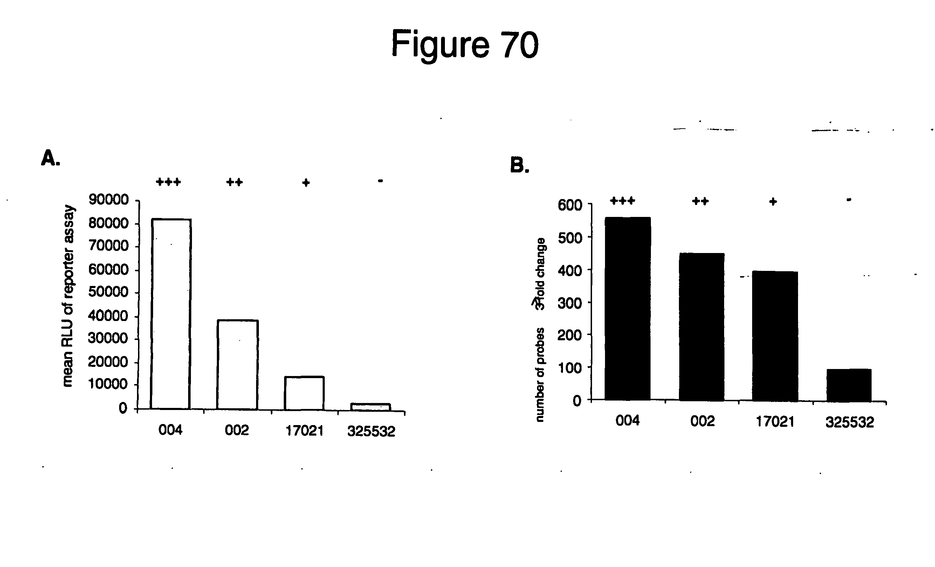

Heatmap analysis was also performed to examine induction of a type I IFN signature in PBMCs of a healthy donor by serum of an SLE patient and neutralization of the type I IFN signature by MEDI-545. See Figure 67. The anti-IFN-α mAb treatment (lanes 4-6) demonstrated strong neutralization of a large number of genes stimulated with the serum of an SLE patient. Furthermore, neutralization by the anti-IFN-α mAb was dose-dependent, which suggests that these genes could be good candidates for PD. The reference mAb itself inhibited the overexpression of some of the genes upregulated when challenged with SLE patient sera; some of these were identified as type I IFN-inducible genes. However, the effect of anti-IFN-α mAb was much broader, with strong neutralization observed in a large number of genes of which neither the reference mAb nor anti-IFN-γ mAb had any significant effect (lane 2; lanes 4-6). It should be noted that treatment with anti-IFN-αR mAb (lane 7) induced more neutralization than anti-IFN-α mAb, which suggests the presence of other type 1 IFN family members in the serum of the SLE patients, in addition to IFN-α. (b) Further investigation was conducted to identify early and late transcriptional responses in healthy donor PBMCs stimulated with SLE patient serum. In this study, four SLE patient serum samples, with varying levels of IFNα activity, were used to stimulate PBMCs isolated from a healthy donor. The varying levels of IFNα activity in the four SLE serum samples were determined in a luciferase reporter gene assay as described in Example 20. Briefly, HEK293H cells were stably transfected with a luciferase construct (*Gaussia princeps)* under the control of the IFN-stimulated response element (ISRE). Transfected cells were incubated with 50% patient sera and luciferase activity was detected in the culture supernatants 24 h later. Samples generating a signal greater than 1.5X negative control wells (normal human serum) were considered positive. To determine which class of type I IFN was responsible for the positive response, cells were treated with anti-type I and anti-type II IFN mAbs. Figure 70a shows the range of levels of type I IFN activity in each of the four SLE patient serum samples.

Each of the four SLE patient serum samples was co-incubated with PBMCs isolated from a healthy volunteer. The PBMCs from the healthy volunteer (previously determined to be IFN-signature negative) were isolated using Ficoll gradient centrifugation. Isolated PBMCs were incubated with 25% SLE patient serum or with 25% autologous patient serum (as a negative control). Following the incubation, cells were harvested with Trizol LS and stored at -70°C for RNA isolation. Total RNA was extracted and RNA purity and concentration were determined spectrophotometrically (260/280>1.9). The generation and hybridization of biotin-labeled amplified complementary RNA (cRNA) were conducted according to manufacturer's instructions (Affymetrix, Santa Clara, CA). Data was generated by implementing a 3-fold (up-regulation) expression cutoff between SLE serum stimulation compared to autologous serum control samples (q value ≤0.05). Figure 70b shows the number of probes detected as 3-fold or more upregulated in the healthy volunteer PBMCs by each of the four SLE patient serum samples. The number of probes detected as 3-fold or more upregulated by an SLE patient serum sample correspondingly increased with the level of type I IFN activity detected in the SLE serum sample.

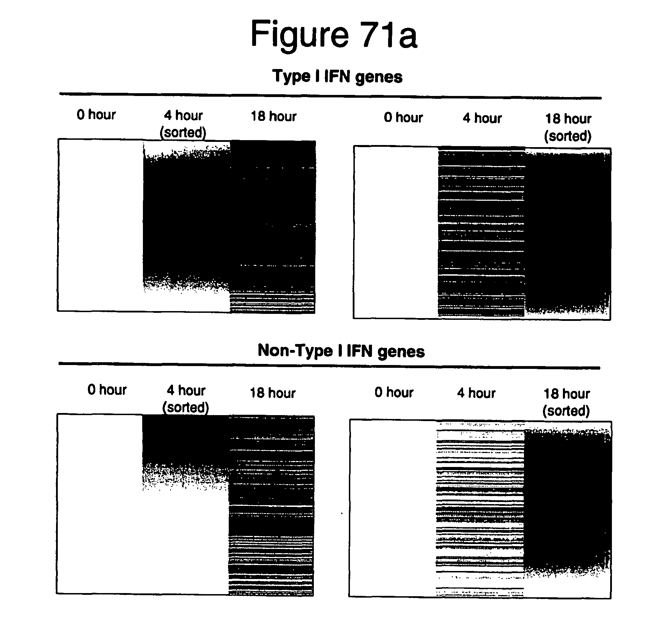

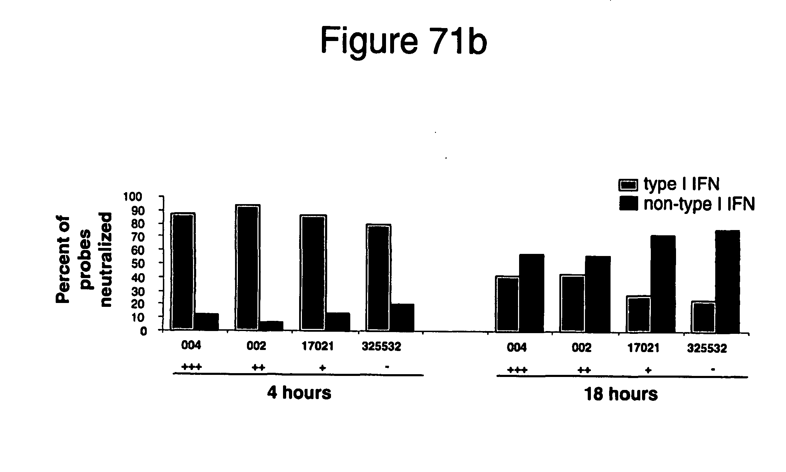

The role of type I IFNs in inducing the 3-fold or more upregulation of probes by the SLE patient serum samples was next investigated. PBMCs isolated from a healthy volunteer, discussed above, were incubated with 25% SLE patient serum in the presence or absence of neutralizing antibodies against IFN-α, or irrelevant mAb, for 4 or 18 hours. As a negative control, PBMC were incubated with 25% of autologous patient serum. Following the incubation, cells were harvested with Trizol LS and stored at -70°C for RNA isolation. Total RNA was extracted and RNA purity and concentration were determined spectrophotometrically (260/280>1.9). The generation and hybridization of biotin-labeled amplified complementary RNA (cRNA) were conducted according to manufacturer's instructions (Affymetrix, Santa Clara, CA). ArrayAssist® Lite software was used to calculate probe-level summaries from the array cell intensity files and R packages were used to identify differentially regulated genes (3-fold or greater upregulation in expression between SLE serum stimulation compared to autologous serum control samples (q value ≤ 0.05); R Development Core Team, New Zealand). Percent neutralization was then determined by calculating the percent change for each upregulated probe treated with and without anti-IFNα antibody. Figure 71a provides heat maps showing the percent neutralization of probes that were identified as upregulated following anti-IFNα treatment for type I IFN genes (689 probes) and non-type I IFN genes (probes induced by SLE serum outside of type I IFN gene list) 4 and 18 h post incubation. Figure 71b shows, for each of the four SLE patient serum samples, the percentage of type I IFN gene signature or non-type I IFN gene signature probes that were neutralized by the anti-IFNα treatment following both the 4 and 18 hour incubations. It appears that the majority of genes neutralized by anti-IFNα treatment of SLE serum-treated healthy volunteer's PBMCs 4 hours post-incubation were type I IFN genes, while the majority of genes neutralized by anti-IFNα treatment of SLE serum-treated healthy volunteer's PBMCs 18 hours post-incubation were non-type I IFN genes.

Genes, whether type I IFN genes or non-type I IFN genes, that were both upregulated and neutralized by anti-IFNα treatment at 18 hours, but that were not upregulated at 4 hours (i.e., "unique genes") were identified for each SLE patient serum sample. Figure 72 provides the (a) type I IFN genes and (b) non-type I IFN genes that were identified as unique genes. Shaded areas indicate greater than 50% neutralization by anti-IFNα in that patient sample.

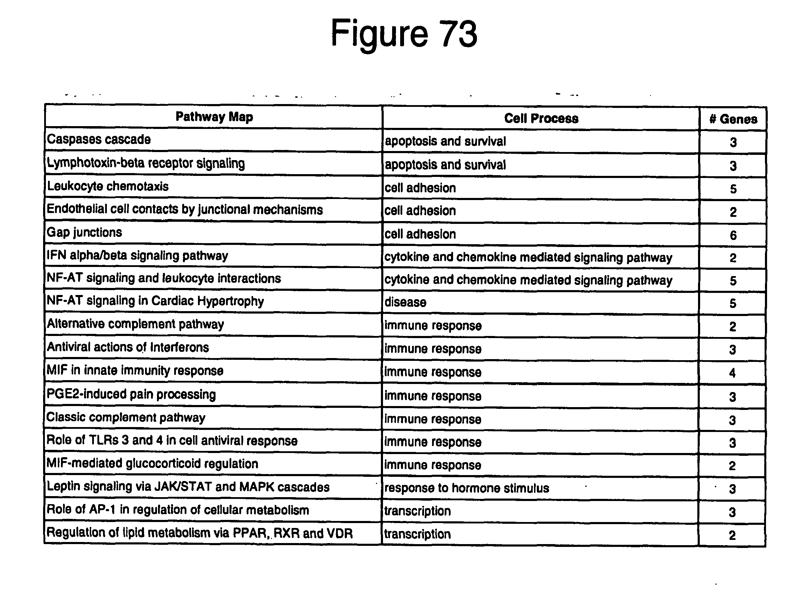

Cell pathways and processes neutralized by anti-IFNα treatment at the 18 hr time point are involved in cytokine and chemokine signaling pathways, immune regulation, cell adhesion, and cell survival. See Figure 73, which provides a table showing the pathway analysis of altered genes and proteins at the 18 hr time point. Pathways highlighted in yellow were also significantly altered in SLE serum samples. The cell pathways and processes neutralized by anti-IFNα treatment at the 18 hr time point were analyzed with the MetaCore integrated software suite from GeneGo, Inc. using the identified unique genes. Only pathways with p-values ≤ 0.05 were considered significant. The pathways shown were altered in at least 2 out of 4 SLE serum samples.

### Example 6: Administering MEDI-545 to Lupus Patients Neutralizes the IFNα-Inducible Candidate PD Marker Expression Pattern

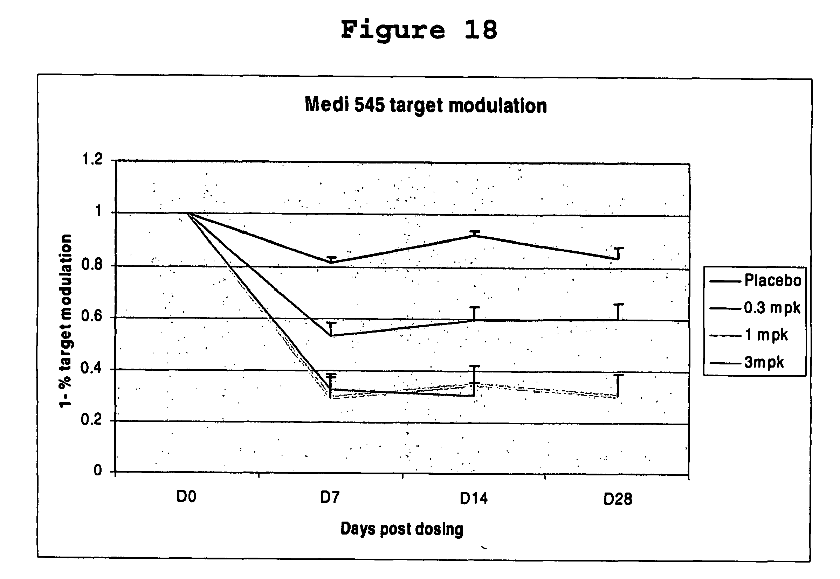

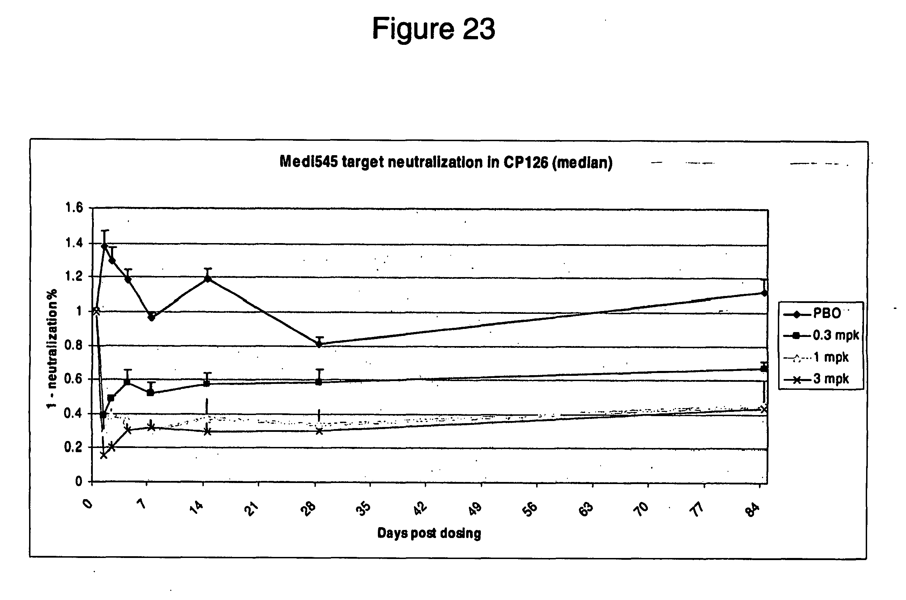

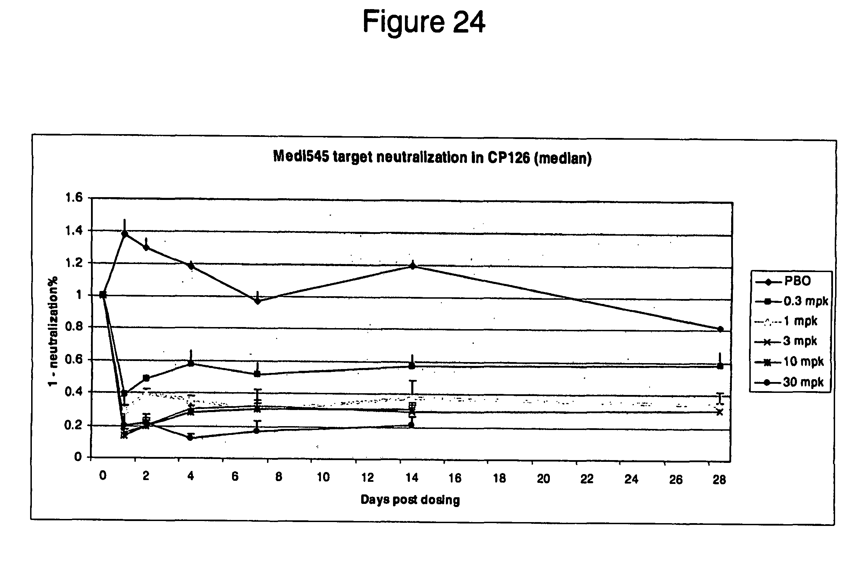

Whole blood of lupus patients receiving placebo, 0.3 mg/kg, 1.0 mg/kg, and 3.0 mg/kg MEDI-545 were analyzed for expression of IFNα-inducible PD markers over the course of 28 days. Whole blood (∼2.5 mL) was drawn into PAXgene RNA tubes and processed as outlined above. With increasing doses of MEDI-545, up-regulated expression of the top 25 PD markers was neutralized. See Figure 18, Figure 23, and Figure 24 which provide graphical representations of neutralization of these top 25 PD markers following administration of varying concentrations of the MEDI-545 IFNα Ab over various lengths of time. The top 25 PD markers measured in this study are provided in Table 19.

**Table 19: Top 25 IFN-Induced PD Markers in Lupus Patients**

| Probe ID | UniGene ID | Gene Title | Gene Symbol |
|---|---|---|---|
| 202086_at | Hs.517307 | myxovirus (influenza virus) resistance 1, interferon-inducible protein p78 (mouse) /// myxovirus (influenza virus) resistance 1, interferon-inducible protein p78 (mouse) | MX1 |
| 202145_at | Hs.521903 | lymphocyte antigen 6 complex, locus E | LY6E |
| 202411_at | Hs.532634 | interferon, alpha-inducible protein 27 | IFI27 |
| 202869_at | Hs.524760 | 2',5'-oligoadenylate synthetase 1, 40/46kDa | OAS1 |
| 203153_at | Hs.20315 | interferon-induced protein with tetratricopeptide repeats 1 /// interferon-induced protein with tetratricopeptide repeats 1 | IFIT1 |
| 204415_at | Hs.523847 | interferon, alpha-inducible protein 6 | IFI6 |
| 204439_at | Hs.389724 | interferon-induced protein 44-like | IFI44L |
| 205483_s_at | Hs.458485 | ISG15 ubiquitin-like modifier | ISG15 |
| 205569_at | Hs.518448 | lysosomal-associated membrane protein 3 | LAMP3 |
| 205660_at | Hs.118633 | 2'-5'-oligoadenylate synthetase-like | OASL |
| 213797_at | Hs.17518 | radical S-adenosyl methionine domain containing 2 | RSAD2 |
| 214059_at | Hs.82316 | Interferon-induced protein 44 | IFI44 |
| 217502_at | Hs.437609 | interferon-induced protein with tetratricopeptide repeats 2 | IFIT2 |
| 218400_at | Hs.528634 | 2'-5'-oligoadenylate synthetase 3, 100kDa | OAS3 |
| 219211_at | Hs.38260 | ubiquitin specific peptidase 18 | USP18 |
| 219519_s_at | Hs.31869 | sialic acid binding Ig-like lectin 1, sialoadhesin /// sialic acid binding Ig-like lectin 1, sialoadhesin | SIGLEC1 |
| 219863_at | Hs.26663 | hect domain and RLD 5 | HERC5 |
| 222154_s_at | Hs.120323 | DNA polymerase-transactivated protein 6 | DNAPTP6 |
| 226702_at | Hs.7155 | hypothetical protein LOC129607 | LOC129607 |
| 227609_at | Hs.546467 | epithelial stromal interaction 1 (breast) | EPSTI1 |
| 229450_at | --- | --- | --- |
| 235276_at | --- | --- | --- |
| 239979_at | Hs.546467 | Epithelial stromal interaction 1 (breast) | EPSTI1 |
| 242234_at | Hs.441975 | XIAP associated factor-1 | BIRC4BP |
| 44673_at | Hs.31869 | sialic acid binding Ig-like lectin 1, sialoadhesin | SIGLEC1 |

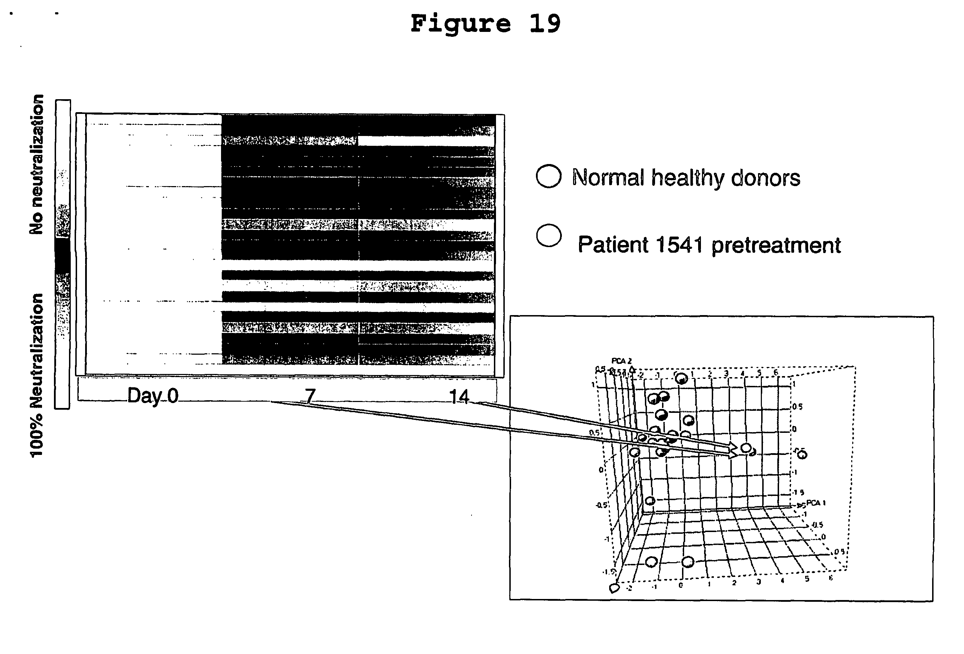

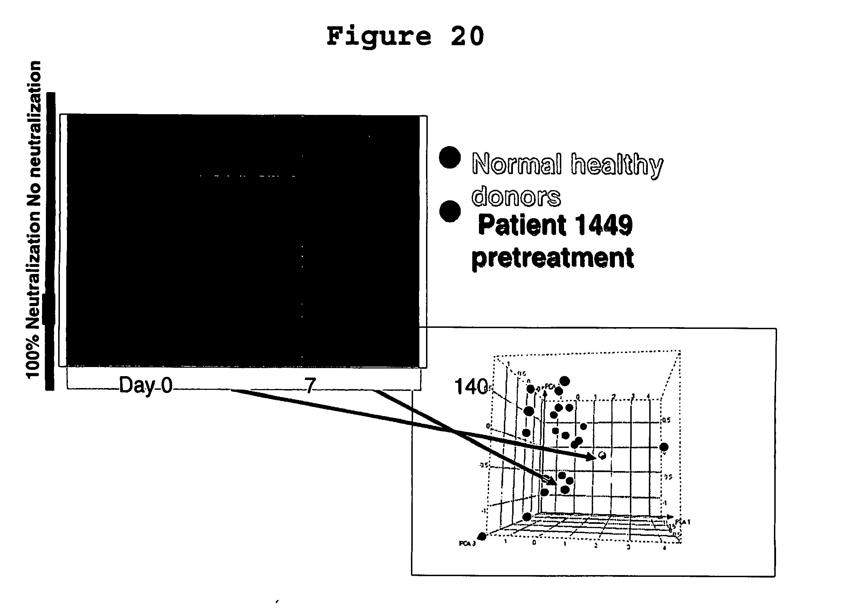

The neutralization of IFN-induced PD markers by MEDI-545 for several individual lupus patients was examined and is presented in Figures 19-21. Figures 19 and 20 are heatmaps showing the neutralization of the top 25 PD markers (see Table 19) for two individual lupus patients (Figure 19, patient 1541; and Figure 20, patient 1449). Each of these lupus patients received 3 mg/kg MEDI-545. Each exhibited neutralization of the top 25 inducible PD markers at 7 and 14 days post-MEDI-545 treatment.

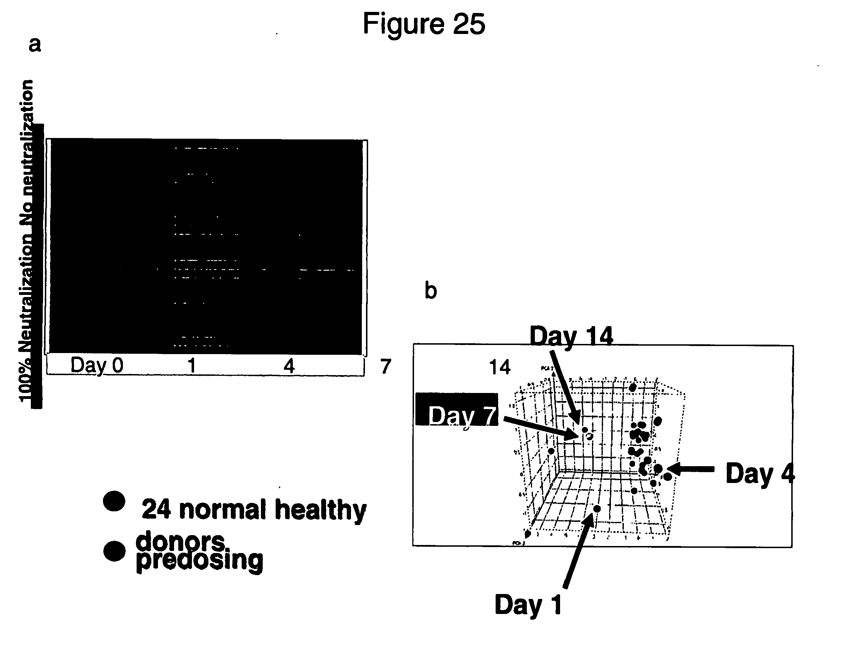

Neutralization of the top 25 type I IFN inducible genes in whole blood of an SLE patient treated with high dose (30 mg/kg) MEDI-545 was also examined. A heatmap of neutralization of the top 25 type I IFN inducible genes at 1, 4, 7, and 14 days following administration of MEDI-545 is presented in Figure 25(a). Neutralization of all genes can be seen following administration of MEDI-545. Figure 25(b) is a PCA of target modulation based on the top 25 type I IFN inducible genes. The PCA diagram shows the progression of the treated SLE patient from a position directly opposite that of normal healthy donors prior to administration of MEDI-545 to a position where it clusters with the healthy donors after administration of MEDI-545.

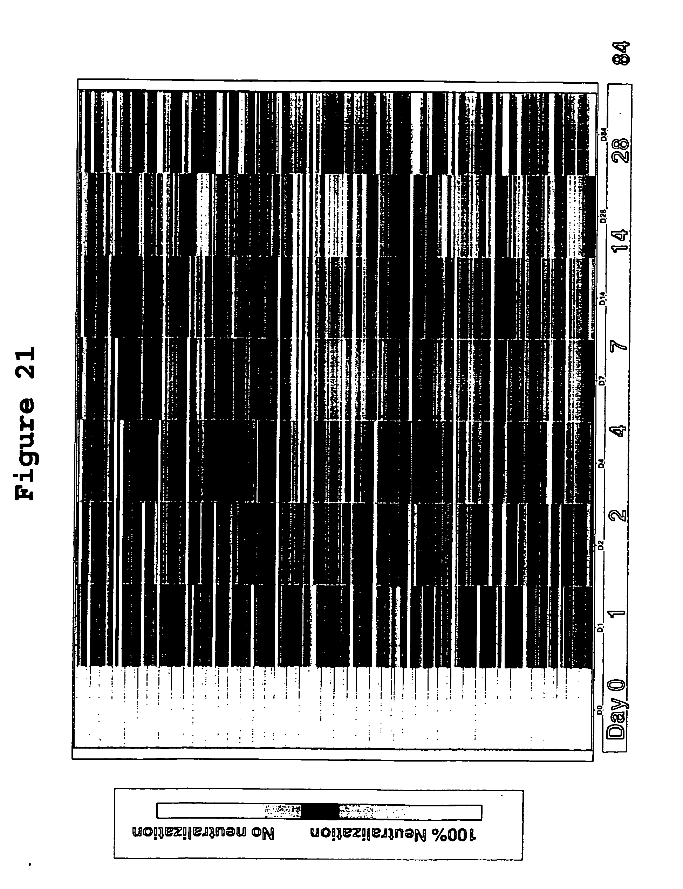

The neutralization of 165 PD markers by MEDI-545 was examined in a further lupus patient dosed with a lower, 0.3 mg/kg dose, of Ab. See Figure 21. MEDI-545 neutralized most of the 165 candidate PD markers in this lupus patient. The 165 candidate PD markers are shown as the first 165 entries of Table 20.

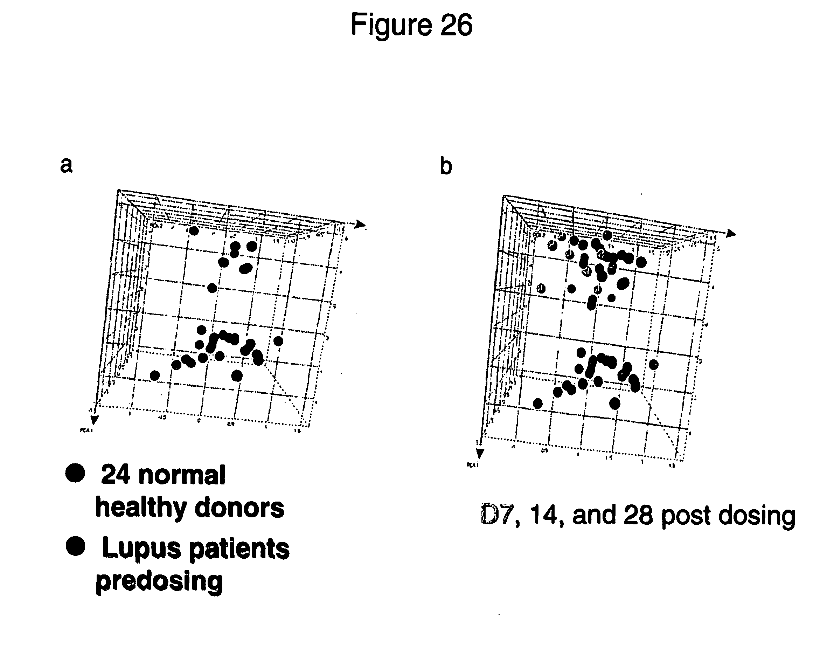

The neutralization of type I IFN inducible probes sets was not observed in SLE patients treated with placebo control. Compare PCA plots of SLE patients before (a) and after (b) dosing with placebo in Figure 26. Thus, the neutralization of the type-I IFN PD markers was due to the MEDI-545 antibody.

Table 22 provides a list of the 63 type I IFN inducible probes upregulated in whole blood of lupus patients and neutralized by MEDI-545 or placebo by at least 30% at day 7, day 14, or day 28 post administration. Each set of columns provides neutralization data for each of the indicated genes at 7, 14, and 28 days post-administration. The first set of columns provides percentage neutralization of each of the indicated genes for lupus patients having a type I IFN signature and that were treated with MEDI-545. It can be noted that for each of the indicated genes, neutralization ranged from 30% to 68% at day 7 post-administration. Meanwhile, at day 7 in the placebo treated group, neutralization of the same genes ranged from 0% to 27%.

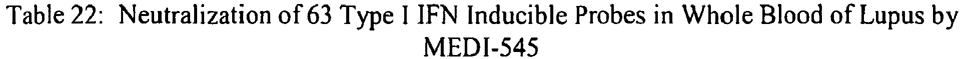

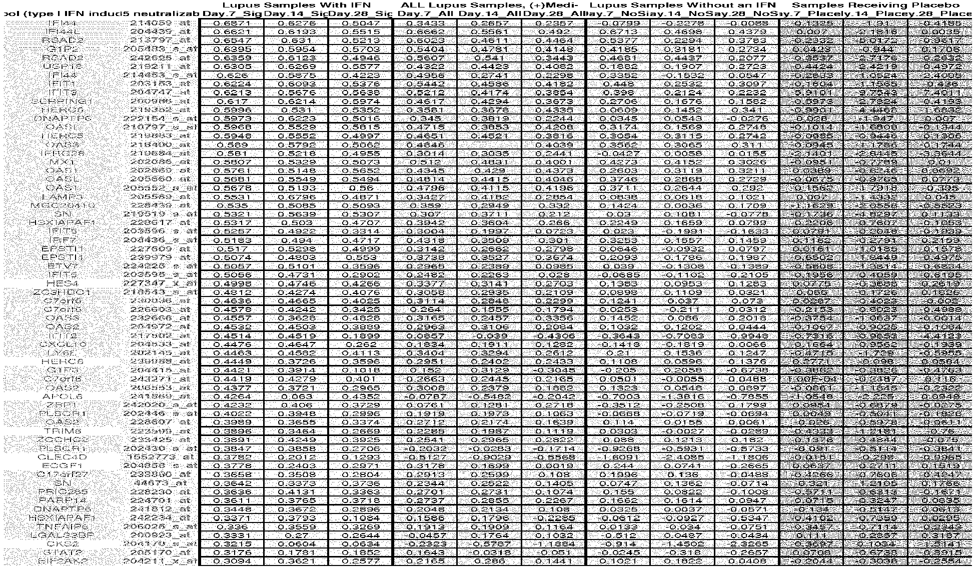

**Table 33 provides the results of a separate study which determined the top 50 genes neutralized in SLE patient whole blood 7 days after MEDI-545 treatment. Only three genes of the 50 genes, ZCCHC2, REC8L1, and GCLM, were not IFN-α/β-inducible Table 33: Top 50 probes neutralized 7 days post-dose in SLE patients receiving MEDI-545 treatment**

| **Probe ID** | **UniGene ID** | **Gene Title** | **Gene Symbol** | **Avg Probe Rank (By % Neutralization)** | **Final Probe Rank** |
|---|---|---|---|---|---|
| 219352_at | Hs.529317 | hect domain and RLD 6 | HERC6 | 2277.0 | 1 |
| 208436_s_at | Hs.166120 | interferon regulatory factor 7 | IRF7 | 2497.5 | 2 |
| 210797_s_at | Hs.110633 | 2 5 oligoadenylate synthetase-like | OASL | 2708.2 | 3 |
| 205483_s_at | Hs.458485 | ISG15 ubiquitin like modifier | ISG15 | 2735.7 | 4 |
| 204439_at | Hs.389724 | interferon induced protein 44 like | IFI44L | 3194.9 | 5 |
| 219211_at | Hs.38260 | ubiquitin specific peptidase 18 | USP18 | 3458.6 | 6 |
| 218543_s_at | Hs.12646 | poly (ADP ribose) polymerase family, member 12 | PARP12 | 3472.3 | 7 |
| 205241_at | Hs.567405 | SCO cytochrome oxidase deficient homolog 2 (veast) | SCO2 | 3825.7 | 8 |
| 204747_at | Hs.47338 | interferon induced protein with tetratricopeptide repeats 3 | IFIT3 | 3987.2 | 9 |
| 219519_s_at | Hs.31869 | sialic acid binding Ig like lectin 1 sialoadhesin | SIGLEC1 | 4207.2 | 10 |
| 228230_at | Hs.517180 | peroxisomal proliferator activated receptor A interacting complex 285 | PRIC285 | 4373.1 | 11 |
| 235276_at | --- | --- | --- | 4438.7 | 12 |
| 214059_at | Hs.82316 | Interferon induced protein 44 | IFI44 | 4477.4 | 13 |
| 222154s_at | Hs.120323 | DNA polymerase transactivated protein 6 | DNAPTP6 | 4531.3 | 14 |
| 202145_at | Hs.521903 | lymphocyte antigen 6 complex locus E | LY6E | 4618.6 | 15 |
| 223849_s_at | Hs.514941 | Mov10 Moloney leukemia virus 10 homolog (mouse) | MOV10 | 4691.0 | 16 |
| 219364_at | Hs.55918 | likely ortholog of mouse D11Igp2 | LGP2 | 4717.4 | 17 |
| 224503_s_at | Hs.114191 | zinc finger, CCHC domain containing | ZCCHC2 | 4926.5 | 18 |
| 228617_at | Hs.441975 | XIAP associated factor 1 | BIRC4BP | 4942.0 | 19 |
| 53720_at | --- | hypothetical protein FLJ11286 | FLJ11286 | 5046.2 | 20 |
| 218400_at | Hs.528634 | 2 5 oligoadenylate synthetase 3 100kDa | OAS3 | 5136.7 | 21 |
| 235508_at | Hs.526464 | promyelocytic leukemia | PML | 5328.7 | 22 |
| 232155_at | Hs.514554 | KIAA1618 | KIAA1618 | 5344.5 | 23 |
| 202086_at | Hs.517307 | myxovirus (influenza virus) resistance 1 interferon inducible protein p78 (mouse) | MX1 | 5484.4 | 24 |
| 242625_at | Hs.17518 | radical S adenosyl methionine domain containing 2 | RSAD2 | 5522.4 | 25 |
| 209417_s_at | Hs.632258 | interferon induced protein 35 | IFI35 | 5529.4 | 26 |
| 228439_at | Hs.124840 | basic leucine zipper transcription factor ATF like 2 | BATF2 | 5563.0 | 27 |
| 221766_s_at | Hs.10784 | family with sequence similarity 46 member A | FAM46A | 5607.1 | 28 |
| 202446_s_at | Hs.130759 | phospholipid scramblase 1 | PLSCR1 | 5911.3 | 29 |
| 205660_at | Hs.118633 | 2 5 oligoadenylate synthetase like | OASL | 6001.3 | 30 |
| 205875_s_at | Hs.344812 | three prime repair exonuclease 1 | TREX1 | 6062.0 | 31 |
| 34689_at | Hs.344812 | three prime repair exonuclease 1 | TREX1 | 6097.0 | 32 |
| 202869_at | Hs.524760 | 2 5 oligoadenylate synthetase 1, 40/46kDa | OAS1 | 6101.5 | 33 |
| 214453_s_at | Hs.82316 | interferon-induced protein 44 | IFI44 | 6210.4 | 34 |
| 1555491_a_at | --- | hypothetical protein FLJ11286 | FLJ11286 | 6235.7 | 35 |
| 230036_at | Hs.489118 | sterile alpha motif domain containing 9 like | SAMD9L | 6254.1 | 36 |
| 222217s_at | Hs.438723 | solute carrier family 27 (fatty acid transporter) member 3 | SLC27A3 | 6257.9 | 37 |
| 201641_at | Hs.118110 | bone marrow stromal cell antigen 2 | BST2 | 6371.4 | 38 |
| 218599_at | Hs.419259 | REC8-like 1 (yeast) | REC8L1 | 6423.3 | 39 |
| 230327_at | Hs.531314 | glutamate-cysteine ligase, modifier subunit | GCLM | 6500.4 | 40 |
| 225291_at | Hs.388733 | polyribonucleotide nucleotidyltransferase 1 | PNPT1 | 6537.6 | 41 |
| 208581_x_at | Hs.374950 | metallothionein 1X | MT1X | 6541.4 | 42 |
| 212380_at | Hs.520102 | KIAA0002 | KIAA0082 | 6547.1 | 43 |
| 227347_x_at | Hs.154029 | hairy and enhancer of split 4 (Drosophila) | HES4 | 6557.3 | 44 |
| 1557116_at | Hs.257352 | apolipoprotein L 6 | APOL6 | 6571.1 | 45 |
| 231769_at | Hs.464419 | F box protein 6 | FBX06 | 6683.0 | 46 |
| 200986_at | Hs.384598 | serpin peptidase inhibitor clade G (C1 inhibitor) member 1 (angioedema hereditary) | SERPING1 | 6688.7 | 47 |
| 33304_at | Hs.459265 | interferon stimulated exonuclease gene 20kDa | ISG20 | 6853.8 | 48 |
| 209593_s_at | Hs.252682 | torsin family 1 member B (torsin D) | TOR1B | 6866.4 | 49 |
| 202307_s_at | Hs.352018 | transporter 1 ATP binding cassette sub family B (MDR/TAP) | TAP1 | 6909.0 | 50 |

### Example 7: The Majority of Lupus Patients Exhibit a Type I IFN-Inducible PD Marker Expression Pattern

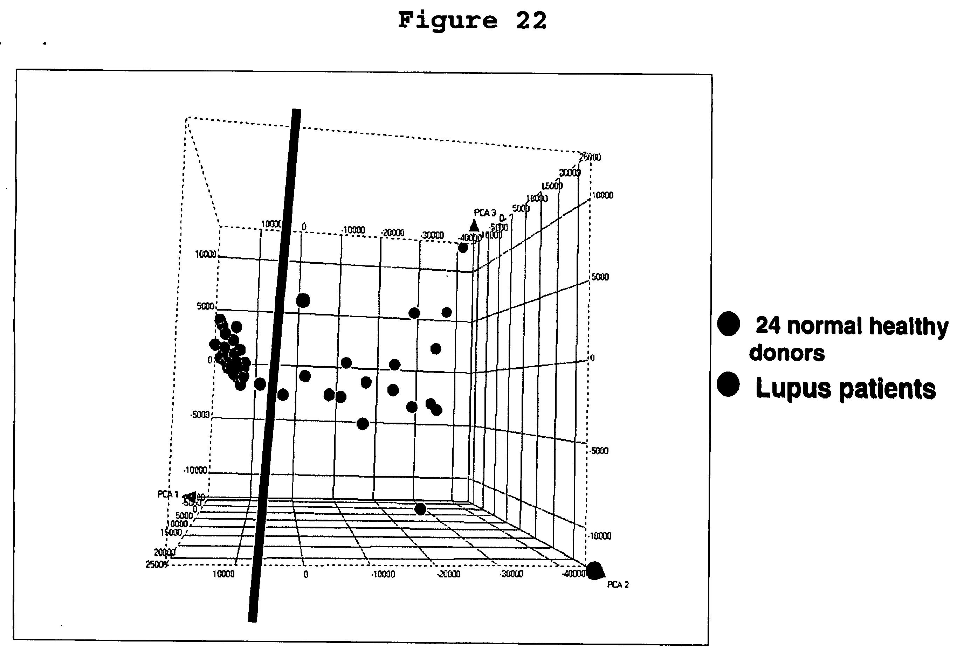

Using 169 probe sets to detect expression of a number of PD markers, gene expression in whole blood samples of 35 lupus patients was analyzed using PCA (Principal Component Analysis). Principal component analysis is a statistical technique for simplifying a dataset, by reducing multidimensional datasets to lower dimensions for analysis. PCA was conducted on the filtered data set (169 probe sets) using the Spotfire statistical tool. The PCA determined that 24/35 of the lupus patients had a statistically significant PD marker signature. See Figure 22 for PCA analysis results. The 169 probe sets used for this PCA analysis are provided in Table 20.

**Table 20: Gene Expression Detected by 169 Probe Sets in 35 SLE Patients**

| **Probe ID** | **UniGene ID** | **Gene Title** | **Gene Symbol** |
|---|---|---|---|
| 1552772_at | Hs.351811 | C-type lectin domain family 4, member D | CLEC4D |
| 1554343_a_at | Hs.435579 | BCR downstream signaling I | BRDG1 |
| 1555464_at | Hs.163173 | interferon induced with helicase C domain I | IFIH1 |
| 1555728_a_at | Hs.325960 | membrane-spanning 4-domains, subfamily A, member 4 | MS4A4A |
| 1556643_at | Hs.515243 | Hypothetical protein BC011840 | LOC93343 |
| 1557236_at | Hs.257352 | apolipoprotein L, 6 | APOL6 |
| 1559585_at | Hs.535011 | hypothetical protein FU31033 | FLJ31033 |
| 200887_s_at | Hs.565365 | signal transducer and activator of transcription 1, 91kDa | STAT1 |
| 200923_at | Hs.514535 | lectin, galactoside-binding, soluble, 3 binding protein | LGALS3BP |
| 200986_at | Hs.384598 | serpin peptidase inhibitor, clade G (C 1 inhibitor), member I, (angioedema, hereditary) | SERPING1 |
| 201015_s_at | Hs.514174 | junction plakoglobin | JUP |
| 201324_at | Hs.436298 | epithelial membrane protein) I | EMPI |
| 201641_at | Hs.118110 | bone marrow stromal cell antigen 2 | BST2 |
| 201646_at | Hs.349656 | scavenger receptor class B, member 2 | SCARB2 |
| 201761_at | Hs.469030 | methylenetetrahydrofolate dehydrogenase (NADP+ dependent) 2, methenyltetrahydrofolate cyclohydrolase | MTHFD2 |
| 202086_at | Hs.517307 | myxovirus (influenza virus) resistance 1, interferon-inducible protein p78 (mouse) /// myxovirus (influenza virus) resistance 1, interteron-inducible protein p78 (mouse) | MX1 |
| 202145_at | Hs.521903 | lymphocyte antigen 6 complex, locus E | LY6E |
| 202270_at | Hs.62661 | guanylate binding protein I, interferon-inducible, 67kDa /// guanylate binding protein I, interferon-inducible, 67kDa | GBP1 |
| 202411_at | Hs.532634 | interferon, alpha-inducible protein 27 | IFI27 |
| 202430_s_at | Hs.130759 | phospholipid scramblase 1 | PLSCR1 |
| 202446_s_at | Hs.130759 | phospholipid scramblase 1 | PLSCR1 |
| 202759_s_at | Hs.591908 | A kinase (PRKA) anchor protein 2 /// PALM2-AKAP2 protein | AKAP2 /// PALM2-AKAP2 |
| 202863_at | Hs.369056 | SP100 nuclear antigen | SP100 |
| 202869_at | Hs.524760 | 2',5'-oligoadenylate synthetase 1, 40/46kDa | OAS1 |
| 203153_at | Hs.20315 | interferon-induecd protein with tetratricopeptide repeats I /// interferon-induced protein with - tetratricopeptide repeats I | IFIT1 |
| 203595_s_at | Hs.252839 | interferon-induced protein with tetratricopeptide repeats 5 | IFIT5 |
| 203596_s_at | Hs.252839 | interferon-induced protein with tetratricopeptide repeats 5 | IFIT5 |
| 203771_s_at | Hs.488143 | biliverdin reductase A | BLVRA |
| 204211_x_at | Hs.131431 | eukaryotic translation initiation factor 2-alpha kinase 2 | E1F2AK2 |
| 204224_s_at | Hs.86724 | GTP cyclohydrolase i (dopa-responsive dystonia) | GCH 1 |
| 204326_x_at | Hs.374950 | metallothionein 1 X | MT1 X |
| 204415_at | Hs.523847 | interferon, alpha-inducible protein 6 | IFI6 |
| 204439_at | Hs.389724 | interferon-induced protein 44-like | IFI44L |
| 204533_at | Hs.632586 | chemokine (C-X-C motif) ligand 10 | CXCL10 |
| 204747_at | Hs.47338 | interferon-induced protein with tetratricopeptide repeats 3 | IFIT3 |
| 204972_at | Hs.414332 | 2'-5'-oligoadenylate synthetase 2, 69/71 kDa | OAS2 |
| 204994_at | Hs.926 | myxovirus (influenza virus) resistance 2 (mouse) | MX2 |
| 205098_at | Hs.301921 | chemokine (C-C motif) receptor I | CCR1 |
| 205099_s_at | Hs.301921 | chemokine (C-C motif) receptor I | CCR1 |
| 205170_at | Hs.530595 | signal transducer and activator of transcription 2, 113kDa | STAT2 |
| 205241_at | Hs.567405 | cytochrome oxidase deficient homolog 2 (yeast) | SCO2 |
| 205483_s_at | Hs.458485 | ISG 15 ubiquilin-like modifier | ISG15 |
| 205552_s_at | Hs.524760 | 2',5'-oligoadenylate synthetase 1, 40/46kDa | OAS1 |
| 205569_at | Hs.518448 | lysosomal-associated membrane protein 3 | LAMP3 |
| 205660_at | Hs.118633 | 2'-5'-oligoadenylate synthetase-like | OASL |
| 206025_s_at | Hs.437322 | tumor necrosis factor, alpha-induced protein 6 | TNFAIP6 |
| 206026_s_at | Hs.437322 | tumor necrosis factor, alpha-induced protein 6 | TNFAIP6 |
| 206133_at | Hs.44 975 | XIAP associated lactor-1 | BIRC4BP |
| 206332_s_at | --- | interferon, gamma-inducible protein 16 | IFI16 |
| 206513_at | Hs.281898 | absent in melanoma 2 | AIM2 |
| 206553_at | Hs.414332 | 2'-5'-oligoadenylate synthetase 2, 69/71 kDa | OAS2 |
| 206576_s_at | Hs.512682 | carcinoembryonic antigen-related cell adhesion molecule I (biliary glycoprotein) | CEACAM 1 |
| 206715_at | Hs.125962 | transcription factor EC | TFEC |
| 208087_s_at | Hs.302123 | Z-DNA binding protein I /// Z-DNA binding protein I | ZBP1 |
| 208436_s_at | Hs.166120 | interferon regulatory factor 7 | IRF7 |
| 208581_x_at | Hs.374950 | metallothionein 1X | MT1X |
| 208653_s_at | Hs.591335 | CD164 molecule, sialomucin | CD164 |
| 208966_x_at | --- | interferon, gamma-inducible protein 16 | IFI16 |
| 209417_s_at | Hs.632258 | interferon-induced protein 35 | IFI35 |
| 209498_at | Hs.512682 | carcinoembryonic antigen-related cell adhesion molecule 1 (biliary glycoprotein) | CEACAM 1 |
| 209593_s_at | Hs.252682 | torsin family 1, member B (torsin B) | TOR1 B |
| 210001_s_at | Hs.50640 | suppressor of cytokine signaling 1 | SOCS1 |
| 210705_s_at | Hs.370515 | tripartite motif-containing 5 | TRIM5 |
| 210797_s_at | Hs.118633 | 2'-5'-oligoadenylate synthetase-like | OASL |
| 210873_x_at | Hs.348983 | apolipoproacin B mRNA editing enzyme, catalytic polypeptide-like 3A | APOBEC3A |
| 210985_s_at | Hs.369056 | SP100 nuclear antigen | SP100 |
| 211012_s_at | Hs.498345 | promyelocytic leukemia /// hypothetical protein LOC 161527 /// similar to promyelocytic leukemia protein isoform 9 | L /// LOC161527 /// LOC652671 |
| 211456_x_at | --- | hypothetical protein LOC650610 | LOC650610 |
| 211889_x_at | Hs.512682 | carcinoembryonic antigen-related cell adhesion molecule 1 (biliary glycoprotein) | CEACAM 1 |
| 212185_x_at | Hs.534330 | metallothioncin 2A | MT2A |
| 212657_s_at | Hs.81134 | interleukin 1 receptor antagonist | ILIRN |
| 212659_s_at | Hs.81134 | interleukin 1 receptor antagonist | ILIRN |
| 212845_at | Hs.98259 | sterile alpha motif domain containing 4A | SAMD4A |
| 213293_s_at | Hs.501778 | tripartite motif-containing 22 | TRIM22 |
| 213294_at | Hs.546523 | Full-length cDNA clone CS0DK002YF13 of HeLa cells Cot 25-normalized of Homo sapiens (human) | -- |
| 213361_at | Hs.193842 | tudor domain containing 7 | TDRD7 |
| 213469_at | Hs.229988 | GP1 deacylase | PGAP1 |
| 213797_at | Hs.17518 | radical S-adenosyl methionine domain containing 2 | RSAD2 |
| 214059_at | Hs.82316 | Interferon-induced protein 44 | IFI44 |
| 214329_x_at | Hs.478275 | tumor neurosis factor (ligand) superfamily, member 10 /// tumor necrosis factor (ligand) superfamily, member 10 | TNFSF10 |
| 214453_s_at | Hs.82316 | interferon-induced protein 44 | IFI44 |
| 214511_x_at | Hs.534956 | Fc fragment of IgG, high affinity Ia, receptor (CD64) /// Fc-gamma receptor I B2 /// similar to Fc-gamna receptor I B2 isoform b | 1 A /// LOC440607 /// LOC652758 |
| 216243_s_at | Hs.81134 | interleukin 1 receptor antagonist | ILIRN |
| 216598_s_at | Hs.303649 | chemokine (C-C motif) ligand 2 | CCL2 |
| 217165_x_at | Hs.513626 | metallothionein I F (functional) | MTI F |
| 217502_at | Hs.437609 | interferon-induced protein with tetratricopeptide repeats 2 | IFIT2 |
| 217933_s_at | Hs.570791 | leucine aminopeptidase 3 | LAP3 |
| 218400_at | Hs.528634 | 2'-5'-oligoadenylate synthetase 3, 100kDa | OAS3 |
| 218543_s_at | Hs.12646 | poly (ADP-ribose) polymerase family, member 12 | PARP12 |
| 218943_s_at | Hs.190622 | DEAD (Asp-Glu-Ala-Asp) box polypeptide 58 | DDX58 |
| 218986_s_at | Hs.591710 | hypothetical protein FU20035 | FU20035 |
| 219062_s_at | Hs.631682 | zinc finger, CCHC domain containing 2 | ZCCHC2 |
| 219209_at | Hs.163173 | interferon induced with helicase C domain I | IFIH1 |
| 219211_at | Hs.38260 | ubiquitin specific peptidase 18 | USP18 |
| 219352_at | Hs.529317 | hect domain and RLD 6 | HERC6 |
| 219364_at | Hs.55918 | likely ortholog of mouse D11lgp2 | LGP2 |
| 219519_s_at | Hs.31869 | sialic acid binding Ig-like lectin I, sialoadhesin /// sialic acid binding Ig-like lectin 1, sialoadhesin | SIGLEC1 |
| 219607_s_at | Hs.325960 | membrane-spanning 4-domains, subfamily A, member 4 | MS4A4A |
| 219684_at | Hs.43388 | receptor transporter protein 4 | RTP4 |
| 219691_at | Hs.65641 | sterile alpha motif domain containing 9 | SAMD9 |
| 219863_at | Hs.26663 | hect domain and RLD 5 | HERC5 |
| 219885_at | --- | schlafen family member 12 | SLFN12 |
| 220059_at | Hs.435579 | BCR downstream signaling I | BRDG1 |
| 220576_at | Hs.229988 | GPI dcacylasc | PGAP1 |
| 221680_s_at | Hs.272398 | ets variant gene 7 (TEL2 oncogene) | ETV7 |
| 221816_s_at | Hs.369039 | PHD finger protein 11 | PHF1 |
| 222154_s_at | Hs.120323 | DNA polymerase-transactivated protein 6 | DNAPTP6 |
| 222631_at | Hs.443733 | phosphalidylinositol 4-kinase type 2 beta | PI4K2B |
| 222793_at | Hs.190622 | DEAD (Asp-Glu-Ala-Asp) box polypeptide 58 | DDX58 |
| 222816_s_at | Hs.631682 | zinc linger, CCHC domain containing 2 | ZCCHC2 |
| 223167_s_at | Hs.473370 | ubiquitin specific peptidase 25 | USP25 |
| 223220_s_at | Hs.518200 | poly (ADP-ribose) polymerase family, member 9 | PARP9 |
| 223434_at | --- | guanylate binding protein 3 | GBP3 |
| 223501_at | --- | --- | --- |
| 223849_s_at | Hs.514941 | Mov10, Moloney leukemia virus 10, homology (mouse) | MOV10 |
| 224225_s_at | Hs.272398 | ets variant gene 7 (TEL2 oncogene) | ETV7 |
| 224701_at | Hs.583792 | poly (ADP-ribose) polymerase family, member 14 | PARP14 |
| 225291_at | Hs.388733 | polyribonucleotide nucleotidyltransferase I | PNPT1 |
| 225415_at | Hs.518201 | deltex 3-like (Drosophila) | DTX3L |
| 225636_at | Hs.530595 | signal transducer and activator of transcription 2, 113kDa | STAT2 |
| 225834_at | Hs.599880 | hypothetical protein LOC652689 /// family with sequence similarity 72, member A /// similar to family with sequence similarity 72, member A /// similar to family with sequence similarity 72, member A | LOC652689 /// FAM 72A /// LOC653594 /// LOC653820 |
| 225869_s_at | Hs.502989 | unc-93 homolog B1 (C. elegans) | UNC93B1 |
| 226103_at | Hs.632387 | nexilin (F actin binding protein) | NEXN |
| 226603_at | Hs.489118 | sterile alpha motif domain containing 9-like | SAM D9L |
| 226702_at | Hs.7155 | hypothetical protein LOC 129607 | LOC 129607 |
| 226757_at | Hs.437609 | interferon-induced protein with tetratricopeptide repeats 2 | IFIT2 |
| 227458_at | --- | --- | --- |
| 227609_al | Hs.546467 | epithelial stromal interaction I (breast) | EPSTI1 |
| 227697_at | Hs.527973 | suppressor of cylokine signaling 3 | SOCS3 |
| 228152_s_at | Hs.53501 | hypothetical protein FLJ31033 | FLJ31033 |
| 228230_at | Hs.517180 | peroxisomal proliferator-activated receptor A interacting complex 285 | PRIC285 |
| 228439_at | Hs.124840 | basic leucine zipper transcription factor, ATF-like 2 | BATF2 |
| 228531_at | Hs.65641 | sterile alpha motif domain containing 9 | SAMD9 |
| 228607_at | Hs.414332 | 2'-5'-oligoadenylate synthetase 2, 69/71kDa | OAS2 |
| 228617_at | Hs.441975 | XIAP associatcd factor-I | BIRC4BP |
| 229450_at | --- | --- | --- |
| 230036_at | Hs.489118 | sterile alpha motif domain containing 9-like | SAM D9L |
| 230314_at | Hs.112420 | Transcribed locus. strongly similar to XP_511805.1 PREDICTED: hypothetical protein XP_511805 [Pan troglodytes] | --- |
| 231769_at | Hs.464419 | F-box protein 6 | FBXO6 |
| 232034_at | Hs.599821 | hypothetical protein LOC203274 | LOC203274 |
| 232155_at | Hs.514554 | KIAA1618 | KIAA1618 |
| 232375_at | Hs.565365 | Signal transducer and activator of transcription 1, 91kDa | STAT1 |
| 232666_at | Hs.528634 | 2'-5'-oligoadenylate synthetase 3, 100kDa | OAS3 |
| 233425_at | Hs.631682 | zinc finger, CCHC domain containing 2 | ZCCHC2 |
| 233880_at | Hs.195642 | chromosome 17 open reading frame 27 | C17or127 |
| 235061_at | Hs.291000 | protein phosphatase I K (PP2C domain containing) | PPMIK |
| 235112_at | Hs.533491 | KIAA1958 | KIAA1958 |
| 235157_at | Hs.583792 | Poly (ADP-ribose) polymerase family, member 14 | PARP14 |
| 235276_at | --- | --- | --- |
| 235643_at | Hs.489118 | sterile alpha motif domain containing 9-like | SAM D9L |
| 236156_at | Hs.127445 | lipase A, lysosomal acid, cholesterol esterase (Wolman disease) | LIPA |
| 236692_at | --- | --- | --- |
| 238439_at | Hs.217484 | ankyrin repeat domain 22 | ANKRD22 |
| 238581_at | Hs.513726 | Guanylate binding protein 5 | GBP5 |
| 238743_at | Hs.546523 | Full-length cDNA clone CSODK002YF13 of HeLa cells Cot 25-normalized of Homo sapiens (human) | --- |
| 239196_at | Hs.217484 | ankyrin repeat domain 22 | ANKRD22 |
| 239277_at | --- | --- | --- |
| 239979_at | Hs.546467 | Epithelial stromal interaction I (breast) | EPSTII |
| 241812_at | Hs.120323 | DNA polymerase-transactivated protein 6 | DNAPTP6 |
| 241916_at | Hs.130759 | Phospholipid scramblase 1 | PLSCR1 |
| 242020_s_at | Hs.302123 | Z-DNA binding protein 1 | ZBP1 |
| 242234_at | Hs.441975 | XIAP associated factor-1 | BIRC4BP |
| 242625_at | Hs.17518 | radical S-adenosyl methionine domain containing 2 | RSAD2 |
| 242898_at | --- | --- | --- |
| 243271_at | Hs.489118 | Sterile alpha motif domain containing 9-like | SAMD9L |
| 44673_at | Hs.31869 | sialic acid binding Ig-like lectin I, sialoadhesin | SIGLEC1 |
| AFFX-HUMISGF3A/M97935-3_at | Hs.565365 | signal transducer and activator of transcription 1, 91kDa | STAT1 |
| AFFX-HUMISGF3A/M97935_5_at | Hs.565365 | signal transducer and activator of transcription 1, 91kDa | STAT1 |
| AFFX-HUMISGF3A/M97935_MB_at | Hs.565365 | signal transducer and activator of transcription 1, 91kDa | STAT1 |

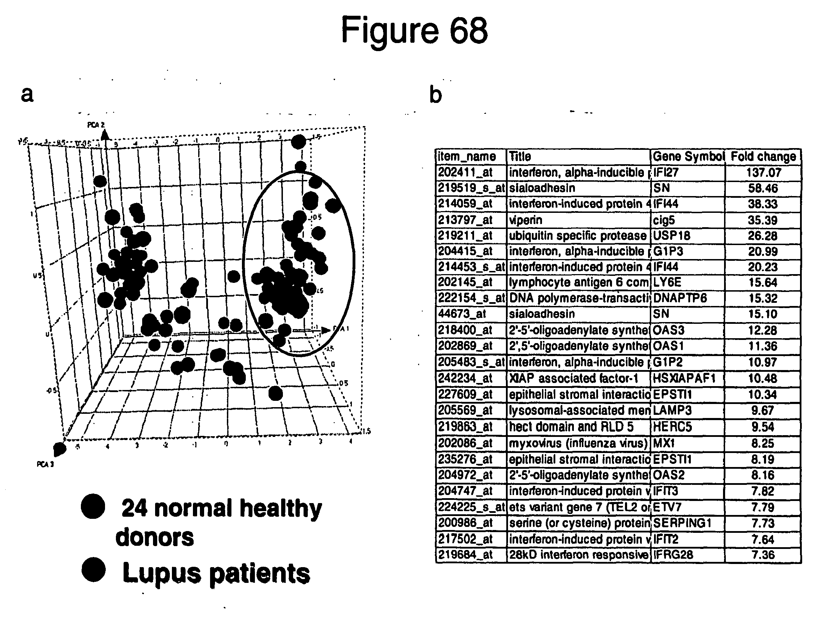

Similarly, using 25 highly upregulated IFN-inducible genes, expression in whole blood samples of lupus patients and normal healthy donors was analyzed using PCA (Principal Component Analysis). The PCA determined that approximately 66% of the lupus patients had a strong/moderate type I IFN inducible signature. See Figure 68a for PCA analysis results and 68b for 25 genes used in the PCA analysis.

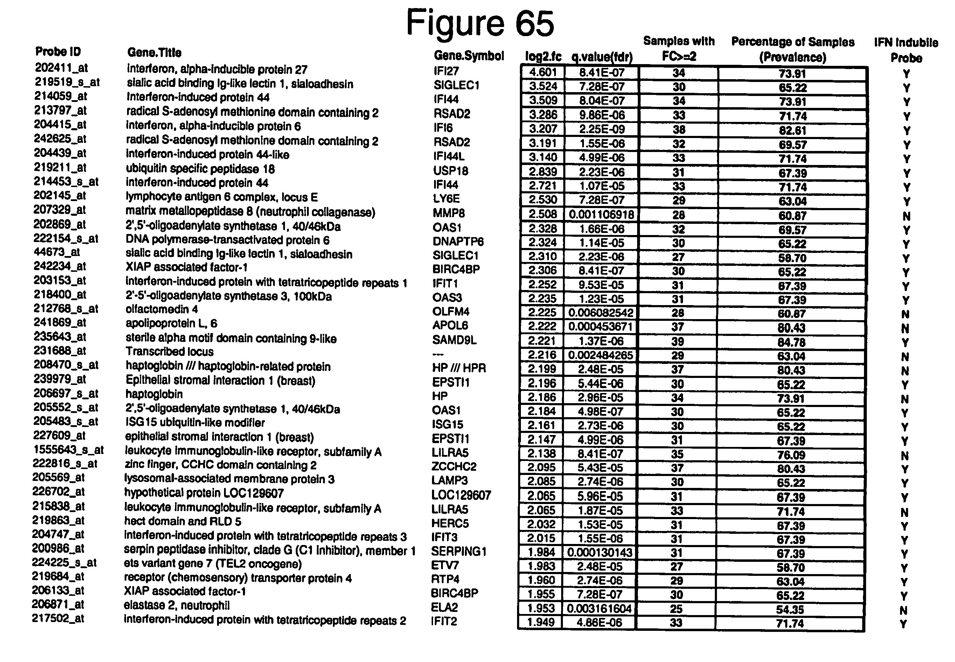

The overexpression of type I IFN genes in SLE patient whole blood for a larger number of patients, determined using an Affymetrix whole genome array, is provided in Table 23. Table 23 and Figure 65 provide further evidence that a high percentage of SLE patients share at least 2-fold overexpression of each individual type I IFN genes.

**Table 23: Overexpressed Type-I IFN Genes in Whole Blood of Lupus Patients**

| **Probe.ID** | **Cene.Title** | **Gene.Symbol** | **Number Of Samples Displaying A Fold-Change >=2** | **% of Samples** | **Average log2 Fold-Change** |
|---|---|---|---|---|---|
| 222816_s_at | zinc finger, CCHC domain containing 2 | ZCCHC2 | 70 | 79.55 | 2.124 |
| 204415_at | interferon, alpha-inducible protein 6 | IFI6 | 67 | 76.14 | 3.007 |
| 217502_at | interferon-induced protein with tetratricopeptide repeats 2 | IFIT2 | 65 | 73.86 | 1.913 |
| 235643_at | strerile alpha motif domain containing 9-like | SAMD9L | 65 | 73.86 | 2.020 |
| 213797_at | radical S-adenosyl methionine domain containing 2 | RSAD2 | 62 | 70.45 | 2.978 |
| 214059_at | interferon-induced protein 44 | IFI44 | 61 | 69.32 | 3.050 |
| 202411_at | interferon, alpha-inducible protein 27 | IFI27 | 60 | 68.18 | 3.937 |
| 204439_at | interferon-induced protein 44-like | IFI44L | 60 | 68.18 | 2.847 |
| 242625_at | radical S-adenosyl methionine domain containing 2 | RSAD2 | 59 | 67.05 | 2.861 |
| 214453_s_at | interferon-induced protein 44 | IFI44 | 59 | 67.05 | 2.463 |
| 203153_at | interferon-induced protein with tetratricopeptide repeats 1 /// in | IFIT1 | 59 | 67.05 | 2.034 |
| 242234_at | XIAP associated factor-1 | BIRC4BP | 59 | 67.05 | 2.066 |
| 203595_s_at | interferon-induced Protein with tetracopeptide repeats 5 | IFIT5 | 59 | 67.05 | 1.603 |
| 202086_at | myxovirus (influenza virus) resistance 1, interferon-inducible p | MX1 | 58 | 65.91 | 1.777 |
| 206133_at | XIAP associated factor-1 | BIRC4BP | 58 | 65.91 | 1.803 |
| 216243_s_at | interleukin 1 receptor antagonist | IL1RN | 58 | 65.91 | 1.278 |
| 219863_at | hect domain and RLD5 | HERC5 | 57 | 64.77 | 1.795 |
| 202869_at | 2'-5'-oligoadenylate synthetase 1, 40/46kDa | OAS1 | 56 | 63.64 | 2.057 |
| 226702_at | hypothetical protein LOC129607 | LOC 129607 | 56 | 63.64 | 1.797 |
| 205483_s_at | ISG15 ubiquitin-like modifier | ISG15 | 56 | 63.64 | 1.979 |
| 204747_at | interferon-induced protein with tetratricopeptide repeats 3 | IFIT3 | 56 | 63.64 | 1.675 |
| 1555464_at | interferon induced with helicase C domain 1 | IFIH1 | 56 | 63.64 | 1.532 |
| 218400_at | 2'-5'-oligoadenylate synthetase 3,100kDa | OAS3 | 55 | 62.50 | 1.932 |
| 227609_at | epithelial stromal interaction 1 (breast) | EPSTI1 | 55 | 62.50 | 1.788 |
| 200986_at | serpin peptidase inhibitor, clade G (C1 inhibitor), member 1, (a) | SERPING1 | 55 | 62.50 | 1.503 |
| 202145_at | lymphocyte antigen 6 complex, locus E | LY6E | 54 | 61.36 | 2.242 |
| 239979_at | Epithelial stromal interaction 1 (breast) | EPSTI1 | 54 | 61.36 | 1.895 |
| 205552_s_at | 2',5'-oligoadenylate synthetase 1, 40/46kDa | OAS1 | 54 | 61.36 | 1.945 |
| 225929_s_at | chromosome 17 open reading frame 27 | C17orf27 | 54 | 61.36 | 1.054 |
| 222154_s_at | DNA polymerase-transactivated protein 6 | DNAPTP6 | 53 | 60.23 | 2.030 |
| 205569_at | membrane protein 3 | LAMP3 | 53 | 60.23 | 1.813 |
| 205660_at | 2'-5'-oligoadenylate synthetase-like | OASL | 53 | 60.23 | 1.677 |
| 219352_at | hect domain and RLD 6 | HERC6 | 52 | 59.09 | 1.663 |
| 210797_sat | 2'-5'oligoadenylate synthetase-like | OASL | 52 | 59.09 | 1.548 |
| 241916_at | Phospholipid scramblase 1 | PLSCR1 | 52 | 59.09 | 1.396 |
| 208087_s_at | Z-DNA binding protein 1 /// Z-DNA binding protein 1 | ZBP1 | 52 | 59.09 | 1.438 |
| 243271_at | Sterile alpha motif domain containing 9-like | SAMD9L | 52 | 59.09 | 1.126 |
| 219519_s_at | sialic acid binding Ig-like lectin 1. sialoadhesin /// sialic acid bin | | 51 | 57.95 | 3.019 |
| 228617_at | XIAP associated factor-1 | BIRC4BP | 51 | 57.95 | 1.473 |
| 202446_s_at | phospholipid scramblase 1 | PLSCR1 | 51 | 57.95 | 1.307 |
| 232095_at | SLIT-ROBO Rho GTPase activating protein 2 | SRGAP2 | 50 | 56.82 | 1.155 |
| 232666_at | 2'-5'-oligoadenylate synthetase 3. 100kDa | OAS3 | 49 | 55.68 | 1.882 |
| 204972_at | 2'-5'-oligoadenylate synthetase 2. 69/71kDa | OAS2 | 49 | 55.68 | 1,642 |
| 202430_s_at | phospholipid scramblase 1 | PLSCR1 | 49 | 55.68 | 1.209 |
| 224701_at | poly (ADP-ribose polymerase family, member 14 | PARP14 | 49 | 55.68 | 1.098 |
| 219211_at | ubiquitin specific peptidase 18 | USP18 | 48 | 54.55 | 2.365 |
| 206553_at | '2'-5'-oligoadenylate synthetase 2, 69/71kDa | OAS2 | 48 | 54.55 | 1.582 |
| 219684_at | receptor transporter Protein 4 | RTP4 | 48 | 54.55 | 1.534 |
| 230000_at | chromosome 17 ooen reading frame 27 | C17orf27 | 47 | 53.41 | 0.936 |
| 44673_at | sialic acid binding Ig-like lectin 1. sialoadhesin | SIGLEC1 | 47 | 53.41 | 1.975 |
| 203596_s_at | interferon-induced protein with tetratricopeptide repeats 5 | IFIT5 | 47 | 53.41 | 1.327 |
| 218986_s_at | hypothetical protein FLJ20035 | FL20035 | 47 | 53.41 | 1.091 |
| 242020_s_at | Z-DNA binding protein 1 | ZBP1 | 47 | 53.41 | 1.195 |
| 212659_s_at | interleukin 1 receptor antagonist | IL1RN | 47 | 53.41 | 1.196 |
| 228439_at | basic leucine zipper transcription factor, ATF-like 2 | BATF2 | 46 | 52.27 | 1.180 |
| 226757_at | interferon-induced protein with tetratricopeptide repeats 2 | IFIT2 | 46 | 52.27 | 0.882 |
| 225291_at | polyribonucleotide nucleotidyltransferase 1 | PNPT1 | 46 | 52.27 | 0.957 |
| 206026_s_at | tumor necrosis factor, alpha-induced protein 6 | TNFAIP6 | 46 | 52.27 | 0.942 |
| 222858_s_at | dual adaptor of phosphotyrosine and 3-phosohoinositides | DAPP1 | 46 | 52.27 | 1.055 |
| 208436_s_at | interferon regulatory factor 7 | IRF7 | 45 | 51.14 | 1.146 |
| 217933_s_at | leucine aminopeptidase 3 | LAP3 | 45 | 51.14 | 0.807 |
| 228152_s_at | hypothetical protein FLJ31033 | FLJ31033 | 45 | 51.14 | 0.834 |
| 230036_at | sterile alpha motif domain containing-9-like | SAMD9L | 44 | 50.00 | 1.097 |
| 228607_at | 2'-5-oligoadenylate synthetase 2.69/71kDa | OAS2 | 44 | 50.00 | 1.113 |
| 218543_s_at | poly (ADP-ribose) polymerase family, member 12 | PARP12 | 44 | 50.00 | 1.111 |
| 226603_at | sterile alpha motif domain containing 9-like | SAMD9L | 44 | 50.00 | 1.033 |
| 204211_x_at | eukaryotic translation initiation factor 2-alpha kinase 2 | EIF2AK2 | 44 | 50.00 | 1.050 |
| 235157_at | Poly (ADP-ribose) polymerase family, member 14 | PARP14 | 44 | 50.00 | 0.940 |
| 209417_s_at | interferon-induced protein 35 | IFI35 | 44 | 50.00 | 0.957 |

Based on the observations of different overexpressed type I IFN genes in SLE patients, described above, a set of 21 type I IFN genes in whole blood of lupus patients was identified as potentially useful. See Table 24.

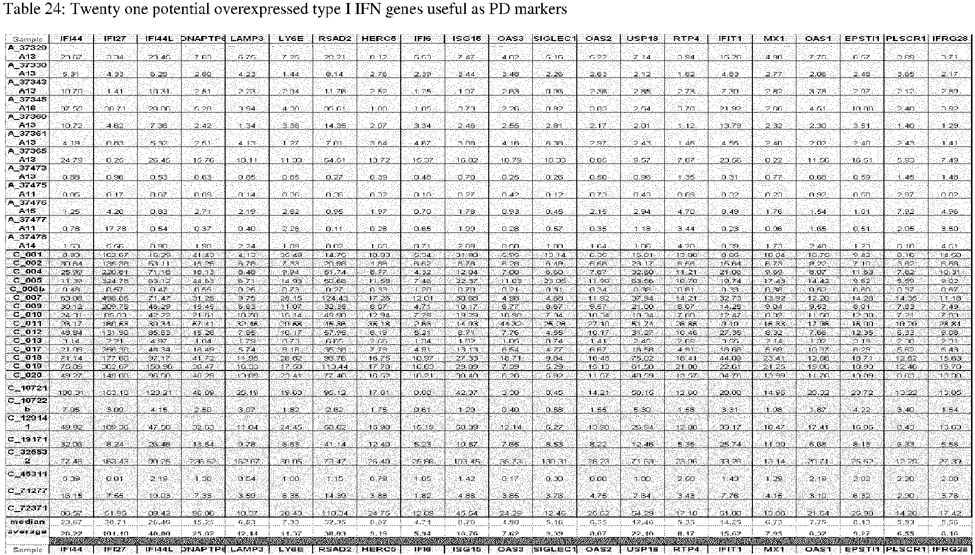

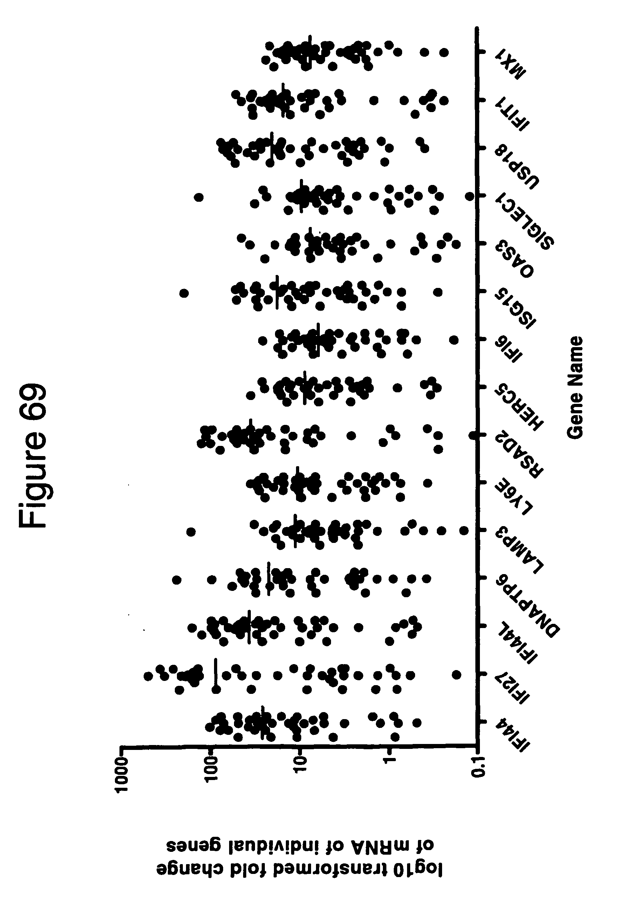

Overexpression of these genes, as detected initially using the Affymetrix arrays, was confirmed by Fluidigm dynamic array, validating their overexpression. See Figure 69.

### Example 8: MEDI-545 Considerably Neutralizes the Tyne I IFN Gene Signature of SLE Patients Having a Strong to Moderate Type I IFN Gene Signature

Patients in a clinical trial were identified as having a strong/moderate type I IFN gene, a weak type I IFN gene signature, or no type I IFN gene signature. These patients were designated into one of these groups based on 149 genes. Table 25 shows the number of lupus patients in the clinical trial that were designated in each of these three groups and indicates the treatment protocol they received.

**Table 25: Patient distribution based on type-I IFN gene signature prior to treatment**

| **Group** | **Strong** & **moderate signature** | **Weak signature** | **No signature** |
|---|---|---|---|
| **PBO** | **10** | **5** | **2** |
| **0.3 mpk** | **5** | **0** | **1** |
| **1 mpk** | **2** | **2** | **2** |
| **3 mpk** | **3** | **2** | **1** |
| **10 mpk** | **4** | **3** | **0** |
| **30 mpk** | **3** | **2** | **1** |
| **Total** | **27** | **14** | **7** |

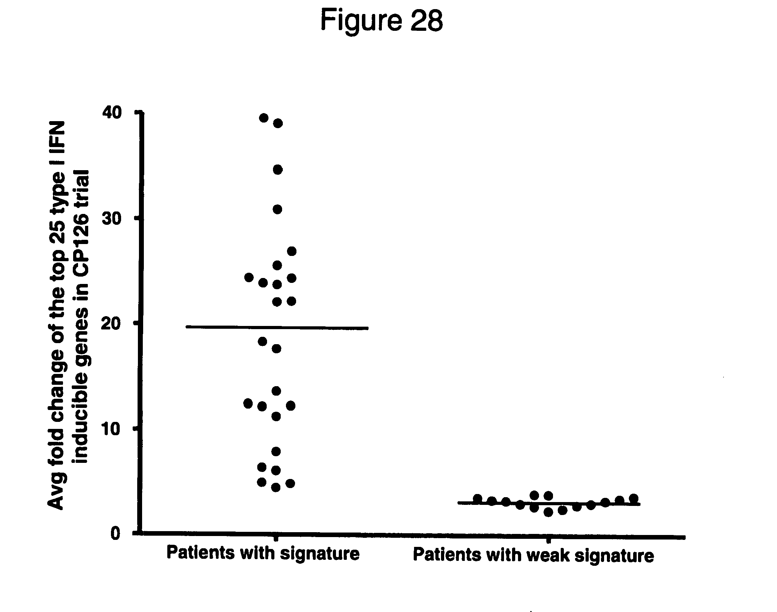

The SLE patients that were designated as having strong and moderate type-I IFN gene signatures all had: an average 4-fold increase in expression of the top 25 most upregulated type I IFN genes; an average 2-fold increase in expression of the top 50 most upregulated type I IFN genes; and a percentage of total examined disease genes being type I IFN inducible of 3.8. The average fold increase in the top 25 type I IFN inducible genes for each patient having a strong/moderate type I IFN signature or a weak signature in the trial is provided in Figure 28.

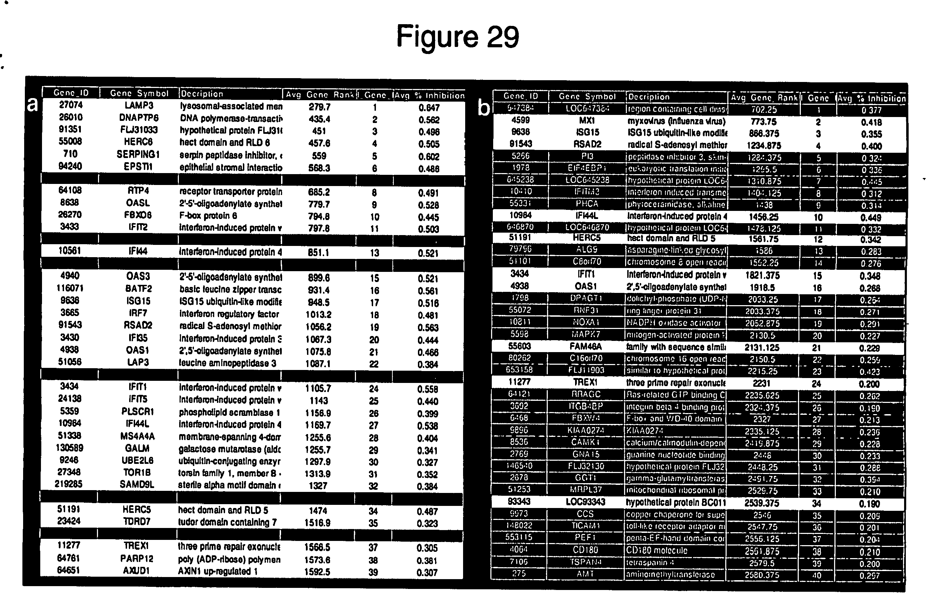

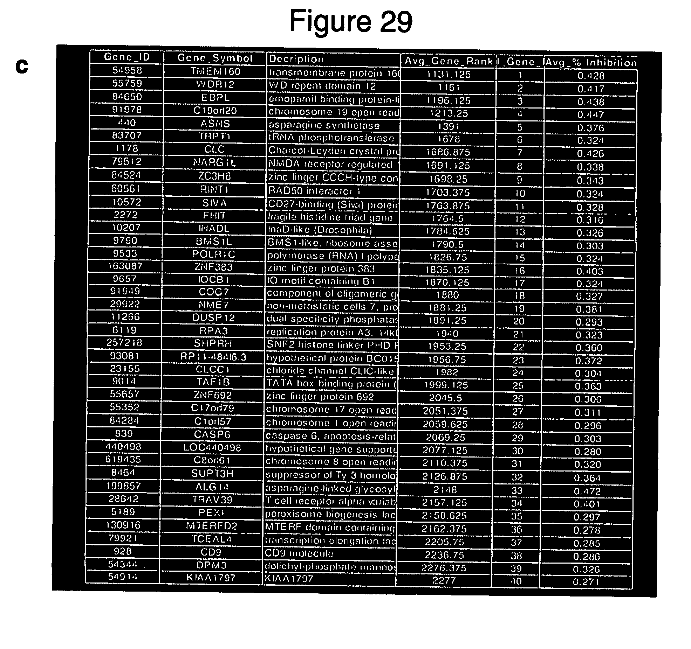

Treatment of these different SLE patient groups provided evidence that neutralization of the type I IFN gene signature by MEDI-545 is drug specific. Figure 29(a) shows that in a group of SLE patients having a type-I IFN gene signature, virtually all of the top 39 genes neutralized 14 days post-MEDI-545 treatment are type I IFN signature genes (see yellow highlighted genes; percentage inhibition of the type I IFN signature genes ranged from 30.5-64.7). By contrast, none of the top 39 neutralized genes in SLE patients who received placebo were type I IFN signature genes. See Figure 29(c). The SLE patients who lacked a type I IFN signature and were treated with MEDI-545 displayed an intermediate neutralization pattern, with some type I IFN signature genes neutralized. (See Figure 29(b); yellow highlighting indicates type I IFN signature genes, which were neutralized from 19%-44.9%).

Further break down of SLE patients into strong, moderate, and weak type-I IFN gene signatures was conducted. Briefly, the 25 most highly overexpressed type I IFN-inducible genes in individual SLE patients generated from the ex vivo stimulation of healthy donor WB with SLE patient sera study were selected and the median fold change of these 25 genes was used to construct a type I IFN gene signature score for each SLE patient. Figure 84 shows the distribution of the type I IFN gene signature scores of the 46 SLE patients profiled. The SLE patients were profiled into 3 groups based on their type I IFN gene signature score: high type I IFN gene signature (score >10); moderate type I IFN gene signature (score 4-10); and weak type I IFN gene signature (score <4).

### Selection of a panel of 21 type I IFN-inducible genes in WB of SLE patients

To select a small, robust panel of type I IFN-inducible genes that could be developed into an HTP assay, the gene panel was narrowed to 21 genes. To identify the 21 potential PD and diagnostic markers, 807 IFN-α/β-inducible probes identified by ex vivo stimulation of healthy donor WB with 10 IFN-α subtypes (2a, 4b, 5, 6, 7, 8, 10, 14, 16, and 17) and IFN-β were used as a candidate marker starting point. The WB samples from a total of 46 SLE patients procured from commercial vendors and 24 healthy normal controls were used to determine the type I IFN-inducible probes that are upregulated in WB of SLE patients. 114 overexpressed probes (q≤0.05; fold change≥2) were identified in WB of SLE patients were type I IFN-inducible using SAM and FDR.

To investigate whether these overexpressed type I IFN-inducible genes in WB of SLE patients were neutralizable by an anti-IFN-α mAb, one healthy donor PBMC was stimulated ex vivo with sera from six individual SLE patients. The healthy donor was prescreened to exclude those donors that might have viral infection. 161 type I IFN-inducible probes were upregulated by ≥2-fold in the PBMC of the healthy donor following stimulation with ≥1 SLE patient serum in which the overexpression of these genes was suppressed by ≥50% and ≥70% by an anti-IFN-α mAb and an anti-IFN-αR mAb, respectively.

The intersection between this list of 161 probes and previously determined list of 114 probes was 80 probes. Each of these 80 probes was ranked by both the average fold change magnitude across all SLE patients and the percentage of patients displaying a change ≥2-fold. Generally, the 21 most prevalently overexpressed type I IFN-inducible genes (that represent unique genes using the NetAffx annotation file for the Affymetrix U133 2.0 plus array; ESTs were excluded) from this ranking were retained for a static list of probes used to measure PD. The type I IFN signature score was then defined by the median of these 21 genes.

With these 21 genes, it was necessary to recalculate the thresholds that had been previously identified for partitioning SLE patients into type I IFN gene signature responses of strong, moderate, or weak (based on the Affymetrix platform) for a lower density platform (TaqMan-based assay). A scaling method was required to convert the type I IFN signature score based on the top 25 differentially expressed genes (independent for each SLE patient) on the Affymetrix platform to the type I IFN signature score based on the 21 genes selected for the TaqMan-based assay. This method was implemented to compensate for 3 primary differences between the 2 platforms: (1) the number of probes used for the type I IFN signature (25 genes dynamically determined for each patient on the Affymetrix platform versus a 21 static gene list on the TaqMan-based assay), (2) the differences in sensitivity between the 2 platforms, and (3) the scales of the dynamic ranges within each platform. First, the fold change values were calculated (on a log₂ scale) for the 155 type I-inducible probes between the 35 randomly selected SLE patients and the average of a set of normal healthy controls. The genes with the top 25-fold change values were determined for each patient on the Affymetrix platform (this gene set is allowed to vary from patient to patient depending on which type I IFN-inducible genes are most highly expressed). Next, the median fold change was calculated from the top 25 genes for each SLE patient. The same calculation was conducted across the same patients using the static 21 gene set on the TaqMan-based assay. This gene set was identical for each patient and the median fold change was calculated based on 21 genes, rather than 25 dynamic genes, as was conducted for the Affymetrix platform. A simple regression model was then computed using these 2 vectors of equal length (35 median fold change values), and the coefficients from the model were used to calculate the conversion factor (from the Affymetrix platform to the TaqMan-based assay) for the response threshold values to partition the SLE patients into a type I IFN gene signature category of strong (>10 on Affymetrix; >5.53 on TaqMan), moderate (between 4 and 10 on Affymetrix; between 1.91 and 5.53 on TaqMan), or weak (<4 on Affymetrix; < 1.91 on TaqMan). Using these scaled threshold values, for the purpose of stratifying SLE patients, the signature (ie, median fold change) that was calculated on the 21 genes from the TaqMan-based assay was comparable to that from the top 25 upregulated type I IFN-inducible genes.

The prevalence and fold change (log₂ based) of the 21 IFN α/β-inducible genes in whole blood of 111 SLE patients is provided in Table 32, below.

**Table 32: Prevalence and fold chance in expression of 21 IFN α/β-inducible genes in SLE patient whole blood**

| **Probe** | **Q value** | **Fold** | **Prevalence** | **Gene name** | **Gene symbol** |
|---|---|---|---|---|---|
| 204415_at | qv<1e-16 | 9.38 | 78.20 | interferon, alpha-inducible protein 6 | IFI6 |
| 213797_at | 2.67E-12 | 8.27 | 71.80 | radical S-adenosyl methionine domain containing 2 | RSAD2 |
| 214059_at | 7.18E-14 | 7.93 | 70.90 | Interferon-induced protein 44 | IFI44 |
| 204439_at | 5.85E-12 | 6.45 | 69.10 | interferon-induced protein 44-like | IFI44L |
| 202411_at | 6.35E-12 | 14.42 | 67.30 | interferon, alpha-inducible protein 27 | IFI27 |
| 202086_at | 1.09E-09 | 3.26 | 66.40 | myxovirus (influenza virus) resistance 1, interferon-inducible protein p78 (mouse) | MX1 |
| 203153_at | 3.90E-07 | 3.52 | 65.50 | interferon-induced protein with tetratricopeptide repeats 1 | IFIT1 |
| 219863_at | 8.05E-11 | 3.27 | 64.50 | hect domain and RLD 5 | HERC5 |
| 205483_s_at | 1.23E-13 | 3.71 | 63.60 | ISG15 ubiquitin-like modifier | ISG15 |
| 205569_at | qv<1e-16 | 3.91 | 62.70 | lysosomal-associated membrane protein 3 | LAMP3 |
| 218400_at | 1-01E-10 | 3.65 | 62.70 | 2'-5'-oligoadenylate synthetase 3, 100kDa | OAS3 |
| 202869_at | 4.95E-11 | 3.77 | 61.80 | 2',5'-oligoadenylate synthetase 1, 40/46kDa | OAS1 |
| 227609_at | 7.41 E-10 | 3.16 | 60.90 | epithelial stromal interaction 1 (breast) | EPSTI1 |
| 204747_at | 9.78E-11 | 3.04 | 60.90 | interferon-induced protein with tetratricopeptide repeats 3 | IFIT3 |
| 202145_at | qv<1e-16 | 4.65 | 60.90 | lymphocyte antigen 6 complex, locus E | LY6E |
| 204972_at | qv<1e-16 | 3.06 | 58.20 | 2'-5'-oligoadenylate synthetase 2, 69/71 kDa | OAS2 |
| 241916_at | 6.29E-07 | 2.46 | 56.40 | Phospholipid scramblase 1 | PLSCR1 |
| 44673_at | qv<1e-16 | 3.91 | 55.50 | sialic acid binding Ig-like lectin 1, sialoadhesin | SIGLEC1 |
| 219211_at | 2.54E-13 | 4.83 | 55.50 | ubiquitin specific peptidase 18 | USP18 |
| 219684_at | 2.75E-07 | 2.47 | 50.00 | receptor (chemosensory) transporter protein 4 | RTP4 |
| 241812_at | 5.25E-07 | 1.84 | 38.20 | DNA polymerase-transactivated protein 6 | DNAPTP6 |

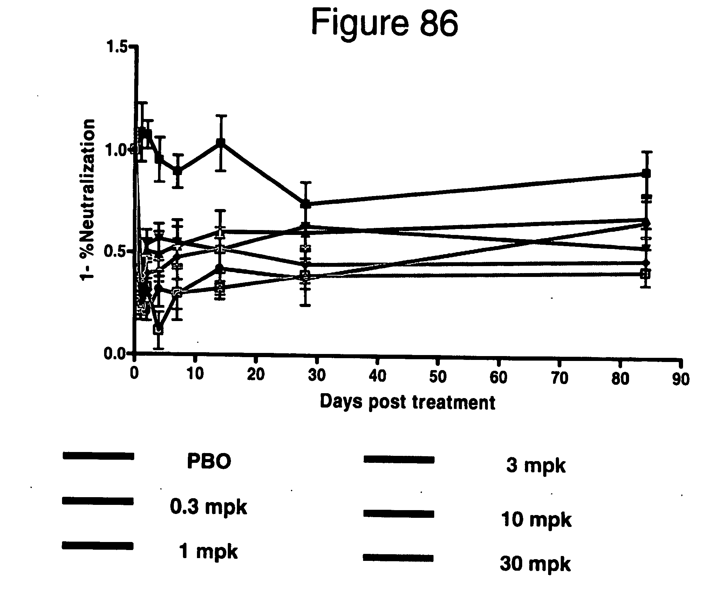

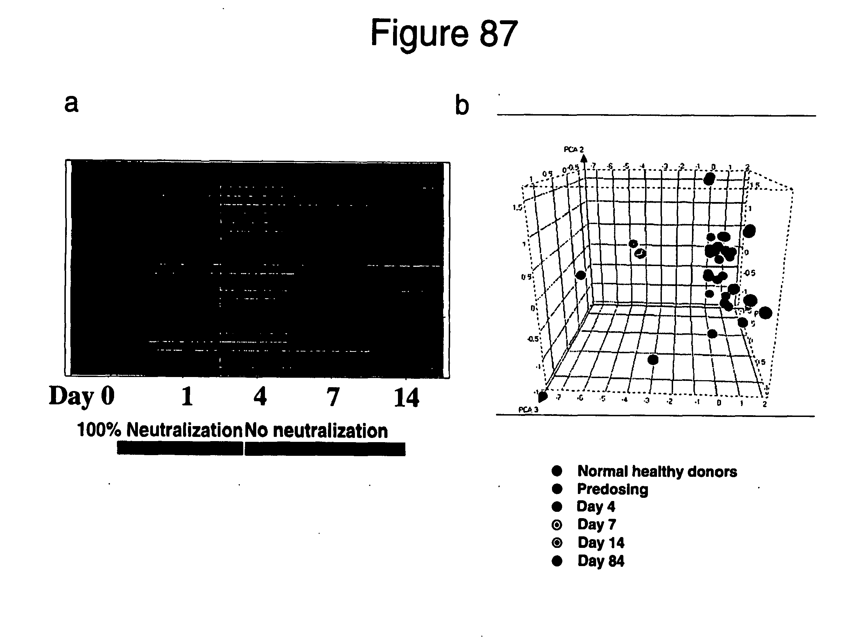

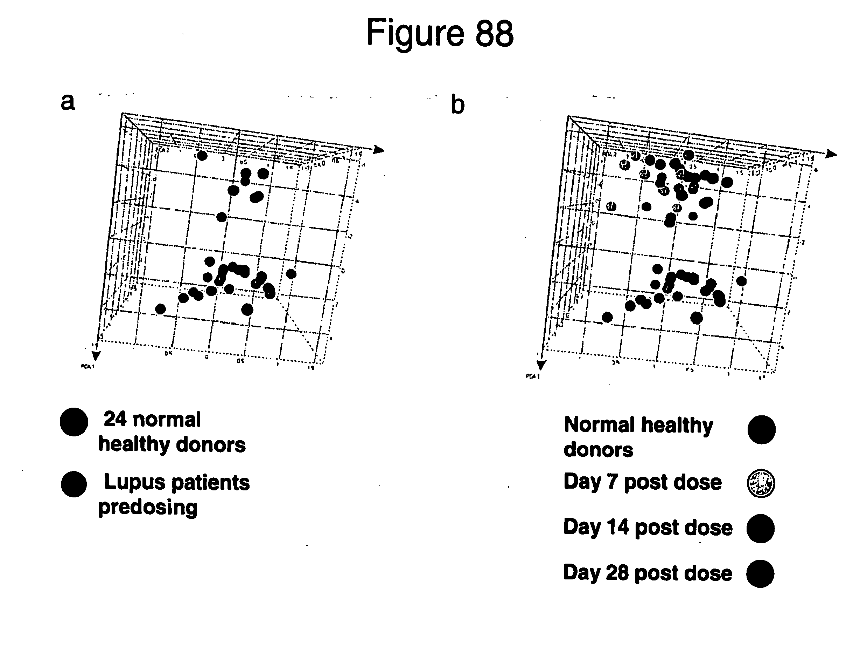

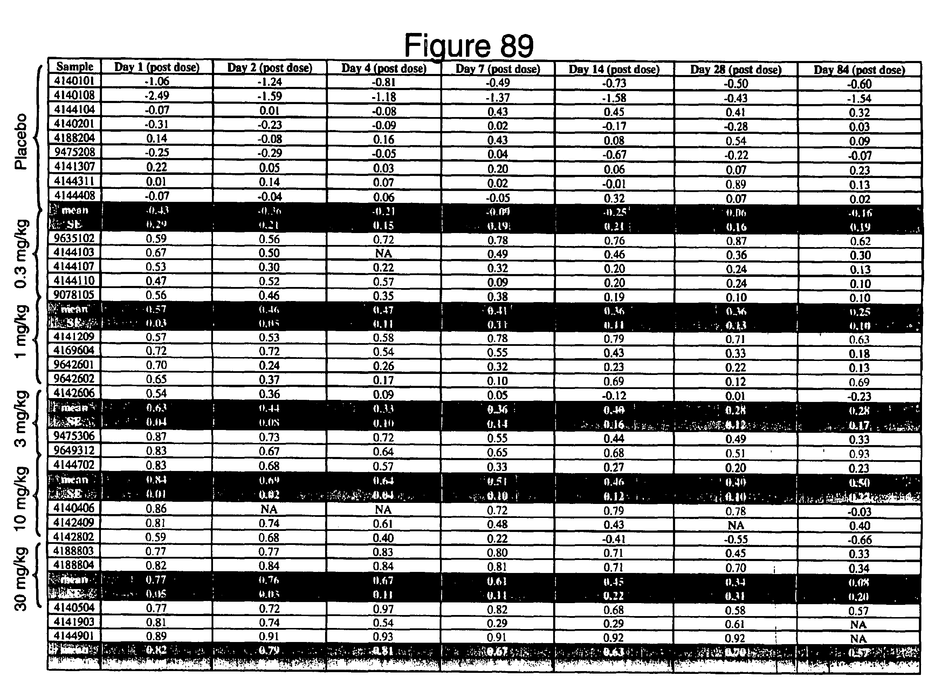

These 21 genes were neutralized in a dose-dependent dependent fashion by MEDI-545. See Figure 86 and 89. Heatmap (Figure 87a) and PCA calculations (Figure 87b) using these 21 genes showed neutralization of the upregulated IFN α/β gene signature in an SLE patient treated with 30 mg/kg MEDI-545, but not in placebo-treated SLE patients (Figure 88). Thus, it is evident that these genes could be used as a PD marker set.

### Stratification of 35 patients, by strength of type I IFN gene signature using the 21 genes

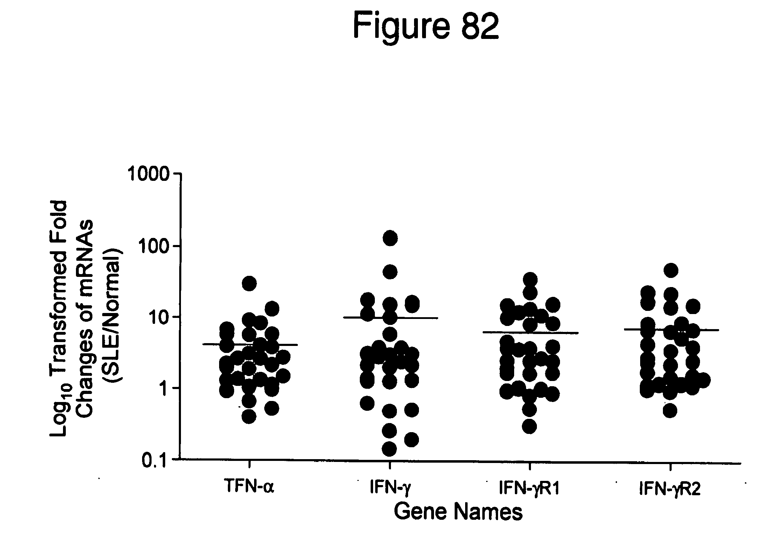

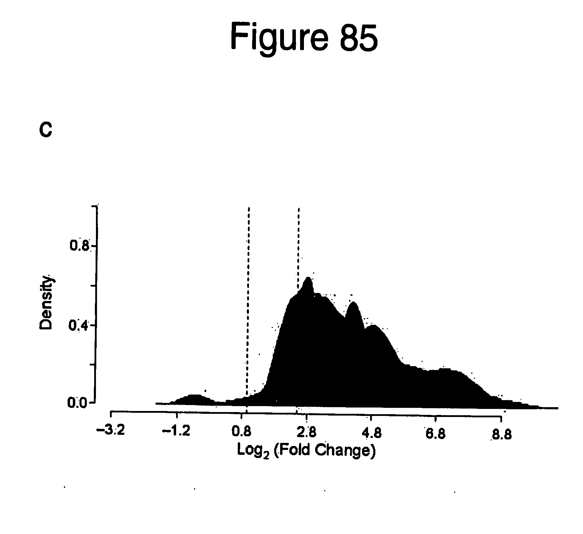

Figure 85 shows the stratification of 35 SLE patients into groups of high (20 patients), moderate (8 patients), and weak (7 patients) type I IFN gene signatures based on the distribution of fold change values (log₂ scale) of all 21 type I IFN-inducible genes and partitioned into each group by the median fold change of this distribution of 21 genes for each patient (vertical dashed lines), as measured by the dynamic array from Fluidigm. From figure 85, it is apparent that each patient distribution exhibits slight differences in skewness and basic shape/form, as this indicates the diversity in the various severity levels of SLE, based on the 21 type IFN-inducible gene selected. In a PCA plot for all SLE patients profiled in this study (n=100) and for the 24 healthy control samples using the 21 type I IFN-inducible genes, a clear distinction between SLE patients with an overexpressed type I IFN gene signature and those with weak or nondetectable type I IFN gene signatures is observed (Figure 82C). Furthermore, the SLE patients with weak or nondetectable type I IFN gene signatures were clustered together with healthy donors. Importantly, the partitioning between these groups using the 21-gene panel of type I IFN-inducible genes was similar to that observed with the larger 114-gene set (Figures 81A and 81B).

### Example 9: Multiple Type-I IFN Subtypes are Up-Regulated in Whole Blood of SLE Patients

To identify the type-I IFN subtypes responsible for the induction of the type-I IFN signature of SLE patients, mRNA levels of type-I IFN genes in SLE patient whole blood were measured.

Gene expression analysis was performed using a TaqMan Low Density Array (TLDA) from Applied Biosystems. Expression of type-I IFNα subtypes 1, 2, 5, 6, 7, 8, 14, 17, and 21 was monitored and compared in whole blood of SLE patients relative to healthy volunteers.

Double-stranded cDNA for each patient sample was pre-amplified using the TaqMan PreAmp Master Mix kit (Applied Biosystems). cDNA was pre-amplified by conducting 10 cycles of PCR on each patient sample using a pooled solution of primers, a pair for each gene analyzed on the array. The pre-amplified cDNA were diluted 1:5 with TE. A 50 µL volume of the diluted pre-amplified cDNA was added to a 50 µL volume of 2x TaqMan Universal PCR Master Mix (Applied Biosystems) and mixed. The array was loaded with the mixture using standard procedures and the loaded array was run on a 7900HT Fast Real-Time PCR System (Applied Biosystems). Data analysis of the resulting Ct values was conducted with the SDSv2.2.2 software tool (Applied Biosystems).

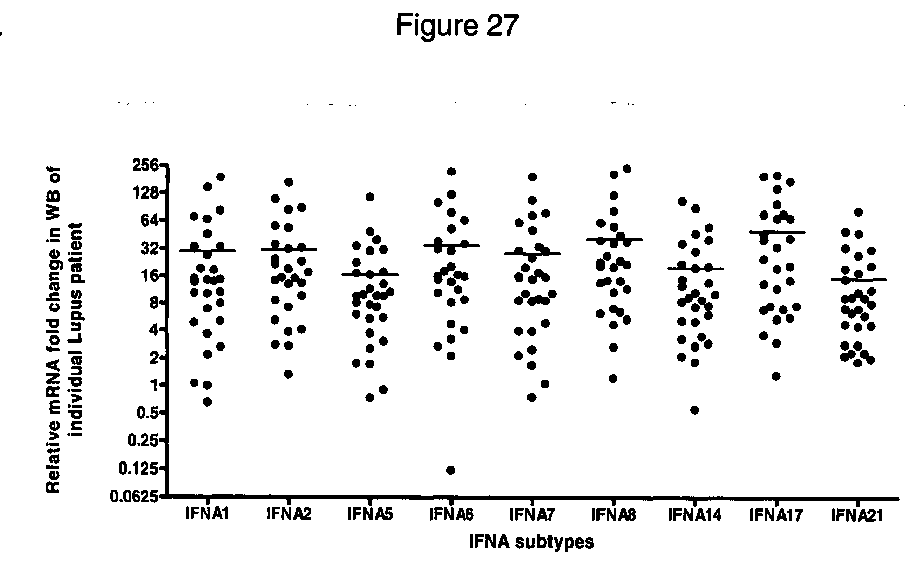

Figure 27 shows the relative overexpression of mRNA of nine IFNα subtypes in the whole blood of lupus patients relative to healthy volunteers. Many of these IFNα subtypes were upregulated at the mRNA level in the whole blood of SLE patients.

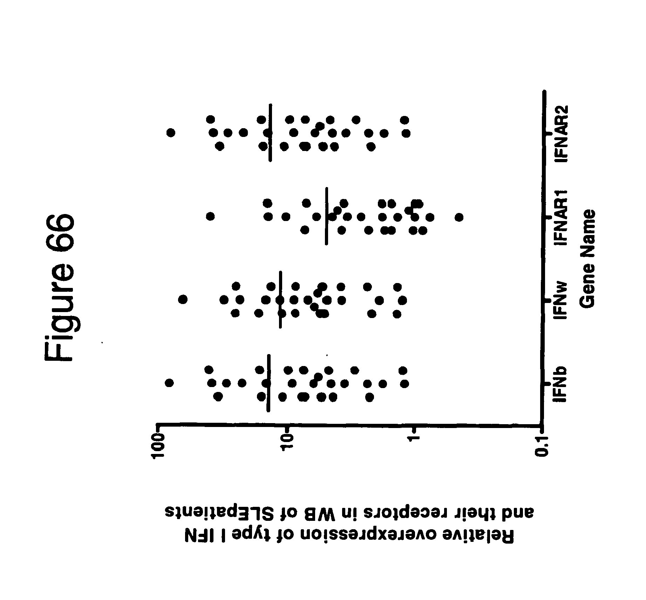

Figure 66 shows that IFNβ, IFNω and IFNAR1 and IFNAR2 genes are also overexpressed in whole blood of lupus patients relative to healthy volunteers.

Figure 82 shows that TNF-α, IFN-γ, IFN-γR1, and IFN-γR2 transcripts were also upregulated in WB of SLE patients (Figure 82). However, the relative magnitude of overexpression of these transcripts was less than that of the type I IFN family members, especially the IFN-α subtypes.

### Example 10: Ex vivo IFN-stimulated whole blood and keratinocytes of healthy individuals identifies a panel of type I IFN-inducible genes relevant to psoriasis

To identify type-I IFN inducible genes over-expressed in keratinocytes of lesions of psoriatic patients, whole blood and keratinocytes of healthy donors were stimulated *ex vivo* with a panel of IFNα subtypes, as well as IFNβ, IFNγ, and TNFα.

### Whole blood

Whole blood was collected from healthy donors in heparinized tubes. The total blood volume collected from each donor was pooled into a single culture flask and 3 mL of the total volume was aliquoted into a single well of a 6-well culture plate. Individual wells of blood were then exposed to a variety of treatments, including: vehicle (1 x PBS), a panel of IFNα subtypes (IFNα2a, -4b, -5, -6, -7, -8, -10, -16, -17), IFNβ, IFNω, IFNλ, IFNγ, leukocyte IFN, or TNFα. Following exposure, the blood was gently mixed by pipetting and incubated at 37°C, 5% CO₂ for 4 hrs (TNFα treatment was conducted for both 2hrs and 4hrs). Following the incubation period, 2.5 mL of blood was transferred to a PAXgene RNA tube and inverted 8-10 times. The PAXgene tubes were incubated at room temperature for two hours and then frozen (-20°C overnight, -70°C for long term storage) until further processing was required. Induction of gene expression by exposure to each of the treatment conditions was performed using Affymetrix GeneChip^{®} human genome U133 plus v2.0 arrays.

The various IFNα subtypes and IFNβ up-regulated 900-1200 probe sets by at least 2 fold. Of these, 689 probe sets (approximately 1.3% of all probe sets on the Affymetrix human genome U133 plus v2.0 array) were uniformly up-regulated by at least 2 fold in all donors by all ten IFNα subtypes and IFNβ. Using the same approach, 336 probe sets were identified as down-regulated by IFNα/β in the *ex vivo* stimulated whole blood.

Alterations in gene expression in healthy patient whole blood stimulated with TNFα were also observed at both the two and four hour time points. In all, 234 and 72 probe sets were up-regulated and down-regulated, respectively, by at least 2 fold in all donors. Furthermore, IFNγ challenge of whole blood for 4 hrs induced up-regulation of 304 probe sets and down-regulation of 52 probe sets by at least 2 fold. Little overlap was observed in the probe sets up-regutated by IFNα/β and TNFα (40 probes). By contrast, greater overlap was observed in the probe sets up-regulated by IFNα/β and IFNγ. 198 probes were up-regulated by at least 2-fold by both IFNα/β and IFNγ. Of the 198 probes up-regulated by at least 2-fold by both IFNα/β and IFNγ, the magnitude of up-regulation by IFNα/β was greater for about 2/3 of these probes (p value less than 0.05) than IFNγ.

Figure 30 provides the hierarchical clustering of 1384 probe sets differentially regulated by either IFNα/β, or IFNγ, or TNFα in *ex vivo* stimulated whole blood. From this hierarchical clustering the similar response of whole blood to challenge with IFNα subtypes and IFNβ can easily be observed, as can the similar but distinctly different effect of IFNγ from IFNα/β, and the drastically different effect of TNFα from IFNα/β.

Figure 31 a provides the hierarchical clustering of the relative expression of only the top 25 type-I IFN inducible probe sets identified in the *ex vivo* stimulated whole blood.

### Keratinocytes

Normal human keratinocytes (EpiDerm system, MatTek, Inc.) were grown under serum-free conditions according to the manufacturers instructions. Briefly, keratinocytes were maintained on tissue culture inserts at the air-liquid interface to maintain a multilayered, fully differentiated epithelial phenotype. Keratinocytes were stimulated with human leukocyte IFN (15, 50, 150, IU/mL, PBL Biomedical Labs), human IFNα2a (15-350 lU/ml, PBL Biomedical Labs), recombinant human TNFα (0.1 ng/ml, R+D Systems) or recombinant human IFNγ (3 ng/ml, R+D Systems). Epidermal cultures were harvested at 2, 4, or 18 hours post treatment for transcript analysis. Over 100 probe sets were identified as overexpressed in keratinocytes cultures stimulated with human IFNα2a and leukocyte IFN.

Figure 31b provides the hierarchical clustering of the relative expression of 25 type-I IFN inducible genes in *ex vivo* stimulated keratinocytes. The 25 type-I IFN inducible probe sets used to prepare the hierarchical clustering are the top 25 type-I IFN inducible probes identified in the *ex vivo* stimulated whole blood (those shown in Figure 31a). Many of the top 25 type-I IFN inducible probe sets in *ex vivo* stimulated whole blood are also induced in *ex vivo* stimulated keratinocytes. See, *e.g*., MX1, IFI27, OASI, IFI6, IFI44L, etc.

In addition, many of these genes were among those most overexpressed in the lesional skin of psoriasis patients. See discussion in Example 11, below.

### Example 11: Whole genome array profiling identified IFNα/β signaling pathway as the most significantly activated pathway in lesional skin of psoriasis patients

A comparison of gene expression profiles of skin samples from healthy donors and paired non-lesional/lesional skin samples from psoriasis patients was performed to identify a type-I interferon induced gene expression signature associated with psoriatic skin lesions. Briefly, skin samples of 21 normal healthy control donors (5 samples obtained from Biochain, 14 from ILSbio, and 2 from Dr. James Krueger's lab) and 26 paired non-lesional/lesional skin samples of 24 psoriatic patients (21 pairs obtained from Asterand, and 5 from Dr. James Krueger's 1ab) were obtained. Three additional lesional skin samples from 3 psoriatic patients were obtained. These 3 additional lesional skin samples lacked a paired non-lesional skin sample because the non-lesional skin sample either did not yield sufficient cRNA for hybridization or the scanned array for the non-lesional skin sample had high scaling factors that were more than 3-fold of average.

Total RNA from the samples was extracted using the Qiagen RNAeasy-Mini kit (Hilden, Germany). The purity and concentration of the extracted RNA were determined spectrophotometrically (260/280 > 1.9). RNA quality was assessed on an Agilent 2100 Bioanalyzer using the RNA 6000 Nano LabChip^{®}. Generation of blotin-labeled amplified cRNA, from 2 ug of total RNA, was accomplished using the Affymetrix GeneChip^{®} One-Cycle cDNA Synthesis kit and the Affymetrix GeneChip^{®} IVT Labeling kit. Concentration and purity of the cRNA product were determined spectrophotometrically.

Twenty micrograms of each biotin-labeled cRNA was fragmented for hybridization on Affymetrix GeneChip^{®} human genome U133 plus v2.0 arrays. Fragmented cRNA was prepared for hybridization as outlined in the Affymetrix GeneChip^{®} manual. Hybridization was conducted overnight in a model 320 rotating hybridization oven set at 45°C. All GeneChip^{®} wash and staining procedures were performed on an Affymetrix Model 450 Fluidics station. Arrays were scanned on an Affymetrix GeneChip^{®} Scanner 3000. Data capture and initial array quality assessment were performed with the GeneChip Operating Software (GCOS) tool.

Stratagene's (La Jolla, CA) ArrayAssist^{®} Lite software was used to calculate probe-level summaries (GC-RMA normalization algorithm) from the array CEL files. R packages (R development core team) samr & qvalue were used to generate differentially regulated genes. PCA and hierarchical clustering analyses were performed in both SpotFire and R (R Development Core Team). SAM & FDR were used to select differentially regulated genes (pairwise comparison between lesional and non-lesional skin, lesional and normal skin, and non-lesional and normal skin). Probe sets with a fold-change of at least 2 and q value less than or equal to 0.05 were considered to be differentially regulated. PCA and hierarchical clustering were performed in both SpotFire and bioconductor R.

Overall, 1408 probe sets were up-regulated and 1465 probe sets were down-regulated in lesional skin compared to non-lesional skin. Although the downregulated genes outnumbered the upregulated genes in the lesional skin, the magnitude of differential regulation of the upregulated genes was much greater as a whole. For example, 318 probe sets were upregulated by at least four fold in the lesional skin, while only 84 probe sets were downregulated by at least four fold in the lesional skin. Among them, 96 probe sets were upregulated by at least eight fold in the lesional skin, while only six probe sets were downregulated by at least eight fold.

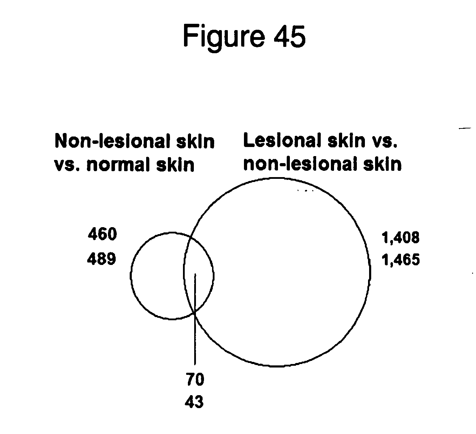

463 probe sets were also up-regulated and 489 probe sets were down-regulated in non-lesional skin compared to normal skin. Figure 45 provides a Venn diagram of the probe sets both upregulated (downregulated) in lesional skin and non-lesional skin relative to normal healthy skin. Only 70 of the 1408 upregulated probe sets in the lesional skin were also upregulated in non-lesional skin. Meanwhile, only 43 of the 1465 probe sets downregulated in the lesional skin were also downregulated in the non-lesional skin. These data suggested that the molecular events and biological changes from the non-lesional skin to lesional skin were quite different from those from the normal skin to the non-lesional skin.

To identify the most statistically significant signaling pathways altered in psoriasis, the list of differentially regulated genes were submitted to GeneGo for pathway and network analysis. Briefly, the pathway and network analysis was conducted with the MetaCore^{™} integrated software suite from GeneGo, Inc. (St. Joseph, MI). The significance, given a particular pathway or network, is approximated by a hypergeometric distribution where the p-value essentially represents the probability of a particular gene set mapping arising by chance, given the numbers of genes in the set of all genes on pathway maps, genes on a particular pathway map and genes in the experiment.

Fifty seven signaling pathways were significantly altered in lesional skin compared to non-lesional skin, a majority of which were involved in immune response and cell cycle. The IFNα/β signaling pathway was the most significantly altered in lesional skin with a *p* value of 3.8 x 10⁻¹³. IFNα/β signaling pathway members such as IFNα, IFNβ, IFNAR1, IFNAR2, STAT1, IRF I , MPL, ISG15, IFI6 were all significantly overexpressed in lesional skin compared to uninvolved skin.

Overall, 22 signaling pathways were activated and 37 signaling pathways were inhibited (p<0.05) in the lesional skin compared to non-lesional skin. All the putative signaling pathways activated were either cytokine and chemokine mediated signaling pathways or were involved in immune responses. For example, IFNγ, TNFα and onconstatin M signaling pathways were activated in the lesional skin of psoriatic patients. Of all the signaling pathways altered in lesional and non-lesional skin, IFNα/β signaling pathway topped the list with a p value of 6.6x10⁻²⁶ (Figure 46). Components of the pathway like IFNα subtypes, IFNβ, IFNAR1, IFNAR2, STAT1, IRF1, MPL, ISG15, IFI6 were all significantly overexpressed in lesional skin compared to non-lesional skin of psoriatic patients.

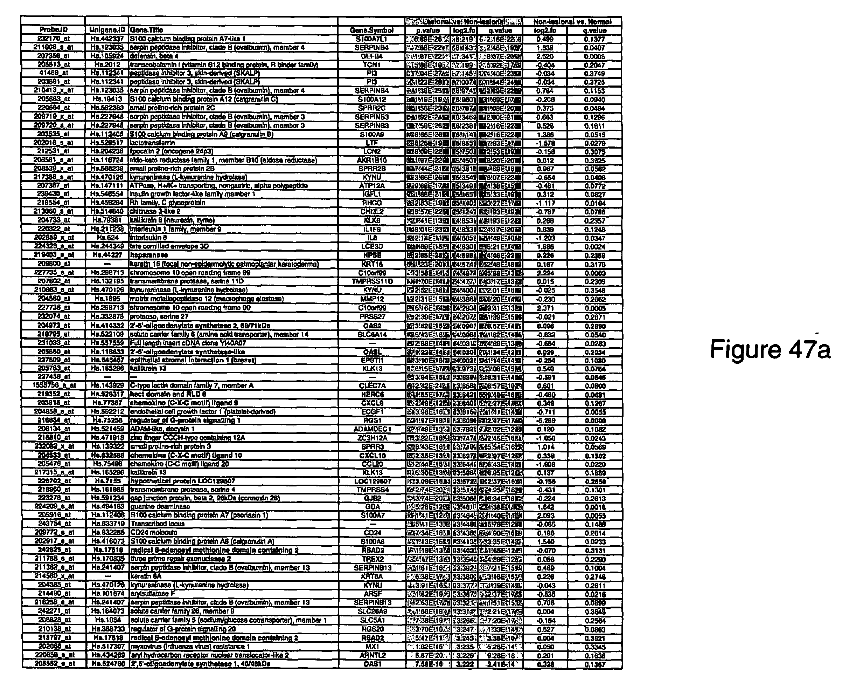

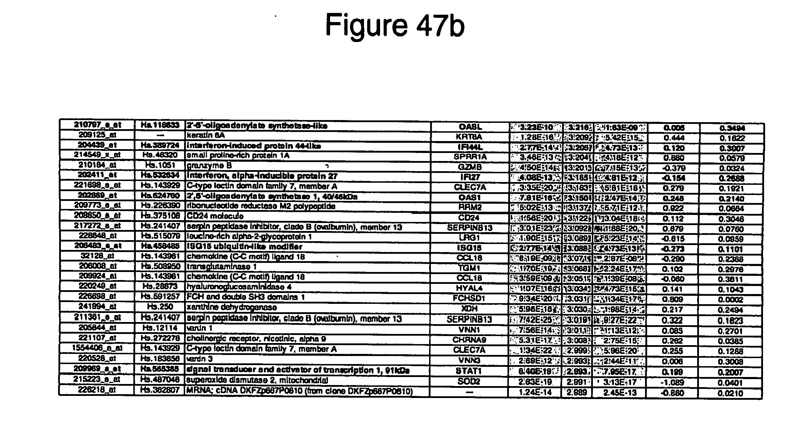

Using the list of probe sets identified to be type-1 IFN inducible in the whole blood and keratinocyte *ex vivo* stimulation studies (Example 10), 164 of the 1408 (approximately 11.7%) probe sets upregulated in lesional relative to non-lesional skin were identified as type-I IFN inducible. Fisher's exact test calculated a *p* value (one-tailed t test) less than 0.0001, suggesting that the observed overexpression of type-I IFN genes in lesional skin of psoriatic patients was statistically significant. The type-I IFN induced genes were also many of the most highly upregulated genes in the lesional relative to non-lesional psoriatic skin. Nineteen percent of the top 100 and 200 most upregulated probe sets in lesional skin relative to non-lesional skin were type-I IFN genes. See Figure 47a and b for the top 100 upregulated probe sets in lesional skin. These genes included STAT1, a key component in fonning the ISGF3 complex; IRF7, a master regulator of the IFNα/β mediated immune response; MYD88, which governs the induction of CD8⁺ T-cell responses with IRF7; IRF1, a transcriptional activator for the type-1 IFN genes; OAS family members OAS1, OAS2, OAS3, mediators of resistance to virus infection; ISG15, a ubiquitin-like protein that becomes conjugated to many cellular proteins upon activation by IFNα/β; and members of the ISG15 signaling pathways such as USP18, UBE2L6, and HERC5. This enrichment of type I IFN genes indicated them as the most overexpressed genes in lesional skin of psoriatic patients.

Table 26 lists, in descending order, the top 50 IFN induced probes in lesional skin compared to non-lesional skin of psoriasis patients. Table 26 not only compares the log2-based fold change (log2 fc) and q value for each of the 50 most upregulated type I IFN inducible genes in lesional relative to non-lesional skin of psoriasis patients, it also compares the log 2-based fold change and q value for these 50 genes in non-lesional skin of psoriasis patients relative to healthy control patients.

**Table 26: The frequency of upregulation of the top 50 type-I IFN induced probes in lesional relative to non-lesional skin in psoriasis patients**

| | | | | Lesional vs. Non-lesional | | Non-lesional vs. Normal | |
|---|---|---|---|---|---|---|---|
| Probe ID | Unigene ID | Gene Title | Gene Symbol | log2 fc | q value | log2 fc | q value |
| 219403_s_at | Hs.44227 | heparanase | HPSE | 4.598 | 4.46E-22 | 0.226 | 0.23589 |
| 204972_at | Hs.414332 | 2-5-oligoadenylate synthetase 2, 69/71kDa | OAS2 | 4.098 | 8.57E-14 | 0.096 | 0.28896 |
| 205660_at | Hs.118533 | 2'-5'-oligoadenylate Synthetase-like | OASL | 4.030 | 1.34E-12 | 0.029 | 0.20341 |
| 227609_at | Hs.546467 | epithelial stromal interaction 1 (breast) | EPSTI1 | 4.002 | 1.14E-14 | -0.254 | 0.10798 |
| 227458_at | --- | --- | --- | 3.859 | 9.31E-14 | -0.591 | 0.05449 |
| 219352_at | Hs.529317 | hecl domain and RLO 6 | HERC6 | 3.842 | 9.49E-16 | -0.460 | 0.04810 |
| 216834_at | Hs.75256 | regulator of G-protein signalling 1 | RGS1 | 3.809 | 2.47E-17 | -5.269 | 0.00000 |
| 204533_at | Hs.632586 | chemokine (C-X-C motif) ligand 10 | CXCL10 | 3.697 | 2.97E-12 | 0.338 | 0.13024 |
| 226702_at | Hs.7155 | hypothetical protein LOC129607 | LOC129607 | 3.572 | 2.37E-16 | -0.156 | 0.26500 |
| 242625_at | Hs.175186 | radical S-adenosyl methionine domain containing 2 | RSAD2 | 3.403 | 1.65E-12 | -0.070 | 0.31309 |
| 213797_at | Hs.17518 | radical S-adenosyl methionine domain containing 2 | RSAD2 | 3.243 | 3.36E-10 | 0.004 | 0.38209 |
| 202086_at | Hs.517307 | myxovirus (influenza virus) resistance 1, interferon-inducible protein p78 (mouse) | MX1 | 3.235 | 5.28E-14 | 0.050 | 0.33453 |
| 205552_s_at | Hs.524760 | 2',5'-oligoadenylate synthetase 1, 40/46kDa | OAS1 | 3.222 | 2.41E-14 | 0.328 | 0.13689 |
| 210797_s_at | Hs.115633 | 2'-5'oligoadenylate synthetase-like | OASL | 3.216 | 1.63E-09 | 0.005 | 0.34940 |
| 204439_at | Hs.389724 | interferon-induced protein 44-like | IFI44L | 3.205 | 4.73E-13 | 0.120 | 0.30073 |
| 202411_at | Hs.532634 | interferon, alpha-inducible protein 27 | IFI27 | 3.165 | 4.81E-12 | -0.154 | 0.26878 |
| 202869_at | Hs.524760 | 2',5'-oligoadenylate synthetase 1, 40/46kDa | OAS1 | 3.150 | 2.47E-14 | 0.248 | 0.21403 |
| 205483_s_at | Hs.458485 | ISG15 ubiquitin-like modifier | ISG15 | 3.088 | 4.73E-13 | -0.273 | 0.11013 |
| 209969_s_at | Hs.565365 | signal transducer and activator of transcription 1, 91kDa | STAT1 | 2.993 | 7.95E-17 | 0.199 | 0.20072 |
| 228531_at | Hs.65641 | sterile alpha motif domain containing 9 | SAMD9 | 2.846 | 5.42E-14 | -0.033 | 0.35359 |
| 204415_at | Hs.523847 | interferon, alpha-inducible protein 5 | IFI6 | 2.769 | 7.23E-09 | -0.045 | 0.29074 |
| 214453_s_at | Hs.82316 | interferon-induced protein 44 | IFI44 | 2.679 | 1.94E-12 | 0.086 | 0.32618 |
| 222838_at | Hs.517265 | SLAM family member 7 | SLAMF7 | 2.659 | 1.60E-16 | -0.046 | 0.31222 |
| 219684_at | Hs.43388 | receptor transporter protein 4 | RTP4 | 2.649 | 3.73E-11 | 0.497 | 0.04912 |
| 203127_s_at | Hs.435661 | serine palmitoyltransferase, long chain base subunit 2 | SPTLC2 | 2.628 | 1.04E-20 | -1.016 | 0.00017 |
| 205569_at | Hs.518448 | lysosomal-associated membrane protein 3 | LAMP3 | 2.569 | 2.64E-09 | 0.293 | 0.22865 |
| 219691_at | Hs.65641 | sterile alpha motif domain containing 9 | SAMD9 | 2.559 | 1.30E-13 | 0.011 | 0.37349 |
| 223220_s_at | Hs.518200 | poly (ADP-ribose) polymerase family, member 9 | PARP9 | 2.553 | 1.08E-15 | 0.069 | 0.31416 |
| AFFX-HUMISG | Hs.565365 | signal transducer and activator of transcription 1, 91kDa | STAT1 | 2.525 | 1.64E-10 | 0.706 | 0.03338 |
| 212268_at | Hs.381167 | serpin peptidase inhibitor, clade B (ovalbumin), member 1 | SERPINB1 | 2.510 | 3.02E-15 | -0.605 | 0.07749 |
| 216202_s_at | Hs.435661 | serine palmitoyltransferase, long chain base subunit 2 | SPTLC2 | 2.507 | 1.17E-13 | -0.682 | 0.01693 |
| 229450_at | --- | **---** | --- | 2.492 | 1.50E-14 | 0.224 | 0.20674 |
| 208436_s_at | Hs.166120 | interferon regulatory factor 7 | IRF7 | 2.448 | 6.90E-15 | -0.578 | 0.01612 |
| AFFX-HUMISG | Hs.565365 | signal transducer and activator of transcription 1, 91kDa | STAT1 | 2.444 | 3.03E-10 | 0.516 | 0.05854 |
| 204747_at | Hs.47338 | interferon-induced protein with tetratricopeptide repeats 3 | IFIT3 | 2.424 | 2.15E-14 | 0.365 | 0.07219 |
| 229390_at | Hs.381220 | hypothetical protein LOC441168 | RP1-93H18.5 | 2.400 | 2.59E-12 | -0.369 | 0.11426 |
| 218400_at | Hs.528634 | 2'-5'-oligoadenylate synthetase 3, 100kDa | OAS3 | 2.397 | 3.83E-14 | 0.179 | 0.11631 |
| 235276_at | --- | **---** | --- | 2.386 | 3.61E-15 | 0.057 | 0.32771 |
| 203153_at | Hs.20315 | interferon-induced protein with tetratricopeptide repeats 1 | IFIT1 | 2.351 | 1.17E-10 | 0.054 | 0.34454 |
| 210873_x_at | Hs.348983 | apolipoprotein B mRNA editing enzyme, catalytic polypeptide-like 3A | APOBEC3A | 2.348 | 1.35E-07 | -0.048 | 0.30119 |
| 204698_at | Hs.459265 | interferon stimulated exonuclease gene 20kDa | ISG20 | 2.337 | 1.50E-12 | -0.644 | 0.05052 |
| 232666_at | Hs.528634 | 2'-5'oligoadenylate synthetase 3. 100kDa | OAS3 | 2.236 | 4.50E-10 | 0.077 | 0.04816 |
| 222881_at | Hs.44227 | heparanase | HPSE | 2.230 | 3.47E-15 | 0.221 | 0.17127 |
| 205241_at | Hs.567405 | SCO cytochrome oxidase deficient homolog 2 (yeast) | SCO2 | 2.208 | 1.90E-17 | -0.285 | 0.08517 |
| AFFX-HUMISG | Hs.565365 | signal transducer and activator of transcription 1,91kDa | STAT1 | 2.205 | 5.29E-10 | 0.397 | 0.10218 |
| 206553_at | Hs.414332 | 2'-5'-oligoadenylate synthetase 2,69/71kDa | OAS2 | 2.183 | 1.34E-09 | 0.043 | 0.14755 |
| 207387_s_at | Hs.1466 | glycerol kinase | GK | 2.160 | 9.38E-14 | 0.014 | 0.37488 |
| 219716_at | Hs.257352 | apolipoprotein L, 6 | APOL6 | 2.123 | 3.03E-11 | -0.126 | 0.19251 |
| 202270_at | Hs.62661 | guanylate binding protein 1, interferon-inducible, 67kDa | GBP1 | 2.113 | 4.67E-14 | -0.053 | 0.31367 |

Removal of ESTs, hypothetical proteins, and duplications of genes due to identification by multiple probe sets produced Table 27. Table 27 provides, in descending order, the top 50 most upregulated type-I IFN genes in lesional skin compared to non-lesional skin. For genes identified by more than one probe set, only the probe set detected as most upregulated is provided.

**Table 27: Top 50 type-I IFN induced genes in lesional relative to non-lesional skin in psoriasis patients**

| Probe.ID | Unigene.ID | Gene.Title | Gene.Symbol | log2.fc | q.value(fdr) | % Prevalance |
|---|---|---|---|---|---|---|
| 219403_s_at | Hs.44227 | heparanase | HPSE | 4.60 | 4.46E-22 | 100.00 |
| 204972_at | Hs.414332 | 2'-5'-oligoadenylate synthetase 2, 69/71kDa | OAS2 | 4.10 | 8.57E-14 | 96.15 |
| 205660_at | Hs.118633 | 2'-5'-oligoadenylate synthetase-like | OASL | 4.03 | 1.34E-12 | 96.15 |
| 227609_at | Hs.546467 | epithelial stromal interaction 1 (breast) | EPSTI1 | 4.00 | 1.14E-14 | 92.31 |
| 219352_at | Hs.529317 | hect domain and RLD 6 | HERC6 | 3.84 | 9.49E-16 | 96.15 |
| 216834_at | Hs.75256 | regulator of G-protein signalling 1 | RGS1 | 3.81 | 2.47E-17 | 100.00 |
| 204533_at | Hs.632586 | chemokine (C-X-C motif) ligand 10 | CXCL10 | 3.70 | 2.97E-12 | 100.00 |
| 242625_at | Hs.17518 | radical S-adenosyl methionine domain containing 2 | RSAD2 | 3.40 | 1.65E-12 | 88.46 |
| 202086_at | Hs.517307 | myxovirus (influenza virus) resistance 1, interferon-inducible protein p78 (mouse) | MX1 | 3.24 | 5.28E-14 | 92.31 |
| 205552_s_at | Hs.524760 | 2',5'-oligoadenylate synthetase 1, 40/46kDa | OAS1 | 3.22 | 2.41E.14 | 96.15 |
| 204439_at | Hs.389724 | interferon-induced protein 44-like | IFI44L | 3.21 | 4.73E-13 | 88.46 |
| 202411_at | Hs.532634 | interferon, alpha-inducible protein 27 | IFI27 | 3.17 | 4.81E-12 | 92.31 |
| 205483_s_at | Hs.458485 | ISG15 ubiquitin-like modifier | ISG1S | 3.09 | 4.73E-13 | 92.31 |
| 209969_s_at | Hs.565365 | signal transducer and activator of transcription 1, 91kDa | STAT1 | 2.99 | 7.95E-17 | 96.15 |
| 228531_at | Hs.65641 | sterile alpha motif domain containing 9 | SAMD9 | 2.85 | 5.42E-14 | 92.31 |
| 204415_at | Hs.523847 | interferon, alpha-inducible protein 6 | IFI6 | 2.77 | 7.23E-09 | 84.62 |
| 214453_s_at | Hs.82316 | interferon-induced protein 44 | IFI44 | 2.68 | 1.94E-12 | 92.31 |
| 222838_at | Hs.517265 | SLAM family member 7 | SLAMF7 | 2.66 | 1.60E-16 | 92.31 |
| 219684_at | Hs.43388 | receptor transporter protein 4 | RTP4 | 2.65 | 3.73E-11 | 88.46 |
| 203127_s_at | Hs.435661 | serine palmitoyltransferase, lonq chain base subunh 2 | SPTLC2 | 2.63 | 1.04E-20 | 100.00 |
| 205569_at | Hs.518448 | lysosomal-associated membrane protein 3 | LAMP3 | 2.57 | 2.64E-09 | 96.15 |
| 223220_s_at | Hs.518200 | poly (ADP-ribose) polymerase family, member 9 | PARP9 | 2.55 | 1.08E-15 | 88.46 |
| 212268_at | Hs.381167 | serpin peptidase inhibitor, clade B (ovalbumin), member 1 | SERPINB1 | 2.51 | 3.02E-15 | 88.46 |
| 208436_s_at | Hs.166120 | interferon regulatory factor 7 | IRF7 | 2.45 | 6.90E-15 | 96.15 |
| 204747_at_at | Hs.47338 | interferon induced protein with tetratricopeptide repeats 3 | IFIT3 | 2.42 | 2.15E-14 | 92.31 |
| 218400_at | Hs.528634 | 2'-5'-oligoadenylate synthetase 3, 100kDa | OAS3 | 2.40 | 3.83E-14 | 100.00 |
| 203153_at | Hs.20315 | interferon-induced protein with tetratricopeptide repeats 1 | IFIT1 | 2.35 | 1.17E-10 | 84.62 |
| 210873_x_at | Hs.348983 | apolipoprotein 8 mRNA editing enzyme, catalytic polypeptide-like 3A | APOBEC3A | 2.35 | 135E-07 | 80.77 |
| 204698_at | Hs.459265 | interferon stimulated exonuclease gene 20kDa | ISG20 | 2.34 | 1.50E-12 | 92.31 |
| 205241_at | Hs.567405 | SCO cytochrome oxidase deficient homolog 2 (yeast) | SCO2 | 2.21 | 1.90E-17 | 96.15 |
| 207387_s_at | Hs.1466 | glycerol kinase | GK | 2.16 | 9.38E-14 | 92.31 |
| 219716_at | Hs.257352 | apolipoprotein L, 6 | APOL6 | 2.12 | 3.03E-11 | 92.31 |
| 202270_at | Hs.62661 | guanylate binding protein 1, interferon-inducible, 67kDa | GBP1 | 2.11 | 4.67E-14 | 92.31 |
| 229625_at | Hs.513726 | Guanylate binding protein 5 | GBP5 | 2.07 | 7.52E-10 | 88.46 |
| 228617_at | Hs.441975 | XIAP associated factor-1 | BIRC4BP | 2.05 | 3.41E-12 | 84.62 |
| 206513_at | Hs.281898 | absent in melanoma 2 | AIM2 | 2.04 | 2.32E-08 | 76.92 |
| 218943_s_at | Hs.190622 | DEAD (Asp-Glu-Ala-Asp) box polypeptide 58 | DDX58 | 2.00 | 1.39E-10 | 88.46 |
| 203148_s_at | Hs.575631 | tripartite motif-containing 14 | TRIM14 | 1.94 | 2.17E-17 | 96.15 |
| 213293_s_at | Hs.501778 | tripartite motif-containing 22 | TRIM22 | 1.89 | 1.36E-12 | 88.46 |
| 214838_at | --- | SFT2 domain containing 2 | SFT2D2 | 1.88 | 5.30E-17 | 92.31 |
| 231769_at | Hs.464419 | F-box protein 6 | FBXO6 | 1.86 | 6.34E-14 | 88.46 |
| 227697_at | Hs.527973 | suppressor of cytokine signaling 3 | SOCS3 | 1.82 | 4.55E-10 | 88.46 |
| 206632_s_at | Hs.226307 | apolipoprotein B mRNA editing enzyme, catalytic polypeptide-like 3B | APOBEC3B | 1.81 | 9.42E-10 | 92.31 |
| 201649_at | Hs.425777 | ubiquitin-conjugating enzyme E2L 6 | UBE2L6 | 1.81 | 2.15E-13 | 84.62 |
| 204702_s_at | Hs.404741 | nuclear factor (erythroid-derived 2)-like 3 | NFE2L3 | 1.80 | 1.71E-16 | 96.15 |
| 202531_at | Hs.436061 | interferon regulatory factor 1 | IRF1 | 1.79 | 2.13E-13 | 80.77 |
| 204994_at | Hs.926 | myxovirus (influenza virus) resistance 2 (mouse) | MX2 | 1.75 | 7.99E-09 | 69.23 |
| 215966_x_at | --- | glycerol kinase pseudogene 3 | GKP3 | 1.73 | 3.33E-11 | 80.77 |
| 207655_s_at | Hs.444049 | B-cell linker | BLNK | 1.71 | 2.28E-14 | 96.15 |
| 216598_s_at | Hs.303649 | chemokine (C-C motif) ligand 2 | CCL2 | 1.71 | 4.80E-07 | 65.38 |

The fold changes (log2 fc) were calculated based on relative transcript level between paired lesional skin and non-lesional skin. Q values were calculated based on FDR. Prevalence tabulated the percentage of the 26 paired lesional and non-lesional skin that had at least 2-fold overexpression of the genes listed in the table.

These top 50 type-I IFN induced genes in lesional relative to non-lesional skin in psoriasis patients were overexpressed, on average, 3.2-fold (CCL2 and BLNK) to 24-fold (HPSE) more in the lesional skin. In addition, all of the genes in the table, except CCL2 and AIM2, were upregulated in at least 84% of the paired lesional/non-lesional skin biopsies (23 of the 26 pairs) of the psoriasis patients. This robust upregulation of a large panel of type-I IFN genes across lesional versus non-lesional skin samples of psoriasis patients provided a strong rationale for their use as PD markers.

As briefly alluded to, above, upregulation of type-I interferon inducible genes was consistently observed across psoriasis patients. Table 28 provides the average and median fold change of the top 25 most upregulated type-I IEN probe sets for each paired lesional/non-lesional skin sample. The top 25 most upregulated type-I IFN probe sets were consistently observed to detect elevated gene expression in the lesional relative to non-lesional skin of each individual psoriasis patient.

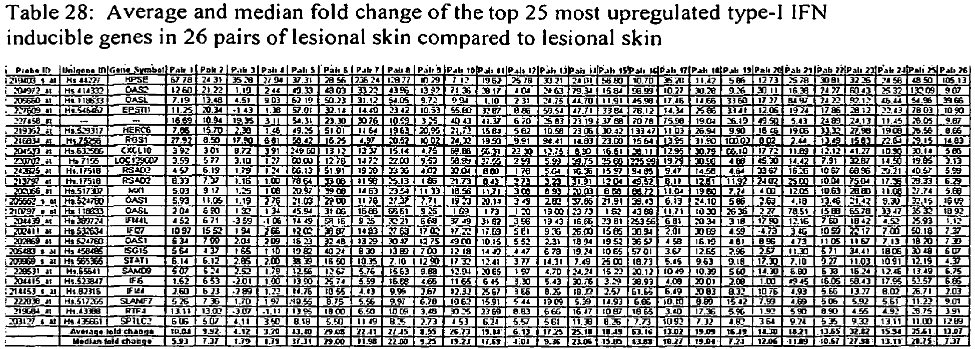

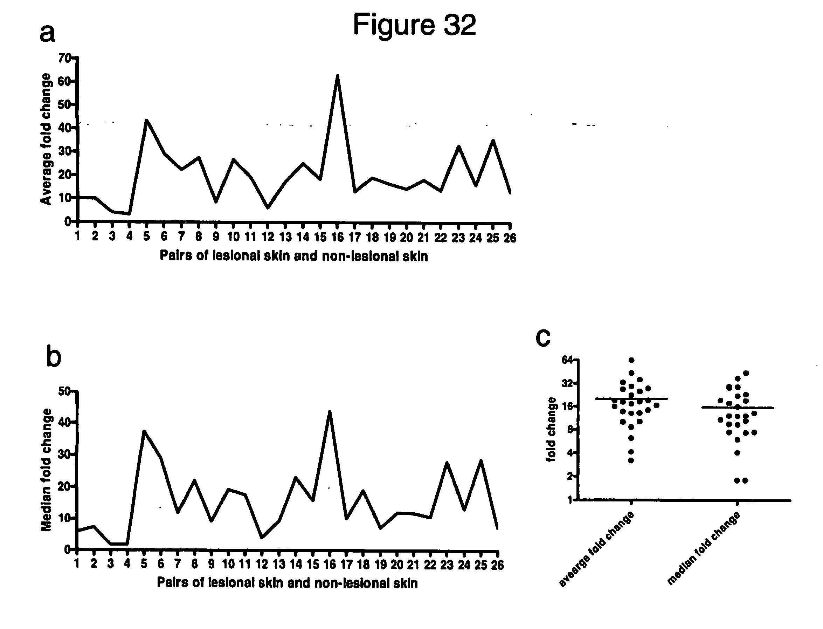

Figure 32 provides a graphic of the distribution of the average and median fold changes among the different pairs of lesional and non-lesional skin. The prevalent and uniform upregulation of the most overexpressed type-I IFN genes in lesional skin of psoriatic patients verified their usefulness as PD markers.

Seventeen probe sets were also observed as underexpressed in lesional skin that were also down-regulated by IFNα/β in the *ex vivo* stimulation studies described in Example 10. These genes include CYP1B1, TGST1, RRAGD, IRS2, MGST1, TGFBR3, and RGS2.

### Example 12: Expression of type-I IFN genes is not significantly altered in normal skin relative to non-lesional skin of psoriatic patients

Although the array data obtained in Example 11 identified overexpression of numerous type-I IFN-inducible genes in lesional relative to non-lesional skin, it identified only 5 probe sets overexpressed in non-lesional skin relative to normal control skin. The *p* value of Fisher's exact test (two-tailed t-test) was 0.581, which suggested that the overexpression of the type-I IFN genes is not statistically significant in the non-lesional skin of the psoriasis patients over normal skin.

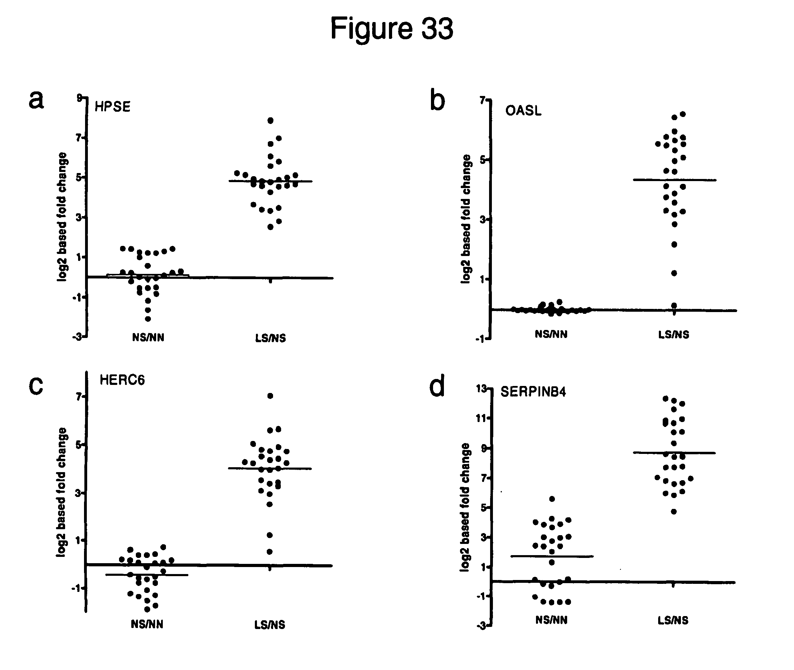

As shown in Table 26 (Example 11), most of the genes identified as being top 50 type-I IFN-induced genes in lesional relative to non-lesional skin were comparably expressed in non-lesional skin relative to normal skin controls (several genes, *e.g*., RGS1, SPTLC2, are downregulated in the non-lesional skin compared to normal skin). Figure 33 provides a graphical representation of the relative expression of 3 type-I IFN inducible genes (HPSE, OASL, and HERC6; included as top 50 type-I IFN-induced probe sets in lesional relative to non-lesional skin), and 1 non type-I IFN inducible gene (SERPINB4) in both (a) lesional skin compared to non-lesional skin and (b) non-lesional skin compared to normal skin. The overexpression of genes HPSE, OASL, and HERC6 in lesional skin compared to non-lesional skin is both statistically significant (as evidenced by the very small *p* value) and large in scale (between 12-250 fold overexpression on average). SERPINB4 is overexpressed in non-lesional skin by about 3-4 fold compared to normal skin, but upregulated by well over 200 fold in lesional skin compared to non-lesional skin.

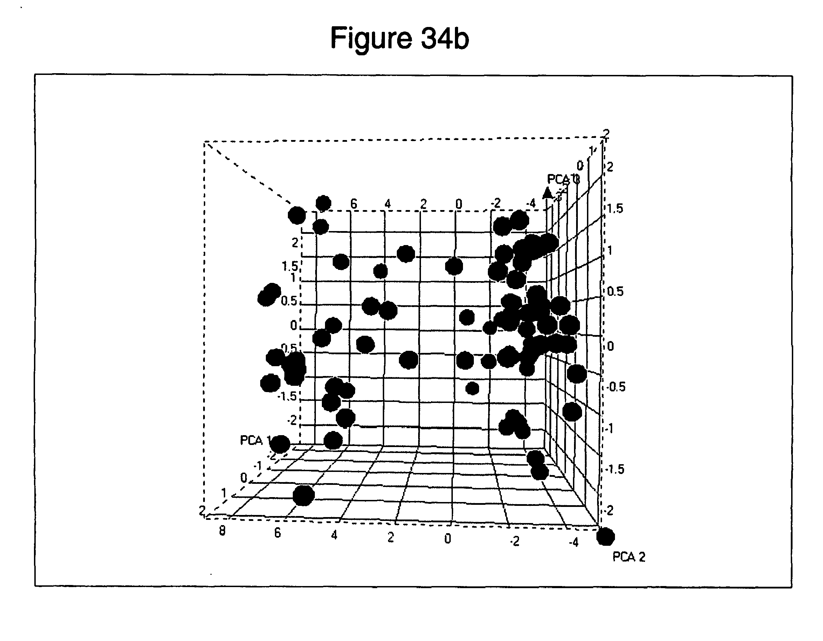

Analysis of normal healthy, lesional psoriasis, and non-lesional psoriasis skin samples using the 164 probe sets identified in Example 11 as type-I IFN inducible, showed a clustering of lesional psoriasis samples and a clustering of non-lesional psoriasis and normal healthy skin samples. Figure 34a provides heatmap of unsupervised hierarchical clustering of all lesional, non-lesional, and normal skin samples profiled using the 164 type-I IFN-inducible probe sets in lesional skin compared to non-lesional skin of psoriasis patients. It can be observed that nearly all (all but three) of the lesional skin samples clustered together, while nearly all of the non-lesional and normal skin samples clustered together. Figure 34b provides a PCA plot of the skin samples using the same 164 upregulated type-I IFN inducible probe sets. Again, the normal skin samples and the non-lesional skin samples mostly clustered together, indicating similar levels of expression of the 164 genes. Also, the majority of the lesional skin samples were separated from the normal and non-lesional skin samples, indicating that the lesional samples exhibited a distinct overexpression of the type-I IFN inducible genes that was separable from the gene expression levels of the normal and non-lesional skin samples.

These observations were further confirmed by gene pathway analysis. GeneGo analysis showed that the possibility of an alteration in the IFNα/β signaling pathway of non-lesional skin of psoriasis patients relative to normal skin had a *p* value close to 1. A distinctive separation of lesional skin samples from non-lesional skin samples and normal skin samples was even observed when clustering samples based on the transcript profile of an entire genome array. See Figure 47.

### Example 13: Validation of type-I IFN-inducible gene up-regulation in psoriatic lesional skin using taqMan-based assays

A BioMark^{™} 48.48 dynamic array (taqMan-based assay) from Fluidigm was used to validate the results of the Affymetrix GeneChip^{®} human genome U133 plus v2.0 arrays, results indicating that type-I IFN genes are up-regulated in lesional psoriatic relative to non-lesional psoriatic or normal skin samples.

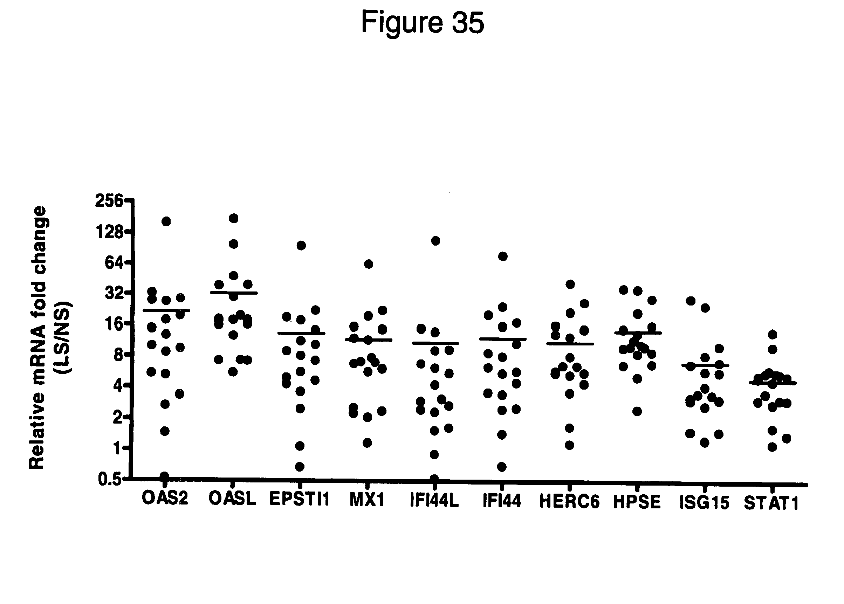

Eighteen pairs of lesional and non-lesional skin samples from 18 psoriasis patients were used for the gene expression analysis. Twenty nine of these genes were type-I IFN inducible genes while 11 were highly upregulated in lesional skin but were not IFN-inducible genes, *e.g*., S100A9, S100A12, SERPINGB4, and KLK13. Each of these genes was selected based on prevalence and significance of overexpression in lesional skin. The overexpression of all genes in the lesional skin was confirmed by taqMan qRT-PCR, the majority of which showed very good correlation between microarray and taqMan assays. Figure 35 provides taqMan data showing overexpression of each of ten (OAS2, OASL, EPSTI1, MX1, IFI44L, IFI44, HERC6, HPSE, ISG15, and STAT1) type-I IFN-inducible genes in lesional skin in the 18 paired lesional/non-lesional samples.

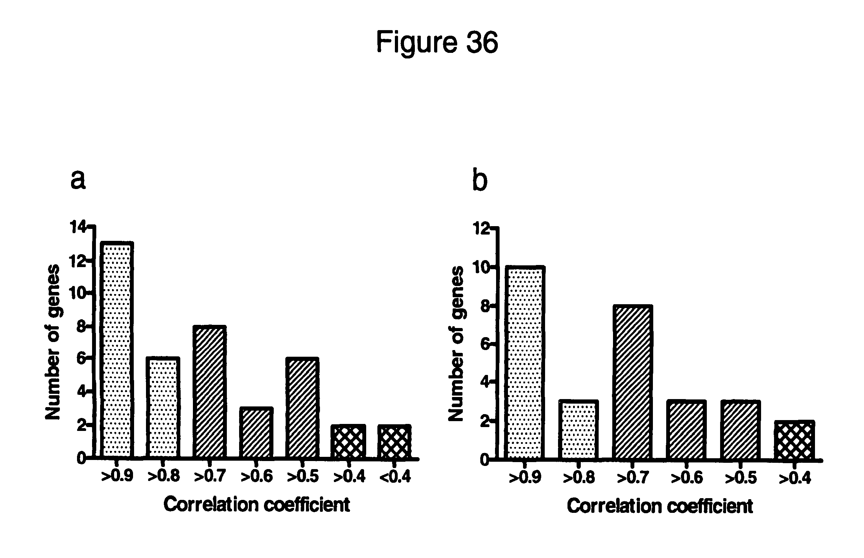

Overall, the taqMan-based assay and Affymetrix array results correlated well, validating the selected genes as overexpressed type-I IFN-induced genes in lesional psoriatic skin. The distribution of correlation coefficients between the taqMan-based assay and the Affymetrix array for the 40 overexpressed genes is provided in Figure 36a. Nineteen of the overexpressed genes had correlation coefficients greater than 0.85, indicating excellent correlation between the microarray and taqMan-based assay. Another 17 genes had high correlation coefficients between the microarray and taqMan-based assay of 0.5 - 0.85. Figure 36b provides the distribution of correlation coefficients between the taqMan-based assay and the Affymetrix array for the 29 type-I IFN-induced genes of the 18 psoriasis patients. Again, many of the genes had high correlation coefficients, greater than 0.90. These genes include, *inter alia,* IFI27, CXCL10, ISG15, and MX1.

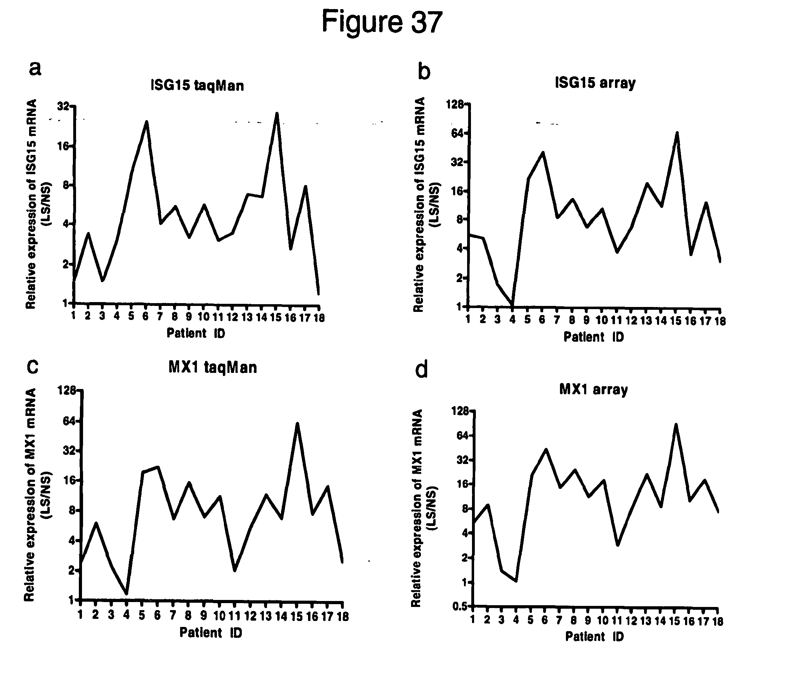

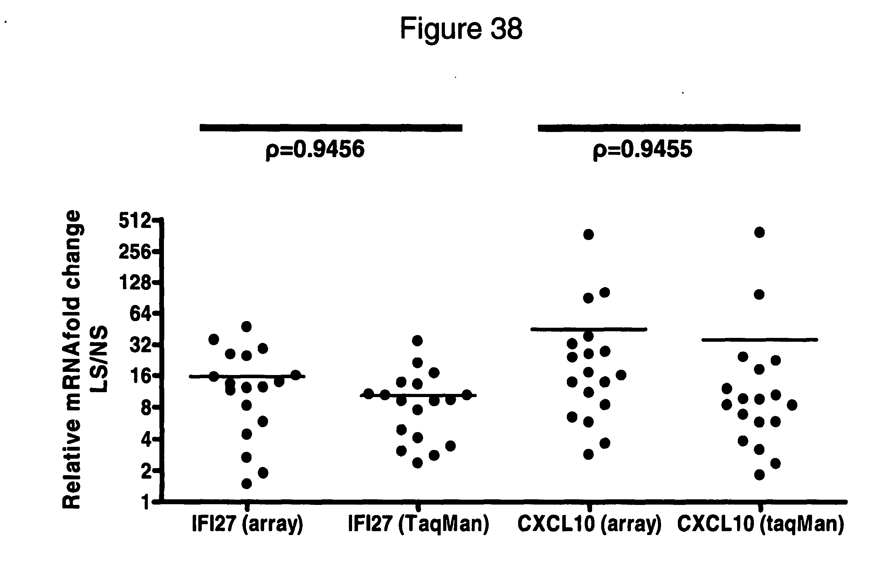

Figures 37a - 37d and 38 provide detailed gene expression data obtained from the microarray and taqMan-based assays for several type-I IFN-inducible genes in the paired lesional/non-lesional samples. These data evidence that similar levels of overexpression of type-I IFN-induced genes in lesional psoriatic skin is detected between the taqMan and array assays, and thus the high correlation coefficients discussed above. Figure 37a and 37b show similar overexpression of ISG 15 in each of the 18 paired lesional/non-lesional skin samples as determined by taqMan (37a) and microarray (37b) analysis. Figure 37c and 37d show similar overexpression of MX1 in each of the 18 paired lesional/non-lesional skin samples as determined by taqMan (37c) and microarray (37d) analysis. The correlation coefficient between the taqMan and microarray was 0.9735 for ISG15 and 0.9836 for MX1. Figure 38 shows measurement of similar overexpression of type-I IFN-inducible genes IFI27 and CXCL10 by taqMan and microarray analysis in each if the 18 paired lesional/non-lesional skin samples. The correlation coefficient between the taqMan and microarray results for IFI27 and CXCL10 was 0.9456 and 0.9455, respectively.

### Example 14: IFNα Ab neutralizes type-I IFNα-induced gene expression in ex vivo stimulated keratinocytes of healthy volunteers

Keratinocytes of healthy volunteers were isolated and stimulated *ex vivo* with escalating doses of IFNα2a and leukocyte IFN to induce an escalating type I IFNα-induced gene expression pattern. Anti-IFNα antibody was able to neutralize the type I IFNα- induced gene expression pattern in a dose-dependent manner.

Normal human keratinocytes (EpiDerm system, MatTek, Inc.) were grown under serum-free conditions according to manufacturer's instructions. Briefly, keratinocytes were maintained on tissue culture inserts at the air-liquid interface to maintain a multilayered, fully differentiated epithelial phenotype. Keratinocytes were stimulated with human leukocyte IFN (15-150 IU/ml, PBL Biomedical Labs) and human IFNα2a (15-350 IU/ml, PBL Biomedical Labs). In some wells a humanized anti-human IFNα monoclonal antibody (0.01-100 µg/ml; MEDI-545, Medlmmune, Inc) or isotype matched control antibody of irrelevant specifcity (R347, MedImmune, Inc) was added simultaneously with cytokine stimulus. Epidermal cultures were harvested at 2, 4, or 18 hours post treatment for transcript analysis. Expression of type-I IFN-induced genes was measured using a BioMark^{™} 48.48 dynamic array.

Expression of a majority of type-I IFN-induced genes was upregulated in the IFNα2a and leukocyte interferon stimulated keratinocytes in a dose-dependent manner. This upregulation of type-I IFN-induced genes, by either IFNα2a or leukocyte interferon, was likewise inhibited in a dose-dependent manner by IFNα monoclonal antibody (MEDI-545). Control antibody, R347, did not have a significant effect on neutralization of the type-I IFN-induced genes.

Dose-dependent neutralization of three type-I IFN-induced genes (ISG15, USP18, and IFIT2) by MEDI-545 in IFNα2a or leukocyte IFN stimulated keratinocytes is provided in Figure 39. Figure 39 (a), (c), and (e) show that MEDI-545 neutralizes overexpression of type-I IFN induced genes ISG15, USP18, and IFIT2, respectively, in keratinocytes stimulated with 350 IU/mL IFNα2a. Each of these genes was neutralized 100% by MEDI-545. Figure 39 (b), (d), and (f), show that MEDI-545 neutralizes overexpression of type-I IFN induced genes ISG15, USP18, and IFIT2, respectively, in keratinocytes stimulated with 150 I.U./mL leukocyte IFN. Neutralization of these genes by MEDI-545 was between 70 and 100%, which is not surprising because leukocyte IFN contains both IFNα and IFNβ. MEDI-545 neutralizes a majority of IFNα subtypes efficiently, but not IFNβ. These neutralization data provide further evidence that the type-I IFN-inducible genes identified in *ex vivo* stimulated whole blood and keratinocytes (Example 10) are type-I IFN-inducible genes. It also provides further support that upregulated expression of these genes in lesional psoriatic skin relative to non-lesional skin due to type-I IFN induction.

### Example 15: Multiple type-I IFN subtypes are up-regulated in lesional skin of psoriasis patients

To identify the type-I IFN subtypes responsible for the induction of the type-I IFN signature in lesional skin of psoriasis patients, mRNA levels of type-I IFN genes in psoriatic lesions were measured.

Gene expression analysis was performed using a TaqMan Low Density Array (TLDA) from Applied Biosystems. Expression of 23 genes, including type-I IFNα subtypes 1, 2, 5, 6, 7, 8, 14, 17, and 21; type-I IFNs IFNβ, κ, and ω; IFNγ; IFNα receptors IFNAR1 and IFNAR2; IFNγ receptors IFNGR1 and IFNGR2; type-I IFNα inducible genes RSAD2, OAS3, IFI44, MX1, and CXCL10; and TNFα was monitored and compared in paired lesional and non-lesional skin of 18 psoriasis patients.

Double-stranded cDNA for each patient sample was pre-amplified using the TaqMan PreAmp Master Mix kit (Applied Biosystems). cDNA was pre-amplified by conducting 10 cycles of PCR on each patient sample using a pooled solution of primers, a pair for each gene analyzed on the array. The pre-amplified cDNA were diluted 1:5 with TE. A 50 µL volume of the diluted pre-amplified cDNA was added to a 50 µL volume of 2x TaqMan Universal PCR Master Mix (Applied Biosystems) and mixed. The array was loaded with the mixture using standard procedures and the loaded array was run on a 7900HT Fast Real-Time PCR System (Applied Biosystems). Data analysis of the resulting Ct values was conducted with the SDSv2.2.2 software tool (Applied Biosystems).

Figure 40a shows the relative overexpression of mRNA of nine IFNα subtypes in the lesional skin compared to either non-lesional skin or normal skin. With the exception of IFNα5 (upregulated by about 4.6 fold; median fold change, *p*<0.001), none of the IFNα subtypes were significantly altered at the mRNA level in the non-lesional skin compared to that in the normal skin (*p*<0.05). However, all of these IFNα subtypes were upregulated at the mRNA level in the lesional skin compared to that in the normal skin (or non-lesional skin), with the overexpression of IFNα1, IFNα5, IFNα8, IFNα14, IFNα17, IFNα21 being statistically significant (*p*<0.05). Figure 40b shows that the overexpression of other members of type I IFN family members, IFNβ, -κ, and -ω mRNA in the lesional skin compared to either non-lesional skin or normal skin. The alterations of IFNβ and IFNω mRNAs in the non-lesional skin were not significant. However, the upregulation of these mRNAs were significant in the lesional skin compared to normal skin (*p* values of 0.022 and 0.049 respectively). IFNκ mRNA was upregulated by about 1.6 fold (median fold change, *p*=0.03) in the non-lesional skin, and was sharply upregulated by 62.6 fold (median fold change) in the lesional skin compared to normal skin (*p*<0.001). Additionally, the receptors for type I IFN, IFNAR1 and IFNAR2 were also significantly overexpressed in the lesional skin of psoriatic patients at transcript level (*p* values<0.001; Figure 40c). While IFNAR2 upregulation was significant in the non-lesional skin, IFNAR1 was not (Figure 40c). Collectively, these data provided strong evidence that mRNA levels of type I IFN family members were comparable between the non-lesional skin and healthy normal skin (with the exception of IFNα5 and IFNκ), and were uniformly overexpressed in the lesional skin of psoriatic patients.

Table 29, lists the correlation coefficients of the overexpression of type-I IFN family member (type-I IFNα subtypes 1, 2, 5, 6, 7, 8, 14, 17, and 21; and IFNβ, IFNκ, and IFNω) mRNAs in lesional skin compared to non-lesional skin of psoriatic patients. Of the 12 type-I IFN family members measured, overexpression of IFNα1, 2, 8, and 14 in lesional skin correlated most consistently with overexpression of other members in the type-I IFN family, with the exception of IFNα5 which showed the weakest correlation with other type-1 IFN family members.

**Table 29: Correlation coefficient of overexpression of type-I IFN family members in lesional skin of psoriasis patients**

| | **IFNA1** | **IFNA2** | **IFNA5** | **IFNA6** | **IFNA7** | **IFNA8** | **IFNA14** | **IFNA17** | **IFNA21** | **IFNB1** | **IFNK** | **IFNW1** |
|---|---|---|---|---|---|---|---|---|---|---|---|---|
| **INFA1** | **1** | | | | | | | | | | | |
| **IFNA2** | **0.66** | **1** | | | | | | | | | | |
| **IFNA5** | **0.11** | **0.20** | **1** | | | | | | | | | |
| **IFNA6** | **0.45** | **0.47** | **-0.01** | **1** | | | | | | | | |
| **IFNA7** | **0.77** | **0.79** | **0.09** | **0.68** | **1** | | | | | | | |
| **IFNA8** | **0.64** | **0.99** | **0.19** | **0.49** | **0.84** | **1** | | | | | | |
| **IFNA14** | **0.84** | **0.94** | **0.28** | **0.44** | **0.72** | **0.94** | **1** | | | | | |
| **IFNA17** | **1.00** | **0.96** | **0.15** | **0.07** | **0.77** | **0.97** | **0.94** | **1** | | | | |
| **IFNA21** | **0.71** | **0.49** | **0.50** | **0.42** | **0.81** | **0.49** | **0.61** | **0.75** | **1** | | | |
| **IFNB1** | **0.54** | **0.86** | **0.28** | **0.33** | **0.69** | **0.96** | **0.80** | **0.93** | **0.54** | **1** | | |
| **IFNK** | **0.78** | **0.73** | **0.09** | **0.59** | **0.27** | **0.73** | **0.77** | **0.03** | **0.22** | **0.54** | **1** | |
| **IFNW1** | **0.73** | **0.72** | **0.44** | **0.22** | **0.75** | **0.70** | **0.77** | **0.93** | **0.90** | **0.73** | **0.26** | **1** |

### Example 16: Co-overexpression of type-I IFN. type-II IFN, and TNFα and their gene signatures in lesional skin or psoriasis patients

The involvement of IFNγ and TNFα mRNA signaling pathways was also evaluated in the paired lesional/non-lesional psoriasis and normal skin samples. As discussed in Example 15, above, TLDA from Applied Biosciences was used to measure IFNγ, IFNGR1 and IFNGR2, and TNFα mRNA in lesional and non-lesional skin of psoriasis patients and in normal healthy skin.

Unlike the observations for type-I IFN mRNA expression levels, IFNγ, IFNGR1, IFNGR2, and TNFα mRNAs were significantly overexpressed in non-lesional skin compared to healthy normal skin (Figure 41; *p* values of 0.02, <0.001, <0.001 and <0.001 respectively). TNFα mRNA was upregulated by about 5.7 fold, while IFNγ, IFNGR1 and IFNGR2 mRNAs were upregulated by about 1.5, 2.2, and 2.8 fold compared to that in the normal skin (median fold change; Figure 41). However, like the type I IFNs, these genes were upregulated in the lesional skin compared to either non-lesional skin (*p* values of 0.04, 0.01, 0.001 and 0.007 respectively) or normal skin (*p* values<0.001 for all of them; Figure 41). TNFα, IFNγ, IFNGR1 and IFNGR2 mRNAs were upregulated by about 33.5, 116.7, 11.6, and 8.4 fold in the lesional skin compared to that in the normal skin. These observations indicated that the mRNA expression patterns for IFNγ and TNFα are different from those of type I IFN family members, which were comparable between healthy skin and non-lesional skin (with the exception of IFNα5 and IFNκ), but upregulated in the lesional skin compared to non-lesional skin of psoriasis patients.

### Example 17: Identification genes induced by type II IFN and TNFα in ex vivo 'stimulated whole blood and which are induced in skin lesions of psoriasis patients

As described in Example 10, whole blood of healthy donors was stimulated *ex vivo* with a panel of IFNα subtypes, as well as IFNβ, IFNγ, and TNFα. Stimulating whole blood *ex vivo* with IFNγ or TNFα identified probe sets associated with potential IFNγ- or TNFα-inducible genes. Three hundred four probe sets were identified as at least 2-fold upregulated by IFNγ four hours post-stimulation. Two hundred thirty four probe sets were identified as at least 2-fold upregulated by TNFα both 2 and 4 hours post-stimulation.

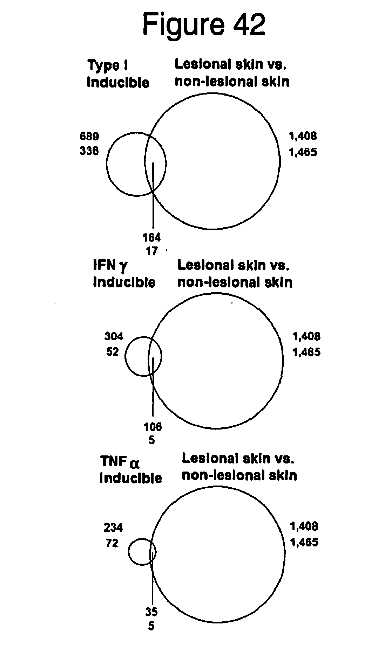

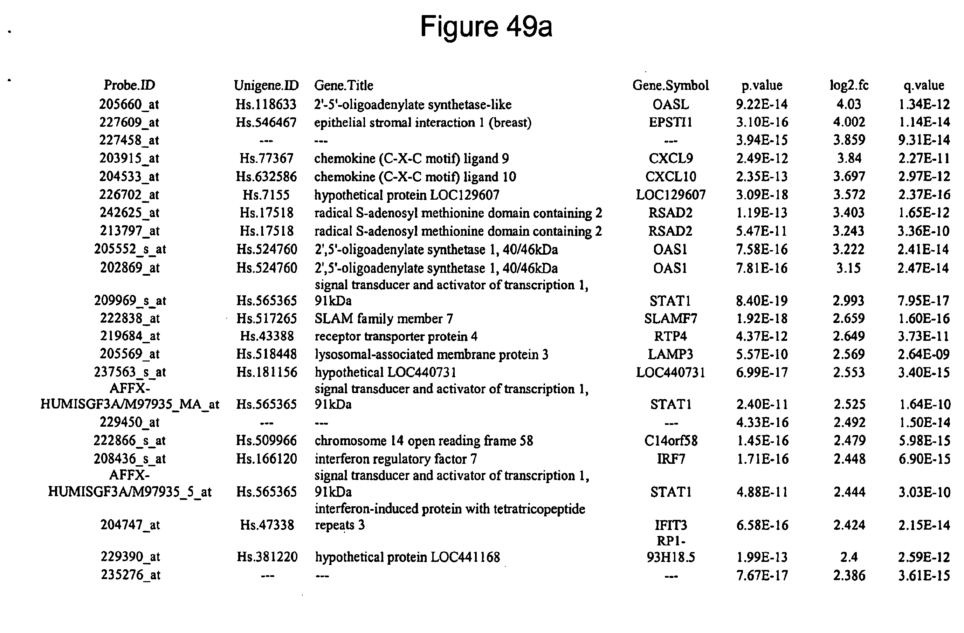

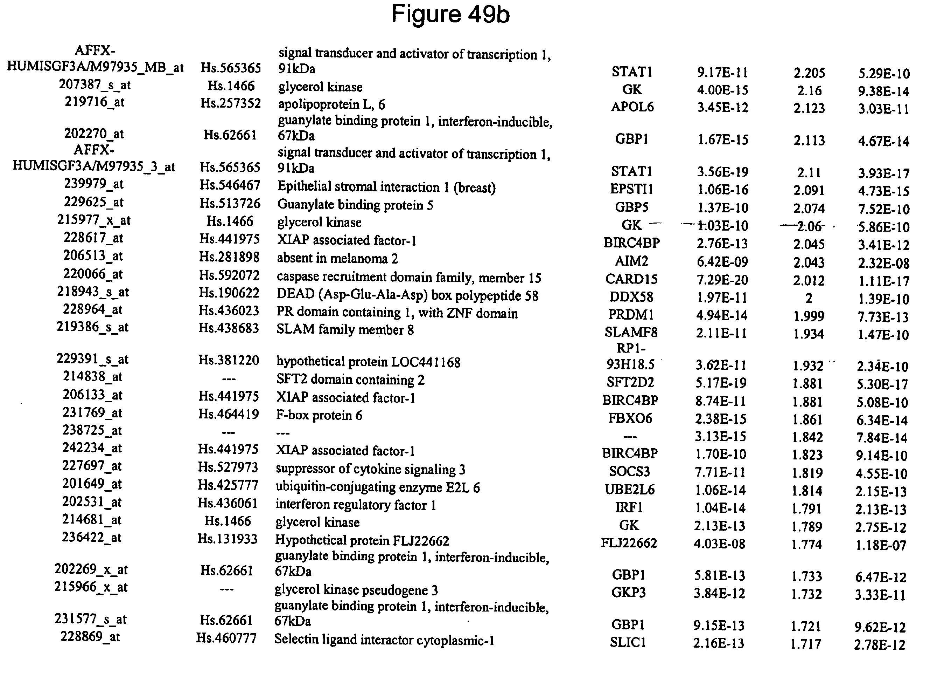

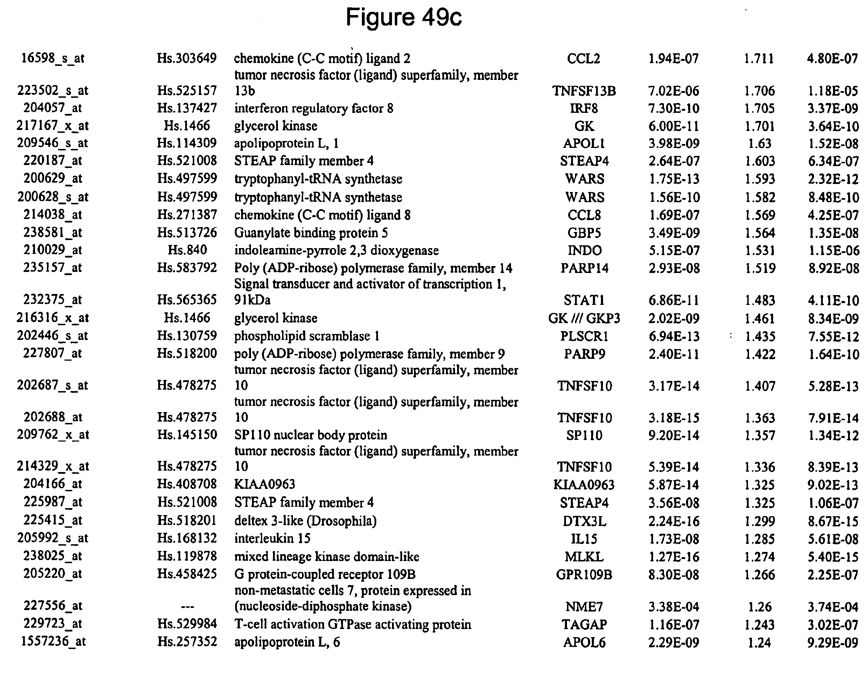

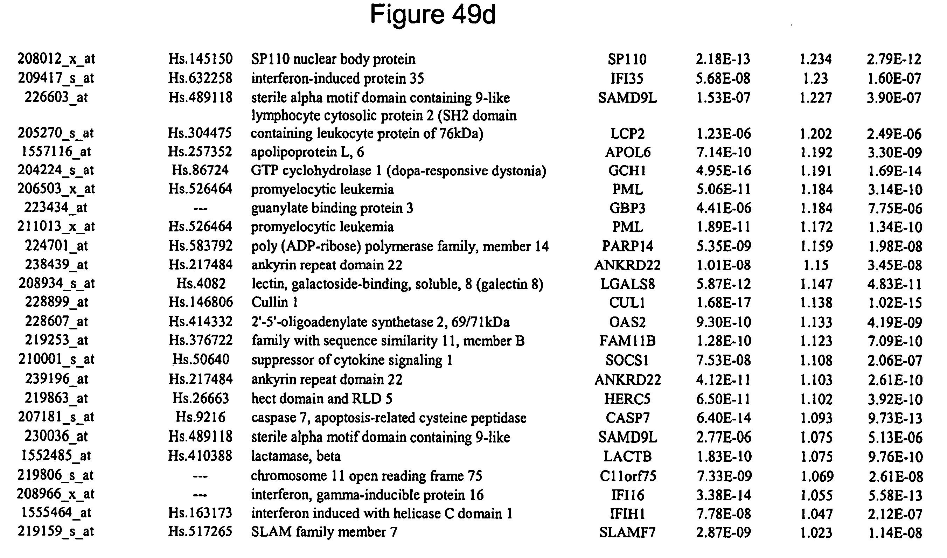

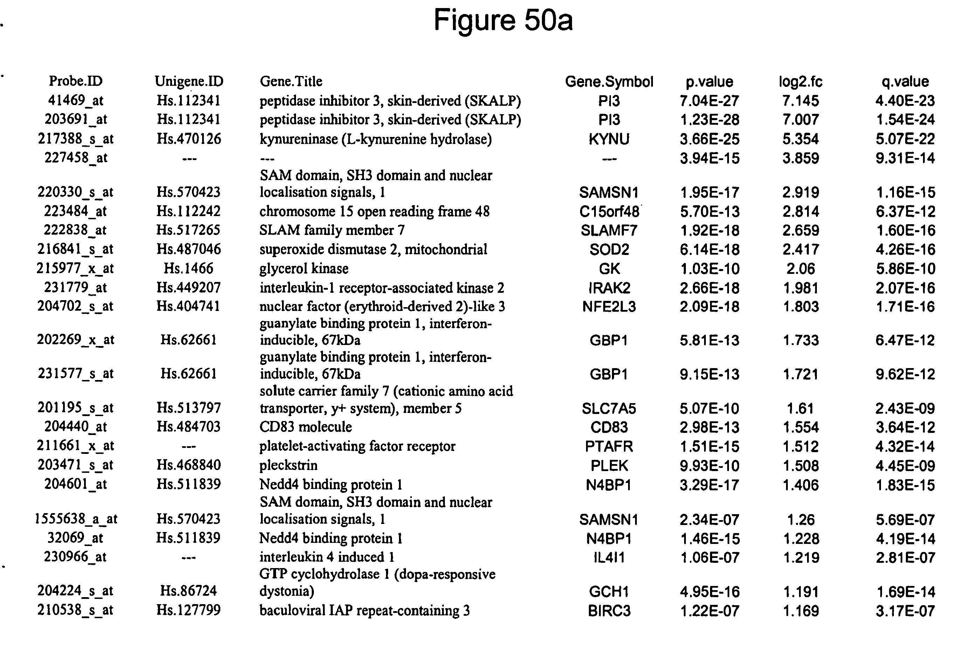

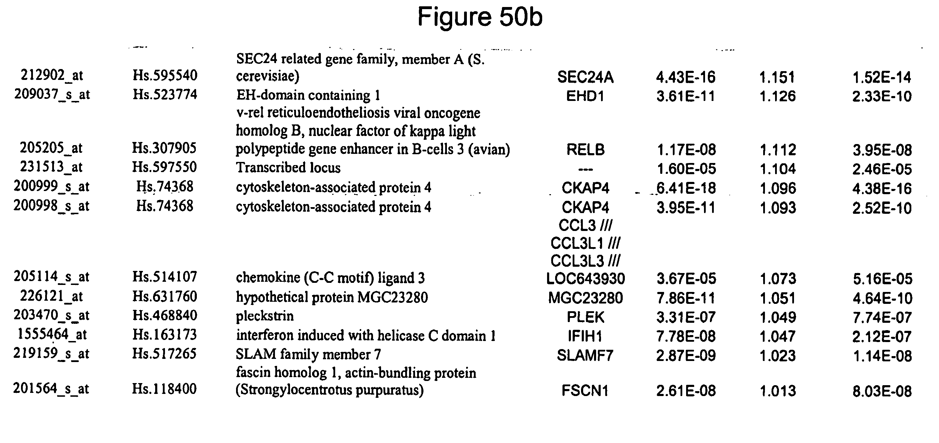

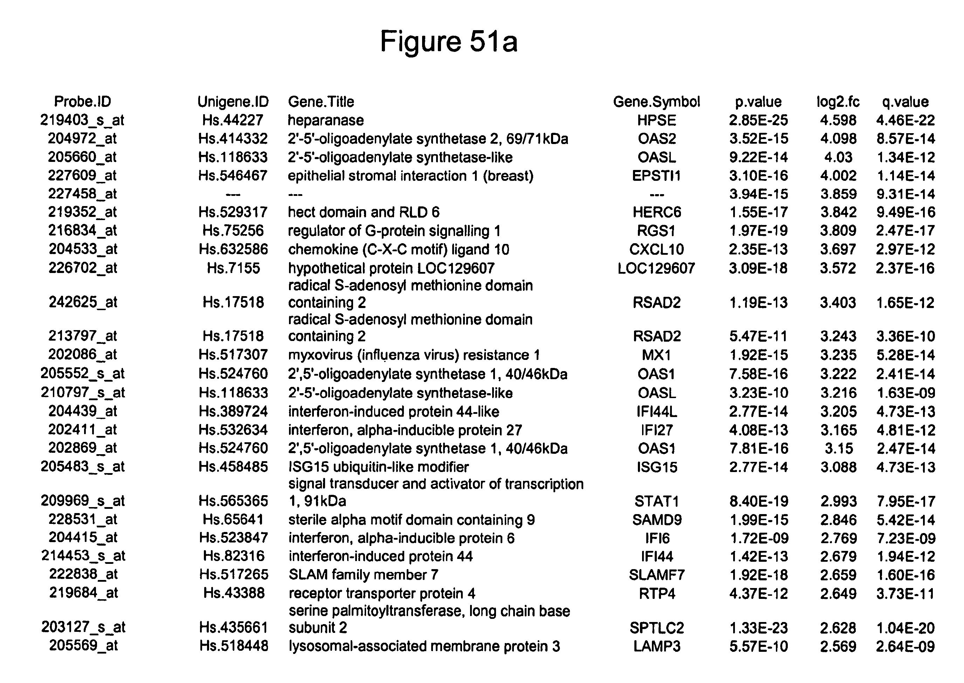

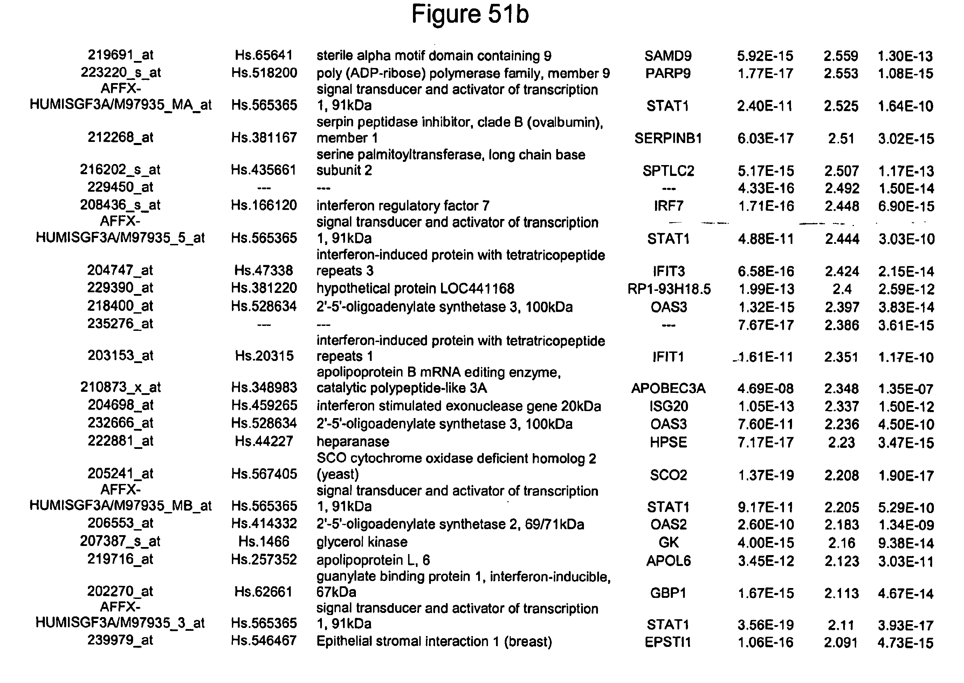

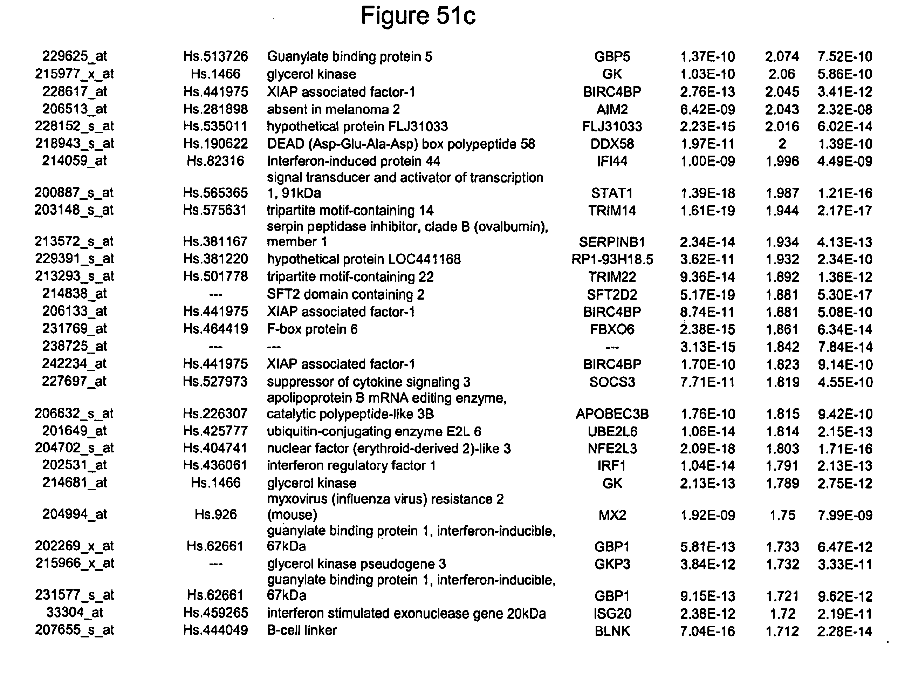

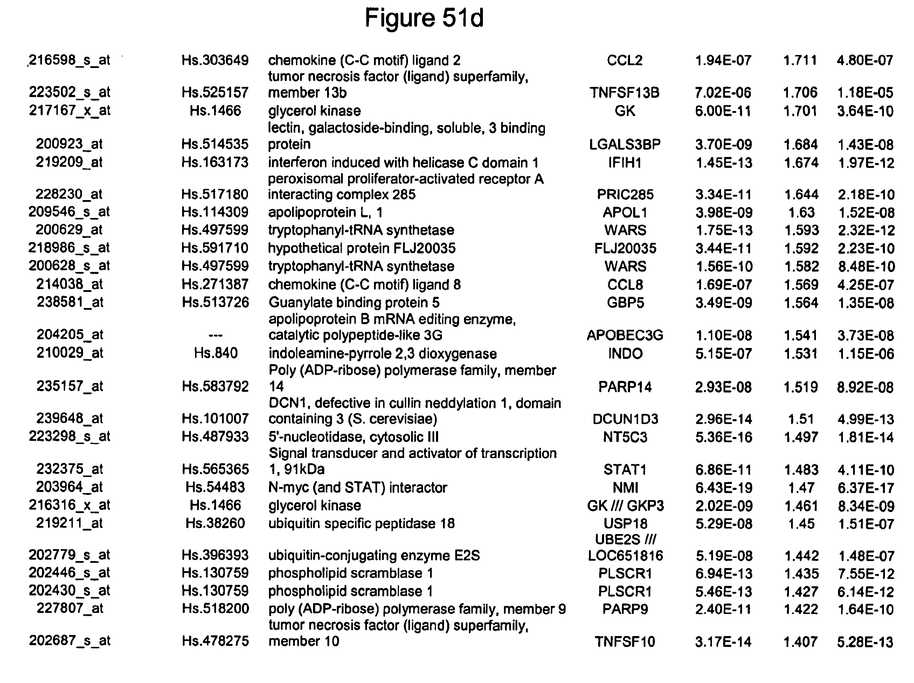

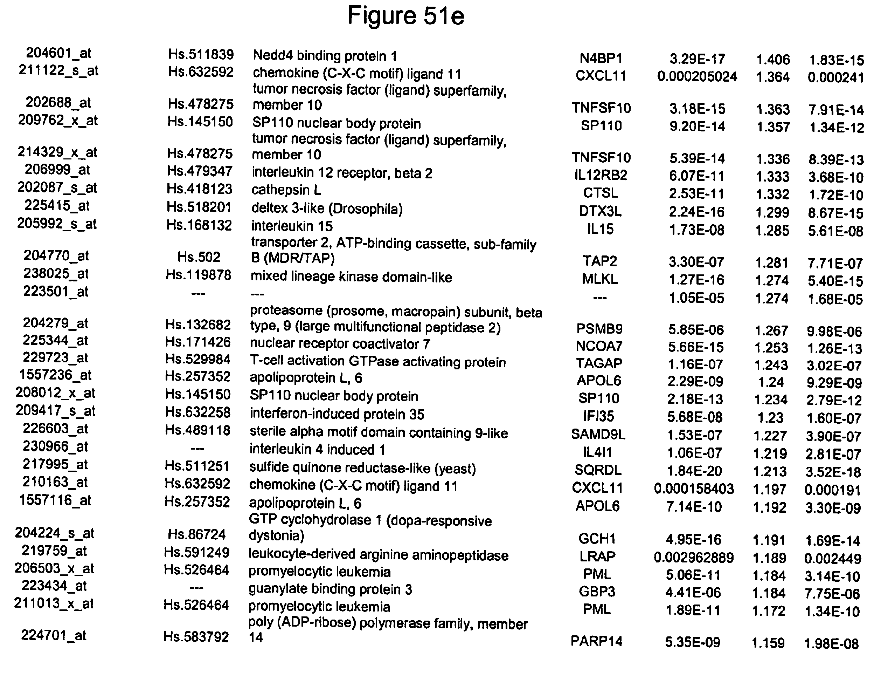

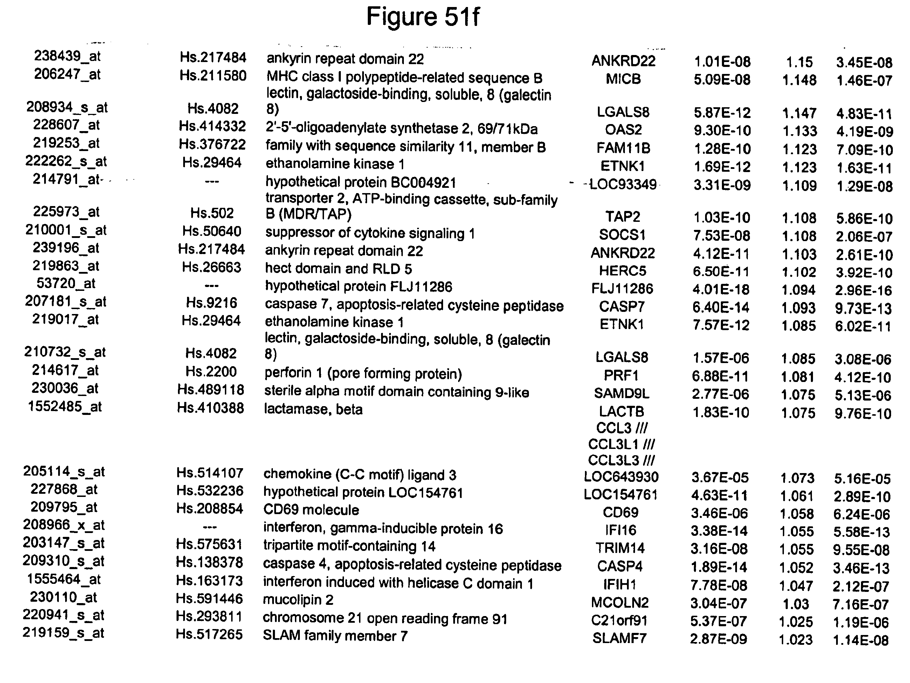

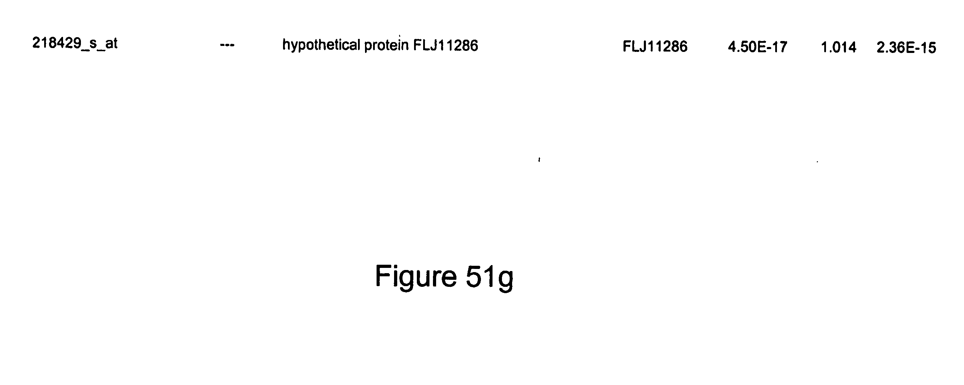

The probe sets identified as associated with *ex vivo* IFNγ or TNFα induction were compared with the total 1408 probe sets (Example 11) found to be upregulated in lesional skin relative to non-lesional skin of psoriasis patients. Using this method, 106 and 35 of the probe sets included in the total 1408 upregulated in lesional skin were identified as IFNγ or TNFα inducible, respectively (Figure 42). The 106 probe sets identified as IFNγ inducible are provided in Figure 49. The 35 probe sets identified as TNFα inducible are provided in Figure 50. The 164 probes sets shown in Figure 42 as identified as type-I IFN inducible are provided in Figure 51. The Fisher's exact test indicated that the *p* values (one-tailed t-test) of the overexpression of IFNγ or TNFα inducible genes in lesional skin were both less than 0.0001. The overexpression of IFNy and TNFα inducible genes was significant.

Also using the list of probe sets identified to be type I IFN, IFNγ and TNFα inducible from the *ex vivo* studies, type I IFN, IFNγ and TNFα inducible genes upregulated at least 2-fold in each of the lesional relative to non-lesional skin sample were identified. Figure 43 shows the number of type I IFN, IFNγ and TNFα inducible genes upregulated in each of the 26 paired lesional and non-lesional skin. The larger the number of type I I FN inducible genes upregulated in a particular lesional skin biopsy usually gave rise to the overexpression of larger numbers of IFNγ and TNFα inducible genes in the same lesional skin biopsy. This observation was confirmed by the strong correlation in the co-activation of these three sets of genes with correlation coefficients of 0.9811, 0.9179 and 0.9372 using two-tail paired t-test to compare the upregulation of type I IFN and IFNγ, type I IFN and TNFα, and IFNγ and TNFα inducible genes in lesional skin compared to non-lesional skin (Figure 43a).

Similar analysis was carried out for the downregulated genes in the lesional skin compared to the non-lesional skin of psoriatic patients (Figure 42). Of the 1465 total probe sets downregulated in lesional relative to non-lesional skin, only 17, 5, and 5 of them were type I IFN, IFNγ and TNFα inducible.

Although IFNγ and TNFα mRNAs were found to be upregulated in the non-lesional skin of psoriatic patients when compared to healthy normal skin, IFNγ and TNFα inducible genes did not appear to be significantly overexpressed in the non-lesional skin (Figure 42). The absence of type I IFN, IFNγ and TNFα inducible gene signatures in the non-lesional skin compared to normal skin, even when IFNγ and TNFα mRNAs are overexpressed in the non-lesional skin, suggested that either IFNγ and TNFα proteins were not made in the non-lesional skin, or other signaling molecules might have inhibitory effect on the IFNγ and TNFα pathways in the non-lesional skin of psoriatic patients.

### Example 18: Immunohistochemical analysis of biopsies from lesional psoriatic skin, non-lesional psoriatic skin, and skin from normal donors shows increased protein levels of type I IFN-induced genes

To determine whether some of the highly overexpressed type I IFN inducible genes in psoriatic skin gave rise to similar changes in the expression of the proteins, immunohistochemical analyses were carried out to assess the presence of STAT1 and ISG15 protein in the skin. Furthermore, analysis of the cellular infiltrates (pDCs, mDCs and CD4-positive cells) was carried to compare the number of IFN-producing cell types and inflammatory cells in the biopsies of the lesional vs. non-lesional and normal skin.

Snap-frozen lesional psoriatic, non-lesional psoriatic, and normal skin biopsies were divided in half. One-half of each sample was embedded in O.C.T., sectioned at 5 µM, placed on a "plus" slide, and fixed in cold acetone. The sectioned samples were incubated with primary antibodies (specific for BDCA2, CD83, CD4, STAT1, and ISG15) for 4 hours and washed with TBS. The slides were then incubated with peroxidase-labeled polymer conjugated to goat anti-mouse immunoglobulin antibody (Envision+; Dakocytomation, Carpenteria, CA) for 30 minutes and washed with Tris-buffered saline, pH 7.2. Detection was performed with 3,3'-diaminobenzidiine tetrahydrochloride (DAB+; DakoCytomation) as the chromogen. Slides were washed with dH₂O), counterstained with hematoxylin, dehydrated and coverslipped.

In all psoriasis patients for which paired lesional/non-lesional samples could be evaluated, lesional skin contained increased numbers of pDCs, and/or mDCs, increased numbers of CD4+ cells, as well as the significant upregulation of STAT-1 and ISG 15 protein in the epidermis and dermis compared to non-lesional biopsies. By contrast, skin biopsies from normal donors did not contain appreciable numbers of pDCs, mDCs or staining for STAT-1 and ISG15. See Figure 44 for example immunohistochemistry slides.

### Example 19: Immunohistochemical and gene expression analysis of biopsies from SLE patient skin lesions show reduced expression of type I IFN-induced genes at the protein and transcript level following treatment with MEDI-545

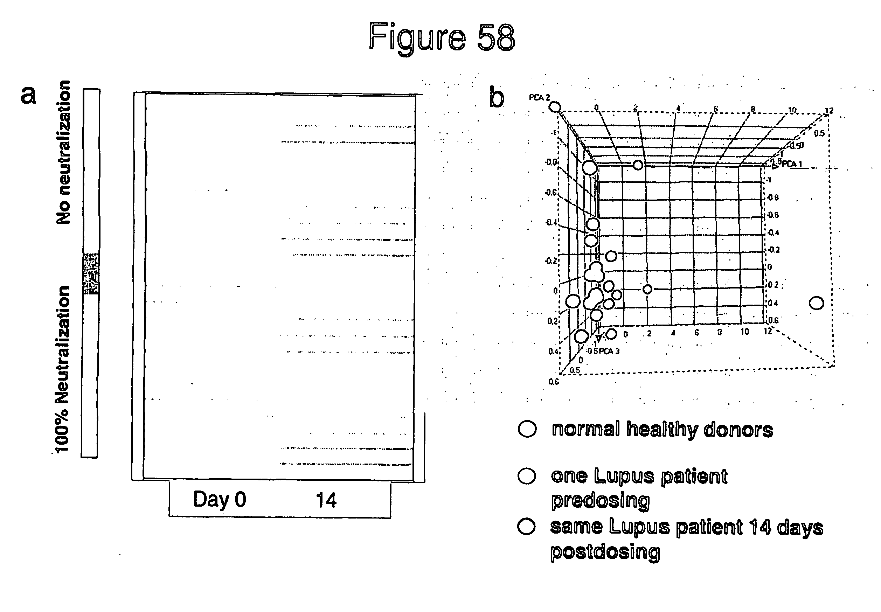

To determine whether transcripts of the top 25 type I IFN inducible genes in skin lesions of an SLE patient were neutralized by MEDI-545, biopsies from patients treated with 10mg/kg MEDI-545 were examined. A heatmap of neutralization of the top 25 type I IFN inducible genes in skin lesions at 0 and 14 days post-treatment is provided in Figure 58(a). All of the top 25 genes are neutralized 14 days following administration of MEDI-545. A PCA diagram of target modulation based on these top 25 type I IFN-inducible genes is provided in Figure 58(b). The PCA diagram shows the progression of the treated SLE patient from a position directly opposite that of normal healthy donors prior to administration of MEDI-545 to a position nearing that of the healthy donors 14 days after administration of MEDI-545.

To determine whether levels of some of the proteins expressed from these highly overexpressed type I IFN inducible genes were also reduced by treatment with 10 mg/kg MEDI-545, immunohistochemical analyses were carried out to detect HERC5, ISG15, and IP10 protein in SLE skin lesions of patients treated with MEDI-545 and placebo. Furthermore, analysis of the cellular infiltrates (pDCs, mDCs and CD4-positive cells) was carried out to compare the number of IFN-producing cell types and inflammatory cells in the biopsies of the SLE skin lesions of MEDI-545 treated patients and placebo treated controls.

Snap-frozen skin lesion samples of MEDI-545 treated SLE patients and placebo treated SLE patients were divided in half. One-half of each sample was embedded in O.C.T., sectioned at 5 µM, placed on a "plus" slide, and fixed in cold acetone. The sectioned samples were incubated with primary antibodies (specific for BDCA2, CD83, CD4, IP10, and ISG15) for 4 hours and washed with TBS. The slides were then incubated with peroxidase-labeled polymer conjugated to goat anti-mouse immunoglobulin antibody (Envision+; Dakocytomation, Carpenteria, CA) for 30 minutes and washed with Tris-buffered saline, pH 7.2. Detection was performed with 3,3'-diaminobenzidiine tetrahydrochloride (DAB+; DakoCytomation) as the chromogen. Slides were washed with dH₂O), counterstained with hematoxylin, dehydrated and coverslipped.

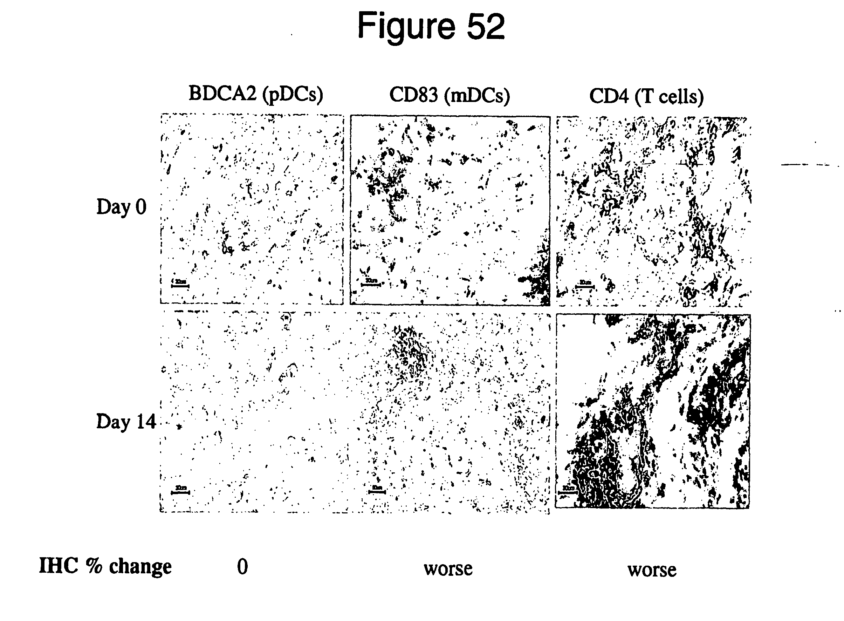

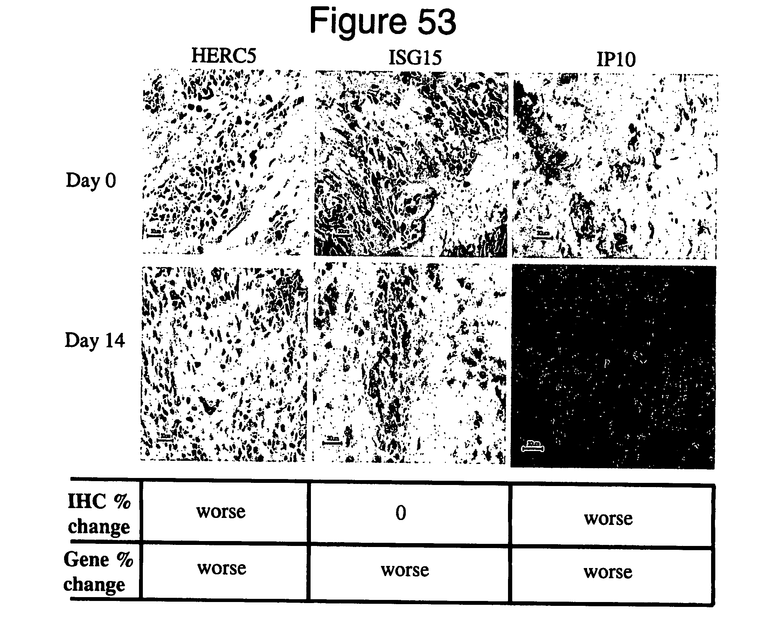

In placebo-treated SLE patients both cellular infiltrates and levels of proteins expressed from overexpressed type 1 IFN inducible genes increased (or worsened) over the course of 14 days. See Figure 52 which shows an increase in (worsening of) mDC (CD83 staining) and T cell (CD4 staining) infiltration in skin lesions. Figure 52 also shows no change in pDC (BDCA2 staining) infiltration in the placebo-treated SLE patient skin lesions over the 14 days. See also Figure 53 which shows an increase in staining for proteins expressed from overexpressed type I IFN inducible genes HERC and IP10. No change in staining for ISG15 was observed.

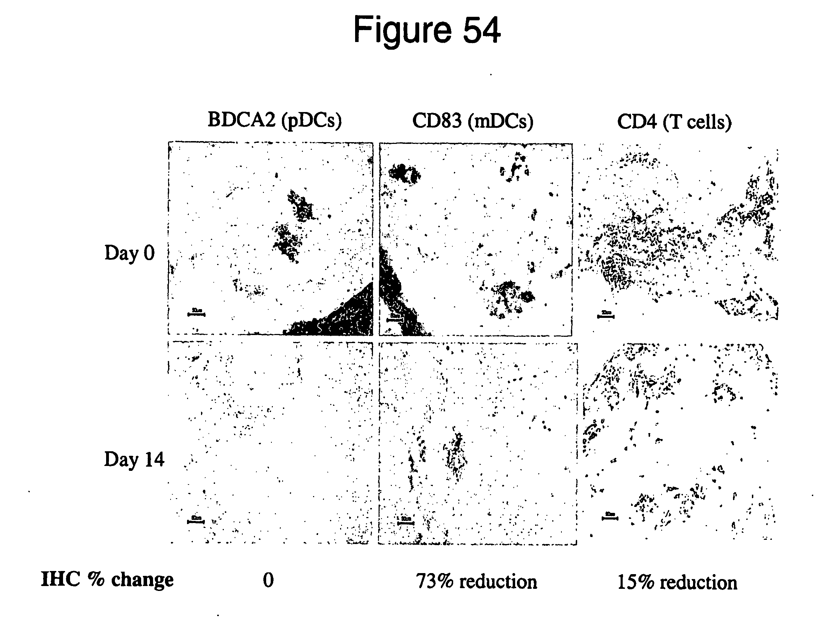

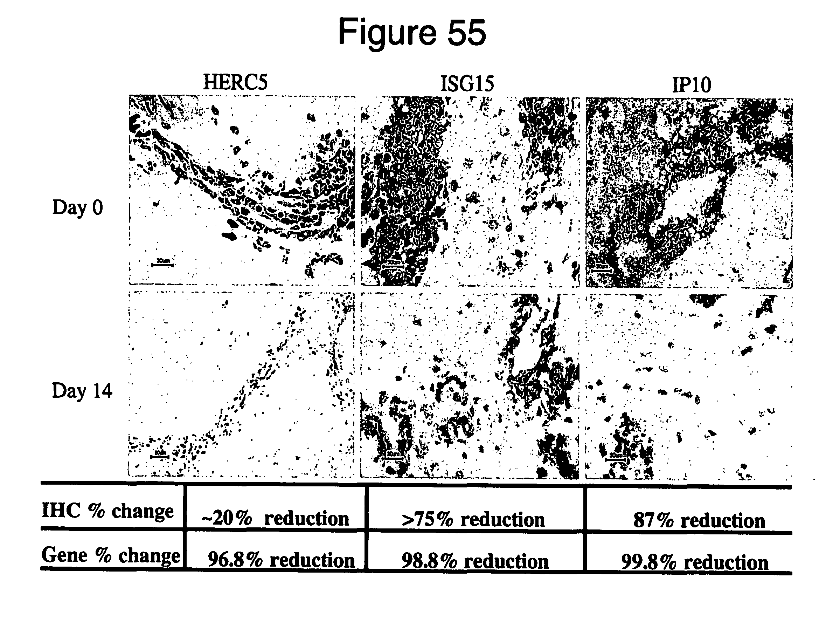

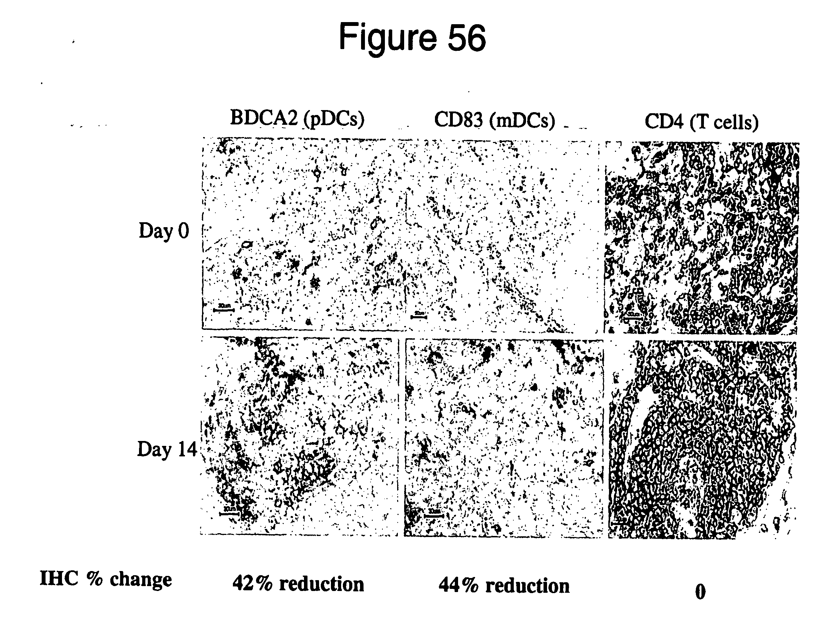

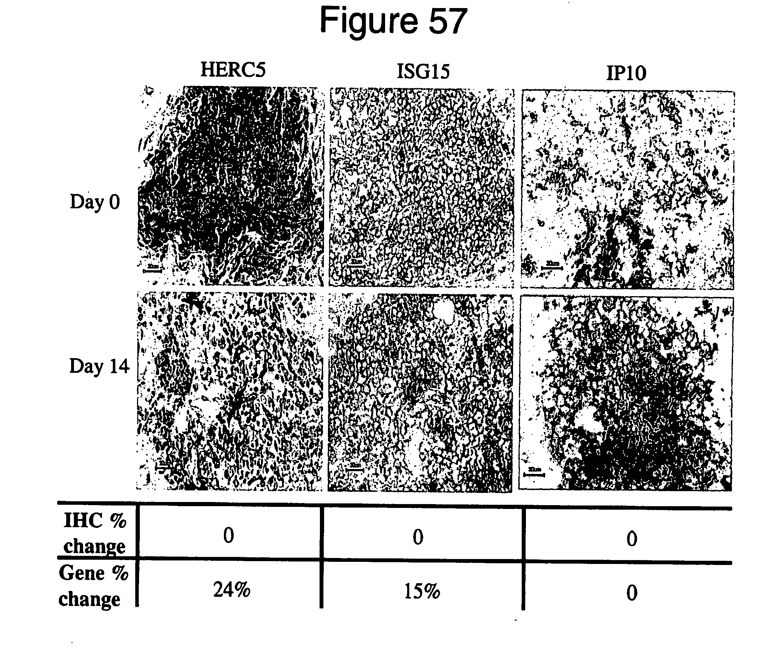

By contrast, in patients treated with 10 mg/kg MEDI-545 levels of infiltrates and proteins expressed from overexpressed type I IFN inducible genes were decreased by varying degrees. See Figures 54 and 55, which provide immunohistochemical data from a first SLE patient treated with MEDI-545 and Figures 56 and 57, which provide immunohistochemical data from a second SLE patient treated with MEDI-545.

### Example 20: Assay for sensitive detection of type I and type II IFNs

To devise an assay to sensitively detect type I and type II IFNs a construct containing the gene for a luciferase enzyme isolated from the marine organism *Gaussia princeps* (Targeting Systems; Santee, CA) under the control of an interferon-stimulated response element (ISRE) (TAGTTTCACTTTCCC)₅; Biomyx; San Diego, CA) was cloned. HEK293H cells were stably transfected with the construct and these cells were used for the IFN detection assays.

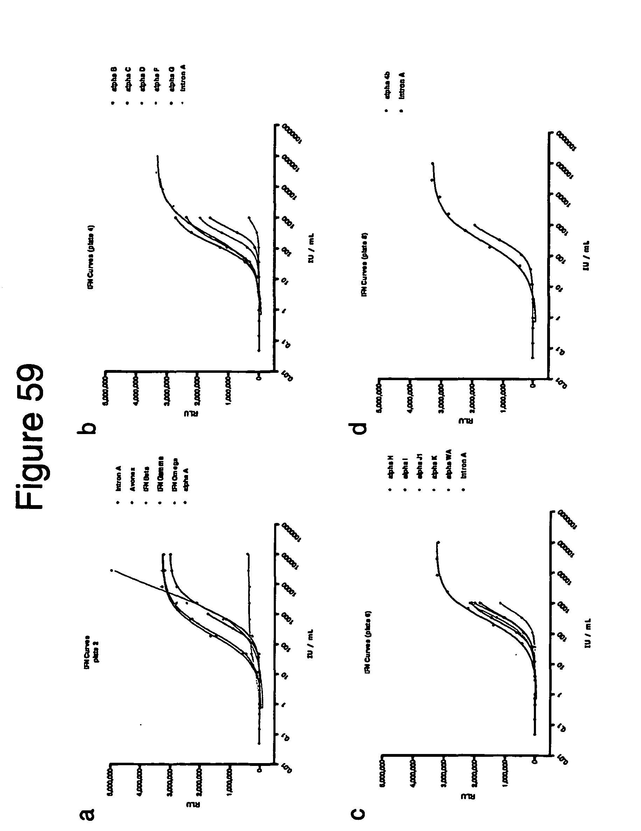

25,000 of the stably transfected HEK293H cells were seeded per assay well in 50uL of cell culture medium overnight in a CO₂ incubator. The following day, patient serum samples (or normal pooled human serum spiked with the various sub-types of IFN alpha or IFN-beta, IFN-omega, IFN-gamma) were screened for detection of the various subtypes of IFN by adding 50uL of undiluted patient or spiked serum to the assay wells containing the seeded cells (final concentration of 50% patient sera in the wells for 24 hours). IFN-induced luciferase activity was detected the following day, by observing chemiluminescence in the culture supernatants. Chemiluminescence was observed by transferring 50uL of supernatant from the wells to a B&W Isoplate, adding 50uL of chemiluminescent substrate, and detecting luminescence at 6 minutes. Samples generating a signal greater than 1.5-times the Negative Control wells on each assay plate are classified as Positive for IFN activity. See Figure 59a-d, which provide detected levels of type I and type II IFN activity in the IFN bioassay for different plates of cells treated with patient serum and spiked control serum. Each of panels a-d show that increased dose of IFN in the assay results in increased detection of IFN activity..

In samples where IFN activity is detected, antibodies that specifically neutralize various Type I and Type II IFNs can then be used to determine which IFN was responsible for the positive response. Anti-IFN-type specific antibodies are pre-incubated with either the positive serum sample(s) (in the case of MEDI 545, anti-IFN beta, anti-IFN gamma and anti-IFN omega that bind to the IFN ligand itself) or with the cells (in the case of MEDI 546 that binds to the Type I interferon receptor on the HEK293H cells) followed by addition of the samples to the cells and chemiluminescence determination as above. Spiked samples that demonstrate lower chemiluminescence following specific antibody treatment are considered to be positive for the presence of the particular IFN(s) that is neutralized by the IFN-specific antibodies.

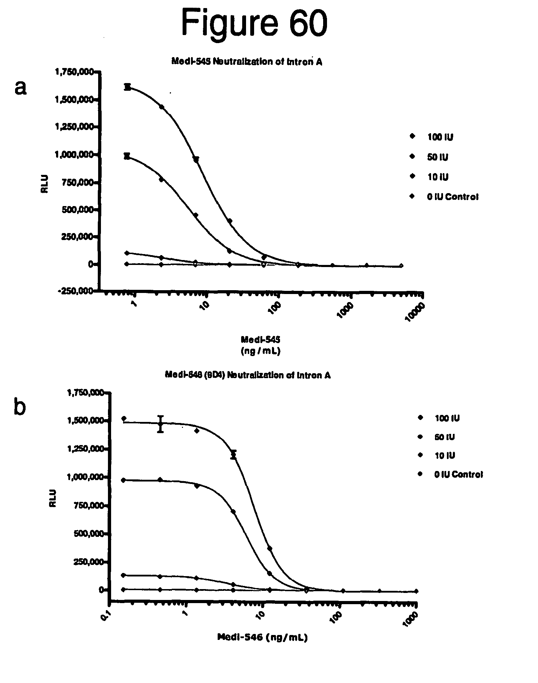

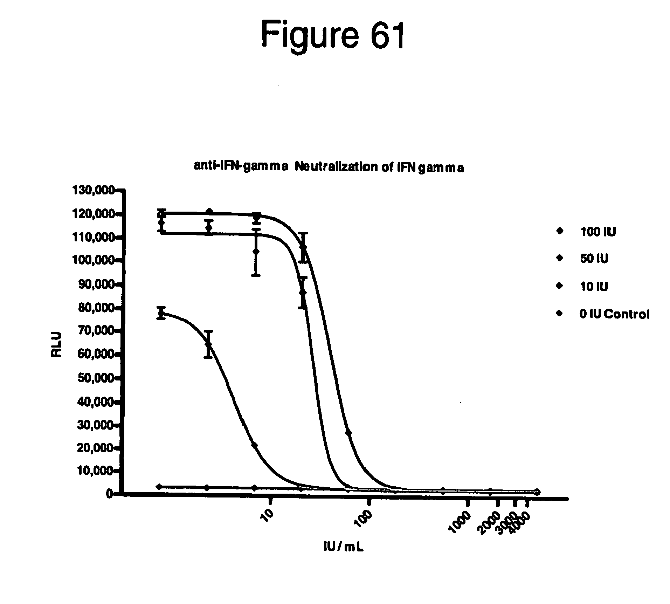

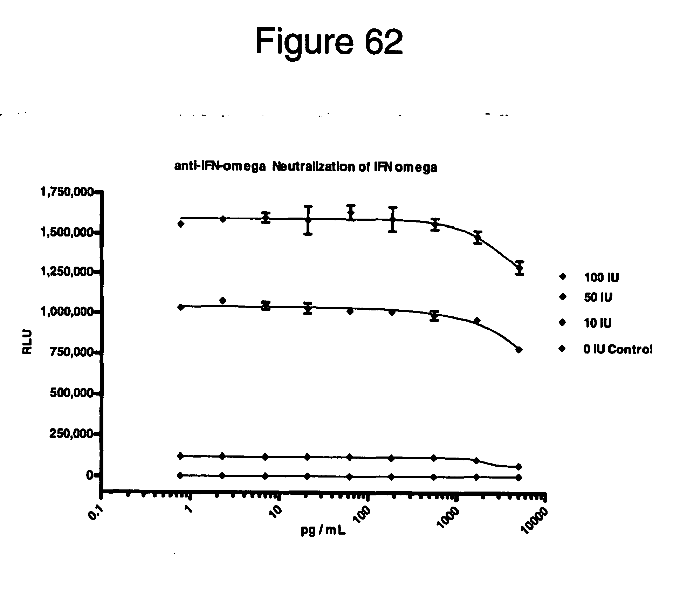

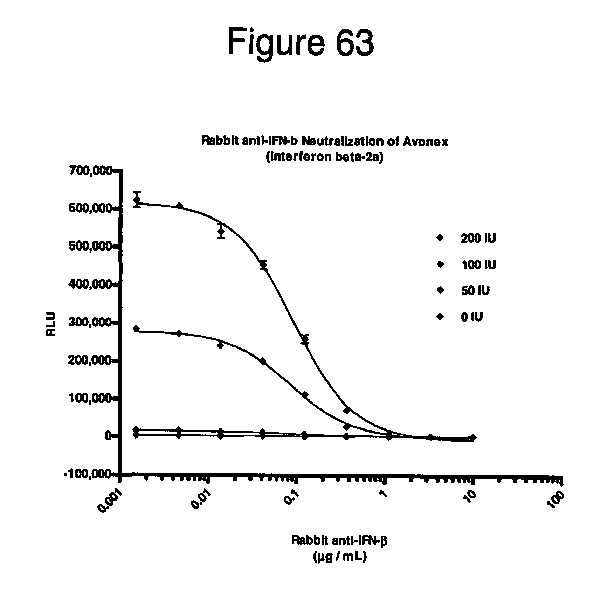

Figure 60(a) shows that increasing dose of MEDI-545 in the treated wells increasingly neutralizes of IFN activity as does increasing dose of MEDI-546 (Figure 60(b)). Figures 61-63 show that IFNγ, IFNω, and IFNβ, respectively, are neutralized by antibodies specific for IFNγ, IFNω, and IFNβ, as expected.

### Example 21: Alterations of Levels of Soluble Proteins in Serum of Lupus Patients

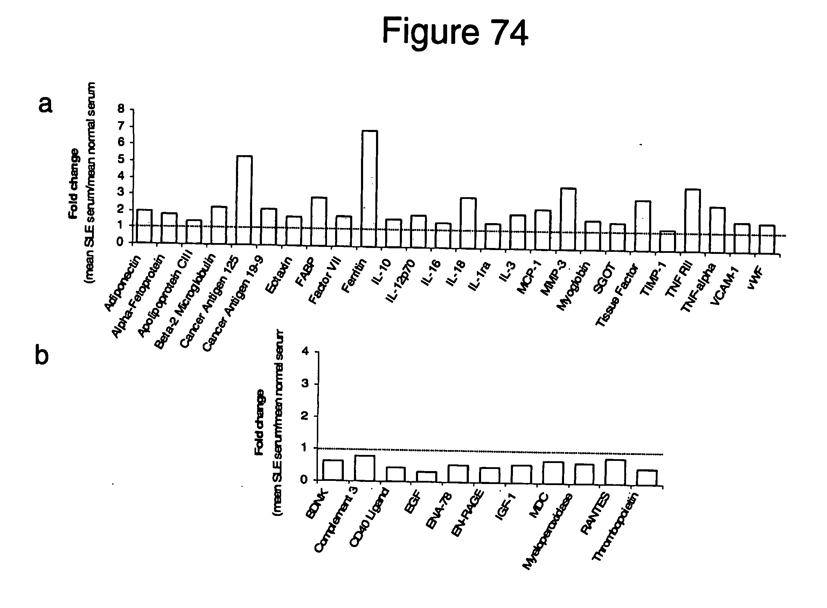

Serum was collected from SLE (n=40) and CLE (n=5) patients that had a history of at least 4 of 11 positive ACR classification criteria and demonstrated active disease manifestations at the time of sample collection. Ninety-five percent were female, with mean ± SD age of 41±15 years. Seventy-six percent were currently receiving oral corticosteroids in doses ranging from 1 mg/d to 30 mg/d prednisone, with 2 SLE patients also receiving pulse intravenous steroids. Fifty-nine percent were receiving at least 1 potential disease-modifying medication other than corticosteroids. Luminex xMAP technology was used to detect changes in 89 analytes and was performed by Rules Based Medicine (see the world wide web at domain name rulesbasedmedicine . com). Results for each analyte were compared to the mean of a panel of normal human serum (n=17) and significance was determined using a paired t-test. Figure 74 shows analytes whose levels were significantly (a) increased or (b) decreased from the mean of the normal serum (p value ≥ 0.05). Significant alterations in levels of cytokines chemokines, metabolic proteins, and other soluble mediators were detected in serum of lupus patients.

### Example 22: Alternative Assay, Panomics OuantiGenePlex Assay, Verifies IFN-Induced Gene Expression Analysis Results

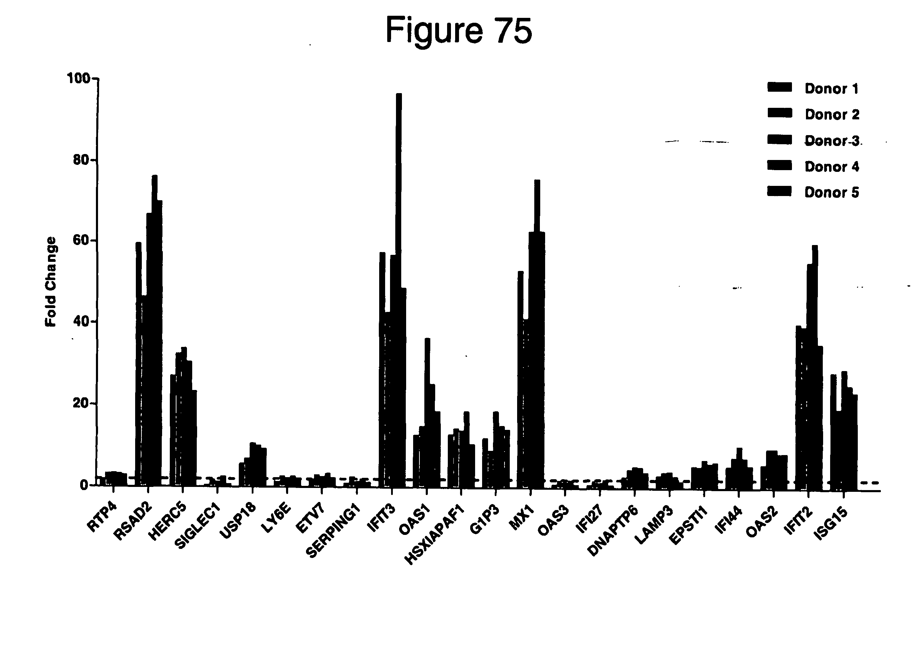

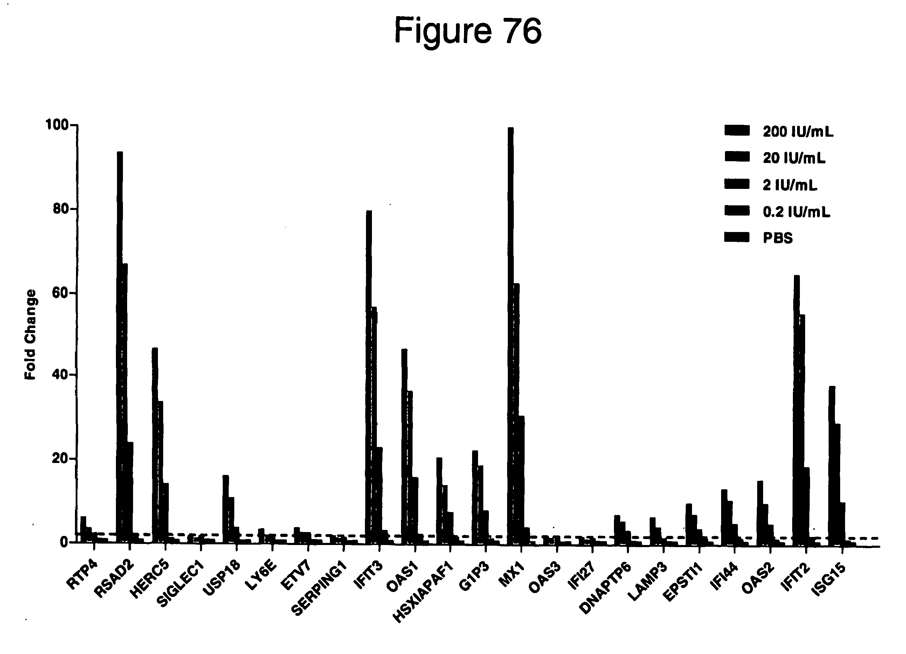

The QuantiGenePlex assay was first performed to assess the ability of QuantiGenePlex to detect 22 IFN-inducible transcripts in whole blood stimulated with IFNα2b. The 22 IFN-inducible transcripts detected by this initial QuantiGenePlex assay were selected based on their consistent up-regulation in SLE patients and are shown on the x-axis of the graphs shown in Figures 75 and 76.

Stimulation of the whole blood was performed by incubating freshly drawn Na-EDTA whole blood from 5 healthy donors with 20 IU/mL IFNα2b for 4 hours. Following this incubation, 2.5 mL of the stimulated whole blood was added to PAXgene tubes, mixed, and held overnight at room temperature. After overnight incubation, the samples were frozen at - 80°C. These sample-handling procedures were selected to mimic those to be used during clinical trials.

PAXgene blood was analyzed for expression levels of the IFN-inducible transcripts. PAXgene blood (500 µL) was pelleted and then lysed in 139 µL of buffer according to the QuantiGenePlex PAXgene Blood Lysis Protocol. Processed blood from each donor was split into duplicate wells and hybridized overnight with a multiplex probe set for the 22 IFN-inducible genes. Gene expression was assessed the following day using a Luminex 100 instrument with BioRad BioPlex software. Fold changes were assessed for each individual based on the increase in signal observed between IFN-stimulated and PBS-stimulated control wells. Figure 75 shows the fold-change in expression of each of the 22 IFN-inducible genes following IFN stimulation of each of the 5 healthy volunteer whole blood samples. The dashed line indicates a 2-fold change over PBS-stimulated control samples.

Whole blood of a single volunteer was further stimulated over a dose range of 0.2 to 200 IU/mL IFNα2b to determine whether upregulation of the IFN-inducible genes by IFNα2b was dose-dependent and could be detected by the QuantiGenePlex assay. For each of the 22 transcripts, a dose-dependent induction was observed. See Figure 76, which provides the fold change in expression for each of the 22 transcripts at each IFNα2b dosage. Maximal transcript induction of nearly 100-fold was observed for RSAD2, IFIT3, and MX1. Using a 2-fold increase over baseline as a cutoff criterion, 19/22 genes were detected in samples spiked with 2 IU/mL of IFN and 5/22 were detected in samples spiked with 0.2 IU/mL IFN. Expression of SIGLEC1, LY6E, SERPING1, OAS3 and IFI27 transcripts were poorly induced by IFNα2b stimulation. These low levels of induction may indicate a lack of sensitivity of the assay to these targets or differences in gene expression between actual SLE disease (from which this panel of transcripts was chosen) and *ex vivo* stimulation with a single IFNα subtype, IFNα2b. Dashed line indicates a 2-fold change over PBS-stimulated control samples.

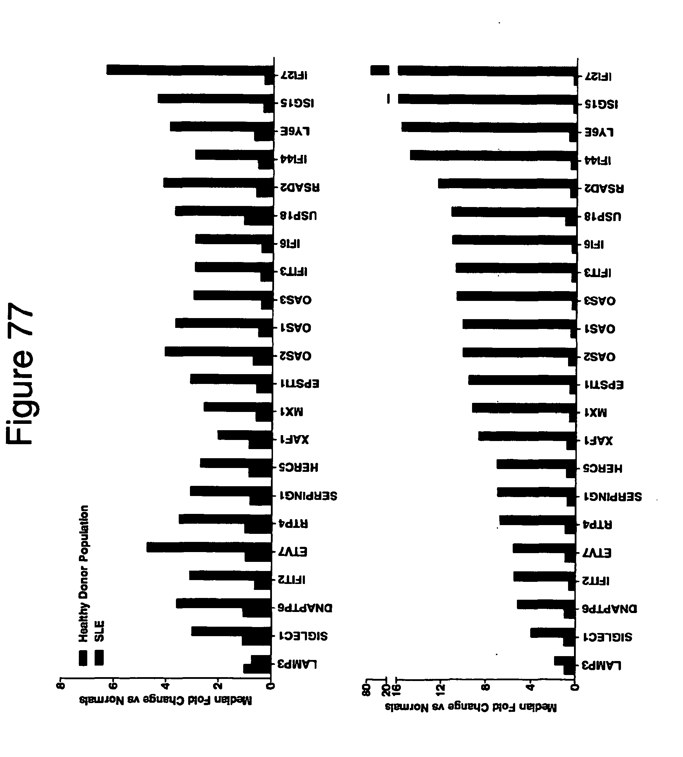

Next, the QuantiGenePlex assay was used to detect levels of IFN-inducible transcripts in whole blood of SLE patients. Twenty of the 22 probes from the original QuantiGenePlex kit, probes identified in Figures 75 and 76, were retained in the QuantiaenePlex assay used for this data analysis. Two probes, HSXIAPAF1 and GIP3, were substituted with different probes, XAF1 and IF16. Using this panel of 22 probes, a baseline gene signature was established based on whole blood samples of ten healthy donors (blue bars in each panel). The baseline gene signature, based on the whole blood samples of the healthy donors, was compared to (1) the gene signature of an SLE patient that had detectable IFN serum activity and (2) the gene signature of an SLE patient that did not have detectable IFN serum activity. IFN serum activity was detected in the SLE patient serum samples using the assay described in Example 20. Figure 77a shows a comparison of the gene signature of an SLE patient (red bars) having no detectable serum IFNα activity (i.e. serum IFN activity <2.5 IU/mL) relative to the baseline gene signature (blue bars). With the exception of LAMP3, all transcript levels were detected as elevated in blood from the SLE patient with no IFN serum activity. Figure 77b shows a comparison of the gene signature of an SLE patient with high levels of serum IFNα activity (red bars) relative to the baseline gene signature (blue bars). All transcripts were elevated at least 2-fold in the blood of the patient with high IFN serum activity, with maximal inductions of nearly 80-fold for IFI27.

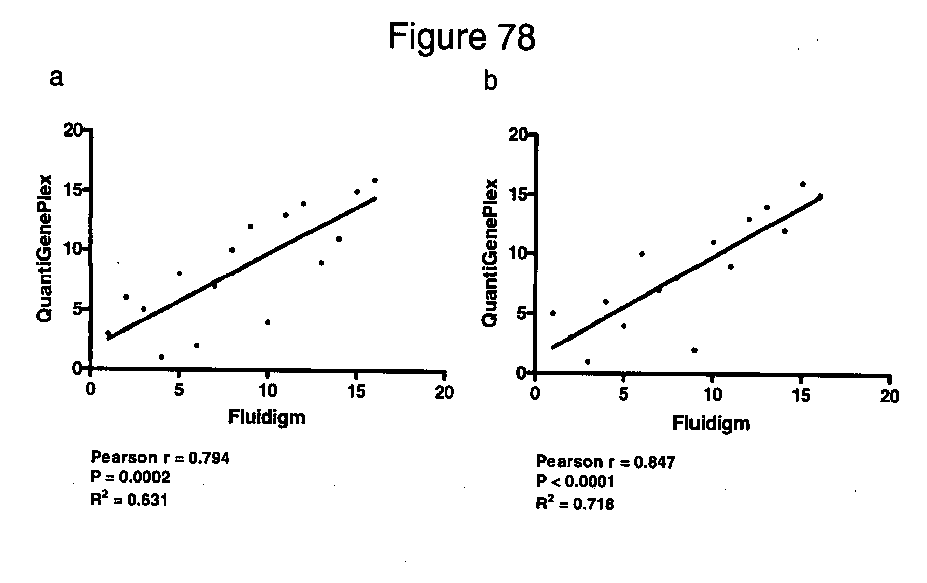

The data obtained from the QuantiGenePlex assay was next evaluated for its comparability to data obtained from a Fluidigm Real-Time PCR assay. QuantiGenePlex and Fluidigm methods were each used to analyze and compare transcript levels in PAXgene-preserved whole blood samples from 16 SLE patients participating in a Phase I clinical trial (of a monoclonal antibody against IFNα) relative to a composite median gene score from 10 healthy donors. Fluidigm analyses were carried out using a mixture of TaqMan Gene Expression assays, including 4 reference control genes prepared using the TaqMan PreAmp Master Mix Kit (Applied Biosystems). Dynamic arrays were loaded using a NanoFlex 4-IFC Controller (Fluidigm Corp) and real-time reactions were performed using a BioMark Real-Time PCR System. Results were analyzed using BioMark Real-Time PCR Analysis software. Delta-delta Cts (DDCt) were calculated using the mean of 4 reference genes (GAPDH, TFRC, b2M, and 18S) and a calibrator sample. The results obtained using whole blood samples from SLE patients demonstrated a high degree of correlation between QuantiGenePlex and Real-Time PCR approaches to detect disease-related gene expression profiles. Figure 78 shows the (a) composite median and (b) mean-fold changes of all genes in the panels that were calculated and compared by Pearson's correlation analysis. Significant correlation was observed between QuantiGenePlex and Fluidigm when median (p=0.0002) and mean (p<0.0001) fold changes were compared for the panel of genes.

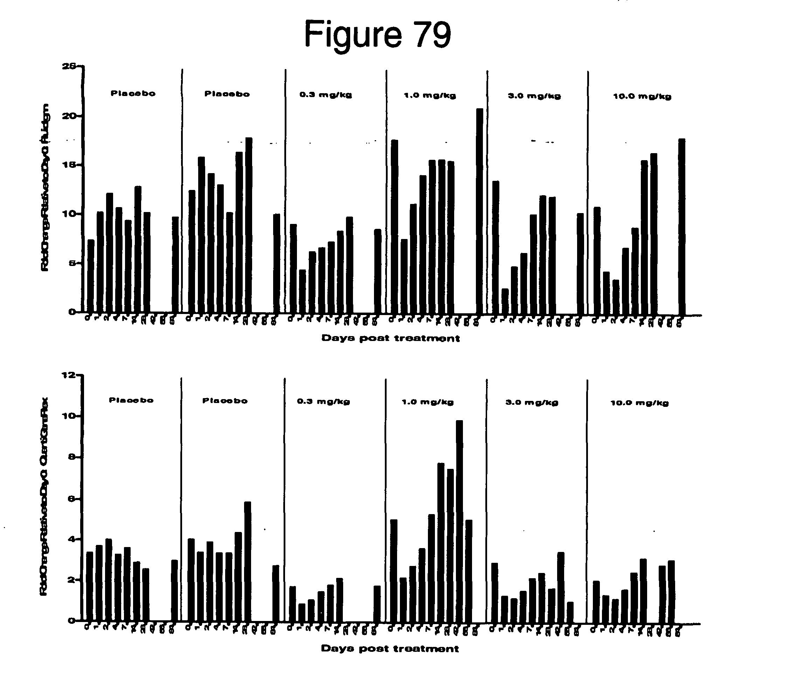

Data obtained from the QuantiGenePlex and Fluidigm Real-Time PCR assays were further compared in their ability to detect changes in transcript levels in SLE patient samples over the course of treatment in a clinical trial. For this comparison, SLE patient samples were collected d irectly into PAXgene tubes on Day 0 (pre-dose) and multiple subsequent time points following administration of a single dose of an anti-IFNα monoclonal antibody or placebo. For each sample, an aggregate median fold-change was calculated from the panel of 22 genes and compared to the pre-dose sample for that patient. Figure 79a shows the changes in gene signature for placebo- or antibody-treated SLE patients using Fluidigm technology. Figure 79b shows the changes in gene signature of the placebo- or antibody-treated SLE patients using QuantiGenePlex technology. For each non-placebo subject, a decrease in IFN gene signature is observed within 24 hours following drug administration and is consistent between Fluidigm and QuantiGenePlex. Subsequent changes in transcript levels post-administration were also highly similar between QuantiGenePlex and Fluidigm technologies.

## Claims

1. A method of monitoring disease progression of a patient receiving treatment with a therapeutic agent that binds to and modulates IFNα activity comprising:
obtaining a first IFNα-inducible PD marker expression profile in a first sample from the patient;
administering a therapeutic agent that binds to and modulates IFNα activity;
obtaining a second IFNα-inducible PD marker expression profile in a second sample from the patient; and
comparing the first and the second IFNα-inducible PD marker expression profiles,
wherein a variance in the first and the second IFNα-inducible PD marker expression profiles indicates a level of efficacy of the therapeutic agent that binds to and modulates IFNα activity

2. The method of claim 1, wherein the type I IFNα-inducible PD marker expression profile comprises up-regulated expression or activity of genes IFI6, RSAD2, IFI44, IFI44L, and IFI27.

3. The method of claim 1 or 2, wherein the variance is a decrease in the up-regulated expression or activity levels of the genes.

4. The method of claim 3, wherein the decrease is at least 10%, at least 20%, at least 25%, at least 30%, at least 40%, at least 45%, at least 50%, at least 60%, at least 70%, at least 75%, at least 80%, at least 85%, at least 90%, at least 95%, at least 96%, at least 97%, at least 98%, at least 99%, or 100%.

5. The method of any of the previous claims, wherein the disease is lupus, idiopathic inflammatory myositis, Sjogren's syndrome, vasculitis, sarcoidosis, and psoriasis.

6. The method of any of the previous claims, wherein the therapeutic agent is an antibody.

7. The method of claim 6, wherein the antibody is MEDI-545.

8. The method of any of the previous claims, wherein the first and the second sample are whole blood or serum.

9. The method of any of the previous claims, wherein the second sample is obtained at least one week, at least two weeks, at least three weeks, at least one month, or at least two months following administration of the therapeutic agent.
